# EUROPEAN PATENT SPECIFICATION

(11) **EP 3 417 296 B1**
(45) Date of publication and mention of the grant of the patent: **25.08.2021**
(21) Application number: 17753595.2
(22) Date of filing: 16.02.2017
(51) Int. Cl.: G01N 33/68, G01N 33/574, C12Q 1/68, C12N 15/113, A61P 35/00

(54) **EPIGENOMIC PROFILING REVEALS THE SOMATIC PROMOTER LANDSCAPE OF PRIMARY GASTRIC ADENOCARCINOMA**
EPIGENOMISCHE PROFILIERUNG ZUR OFFENBARUNG DER SOMATISCHEN PROMOTORLANDSCHAFT VON PRIMÄREM MAGENADENOKARZINOM
PROFILAGE ÉPIGÉNOMIQUE RÉVÉLANT LE PAYSAGE DE PROMOTEUR SOMATIQUE D'ADÉNOCARCINOME GASTRIQUE PRIMAIRE

(30) Priority: 16.02.2016 SG 10201601142V
(43) Date of publication of application: 26.12.2018
(73) Proprietor: Agency for Science, Technology and Research, Singapore 138632 (SG)
(72) Inventor: TAN, Patrick, Singapore 138672 (SG); QAMRA, Aditi, Singapore 138672 (SG); XING, Manjie, Singapore 138672 (SG); OOI, Wen Fong, Singapore 138672 (SG)
(74) Representative: Elend, Almut Susanne
(86) International application number: PCT/SG2017/050072
(87) International publication number: WO 2017/142484

(56) References cited:
- WO-A1-2011/137302
- WO-A1-2015/102536
- WO-A1-2015/102536
- US-A1- 2012 028 817
- MASAFUMI MURATANI ET AL: "Nanoscale chromatin profiling of gastric adenocarcinoma reveals cancer-associated cryptic promoters and somatically acquired regulatory elements", NATURE COMMUNICATIONS, vol. 5, 10 July 2014 (2014-07-10), pages 1-14, XP055355507, DOI: 10.1038/ncomms5361
- PREVIATI MAURIZIO ET AL: "Next generation analysis of breast cancer genomes for precision medicine", CANCER LETTERS, NEW YORK, NY, US, vol. 339, no. 1, 20 July 2013 (2013-07-20) , pages 1-7, XP028704929, ISSN: 0304-3835, DOI: 10.1016/J.CANLET.2013.07.018
- MURATANI M. ET AL.: 'Nanoscale Chromatin Profiling of Gastric Adenocarcinoma Reveals Cancer-associated Cryptic Promoters and Somatically Acquired Regulatory Elements' NATURE COMMUNICATIONS vol. 5, 10 July 2014, pages 1 - 14, XP055355507
- SUZUKI A. ET AL.: 'Aberrant Transcriptional Regulations in Cancers: Genome, Transcriptome and Epigenome Analysis of Lung Adenocarcinoma Cell Lines' NUCLEIC ACIDS RESEARCH vol. 42, no. 22, 06 November 2014, pages 13557 - 13572, XP055409780
- LU T-Y. ET AL.: 'DNA Methylation and Histone Modification Regulate Silencing of OPG During Tumor Progression' J. CELL . BIOCHEM. vol. 108, no. 1, 29 June 2009, pages 315 - 325, XP055409781
- CHANG Q. ET AL.: 'Sustained JNK1 Activation is Associated with Altered Histone H3 Methylations in Human Liver Cancer' J. HEPATOL., [Online] vol. 50, no. 2, 16 October 2008, pages 323 - 333, XP025949524 Retrieved from the Internet: <URL:https://www.ncbi.nlm.nih.gov/pmc/artic les/PMC4417500> [retrieved on 2017-05-16]
- MESSIER T.L. ET AL.: 'Histone H3 Lysine 4 Acetylation and Methylation Dynamics Define Breast Cancer Subtypes' ONCOTARGET vol. 7, no. 5, 15 January 2016, pages 5094 - 5109, XP055409784
- HEINTZMANN N.D. ET AL.: 'Distinct and Predictive Chromatin Signatures of Transcriptional Promoters and Enhancers in the Human Genome' NATURE GENETICS vol. 39, no. 3, 04 February 2007, pages 311 - 318, XP002517419
- CHENG J. ET AL.: 'A Role for H3K4 Mono-methylation in Gene Repression and Partitioning of Chromatin Readers' MOL. CELL, [Online] vol. 53, no. 6, 20 March 2014, pages 979 - 992, XP028635729 Retrieved from the Internet: <URL:https://www.ncbi.nlm.nih.gov/pmc/artic les/PMC4031464> [retrieved on 2017-05-16]
- QAMRA A. ET AL.: 'Epigenomic Promoter Alterations Amplify Gene Isoform and Immunogenic Diversity in Gastric Adenocarcinoma' CANCER DISCOVERY 20 March 2017, pages 1 - 63, XP055409789

## Description

### CROSS-REFERENCE TO RELATED APPLICATIONS

This application claims the benefit of priority of Singapore application No. 10201601142V, filed 16 February 2016.

### FIELD OF THE INVENTION

The invention relates to a method for determining the presence or absence of at least one promoter in a cancerous biological sample relative to a non-cancerous biological sample.

### BACKGROUND OF THE INVENTION

Gastric cancer (GC) is the third leading cause of global cancer mortality with high prevalence in many East Asian countries. GC patients often present with late-stage disease, and clinical management remains challenging as exemplified by several recent negative Phase II and Phase III clinical trials. At the molecular level, studies have identified characteristic gene mutations, copy number alterations, gene fusions, and transcriptional patterns in GC. However, few of these have been clinically translated into targeted therapies, with the exception of HER2-positive GC and traztuzumab. There is thus a strong need for additional and more comprehensive explorations of GC, as these may highlight new biomarkers for disease detection, predicting patient prognosis or responses to therapy, as well as new therapeutic modalities.

Promoter elements are *cis*-regulatory elements which function to link gene transcription initiation to upstream regulatory stimuli, integrating inputs from diverse signaling pathways. Promoters represent an important reservoir of biological, functional, and regulatory diversity, as current estimates suggest that 30-50% of genes in the human genome are associated with multiple promoters, which can be selectively activated as a function of developmental lineage and cellular state. Differential usage of alternative promoters causes the generation of distinct 5' untranslated regions (5' UTRs) and first exons in transcripts, which in turn can influence mRNA expression levels, translational efficiencies, and generation of different protein isoforms through gain and loss of 5' coding domains. To date, promoter alterations in cancer have been largely studied on a gene-by-gene basis, and very little is known about the global extent of promoter-level diversity in GC and other solid malignancies.

Accordingly, there is a need for a method of profiling promoter elements in cancer.

### SUMMARY

In one aspect there is provided a method for determining the presence or absence of at least one promoter in a cancerous biological sample relative to a non-cancerous biological sample, comprising: contacting the cancerous biological sample with at least one antibody specific for histone modifications H3K4me3 and H3K4me1; isolating nucleic acid from the cancerous biological sample having a signal ratio of H3K4me3 relative to H3K4me1 greater than 1, wherein the isolated nucleic acid comprises at least one region specific to said histone modifications; detecting a signal intensity of H3K4me3 in the isolated nucleic acid; and determining the presence or absence of at least one promoter in the cancerous biological sample based on the change in the signal intensity of H3K4me3 relative to the signal intensity of H3K4me3 in a non-cancerous biological sample.

In another aspect there is provided a method for determining the prognosis of cancer in a subject, comprising, contacting a cancerous biological sample obtained from the subject with at least one antibody specific for histone modification H3K4me3 and H3K4me1; isolating nucleic acid from the cancerous biological sample having a signal ratio of H3K4me3 relative to H3K4me1 greater than 1, wherein the isolated nucleic acid comprises at least one region specific to said histone modifications; detecting a signal intensity of H3K4me3 in the isolated nucleic acid; and determining the presence or absence of at least one cancer-associated promoter in the cancerous biological sample based on the change in the signal intensity of H3K4me3 relative to the signal intensity of H3K4me3 in a reference nucleic acid sequence, wherein the presence or absence of the at least one cancer-associated promoter in the cancerous biological sample is indicative of the prognosis of the cancer in the subject.

In another aspect there is provided a biomarker for detecting cancer in a subject, the biomarker comprising at least one promoter having a change in signal intensity of H3K4me3 in a cancerous biological sample relative to a non-cancerous biological sample.

In another aspect there is provided a method for modulating the activity of at least one cancer-associated promoter in a cell, comprising administering an inhibitor of EZH2 to the cell.

In another aspect there is provided a method for modulating the immune response of a subject to cancer, comprising administering to the subject an inhibitor of EZH2, wherein the EZH2 is associated with at least one cancer-associated promoter in the subject.

In another aspect there is provided a method for determining the presence or absence of at least one cancer-associated promoter in a cancerous biological sample relative to a non-cancerous biological sample, comprising: contacting the cancerous biological sample with at least one antibody specific for histone modifications H3K4me3 and H3K4me1; isolating nucleic acid from the cancerous biological sample having a signal ratio of H3K4me3 relative to H3K4me1 greater than 1, wherein the isolated nucleic acid comprises at least one region specific to said histone modifications; detecting a signal intensity of H3K4me3 in the isolated nucleic acid at a read depth of 20M; and determining the presence or absence of at least one cancer-associated promoter in the cancerous biological sample based on the change in the signal intensity of H3K4me3 relative to the signal intensity of H3K4me3 in a non-cancerous biological sample.

In one aspect, there is provided a biomarker comprising at least one promoter having a change in signal intensity of H3K4me3 in a cancerous biological sample relative to a non-cancerous biological sample for use in detecting cancer in a subject.

In one aspect, there is provided a use of a biomarker comprising at least one promoter having a change in signal intensity of H3K4me3 in a cancerous biological sample relative to a non-cancerous biological sample in the manufacture of a medicament for detecting cancer in a subject.

In one aspect, there is provided an inhibitor of EZH2 for use in modulating the activity of at least one cancer-associated promoter in a cell.

In one aspect, there is provided a use of an inhibitor of EZH2 in the manufacture of a medicament for modulating the activity of at least one cancer-associated promoter in a cell.

In one aspect, there is provided an inhibitor of EZH2 for use in modulating the immune response of a subject to cancer, wherein the EZH2 is associated with at least one cancer-associated promoter in the subject.

In one aspect, there is provided a use of an inhibitor of EZH2 in the manufacture of a medicament for modulating the immune response of a subject to cancer, wherein the EZH2 is associated with at least one cancer-associated promoter in the subject.

### DEFINITIONS

The following are some definitions that may be helpful in understanding the description of the present invention. These are intended as general definitions and should in no way limit the scope of the present invention to those terms alone, but are put forth for a better understanding of the following description.

As used herein, the term "promoter" is intended to refer to a region of DNA that initiates transcription of a particular gene.

As used herein, the term "cancerous" relates to being affected by or showing abnormalities characteristic of cancer.

As used herein, the term "biological sample" refers to a sample of tissue or cells from a patient that has been obtained from, removed or isolated from the patient. The term "obtained or derived from" as used herein is meant to be used inclusively. That is, it is intended to encompass any nucleotide sequence directly isolated from a biological sample or any nucleotide sequence derived from the sample.

As used herein, the term "antibody" or "antibodies" as used herein refers to molecules with an immunoglobulin-like domain and includes antigen binding fragments, monoclonal, recombinant, polyclonal, chimeric, fully human, humanised, bispecific and heteroconjugate antibodies; a single variable domain, single chain Fv, a domain antibody, immunologically effective fragments and diabodies.

The term "specifically binds" as used throughout the present specification in relation to antigen binding proteins means that the antigen binding protein binds to a target epitope on an antigen with a greater affinity than that which results when bound to a non-target epitope. In certain embodiments, specific binding refers to binding to a target with an affinity that is at least 10, 50, 100, 250, 500, or 1000 times greater than the affinity for a non-target epitope. For example, binding affinity may be as measured by routine methods, e.g., by competition ELISA or by measurement of Kd with BIACORE™, KINEXA™ or PROTEON™.

As used herein, the term "isolated" relates to a biological component (such as a nucleic acid molecule, protein or organelle) that has been substantially separated or purified away from other biological components in the cell of the organism in which the component naturally occurs, i.e., other chromosomal and extra-chromosomal DNA and RNA, proteins and organelles. Nucleic acids and proteins that have been "isolated" include nucleic acids and proteins purified by standard purification methods. The term also embraces nucleic acids and proteins prepared by recombinant expression in a host cell as well as chemically synthesized nucleic acids.

As used herein, the term "nucleic acid" refers to a deoxyribonucleotide or ribonucleotide polymer in either single or double stranded form, and unless otherwise limited, encompassing known analogues of natural nucleotides that hybridize to nucleic acids in a manner similar to naturally occurring nucleotides. "Nucleotide" includes, but is not limited to, a monomer that includes a base linked to a sugar, such as a pyrimidine, purine or synthetic analogs thereof, or a base linked to an amino acid, as in a peptide nucleic acid (PNA). A nucleotide is one monomer in a polynucleotide. A nucleotide sequence refers to the sequence of bases in a polynucleotide.

As used herein, the term "prognosis" or grammatical variants thereof, as used herein refers to a prediction of the probable course and outcome of a clinical condition or disease. A prognosis of a patient is usually made by evaluating factors or symptoms of a disease that are indicative of a favorable or unfavorable course or outcome of the disease. The term "prognosis" does not refer to the ability to predict the course or outcome of a condition with 100% accuracy. Instead, the term "prognosis" refers to an increased probability that a certain course or outcome will occur; that is, that a course or outcome is more likely to occur in a patient exhibiting a given condition, when compared to those individuals not exhibiting the condition.

As used herein, the term "modulating" is intended to refer to an adjustment of the immune response to a desired level.

As used herein, the term "annotated promoter" refers to a promoter mapping close (<500bp) to a known Gencode transcription start site (TSS).

The term "unannotated promoter" refers to a promoter mapping to genomic regions devoid of known Gencode TSSs.

As used herein, the term "canonical" in the context of a promoter refers to a promoter region exhibiting unaltered H3K4me3 peaks.

As used herein, the term "detectable label" or "reporter" refers to a detectable marker or reporter molecules, which can be attached to nucleic acids. Typical labels include fluorophores, radioactive isotopes, ligands, chemiluminescent agents, metal sols and colloids, and enzymes. Methods for labeling and guidance in the choice of labels useful for various purposes are discussed, e.g., in Sambrook et al., in Molecular Cloning: A Laboratory Manual, Cold Spring Harbor Laboratory Press (1989) and Ausubel et al., in Current Protocols in Molecular Biology, Greene Publishing Associates and Wiley-Intersciences (1987).

As used herein, the term "hypomethylated" refers to a decrease in the normal methylation level of DNA.

As used herein, the term "hypermethylated" refers to an increase in the normal methylation level of DNA.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/-0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and subgeneric groupings falling within the generic disclosure also form part of the disclosure. This includes the generic description of the embodiments with a proviso or negative limitation removing any subject matter from the genus, regardless of whether or not the excised material is specifically recited herein.

Unless the context requires otherwise or specifically stated to the contrary, integers, steps, or elements of the invention recited herein as singular integers, steps or elements clearly encompass both singular and plural forms of the recited integers, steps or elements.

The word "substantially" does not exclude "completely" e.g. a composition which is "substantially free" from Y may be completely free from Y. Where necessary, the word "substantially" may be omitted from the definition of the invention.

The invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

### BRIEF DESCRIPTION OF THE DRAWINGS

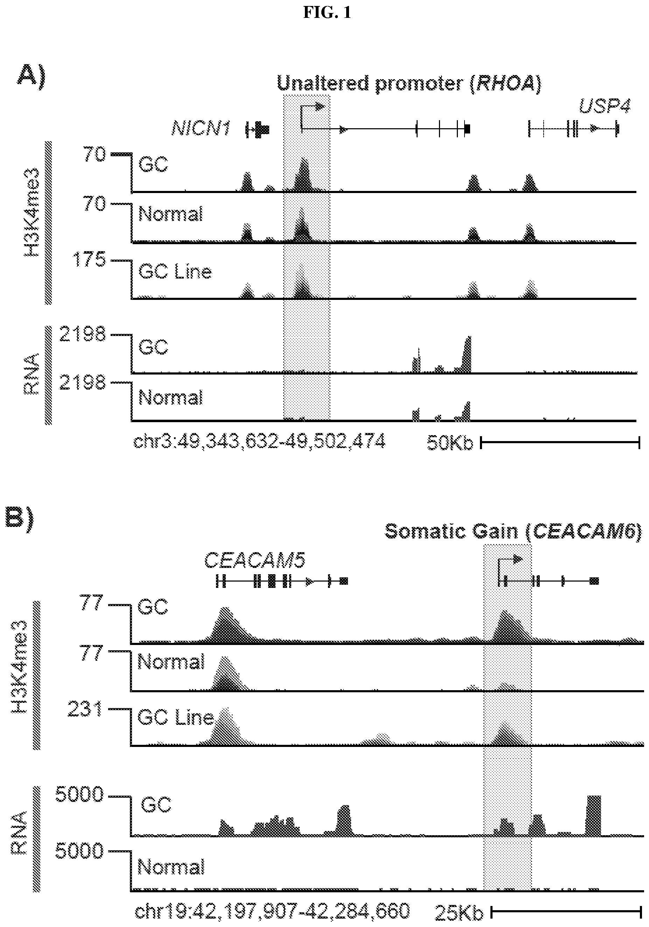

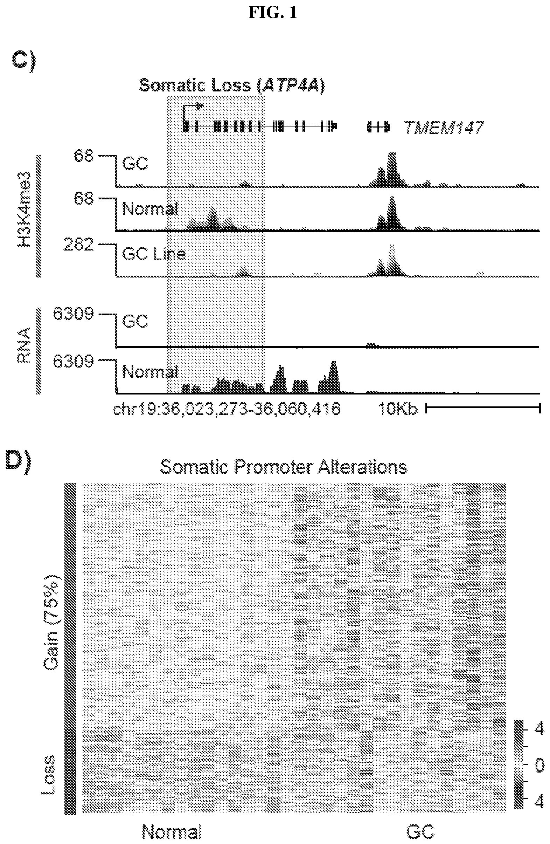

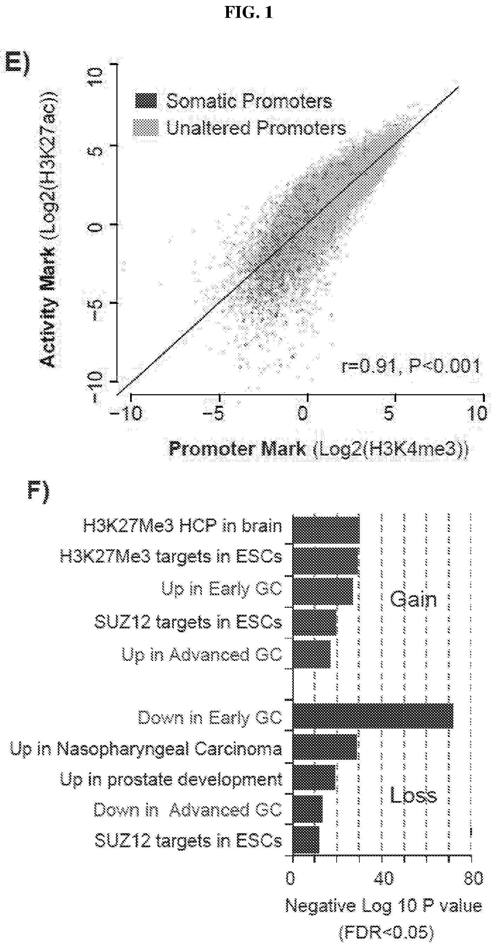

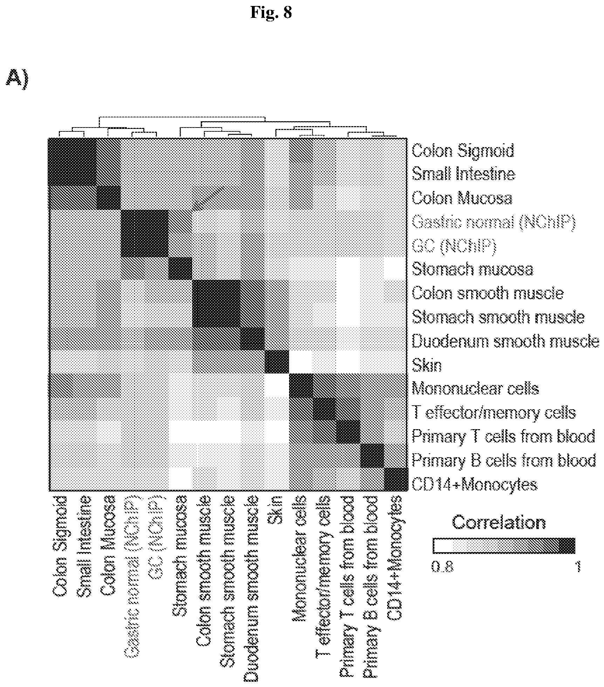

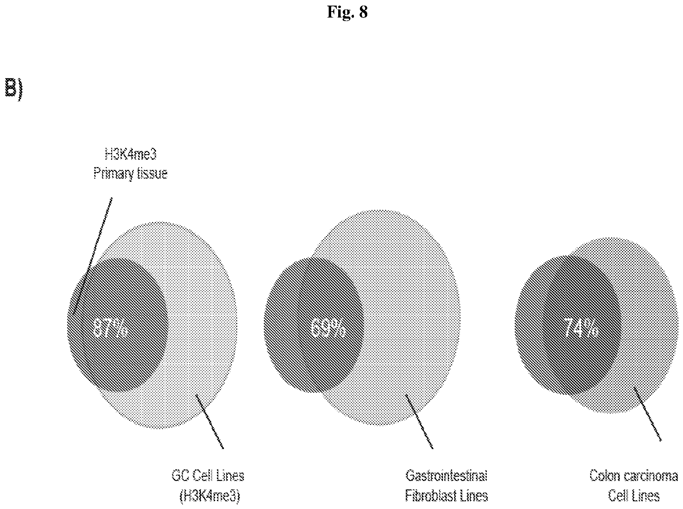

The invention will be better understood with reference to the detailed description when considered in conjunction with the non-limiting examples and the accompanying drawings, in which
**Figure 1****: Somatic Promoter Alterations in Primary Gastric Adenocarcinoma.**
   A) Example of an unaltered GC promoter. The UCSC genome track of the *RHOA* TSS (shaded box) highlights similar H3K4me3 signals in GC and matched normal samples. Similar signals are seen in GC lines. The bottom two tracks display similar levels of RNA expression in the same GC and matched normal sample (RNAseq).
   B) Example of a gained somatic promoter. The UCSC genome track of the *CEACAM6* TSS (shaded box) highlights gain of H3K4me3 signals in GC samples and GC lines, compared to matched normal samples. In contrast, no changes are observed at the TSS of *CEACAM5,* an adjacent gene. Concordant tumor-specific gain of RNA expression is shown in the bottom 2 tracks displaying RNA-seq profiles of the same GC and matched normal samples.
   C) Example of a lost somatic promoter. The UCSC genome track of the *ATP4A* TSS (shaded box) highlights loss of H3K4me3 signals in GC samples and GC lines compared to matched normal samples. Concordant tumor-specific loss of RNA expression is shown in the bottom 2 tracks displaying RNA-seq profiles of the same GC and gastric normal samples.
   D) Heatmap of H3K4me3 read densities (row scaled) of somatic promoters (rows) in primary GCs and matched normal samples.
   E) Correlation between H3K4me3 promoter signals and H3K27ac activity signals in primary gastric samples (r=0.91, P<0.001). Each data point corresponds to a single H3K4me3 hi/H3K4me1 lo region. Analysis was performed using data from 16 N/T pairs (Table 4).
   F) Top 5 gene sets associated with canonical gained and lost somatic promoters. Genesets associated with genes up and downregulated in GC are rediscovered. Also note that gene sets related to H3K27me3 and SUZ12, a PRC2 component, are enriched.

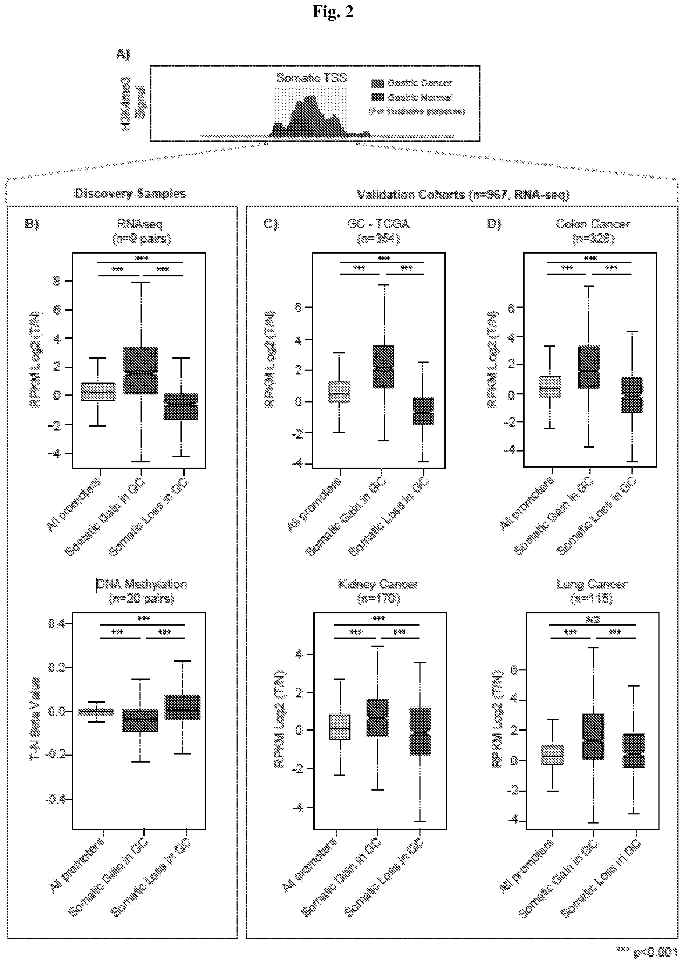

**Figure 2****: Association of Somatic Promoter Alterations with Gene Expression in GC and Other Tumor Types**
   A) Example of a GC somatic promoter. Example is for illustrative purposes only.
   B) Changes in RNA-seq expression (top) and DNA methylation (bottom) in discovery samples between somatic promoters and all promoters. Top - Boxplot depicting changes in RNA-seq expression between 9 paired primary GC and gastric normal samples at genomic regions exhibiting somatic promoters (gained and lost) (***P<0.001, Wilcoxon Test). Bottom - Boxplot depicting changes in DNA methylation (β-values) at regions exhibiting somatic promoters between 20 paired GC and gastric normal samples, compared to all promoters. (***P<0.001, Wilcoxon test)
   C) Independent Validation Cohorts. Boxplot depicting changes in RNA-seq expression at genomic regions exhibiting somatic promoters across 354 (321 GC, 33 normal) TCGA Stomach adenocarcinoma (STAD) samples, compared to all promoters (***P<0.001, Wilcoxon test)
   D) Somatic Promoters in Other Cancer Types. Boxplot depicting changes in RNA-seq expression at genomic regions exhibiting GC somatic promoters compared against all promoters, across 326 TCGA Colon adenocarcinoma (COAD) samples (286 COAD, 40 normal; ***P<0.001, Wilcoxon test), 170 TCGA kidney renal clear cell carcinoma (ccRCC) samples (98 ccRCC and 72 normal; ***P<0.001, Wilcoxon test), and 115 TCGA lung adenocarcinoma (LUAD) samples (58 LUAD, 57 normal; ***P<0.001 somatic gain vs all promoters and somatic gain vs. somatic loss, Wilcoxon test).

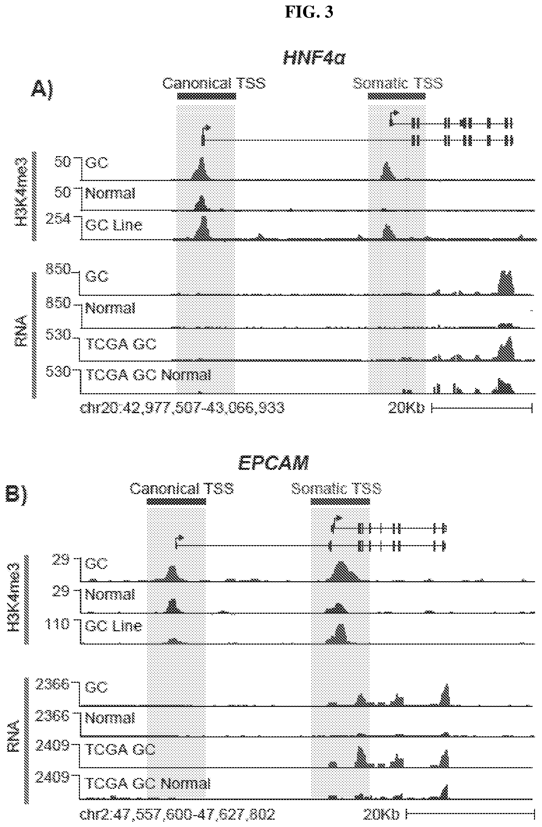

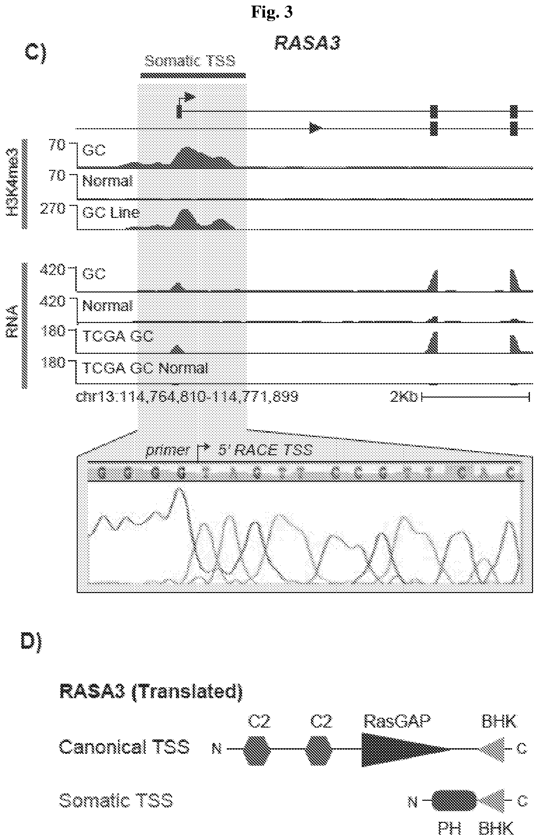

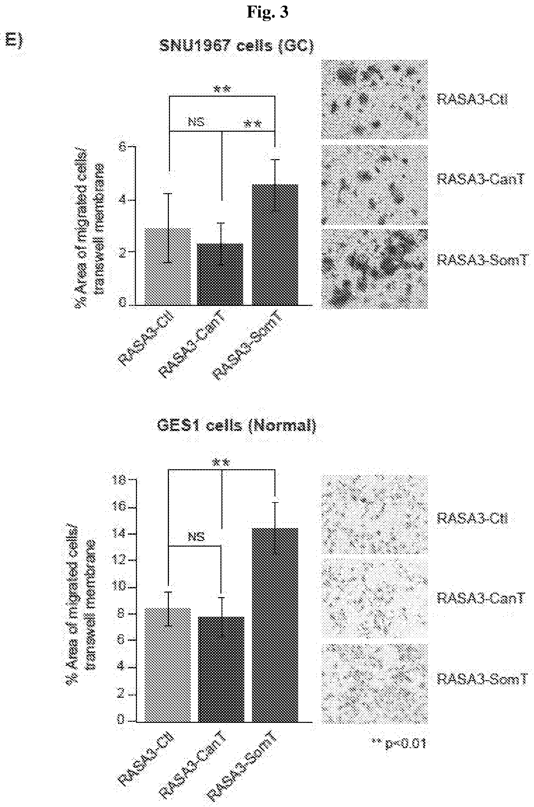

**Figure 3****: Alternative Promoters in GC**
   A) UCSC browser track of the *HNF4α* gene. GC and matched gastric normal samples have equal H3K4me3 signals at the canonical *HNF4α* promoter. However, an alternative promoter, seen by H3K4me3 gain, can be observed at a downstream TSS in GCs compared to matched normals. At the RNA level, both in-house and TCGA STAD samples also show gain of gene expression at the alternate promoter TSS compared to normal samples.
   B) UCSC browser track of the *EPCAM* gene. Another example of alternative promoter usage at a downstream TSS. Gain of H3K4me3 is observed at a TSS downstream of the canonical promoter, while the canonical promoter exhibits equal H3K4me3 signals in GC and gastric normal. Gain of RNA-seq expression can also be observed in GC at the alternative promoter driven transcript in both in-house and TCGA STAD samples.
   C) UCSC browser track of the *RASA3* gene, demonstrating H3K4me3 and RNA-seq signals highlighting gain of promoter activity at an un-annotated TSS (dark grey box) corresponding to a novel N-terminal truncated *RASA3* transcript. Expression of this variant transcript was validated through 5'RACE in GC lines (bottom).
   D) Functional domains of the translated *RASA3* canonical and alternate isoform. The alternate transcript is predicted to encode a *RASA3* protein missing the RASGAP domain.
   E) Effect of overexpression of *RASA3* canonical (CanT) and alternate (SomT) isoforms on the migration capability of SNU1967 (top) and GES1 (bottom) cells. Representative images of *RASA3-*Ct1 (Empty vector), *RASA3*-CanT and *RASA3*-SomT in migration assays (n=3). Barplots show the % area of migrated cells vs the area of transwell membrane. Data is shown as mean ± SD; n=3. (*P<0.05, **P<0.01, ***P<0.001, Student's one sided t-test)

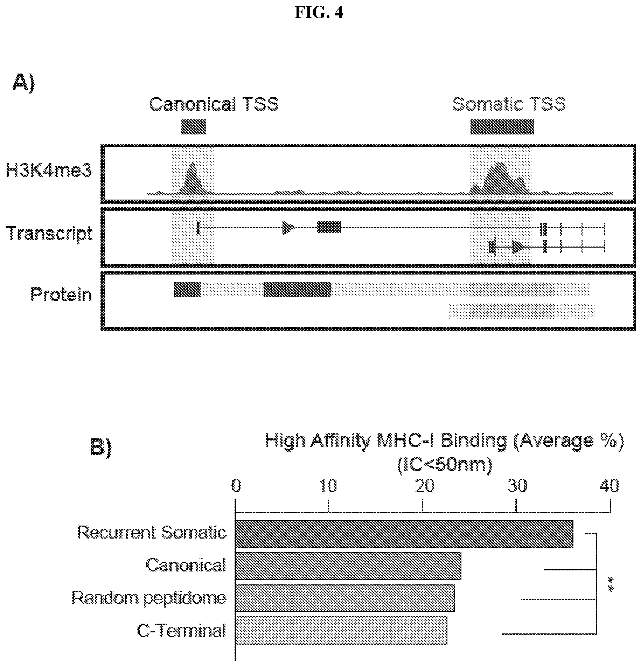

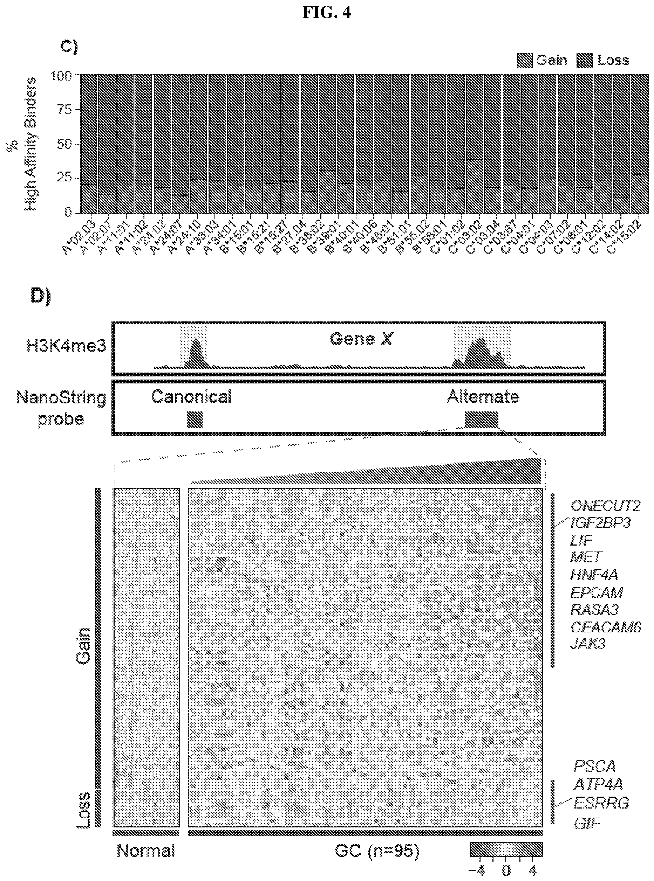

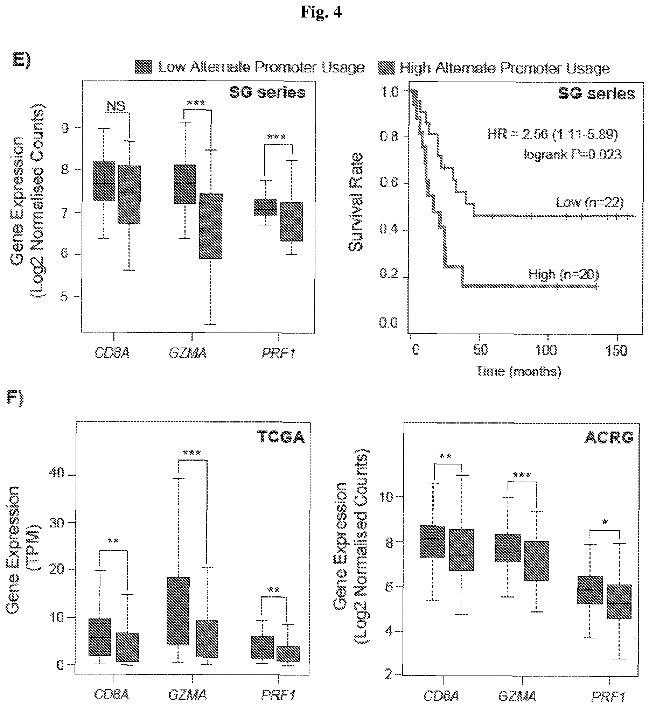

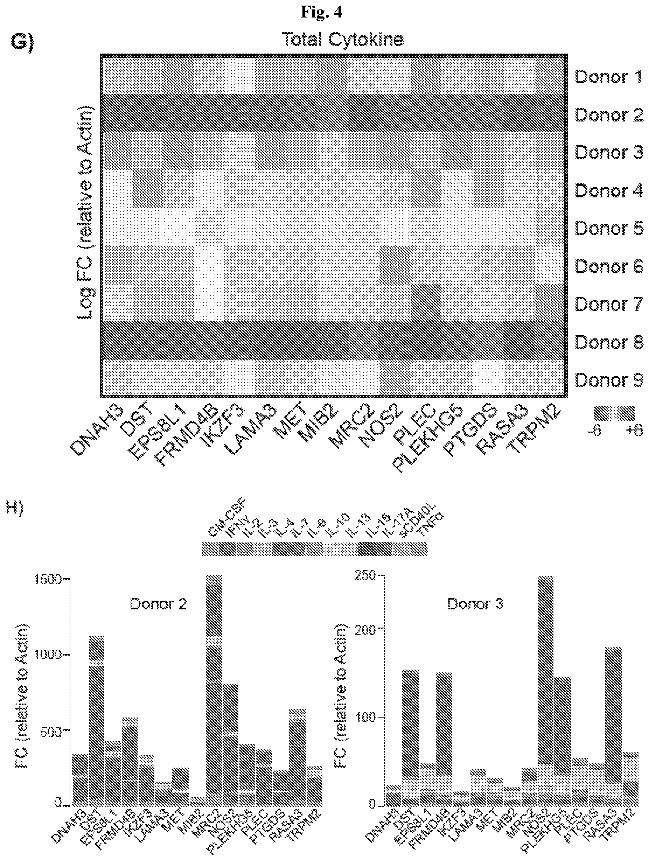

**Figure 4****: Somatic Promoter Alterations Exhibit Immunoediting Signatures**
   A) Schematic outlining alternative promoter usage leading to alternative transcript usage (Transcript box) and N terminally truncated protein isoforms (protein box).
   B) Barplot showing the average % of peptides with predicted high-affinity binding to MHC Class I (HLA-A, B, and C, IC<=50 nm). N-terminal peptides associated with recurrent somatic promoters (alternative promoters) show significantly enriched predicted MHC I binding compared to canonical GC peptides (P<0.01, Fisher's test), random peptides from the human proteome (P<0.001) and C-terminal peptides (P<0.01) derived from the same genes exhibiting the N-terminal alterations. Canonical peptides refer to peptides derived from protein coding genes overexpressed in GC through non-alternative promoters.
   C) Percentage (%) of high affinity peptides predicted to bind different HLA-alleles categorized by somatic gain or loss. Most alleles have a greater number of N-terminal lost peptides predicted to have high binding affinity.
   D) Quantification of somatic promoter expression using Nanostring profiling. Top - Distinct Nanostring probes were designed to measure expression of alternate and canonical promoter driven transcripts. 2 probes were designed for each gene - a canonical probe at the 5' transcript marked by unaltered H3K4me3, and an alternate probe at the 5' transcript of the somatic promoter. Bottom - Heatmap of alternative promoter expression from 95 GCs and matched normal samples. GC samples have been ordered left to right by their levels of somatic promoter usage.
   E) Association between Somatic Promoters and T-cell immune correlates (Singapore (SG) cohort). Top left - Expression of T-cell markers *CD8A* (P=0.1443) and the T-cell cytolytic markers *GZMA* (P=0.0001) and *PRF1* (P=0. 00806) in GC samples with either high or low somatic promoter usage (SG). Samples with high alternative promoter usage show lower expression of immune markers. All P values are from Wilcoxon one sided test. Right - Kaplan-Meier analysis comparing overall survival curves between validation samples with high somatic promoter usage (top 25%) and low somatic promoter usage (bottom 25%) (HR= 2.56, P=0.02).
   F) Association of Somatic Promoters with T-cell Correlates in TCGA and ACRG Cohorts. (Left) Expression of T-cell markers *CD8A* (P=0.02), *GZMA* (P= 0.01) and *PRF1* (P=0.03) in TCGA STAD with either high or low somatic promoter usage. T-cell markers were evaluated by RNA-seq (Transcripts per million, Right) Expression of T-cell markers *CD8A* (P=0.035), *GZMA* (P=0.001) and *PRF1* (P=0.025) in ACRG GC samples with either high or low somatic promoter usage. All P values are from Wilcoxon one sided test.
   G) EpiMAX Heatmap of total cytokine responses (Fold change relative to Actin) for 15 peptide pools against 9 donors.
   H) Individual cytokine responses against 15 peptides for two individual donors (Donor 2 and Donor 3) showing complex cytokine responses (FC≥2).

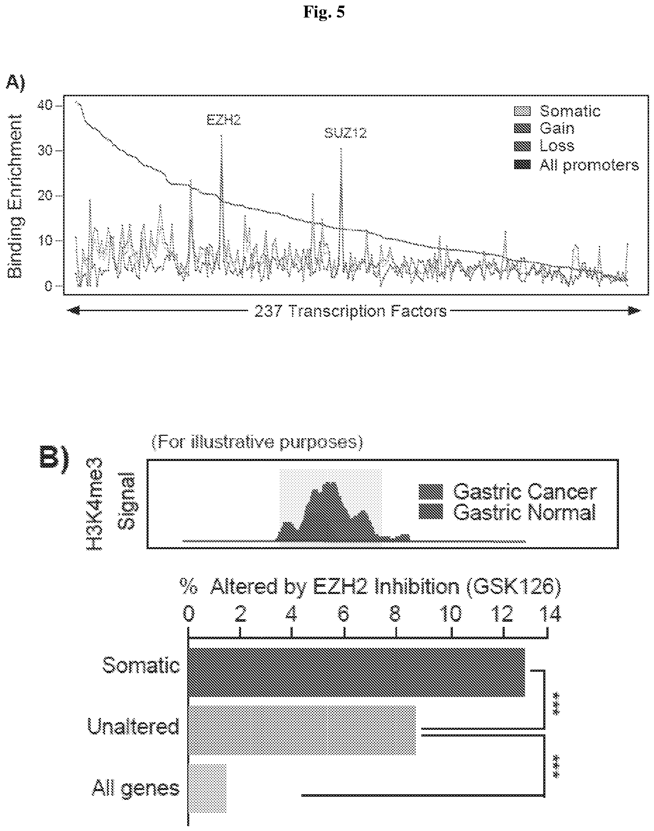

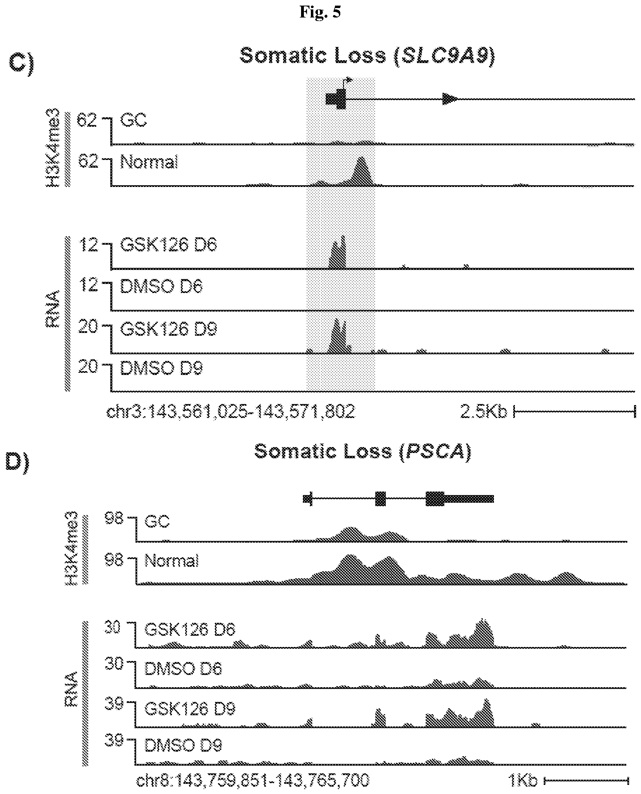

**Figure 5****: Somatic Promoters are Associated with EZH2 Occupancy**
   A) Binding enrichment of ReMap-defined TFBSs at genomic regions exhibiting somatic promoters. TFs were sorted according to their binding frequency at all H3K4me3-defined promoter regions. EZH2 and SUZ12 binding sites significantly overlap regions exhibiting somatic promoters (gained and lost) (P<0.01, Empirical distribution test).
   B) Proportion of RNA transcripts associated with somatic promoters changing upon GSK126 treatment in IM95 cells, compared to RNA transcripts associated with unaltered promoters. The top somatic promoter figure is for illustrative purposes only. Unaltered promoters were defined as all gene promoters except the somatic promoters. The proportion of genes changing upon treatment, as a proportion of all genes, is also shown. Somatic promoters are more likely to change expression after GSK126 treatment relative to unaltered promoters (OR 1.46, P<0.001) or all GSK126 regulated genes (OR 9.21, P<0.001, Fisher Test)
   C) UCSC browser track of the *SLC9A9* TSS, a gene with loss of promoter activity. Gain of expression is seen after inhibition of EZH2 using GSK126 in IM95 cells at both day 6 (D6) and Day 9 (D9) treatment.
   D) UCSC browser track of the *PSCA* TSS, with loss of promoter activity. Gain of expression is seen after inhibition of EZH2 using GSK126 in IM95 cells at both day 6 (D6) and Day 9 (D9) treatment.

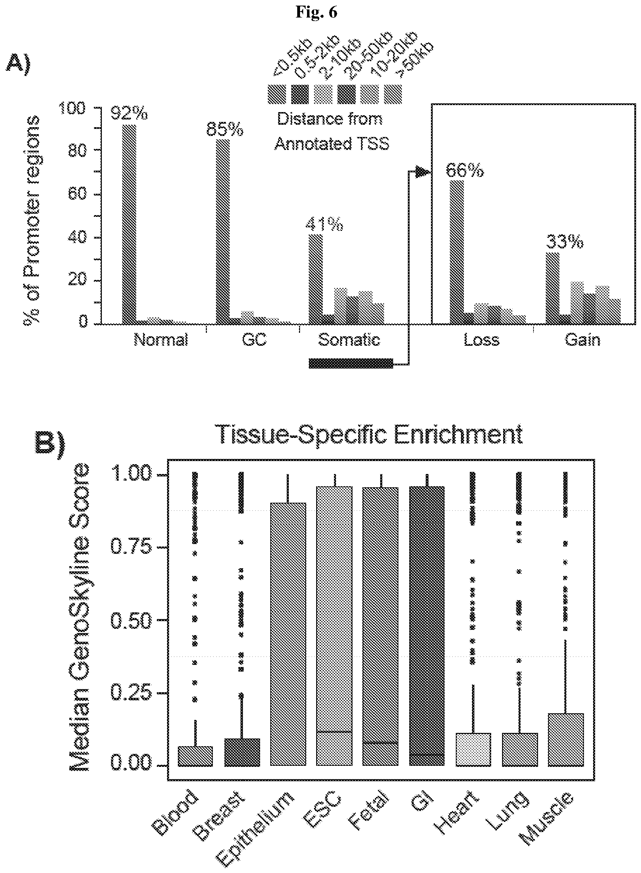

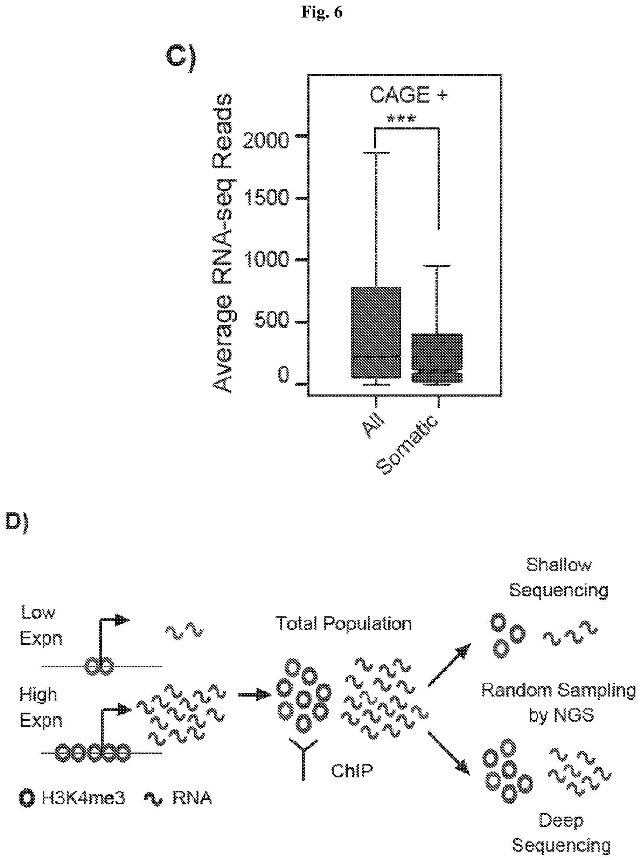

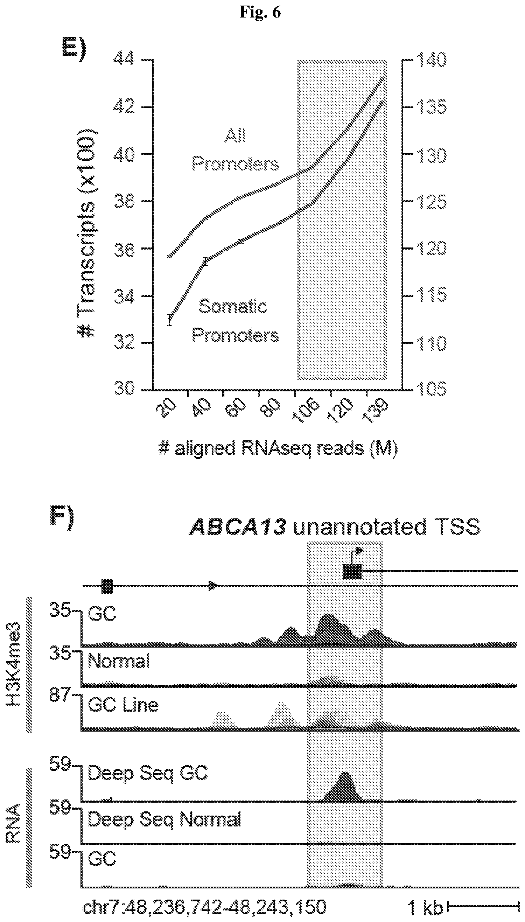

**Figure 6****: Somatic promoters reveal novel cancer-associated transcripts**
   A) Distribution of distances for different promoter categories to the nearest annotated TSSs. (left) The first barplot shows distance distributions for promoters present in gastric normal tissues, the second for promoter present in GC samples, and the third for promoters exhibiting somatic alterations (i.e. different in tumor vs normal). (right) The barplots present distance distributions associated with either lost or gained somatic promoters. A substantial proportion of gained somatic promoters occupy locations distant from previously annotated TSSs
   B) Median functional scores of unannotated promoters as predicted by GenoSkyline across 7 different tissues. Unannotated promoters exhibited high functional scores for GI, fetal and ESC tissues.
   C) Boxplot depicting average RNA-seq reads for CAGE-validated promoters, comparing either all promoters or somatic promoters and also supported by CAGE data. (**P<0.001, Wilcoxon one sided test). Somatic promoters are observed to have lower levels of RNA-seq expression.
   D) Cartoon depicting proposed effects of dynamic range on NanoChIP-seq and RNA-seq sensitivity in detecting lowly expressed transcripts. Due to a more restricted dynamic range, epigenomic profiling may detect active promoters missed by RNA-sequencing, due to the random sampling of abundantly expressed genes by RNAseq.
   E) Down and Up-sampling analysis. The y-axis depicts the number of transcripts detected that overlap either all promoters or somatic promoters at varying RNA-sequencing depths. Original primary sample RNA-seq data was sequenced at -106M reads which was down-sampled to 20M, 40M and 60M reads. Deep RNA-seq data was additionally generated at -139M read depth.
   F) Cancer-associated transcripts detected at deep but not regular RNA-seq depth. The UCSC genome browser track for *ABCA13* shows an example of a novel transcript detected by NanoChIP-seq at a read depth of 20M but only detected by RNA-sequencing at read depth of -139M (Deep sequencing GC). This transcript is not detected by regular depth RNA-seq (GC).

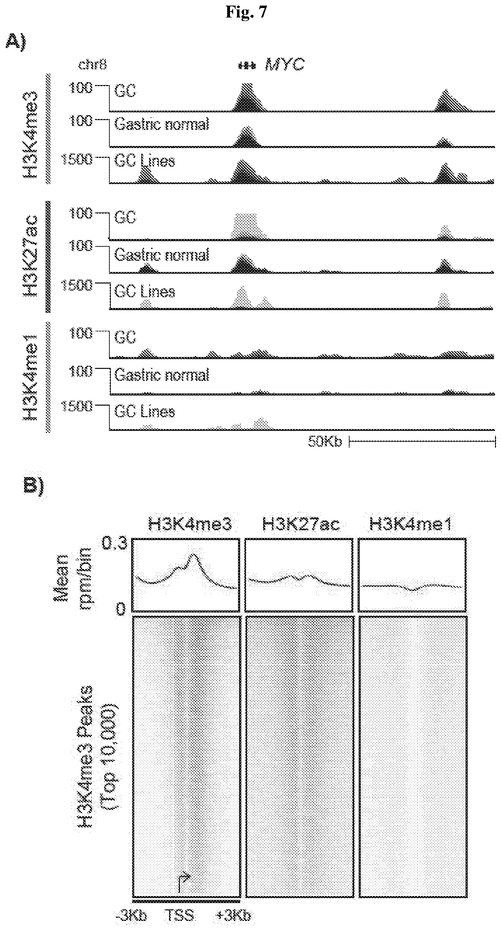

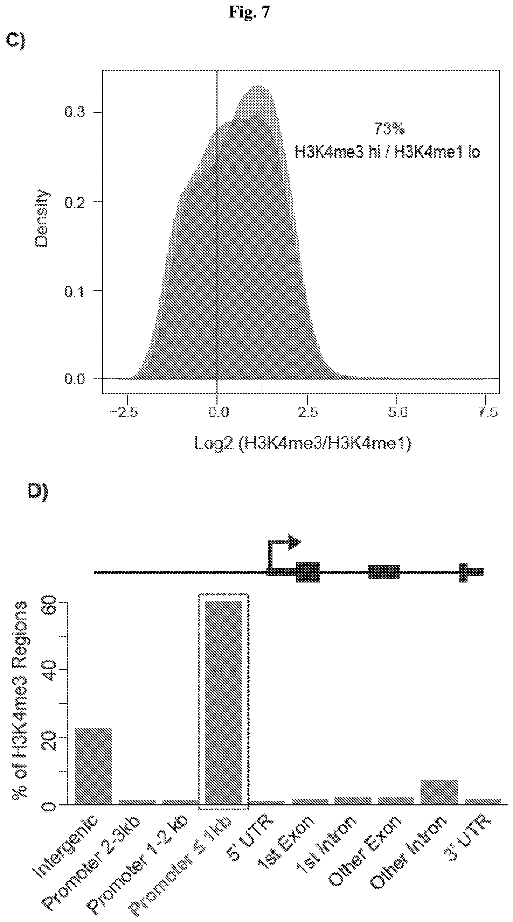

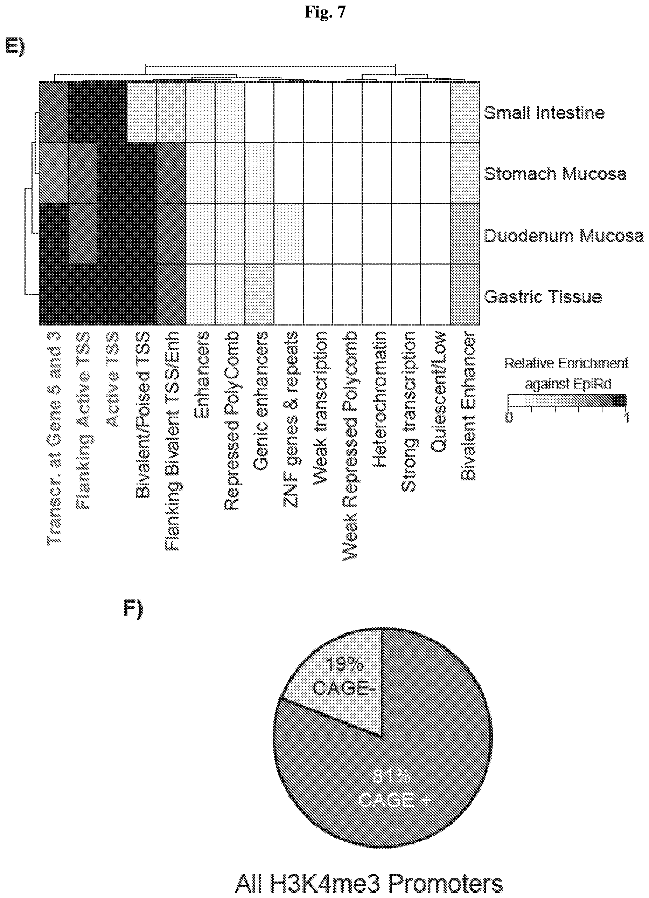

**Figure 7****: Chromatin Profiles of Primary GC**
   A) Chromatin profiles of primary GCs, matched normal gastric mucosae, and GC cell lines for 3 marks (H3K4me3, H3K27ac and H3K4me1). Shown are UCSC genome browser tracks of the GC driver gene *MYC* highlighting strong H3K4me3 and H3K27ac signals and low H3K4me1 at promoter locations
   B) H3K4me3, H3K27ac and H3K4me1 signal distributions at transcription start sites (TSS). Line plots show the distribution of chromatin signals for H3K4me3 hi/H3K4me1 lo regions at TSS regions (+/- 3 kb). Heatmaps were plotted using ngs.plot(6) for the top 10,000 H3K4me3 hi/H3K4me1 lo regions
   C) Density distributions of H3K4me3:H3K4me1 ratios at identified H3K4me3 regions. All regions with H3K4me3/H3K4me1 ratios >1 were selected for further analysis (73%)
   D) Distribution of H3K4me3 hi/H3k4me1 lo regions against representative gene body features (top). The arrow represents the TSS.
   E) Enrichment of H3K4me3 hi/H3K4me1 lo regions against 15 chromatin states (columns) defined in different gastrointestinal tissues from the Epigenome Roadmap database (rows). Each column is scaled from 0 to 1.
   F) Overlap of H3K4me3 hi/H3K4me1 lo regions with FANTOM5 CAGE data
**Figure 8****: Epithelial features of GC promoters**
   A) Spearman correlation heat-map between H3K4me3 signals of primary GC, gastric normal samples (red type, highlighted by red arrow) and various tissue types from the Epigenome Roadmap database across all H3K4me3 hi/H3K4me1 lo regions
   B) Overlap of H3K4me3 hi/H3K4me1 lo regions with H3K4me3 regions identified in GC cell lines (87%), gastrointestinal fibroblast cells (61%) and colon carcinoma lines (74%)

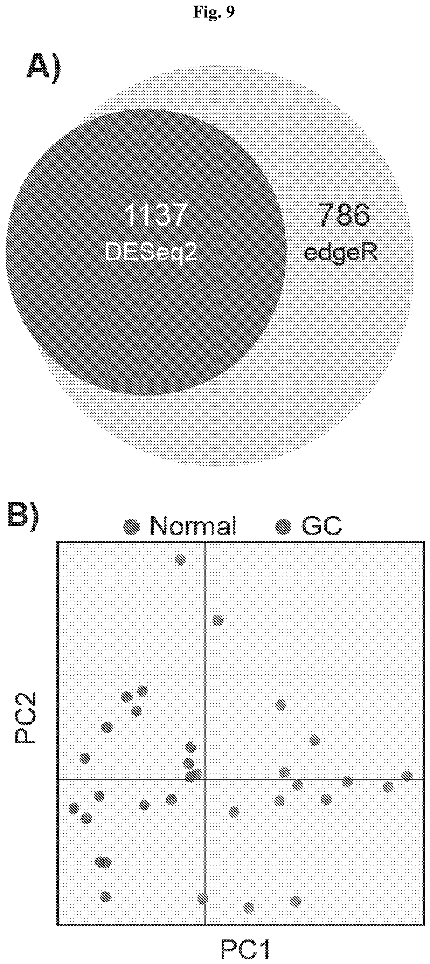

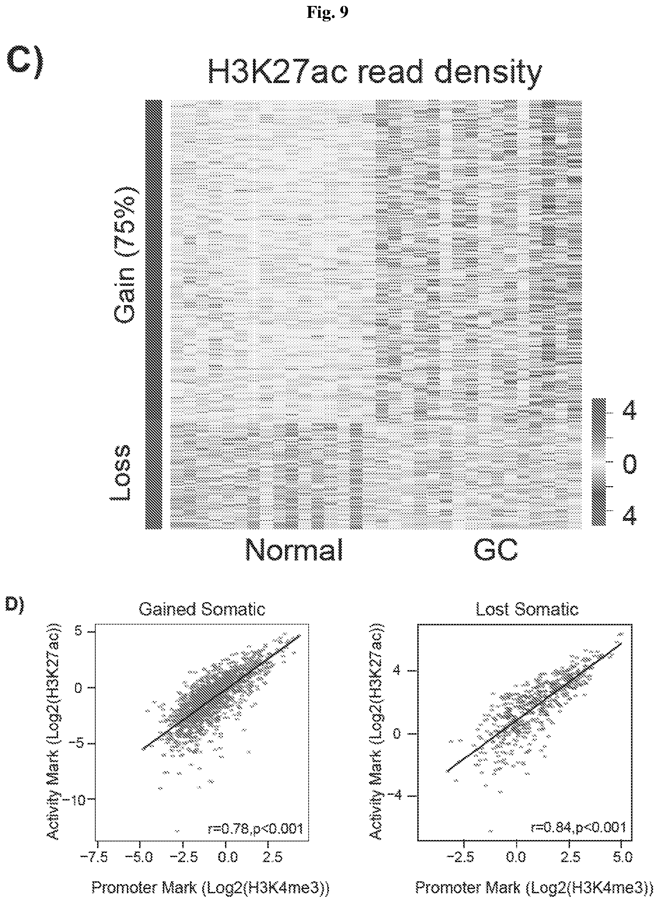

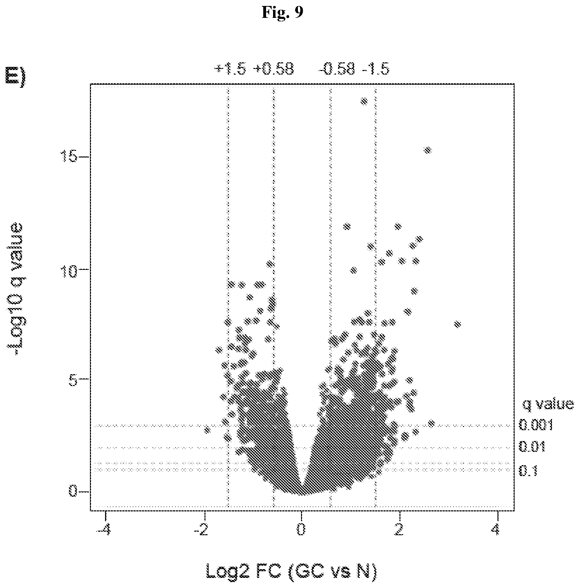

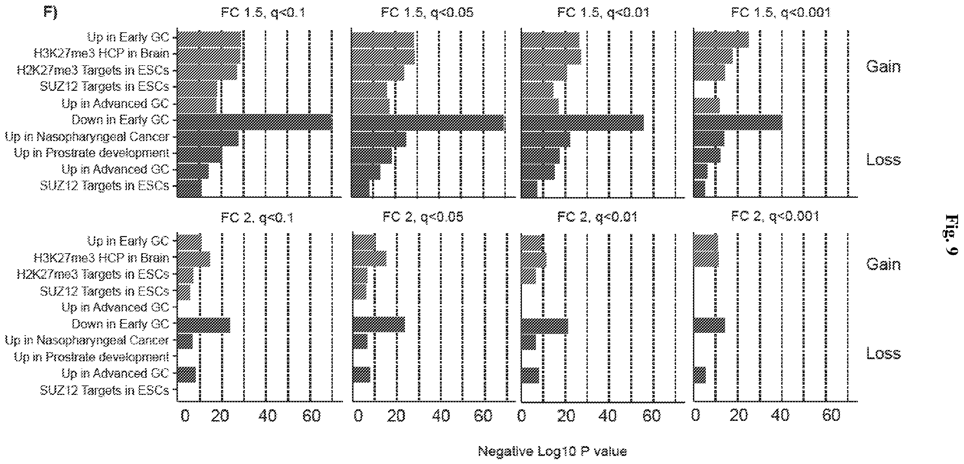

**Figure 9****: GC Somatic Promoter Features**
   A) Differential (somatic) H3K4me3 regions identified from 2 independent algorithms DESeq2 and edgeR. 96% of regions identified from DESeq2 overlapped those identified using edgeR. Both sets were pooled for subsequent analysis.
   B) Principal component analysis of 16 GC and gastric normal samples based on somatic promoters
   C) Heatmap of H3K27ac read densities across 16 GC and gastric normal samples across 1959 somatic promoters.
   D) Correlation between H3K4me3 promoter signals and H3K27ac activity signals in primary gastric samples for gained somatic (Left, r=0.78, p<0.001) and lost somatic (Right, r=0.82, p<0.001) promoters. Each data point corresponds to a single H3K4me3 hi/H3K4me1 lo region. Analysis was performed using data from 16 N/T pairs (Table 4).
   E) Volcano plot of somatic promoters (Top) highlighting the dynamic range of fold changes differences (x-axis) and the false discovery rate (FDR)-adjusted significance (-log10 scale, y axis). The majority of the somatic promoters lie between FC 1 and 2.82, which likely reflects the dynamic range of Chip-seq. The Table (bottom) lists the number of somatic promoters identified at differing levels of stringency. Despite varying FDR thresholds, the majority of differential peaks are still preserved (e.g. 59% at q<0.01).
   F) Enrichment analysis of somatic promoters at varying fold change and FDR (q value) for top 5 genesets (Fig 1F) associated with gained (red) and lost somatic promoters (blue). X axis reflects the -log10 p value for gene-sets found to be enriched in subsets of somatic promoters. Even at stricter fold change (FC 2) and q-value thresholds (0.05, 0.01 and 0.001), similar GC specific and PRC2 associated signatures are still observed.

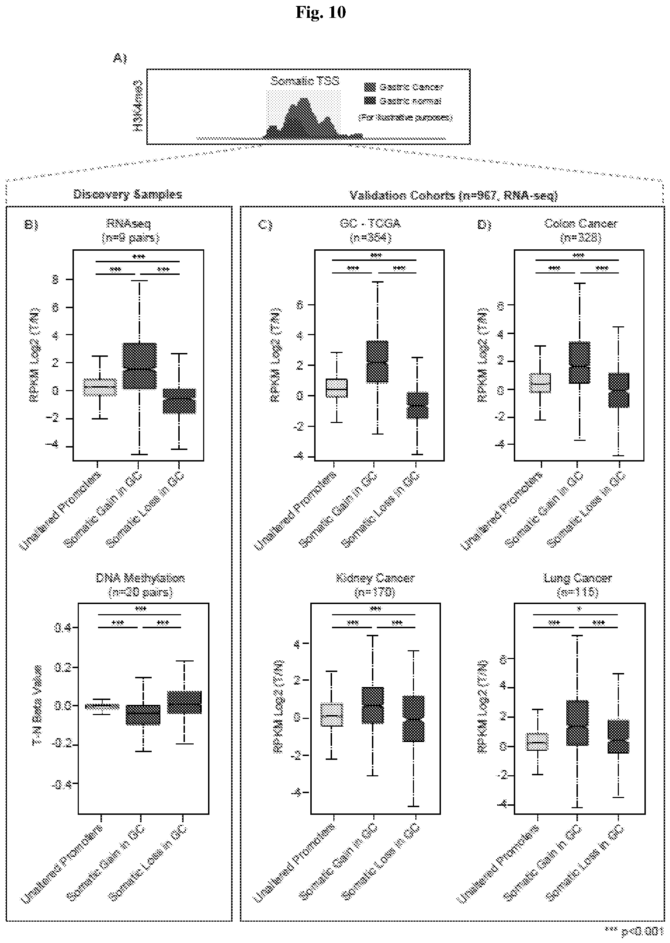

**Figure 10****: Association of Somatic Promoters with Gene Expression in GC and Other Tumor Types**
   A) Example of a GC somatic promoter. Example is for illustrative purposes only.
   B) Changes in RNA-seq expression (top) and DNA methylation (bottom) discovery samples between somatic promoters and unaltered promoters. Top - Boxplot depicting changes in RNA-seq expression between 9 paired primary GC and gastric normal samples at genomic regions exhibiting somatic promoters (gained and lost) (***P<0.001, Wilcoxon Test). Bottom - Boxplot depicting changes in DNA methylation (β-values) at regions exhibiting somatic promoters between 20 paired GC and gastric normal samples, compared to unaltered promoters (***P<0.001, Wilcoxon test)
   C) Independent Validation Cohorts. Boxplot depicting changes in RNA-seq expression at genomic regions exhibiting somatic promoters across 354 (321 GC, 33 normal) TCGA Stomach adenocarcinoma (STAD) samples, compared to unaltered promoters (***P<0.001, Wilcoxon test)
   D) Somatic Promoters in Other Cancer Types. Boxplot depicting changes in RNA-seq expression at genomic regions exhibiting GC somatic promoters compared to unaltered promoters, across 328 TCGA Colon adenocarcinoma (COAD) samples (286 COAD, 40 normal; ***P<0.001, Wilcoxon test), 170 TCGA kidney renal clear cell carcinoma (ccRCC) samples (98 ccRCC and 72 normal; ***P<0.001, Wilcoxon test), and 115 TCGA lung adenocarcinoma (LUAD) samples (58 LUAD, 57 normal; ***P<0.001 Somatic gain vs unaltered and somatic gain vs somatic loss, *P<0.05 Somatic loss vs unaltered, Wilcoxon test).
**Figure 11****: Changes in DNA methylation at CpG island containing promoters**
   A) Boxplot depicting changes in DNA methylation (β-values) at CpG island bearing somatic promoters between 20 paired GC and gastric normal samples, compared to all promoters bearing CpG islands (**P<0.001, Wilcoxon test)
**Figure 12****: Expression distribution of alternative and canonical isoforms**
   A) Barplot showing distribution of T/N ratios of canonical and alternative transcript isoforms for all alternative transcripts (Global - top), *HNF4α* (middle), and *EPCAM* (bottom) using four independent quantification techniques, Cufflinks, MISO, Kallisto and NanoString. The Nanostring platform is introduced in Fig. 4 of the Main Text. ++ Nanostring analysis is confined to queried probes. (*P<0.05, **P<0.01, ***P<0.001, Wilcoxon one sided test).
   B) Boxplot showing the T/N ratio of N-terminal reads mapping to canonical promoters, compared to N-terminal reads mapping to alternative promoters. Alternative promoter driven transcripts exhibit significantly higher T/N ratios (p=0.04, Wilcoxon one sided test).

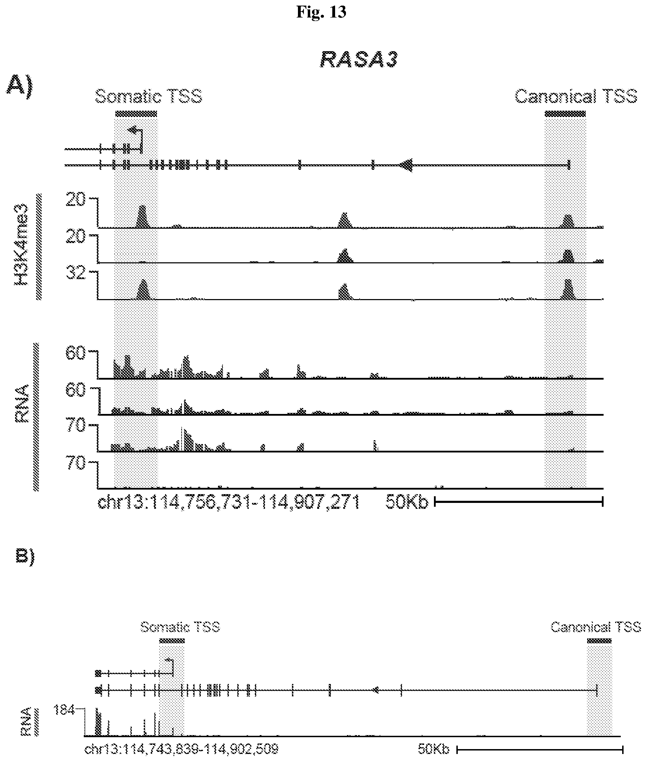

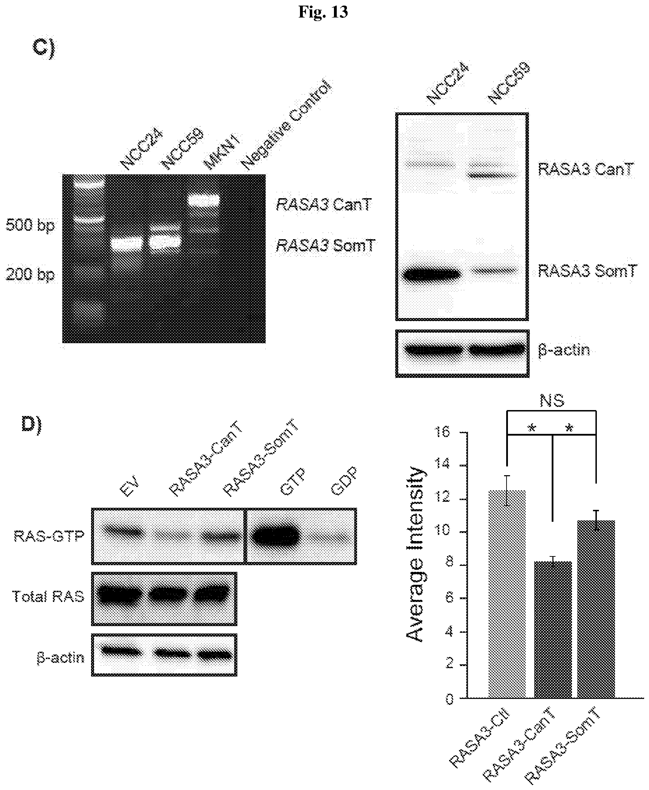

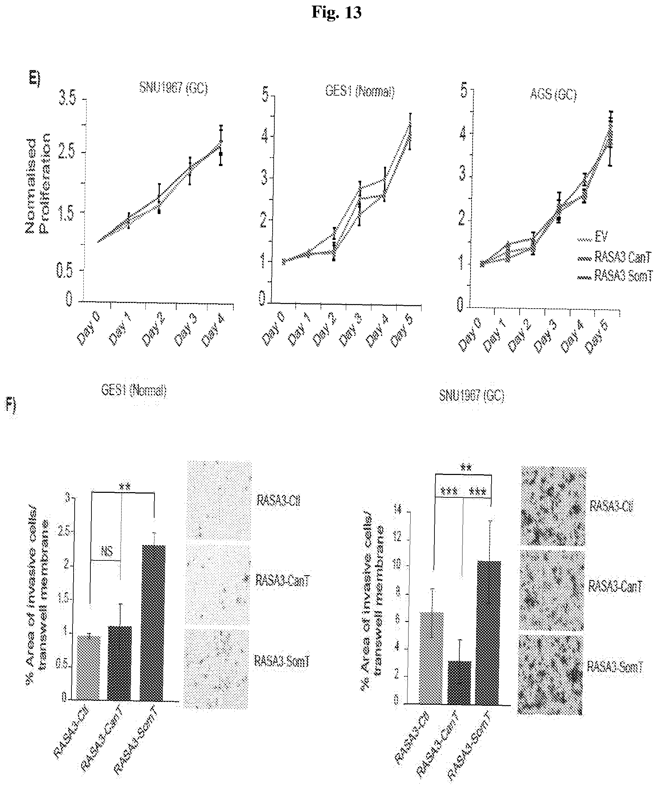

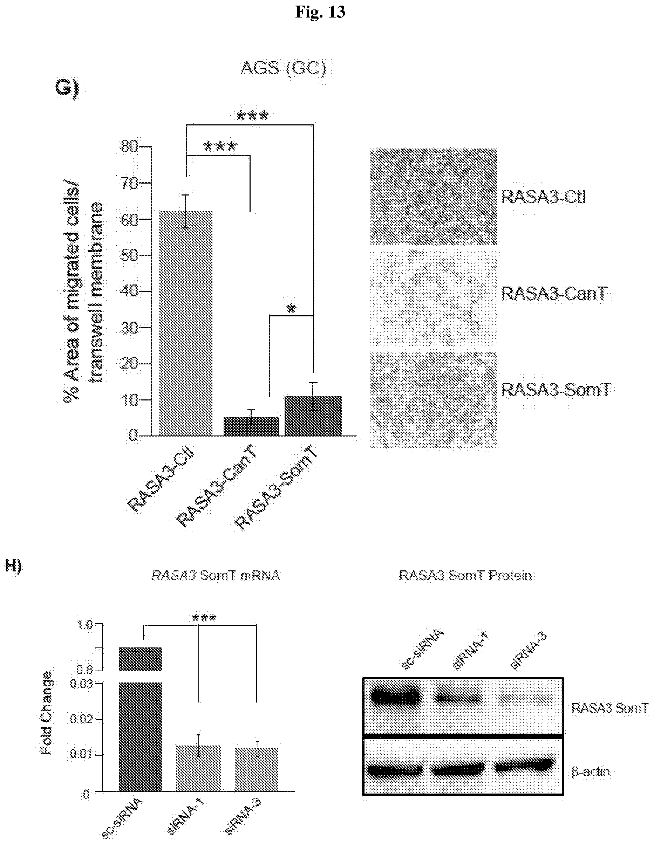

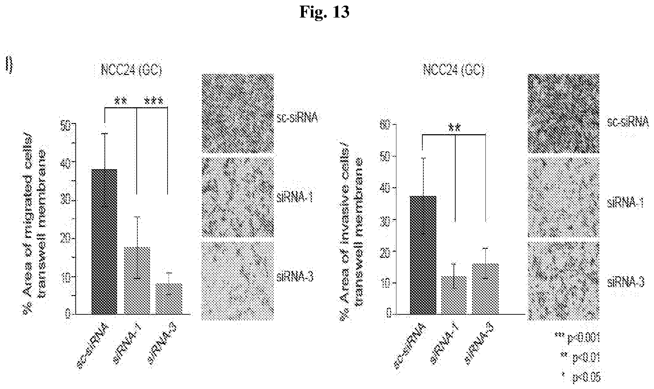

**Figure 13****: Characterization of *RASA3* Isoform**
   A) UCSC browser track of the *RASA3* gene demonstrating H3K4me3 and RNA-seq signals at Somatic and Canonical TSSs. The Canonical TSS has equal signals while the Somatic TSS shows gain of promoter activity at an un-annotated TSS corresponding to a novel N-terminal truncated *RASA3* transcript.
   B) UCSC browser track of the *RASA3* gene demonstrating RNA-seq signals for the NCC24 GC cell line at Somatic and Canonical TSSs. NCC24 only expresses *RASA3* SomT (also see C).
   C) Left - Identification of *RASA3* SomT and CanT transcripts in NCC24 and NCC59 GC cells by 5'RACE. A third line (MKN1), was negative for *RASA3* SomT as shown in the gel picture. A no-RNA template was run as a negative control. Right- Western Blot highlighting expression of RASA3 SomT protein in NCC24 cells.
   D) RAS GTP assays. (left) The Western blot shows levels of RAS in GES1 cells transfected with either empty vector (EV), *RASA3* CanT or *RASA3* SomT (n=3). GES1 cells were serum-starved overnight followed by serum stimulation for 30 minutes prior to harvest and a RAS-GTP pull down assay. Total RAS was measured in corresponding whole cell protein lysates. β-actin was used as a loading control. Positive (GTP) and negative (GDP) controls from the pull down assay are also shown. (right) The barplot quantifies active RAS intensity from three independent pull-down assays, performed in GES1 cells transfected with either empty vector (EV), *RASA3* CanT or *RASA3* SomT under FBS exposed conditions. Data is shown as mean ± SD; n=3. (*P<0.05, Student's two sided t-test).
   E) Cell proliferation assays of SNU1967, GES1 and AGS cells after transfection with *RASA3* CanT and SomT normalized to Day 0. (Data is shown as mean ± SD performed in triplicate, representative of 3 independent experiments).
   F) Effect of overexpression of *RASA3* CanT and SomT isoforms on the invasive capability of GES1 and SNU1967 cells. Representative images of EV, RASA3-WT and *RASA3*-Var in invasion assay (n=3). Barplot showing % area of invaded cells vs the area of transwell membrane. Data is shown as mean ± SD ; n=3. (*P<0.05, **P<0.01, ***P<0.001, Student's one sided t-test).
   G) Effect of overexpression of *RASA3* CanT and SomT protein isoforms on the migration capability of highly migratory *KRAS* mutated AGS cells. Barplot showing % area of migrated cells vs the area of transwell membrane. Data is shown as mean ± SD ; n=3. (*P<0.05, **P<0.01, ***P<0.001, Student's one sided t-test). *RASA3* WT induces more potent migration suppression than RASA3 Var, suggesting that *RASA3* WT is a migration inhibitor.
   H) siRNA-mediated knockdown of RASA3 *SomT* in NCC24 cells. Cells were treated with sc-siRNA (control) and 2 *RASA3* siRNAs (siRNA1 - hs.Ri.RASA3.13 TriFECTa® Kit DsiRNA and siRNA-3 - Silencer® Select Pre-Designed siRNA s355). (Left) Barplots showing fold change differences in mRNA expression of *RASA3* SomT after treatment with siRNA-1 and siRNA-3. Data is shown as mean ± SD ; n=3. (Right) Western blotting results confirming RASA3 SomT protein reductions. Cells were harvested and lysed after 48 hrs of transfection. (***P<0.001, Student's one sided t-test).
   I) Effect of siRNA knockdown of *RASA3* SomT isoform on the migration (left) and invasive (right) capability of NCC24 cells from two independent siRNAs. Representative images of sc-siRNA (control), siRNA-1, and siRNA-3 in migration and invasion assays (n=3). Barplot showing % area of migrated / invaded cells vs the area of transwell membrane. Data is shown as mean ± SD ; n=3. (*P<0.05, **P<0.01, ***P<0.001, Student's one sided t-test).

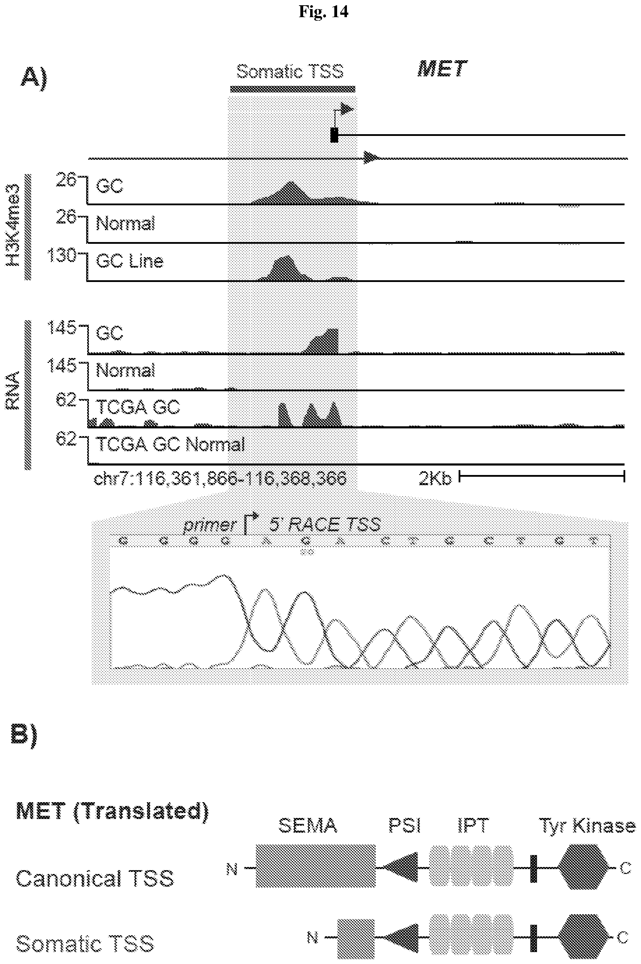

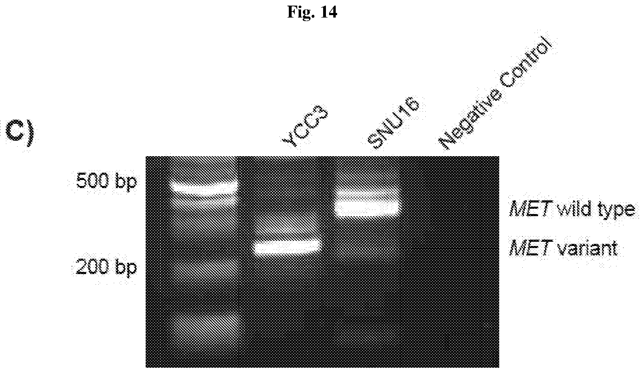

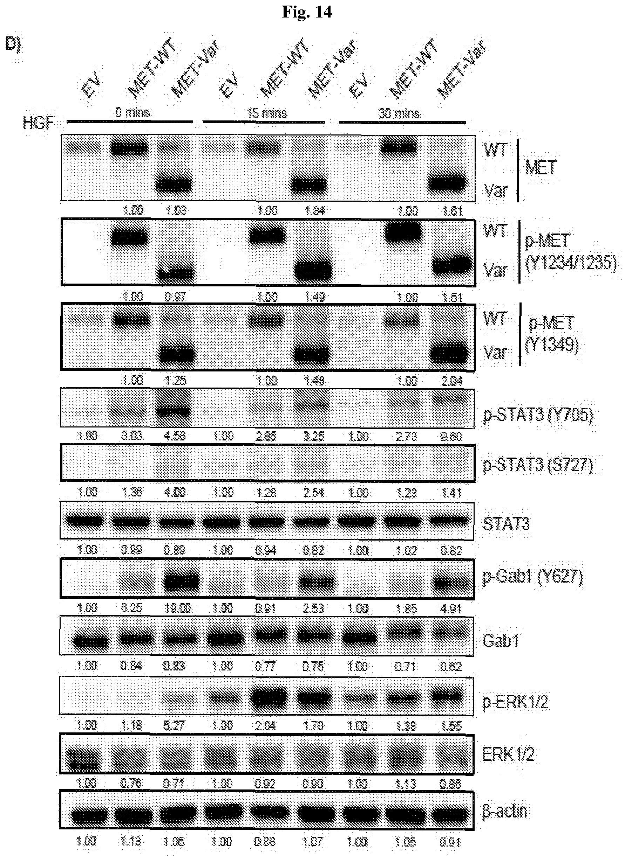

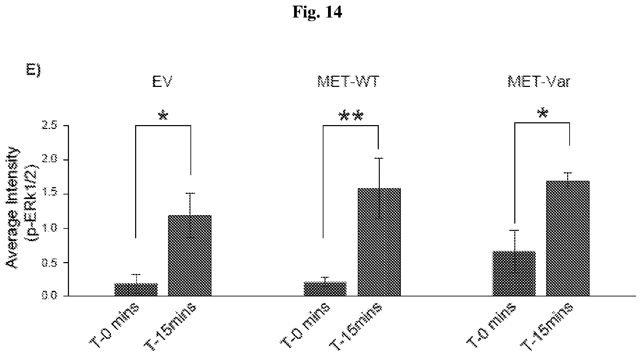

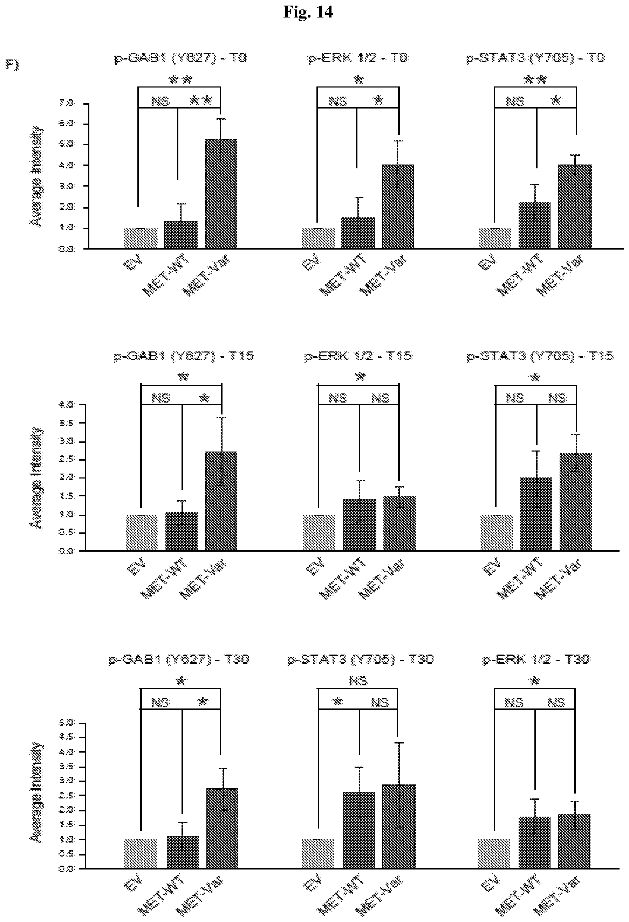

**Figure 14****: Characterization of *MET* Isoforms**
   A) UCSC browser track of the *MET* gene, demonstrating H3K4me3 and RNA-seq signals highlighting gain of promoter activity at an alternative downstream locus (dark grey box).
   B) Functional domains of the MET canonical (WT) and alternative (Var) isoform. The alternative isoform is predicted to encode a MET protein with an N terminally truncated SEMA domain.
   C) Expression of *MET* (Var) transcripts in GC lines, as detected by 5'RACE.
   D) Western blot of HEK293 cells transfected with empty vector (EV), *MET* canonical full length (MET-WT) and truncated Variant (MET-Var) at 0, 15 and 30 minutes of HGF treatment (100 ng/ml) (n=3). GAB1, STAT3 and ERK1/2 are known downstream effectors of MET signaling. Number below each band is the quantified intensity using Image Lab. In both untreated and HGF-treated conditions, MET-Var transfected cells exhibited higher levels of p-Gab1 (Y627), a key mediator of MET signaling (2.48-3.95 fold, p=0.003 (untreated), p<0.05 (T15 and T30). In untreated samples, cells transfected with MET-Var also exhibited higher pERK1/2 levels (2.74 fold) and also higher p-STAT3 (Y705) levels (1.80 fold) compared to MET-WT (p=0.023 and p=0.026 for pERK and p-STAT3 (Y705) respectively).
   E) Bar graphs showing increase in pERK1/2 for EV, MET-WT and MET-Var at T0, T15 and T30, reflecting effects of HGF treatment. Data is shown as mean ± SD; n=3. (*P<0.05, **P<0.01, ***P<0.001, Student's one sided t-test)
   F) Bar graphs showing increase in p-GAB1 (Y627), p-STAT3 (Y705), and pERK1/2 in cells transfected with MET-Var compared to EV and MET-WT. Graphs for all 3 time points are shown. Data is shown as mean ± SD; n=3. (*P<0.05, **P<0.01, ***P<0.001, Student's one sided t-test)

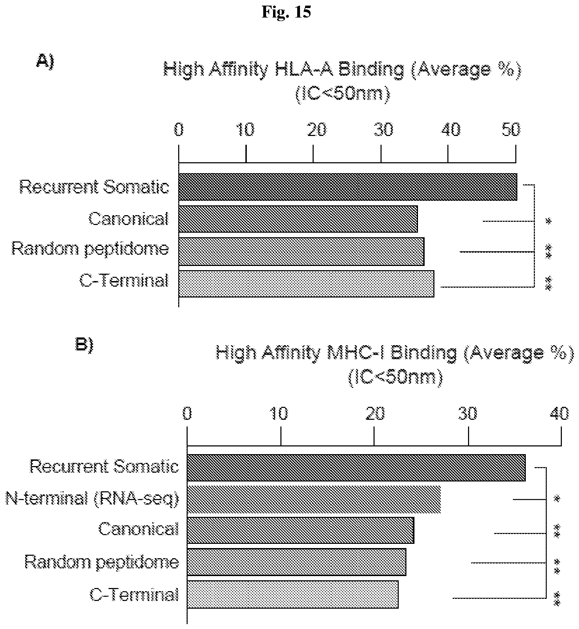

**Figure 15****: Immunogenicity of N-terminal peptides**
   A) Barplot showing average % of N-terminal peptides with predicted high-affinity binding to MHC Class I HLA-A (IC<=50 nm). As comparison, the figure in the Main Text represents average %s based on all three HLA classes (HLA-A, HLA-B, HLA-C). N-terminal peptides associated with recurrent somatic alternative promoters show significantly enriched predicted MHC I binding compared to canonical GC peptides (p<0.01), random peptides from human proteome and C-terminal peptides (p<0.001, Fisher's Test) derived from the same genes exhibiting the N-terminal alterations.
   B) MHC Binding Predictions using N-terminal peptides inferred by RNA-seq analysis alone. Annotated transcripts exhibiting different N-terminal exons in GC vs normals were identified using two different RNA-seq algorithms (DEXSeq(7) and Voom-diffsplice(8)) (FC>=2, FDR 0.05). This analysis identified 96 genes with potential alternative N-terminal transcripts, of which 46 (48%) were predicted to result in differing N terminal peptides (Purple bar).

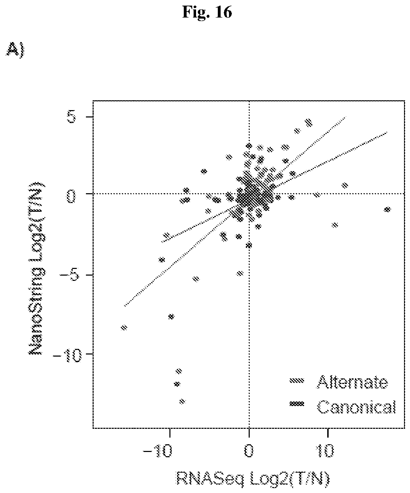

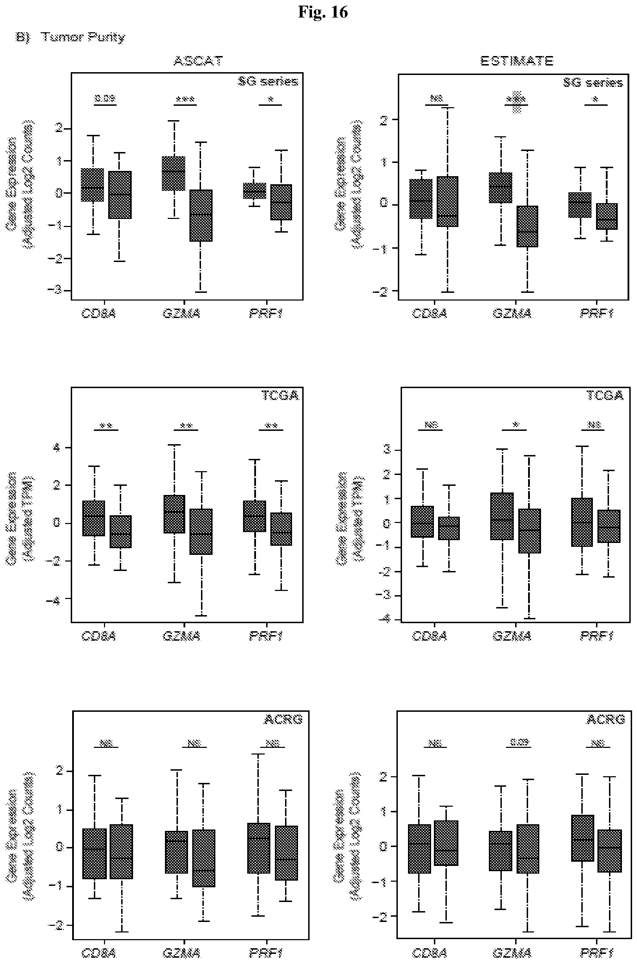

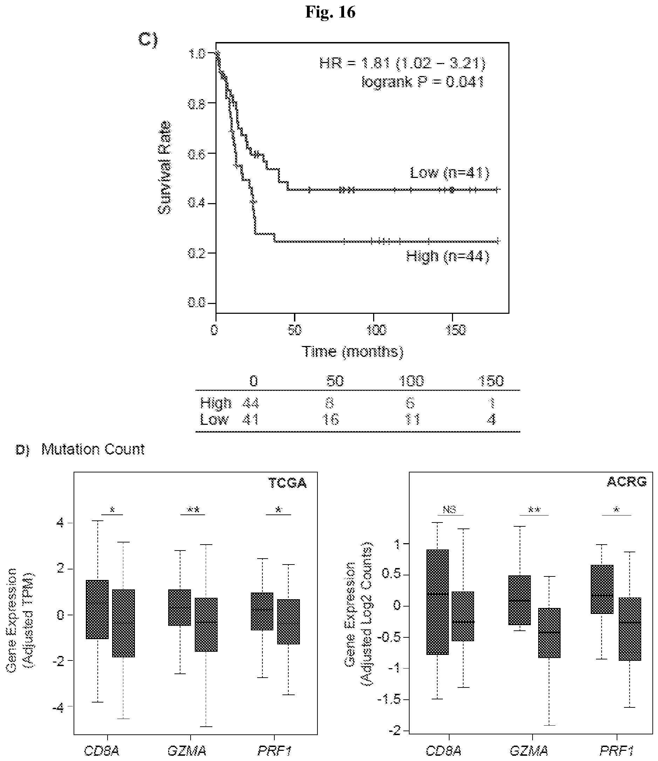

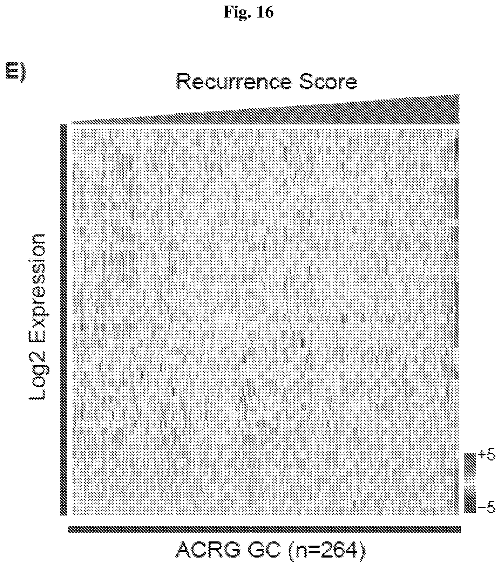

**Figure 16****: Immunogenicity Assay and Nanostring Profiling**
   A) Scatter plot of fold change (T vs N) of expression of alternate and canonical probes from NanoString and RNA-seq data of the same samples. An improved correlation is observed using the alternate probes
   B) Left - Expression of T-cell markers CD8A, GZMA and PRF1 in SG series (top), TCGA STAD (middle) and ACRG cohort (bottom) with high or low somatic promoter usage after adjustment of tumor purities as estimated by ASCAT. P values (Wilcoxon one sided test) are: CD8A - p= 0.09 (SG), 0.004 (TCGA), 0.3 (ACRG); GZMA - 0.0001 (SG), 0.002 (TCGA), 0.166 (ACRG), PRF1 - 0.013 (SG), 0.006 (TCGA), 0.3 (ACRG). Right - Expression of T-cell markers CD8A, GZMA and PRF1 in SG series (top), TCGA STAD (middle) and ACRG cohort (bottom) with high or low somatic promoter usage after adjustment of tumor content as estimated by ESTIMATE, p values (Wilcoxon one sided test) are: CD8A - p= 0.28 (SG), 0.17 (TCGA), 0.37 (ACRG), GZMA - 0.0005 (SG), 0.03 (TCGA), 0.09 (ACRG), PRF1 - 0.02 (SG), 0.22 (TCGA), 0.17 (ACRG). Samples with high alternative promoter usage are in red, while those with low usage are in blue.
   C) Kaplan-Meier analysis comparing overall survival curves between validation samples with high somatic promoter usage and low somatic promoter usage (split by median) (HR= 1.81, P=0.04)
   D) Left - Expression of T-cell markers CD8A, GZMA and PRF1 in TCGA STAD with high or low somatic promoter usage after adjustment of mutation burden. P values (Wilcoxon one sided test) are: P= 0.02 (CD8A), 0.01 (GZMA) and 0.03 (PRF1). Right - Expression of T-cell markers CD8A, GZMA and PRF1 in ACRG cohort with high or low somatic promoter usage after adjustment of mutation burden. P values (Wilcoxon one sided test) are: P= 0.167 (CD8A), 0.009 (GZMA) and 0.03 (PRF1).
   E) Heatmap of alternative promoter expression from 264 ACRG GCs for all gained alternative promoters. GC samples have been ordered left to right by their levels of somatic promoter usage.

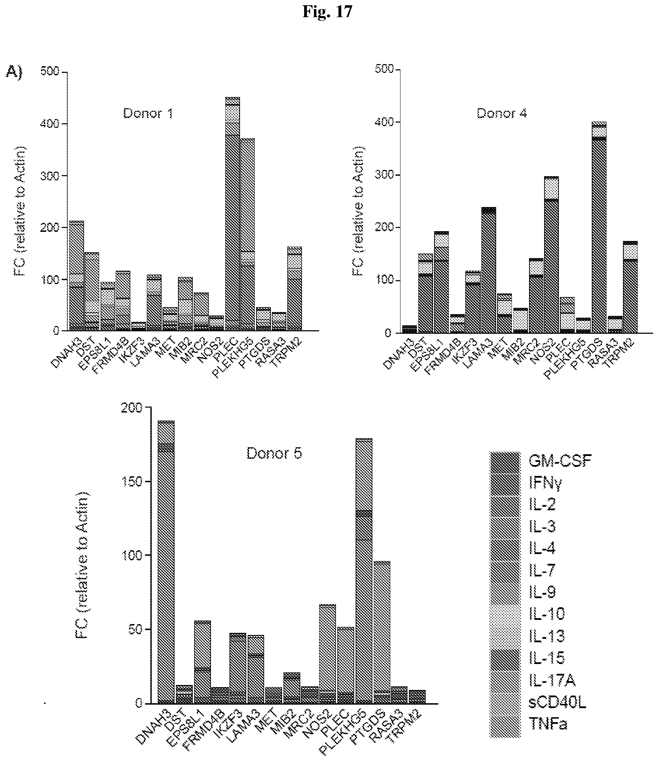

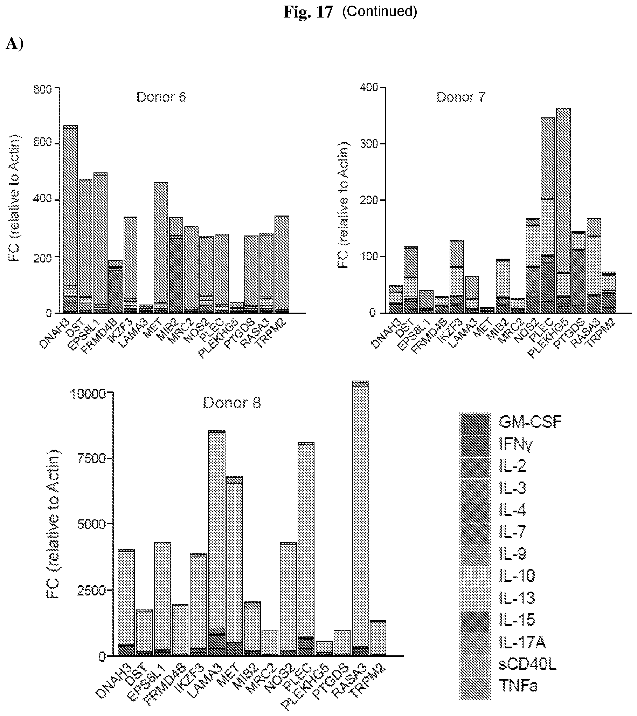

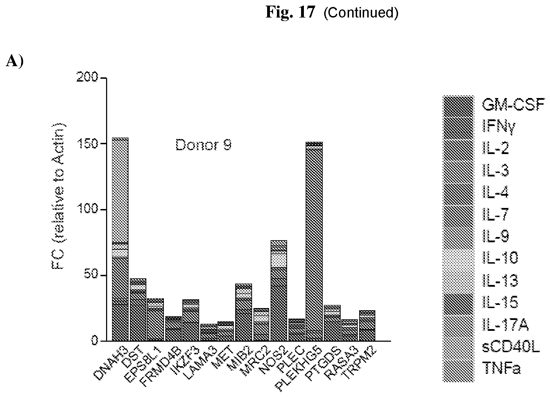

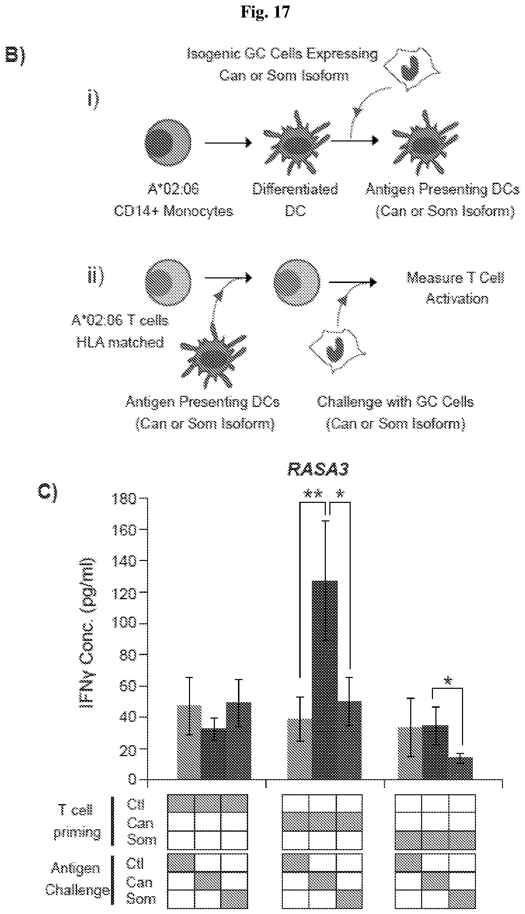

**Figure 17****: Functional Assessment of Peptide Immunogenicity**
   A) Individual cytokine responses against 15 peptides for other normal donor PBMCs tested against different peptide pools.
   B) Experimental Immunogenicity Assay. Experimental design of *in-vitro* assay - i) Immature dendritic cells (DCs) cultured from CD14⁺ monocytes from HLA-A02:06 donors were differentiated in mature DCs (see Methods). Mature DCs were exposed to isogenic GC cell lysates (AGS cells) expressing Canonical (CanT) and Somatic (SomT) *RASA3* isoforms. ii) Antigen presentation and T-cell activation: DCs presenting Can or Som *RASA3* isoforms were co-cultured with HLA-matched T cells, resulting in T-cells primed against CanT or SomT RASA3. Primed T cells were then independently co-cultured with *RASA3* CanT or *RASA3* SomT expressing GC cells for two days, and markers of T-cell activation were assessed.
   C) Concentration of interferon-gamma (IFN-γ) secretion by co-culture of T cells primed with *RASA3* CanT or SomT Isoforms, after antigen challenge. RASA3 CanT primed T cells released significantly more IFN-γ when co-cultured with RASA3 CanT expressing cells, compared to T cells primed with RASA3 SomT and co-cultured with *RASA3* SomT expressing cells (P=0.02, representative of n=3 experiments). IFN-γ levels were determined by ELISA.

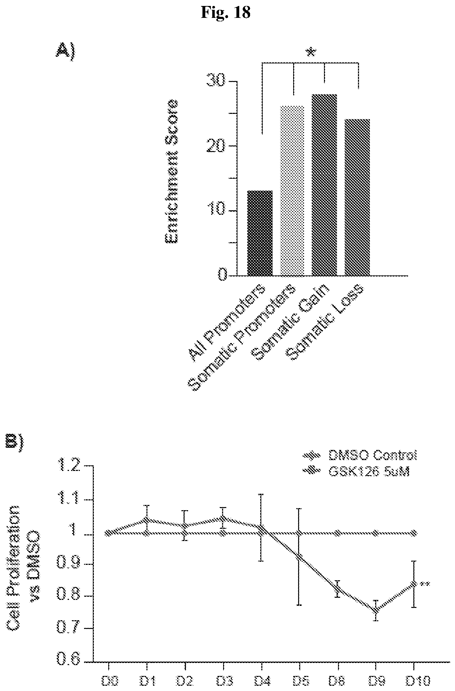

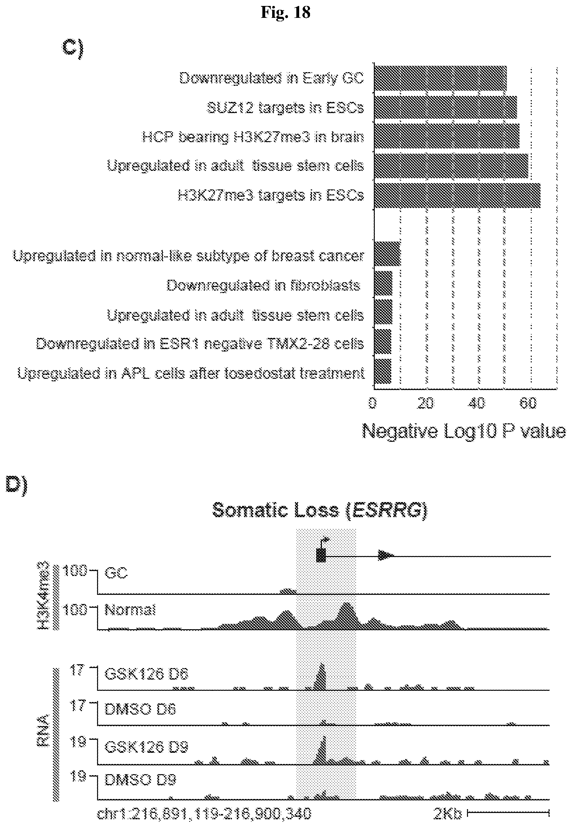

**Figure 18****: EZH2 Inhibition**
   A) Barplot showing increased enrichment of EZH2 binding sites in HFE-145 cells at somatic promoters compared to all promoters (P<0.01).
   B) Growth curves of IM95 GC cells after GSK126 administration. Cell proliferation was monitored from 24 to 216 hours and represented relative to DMSO control treated cells (means ± s.e.m. represents data from three experiments, and each experiment was performed in duplicate)
   C) Top 5 enriched curated gene sets (C2) for the set of genes identified from differential analysis of GSK126 treated vs DMSO control IM95 RNA-seq data at promoter loci.
   D) UCSC browser track of alternative promoter ESRRG with loss of promoter activity (GC (red) and normal gastric tissue (blue) H3K4me3). Gain of expression is seen after inhibition of EZH2 using GSK126 in IM95 cells at both day 6 (D6) and Day 9 (D9) treatment.

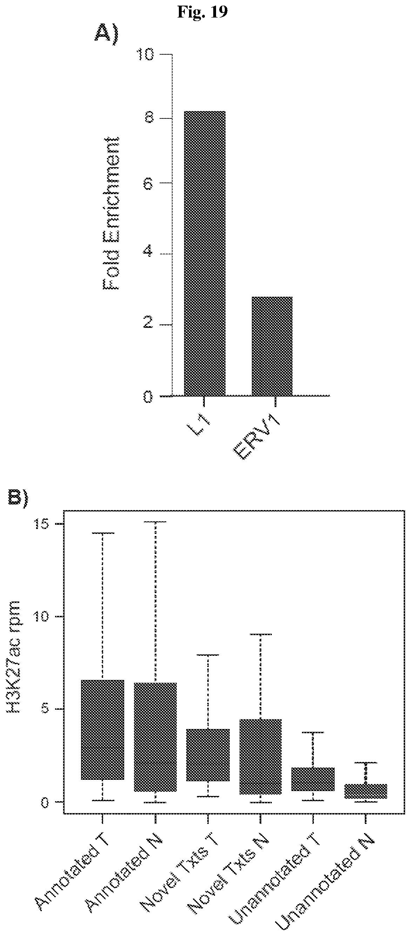

**Figure 19****: Unannotated somatic promoters**
   A) Barplot showing fold enrichment of L1 (FC=8.02, P<0.001) and ERV1 (FC=2.78, P<0.001) repeat elements at unannotated promoter regions compared to all promoters
   B) Boxplot comparing H3K27ac signals (rpm) at unannotated somatic promoters with annotated somatic promoters. Unannotated somatic promoters have lower H3K27ac signals.

### DETAILED DESCRIPTION OF THE PRESENT INVENTION

The invention is defined in the appended claims.

In a first aspect, the present invention refers to a method for determining the presence or absence of at least one promoter in a cancerous biological sample relative to a non-cancerous biological sample. The method comprises contacting the cancerous biological sample with at least one antibody or antibodies specific for histone modifications H3K4me3 and H3K4me1; isolating nucleic acid from the cancerous biological sample having a signal ratio of H3K4me3 relative to H3K4me1 greater than 1, wherein the isolated nucleic acid comprises at least one region or regions specific to said histone modifications; detecting a signal intensity of H3K4me3 in the isolated nucleic acid; and determining the presence or absence of at least one promoter in the cancerous biological sample based on the change in the signal intensity of H3K4me3 relative to the signal intensity of H3K4me3 in a non-cancerous biological sample.

In one embodiment, the cancerous and non-cancerous biological sample may comprise a single cell, multiple cells, fragments of cells, body fluid or tissue. In one embodiment the cancerous and non-cancerous biological sample may be obtained from the same subject.

In one embodiment, the cancerous and non-cancerous biological sample are each obtained from different subjects.

The contacting step in accordance with the method as described herein may comprise the immunoprecipitation of chromatin with the antibodies specific for the histone modifications. Examples of histone modification include but are not limited to H3K27ac, H3K4me3, H3K4me1. In a preferred embodiment, the histone modification is H3K4me3 and/or H3K4me1. In yet another embodiment, the histone modification is H3K27ac.

The method may further comprise mapping at least one promoter from the cancerous biological sample against at least one reference nucleic acid sequence to identify a gene transcript associated with the at least one promoter.

In some embodiments, the at least one reference nucleic acid sequence may comprise a nucleic acid sequence derived from: i) an annotated genome sequence; ii) a *de novo* transcriptome assembly; and/or iii) a non-cancerous nucleic acid sequence library or database.

In one embodiment, the change of signal intensity of H3K4me3 may be greater than a 0.5 fold, greater than a 1 fold, greater than a 1.5 fold, greater than a 2 fold, greater than a 2.5 fold or greater than a 3 fold increase or decrease relative to the signal intensity of H3K4me3 in the non-cancerous biological sample. In a preferred embodiment, the change of signal intensity of H3K4me3 may be greater than a 1.5 fold increase or decrease relative to the signal intensity of H3K4me3 in the non-cancerous biological sample. In another embodiment, the change of signal intensity of H3K4me3 greater than a 0.5 fold, greater than a 1 fold, greater than a 1.5 fold, greater than a 2 fold, greater than a 2.5 fold or greater than a 3 fold increase relative to the signal intensity of H3K4me3 in a non-cancerous biological sample, may correlate to the presence of at least one cancer-associated promoter in the cancerous biological sample.

In a preferred embodiment the change of signal intensity of H3K4me3 greater than a 1.5 fold increase relative to the signal intensity of H3K4me3 in a non-cancerous biological sample, may correlate to the presence of at least one cancer-associated promoter in the cancerous biological sample.

In one embodiment, the activity of the at least one cancer-associated promoter may correlate with an increase of SUZ12 or EZH2 binding sites relative to the total promoter population.

In one embodiment, an increase of SUZ12 or EZH2 binding sites correlates with an upregulation of activity of the at least one cancer-associated promoter. In another embodiment, the increase of SUZ12 or EZH2 binding sites correlates with a downregulation of activity of the at least one cancer-associated promoter.

In one embodiment, the at least one promoter may be a canonical promoter that is positioned within 100 bp, 200 bp, 300 bp, 400 bp, 500bp, 600 bp, 700 bp, 800 bp, 900 bp or 1000 bp from a known gene transcript start site. In a preferred embodiment, the at least one promoter may be a canonical promoter that is positioned within 500bp from a known gene transcript start site. The gene transcript start site may be associated with one or more of a cell-type specification gene, a cell adhesion gene, a cell mediated immunity gene, a gastric cancer-associated or deregulated gene, a PRC2 target gene or a transcription factor. In one embodiment, the gene transcript start site may be associated with an oncogene. The gene transcript start site may be associated with a gene selected from the group consisting of MYC, MET, CEACAM6, CLDN7, CLDN3, HOTAIR, PVT1, HNF4α, RASA3, GRIN2D, EpCAM and a combination thereof.

In one embodiment, the cancer is gastrointestinal cancer, gastric cancer or colon cancer.

In another embodiment, the at least one promoter may be an alternative promoter that may be associated with a canonical promoter, wherein the canonical promoter may be present in both the cancerous biological sample and the non-cancerous biological sample, and i) wherein the alternative promoter may be only present in the cancerous biological sample, or ii) wherein the alternative promoter may be only absent in the cancerous biological sample.

In some embodiments, the at least one promoter is an unannotated promoter that is positioned more than 100 bp, more than 200 bp, more than 300 bp, more than 400 bp, more than 500bp away, more than 600 bp, more than 700 bp, more than 800 bp, more than 900 bp or more than 1000 bp from a gene transcript start site. In a preferred embodiment, the at least one promoter is an unannotated promoter that is positioned more than 500bp away from a gene transcript start site.

In one embodiment, the method as described herein further comprises measuring the expression level of the at least one alternative promoter in the cancerous biological sample and non-cancerous biological sample, wherein the measuring comprises digital profiling of reporter probes; and determining the differential expression level of the at least one alternative promoter relative to the non-cancerous biological sample, based on the digital profiling of the reporter probes, to validate the presence or absence of at least one alternative promoter in the cancerous biological sample relative to a non-cancerous biological sample.

The step of measuring may be conducted using a NanoString™ platform.

In another aspect, the present invention provides a method for determining the prognosis of cancer in a subject. The method comprises contacting a cancerous biological sample obtained from the subject with at least one antibody or antibodies specific for histone modification H3K4me3 and H3K4me1; isolating nucleic acid from the cancerous biological sample having a signal ratio of H3K4me3 relative to H3K4me1 greater than 1, wherein the isolated nucleic acid comprises at least one region or regions specific to said histone modifications; detecting a signal intensity of H3K4me3 in the isolated nucleic acid; and determining the presence or absence of at least one cancer-associated promoter in the cancerous biological sample based on the change in the signal intensity of H3K4me3 relative to the signal intensity of H3K4me3 in a reference nucleic acid sequence, wherein the presence or absence of the at least one cancer-associated promoter in the cancerous biological sample is indicative of the prognosis of the cancer in the subject.

In one embodiment, the at least one cancer-associated promoter may be an alternative promoter that is associated with a canonical promoter, wherein the canonical promoter may be present in both the cancerous biological sample and the reference nucleic acid sequence, and i) wherein the alternative promoter may be only present in the cancerous biological sample, or ii) wherein the alternative promoter may be only absent in the cancerous biological sample.

The presence or absence of the at least one alternative promoter in the cancerous sample may indicative of a poor prognosis of cancer survival in the subject.

In one embodiment the method as described herein further comprises measuring the expression level of the at least one alternative promoter in the cancerous biological sample and the reference nucleic acid sequence, wherein the measuring comprises digital profiling of reporter probes; and determining the differential expression level of the at least one alternative promoter relative to the non-cancerous biological sample, based on the digital profiling of the reporter probes, to validate the presence or absence of at least one alternative promoter in the cancerous biological sample relative to the reference nucleic acid sequence.

The step of measuring may be conducted using a NanoString™ platform.

In another aspect the present disclosure provides a biomarker for detecting cancer in a subject, the biomarker comprising at least one promoter having a change in signal intensity of H3K4me3 in a cancerous biological sample relative to a non-cancerous biological sample.

In one embodiment, the at least one promoter comprises an increase of EZH2 binding sites relative to the total promoter population. In one embodiment, the at least one promoter may be hypomethylated. In another embodiment, the at least one promoter may be hypermethylated.

The at least one promoter may be a canonical promoter that is positioned less than 500bp away from a gene transcript start site. In one embodiment, the gene transcript start site may be associated with one or more of a cell-type specification gene, a cell adhesion gene, a cell mediated immunity gene, a gastric cancer-associated or deregulated gene, a PRC2 target gene or a transcription factor. In one embodiment, the gene transcript start site may be associated with an oncogene.

In one embodiment, the gene transcript start site may be associated with a gene selected from the group consisting of MYC, MET, CEACAM6, CLDN7, CLDN3, HOTAIR, PVT1, HNF4α, RASA3, GRIN2D, EpCAM or a combination thereof.

In one embodiment, the at least one promoter may be an alternative promoter that may be associated with a canonical promoter, wherein the canonical promoter may be present in both a cancerous sample and a non-cancerous sample, and i) wherein the alternative promoter may be only present in a cancerous sample, or ii) wherein the alternative promoter may be only absent in a cancerous sample.

In one embodiment, the at least one promoter may be an unannotated promoter that may be positioned more than 100 bp, more than 200 bp, more than 300 bp, more than 400 bp, more than 500bp, more than 600 bp, more than 700 bp, more than 800 bp, more than 900 bp or more than 1000 bp away from a gene transcript start site. In a preferred embodiment, the at least one promoter may be an unannotated promoter that may be positioned more than 500bp away from a gene transcript start site.

In another aspect, there is provided a method for modulating the activity of at least one cancer-associated promoter in a cell, comprising administering an inhibitor of EZH2 to the cell. In another aspect there is provided a method for modulating the immune response of a subject to cancer, comprising administering to the subject an inhibitor of EZH2, wherein the EZH2 is associated with at least one cancer-associated promoter in the subject.

In one embodiment, the inhibitor of EZH2 may modulate the expression of immunogenic N-terminal peptides.

In one embodiment, the at least one cancer-associated promoter may be an alternative promoter that may be associated with a canonical promoter, wherein the canonical promoter may be present in both a cancerous sample and a non-cancerous sample, and i) wherein the alternative promoter may only be present in a cancerous sample, or ii) wherein the alternative promoter may only be absent in a cancerous sample.

In one embodiment, the alternative promoter is associated with a transcript variant, and wherein the transcript variant encodes a N-terminal protein variant.

In one embodiment, the N-terminal protein variant may be an N-terminal truncated protein or an N-terminal elongated protein. In one embodiment, the inhibitor of EZH2 may be a siRNA or a small molecule.

In one embodiment, the inhibitor of EZH2 may be GSK126.

In another aspect, there is provided use of an inhibitor of EZH2 in the manufacture of a medicament for modulating the activity of at least one cancer-associated promoter in a cell.

In another aspect there is provided use of an inhibitor of EZH2, wherein the EZH2 is associated with at least one cancer-associated promoter in the subject, in the manufacture of a medicament for modulating the immune response of a subject to cancer.

In another aspect, there is provided an inhibitor of EZH2 for use in modulating the activity of at least one cancer-associated promoter in a cell. In yet another aspect, there is provided an inhibitor of EZH2 for use in modulating the immune response of a subject to cancer, wherein the EZH2 is associated with at least one cancer-associated promoter in the subject.

In another aspect there is provided a method for determining the presence or absence of at least one cancer-associated promoter in a cancerous biological sample relative to a non-cancerous biological sample. The method comprises: contacting the cancerous biological sample with antibodies specific for histone modifications H3K4me3 and H3K4me1; isolating nucleic acid from the cancerous biological sample having a signal ratio of H3K4me3 relative to H3K4me1 greater than 1, wherein the isolated nucleic acid comprises regions specific to said histone modifications; detecting a signal intensity of H3K4me3 in the isolated nucleic acid at a read depth of 20M; and determining the presence or absence of at least one cancer-associated promoter in the cancerous biological sample based on the change in the signal intensity of H3K4me3 relative to the signal intensity of H3K4me3 in a non-cancerous biological sample.

### EXPERIMENTAL SECTION

### Methods and Materials

### Primary Tissue Samples and Cell Lines

Primary patient samples were obtained from the SingHealth tissue repository with approvals from institutional research ethics review committees and signed patient informed consent. 'Normal' (non-malignant) samples used in this study refers to samples harvested from the stomach, from sites distant from the tumour and exhibiting no visible evidence of tumour or intestinal metaplasia/dysplasia upon surgical assessment. Tumor samples were confirmed by cryosectioning to contain >60% tumor cells. FU97, IM95, MKN7, OCUM1 and RERF-GC-1B cell lines were obtained from the Japan Health Science Research Resource Bank. AGS, KATOIII and SNU16, Hs 1.Int and Hs 738.St/Int gastrointestinal fibroblast lines were obtained from the American Type Culture Collection. NCC-59, NCC-24 and SNU-1967 and SNU-1750 were obtained from the Korean Cell Line Bank. YCC3, YCC7, YCC21, YCC22 were gifts from Yonsei Cancer Centre, South Korea. HFE145 cells were a gift from Dr. Hassan Ashktorab, Howard University. GES-1 cells were a gift from Dr. Alfred Cheng, Chinese University of Hong Kong. Cell line identifies were confirmed by STR DNA profiling using ANSI/ATCC ASN-0002-2011 guidelines. For our study, MKN7 cells, listed as a commonly misidentified cell line by ICLAC (http://iclac.org/databases/cross-contaminations/), exhibited a perfect match (100%) with MKN7 reference profiles in the Japanese Collection of Research Bioresources Cell Bank. All cell lines were negative for mycoplasma contamination as assessed by the MycoAlert™ Mycoplasma Detection Kit (Lonza) and the MycoSensor qPCR Assay Kit (Agilent Technologies). PBMCs from healthy donors were collected under protocol CIRB Ref No. 2010/720/E.

### Nano-ChIPseq

Nano-ChIP-Seq was performed as described below.

### Primary Tissue and Cell Line Fixation

Fresh-frozen cancer and normal tissues were dissected using a razor blade in liquid nitrogen to obtain ∼5mg sized pieces for each ChIP. Tissue pieces were fixed in 1% formaldehyde/PBS buffer for 10 min at room temperature. Fixation was stopped by addition of glycine to a final concentration of 125 mM. Tissue pieces were washed 3 times with TBSE buffer. For cell lines, 1 million fresh harvested cells were fixed in 1% formaldehyde/medium buffer for 10 minutes (min) at room temperature. Fixation was stopped by addition of glycine to a final concentration of 125 mM. Fixed cells were washed 3 times with TBSE buffer, and centrifuged (5,000 r.p.m., 5 min).

### ChIP

Pelleted cells and pulverized tissues were lysed in 100 µl 1% SDS lysis buffer and sonicated to 300-500bp using a Bioruptor (Diagenode). ChIP was performed using the following antibodies: H3K4me3 (07-473, Millipore); H3K4me1 (ab8895, Abcam); H3K27ac (ab4729, Abcam).

### WGA

After recovery of ChIP and input DNA, whole-genome-amplification was performed using the WGA4 kit (Sigma-Aldrich) and BpmI-WGA primers. Amplified DNAs were purified using PCR purification columns (QIAGEN) and digested with BpmI (New England Biolabs) to remove WGA adapters.

### Library preparation and sequencing

30ng of amplified DNA was used for each sequencing library preparation (New England Biolabs). 8 libraries were multiplexed (New England Biolabs) and sequenced on 2 lanes of a Hiseq2500 sequencer (Illumina) to an average depth of 20-30 million reads per library.

Sequencing reads were trimmed (10bp from front and back) and mapped against human genome reference hg19 using the Burrows-Wheeler Aligner (BWA) (version 0.6.2) 'aln' algorithm. Reading statistics were generated using mapstat from samtools. We filtered reads based on their mapping quality (MAPQ >=10) and used uniquely mapped reads to perform peak calling using CCAT v3.0. We chose a MAPQ value of ≥10 because i) MAPQ≥10 has been previously reported as a reliable value for confident read mapping, ii) MAPQ≥10 has been recommended by the developers of the BWA-algorithm as a suitable threshold for confident mapping, and iii) independent studies comparing various read alignment algorithms have shown that mapping accuracies plateau at a 10-12 MAPQ threshold.

### EZH2 ChIP-seq

Cells were cross-linked with 1% formaldehyde for 10 minutes at room temperature, and stopped by adding glycine to a final concentration of 0.2M. Chromatin was extracted and sonicated to ∼500bp fragments. EZH2 antibodies (Catalog #5246, Cell Signaling) were used for chromatin immunoprecipitation (ChIP). 30ng of ChIPed DNA was used for each sequencing library preparation (New England Biolabs). The library was sequenced on a Hiseq2500 (Illumina). Input DNA from cells prior to immunoprecipitation was used to normalize ChIP-seq peak calling. Prior to sequencing, qPCR was used to verify that positive and negative control ChIP regions were amplified in the linear range. Sequencing reads were mapped against human genome reference hg19 using the Burrows-Wheeler Aligner (BWA) (version 0.7) 'aln' algorithm. Reading statistics were generated using mapstat from samtools. We filtered reads based on their mapping quality (MAPQ >=10) and used uniquely mapped reads to perform peak calling using MACS2.

### Quality Control Assessments of Nano-ChIPseq Data

### ChIP enrichment assessment

We assessed ChIP library qualities (H3K27ac, H3K4me3 and H3K4me1) using two different methods. First, we estimated ChIP qualities, particularly H3K27ac and H3K4me3, by interrogating their enrichment levels at annotated promoters of protein-coding genes. Specifically, we computed median read densities of input and input-corrected ChIP signals around the transcription start sites (TSSs, +/- 500bp) of highly expressed protein-coding genes. For each sample, we then compared read density ratios of ChIP over input as a surrogate of data quality, retaining only those samples where the ChIP/input ratio was greater than 2-fold. Using this criteria, all H3K4me3 and H3K27ac samples (GC lines and primary samples) exhibited greater than 2-fold enrichment, indicating successful enrichment. Second, we used CHANCE (ChIp-seq ANalytics and Confidence Estimation), a software for ChIP-seq quality control and protocol optimization that indicates whether a ChIP library shows successful or weak enrichment. CHANCE assessment confirmed that the large majority (81%) of samples in our study exhibited successful enrichment. Quality status of each library, as assessed by both methods, are reported in Table 1.

**Table 1: Read Mapping statistics of NanoChIP-seq libraries**

| **S.No** | **Patient No** | **Group** | **Sample ID** | **Library ID** | **Histone Modification** | **Total Reads** | **Total Mapped Reads** | **# of Peaks (FDR <5%, CCAT)** | **CHANCE Enrich ment** | **ChIP enrichment aro und TSS (>2 Fold** ) |
|---|---|---|---|---|---|---|---|---|---|---|
| 1 | 1 | N | 2000639 | CHG023 | H3K4Me1 | 116,179,997 | 56,009,114 | 11,438 | successful | yes |
| 2 | 1 | N | 2000639 | CHG079 | H3K4Me3 | 144,760,092 | 45,662,594 | 13,301 | successful | yes |
| 3 | 1 | N | 2000639 | CHG022 | H3K27Ac | 107,005,238 | 47,688,264 | 30,155 | successful | yes |
| 4 | 1 | N | 2000639 | CHG021 | Input | 108,432,681 | 53,434,667 | - | - | - |
| 5 | 1 | T | 2000639 | CHG019 | H3K4Me1 | 139,751,844 | 62,529,719 | 9,133 | successful | yes |
| 6 | 1 | T | 2000639 | CHG078 | H3K4Me3 | 176,761,815 | 52,219,714 | 15,417 | successful | yes |
| 7 | 1 | T | 2000639 | CHG018 | H3K27Ac | 125,811,014 | 56,636,793 | 22,220 | successful | yes |
| 8 | 1 | T | 2000639 | CHG017 | Input | 133,549,980 | 62,465,142 | - | - | - |
| 9 | 2 | N | 2000721 | CHG081 | H3K4Me3 | 123,984,264 | 41,723,243 | 13,046 | successful | yes |
| 10 | 2 | N | 2000721 | CHG031 | H3K4Me1 | 142,898,092 | 61,716,210 | 17,896 | successful | yes |
| 11 | 2 | N | 2000721 | CHG030 | H3K27Ac | 142,881,448 | 56,328,103 | 24,624 | successful | yes |
| 12 | 2 | N | 2000721 | CHG029 | Input | 144,582,591 | 67,254,098 | - | - | - |
| 13 | 2 | T | 2000721 | CHG080 | H3K4Me3 | 128,094,707 | 52,416,345 | 12,751 | successful | yes |
| 14 | 2 | T | 2000721 | CHG026 | H3K27Ac | 132,143,844 | 52,416,345 | 45,274 | successful | yes |
| 15 | 2 | T | 2000721 | CHG027 | H3K4Me1 | 120,824,194 | 54,688,706 | 48,701 | successful | yes |
| 16 | 2 | T | 2000721 | CHG025 | Input | 150,621,523 | 65,242,401 | - | - | - |
| 17 | 3 | N | 2000986 | CHG083 | H3K4Me3 | 145,813,278 | 44,476,466 | 13,305 | successful | yes |
| 18 | 3 | N | 2000986 | CHG039 | H3K4Me1 | 112,190,461 | 52,061,916 | 14,977 | successful | yes |
| 19 | 3 | N | 2000986 | CHG038 | H3K27Ac | 136,195,033 | 47,671,991 | 26,993 | successful | yes |
| 20 | 3 | N | 2000986 | CHG037 | Input | 125,858,642 | 58,503,831 | - | - | - |
| 21 | 3 | T | 2000986 | CHG082 | H3K4Me3 | 199,735,230 | 48,070,517 | 13,296 | successful | yes |
| 22 | 3 | T | 2000986 | CHG035 | H3K4Me1 | 99,757,592 | 48,602,649 | 25,882 | successful | yes |
| 23 | 3 | T | 2000986 | CHG034 | H3K27Ac | 127,564,120 | 45,231,776 | 29,278 | successful | yes |
| 24 | 3 | T | 2000986 | CHG033 | Input | 127,392,001 | 57,846,771 | - | - | - |
| 25 | 4 | N | 980437 | CHG087 | H3K4Me3 | 252,269,976 | 16,106,111 | 6,925 | weak | yes |
| 26 | 4 | N | 980437 | CHG089 | H3K27Ac | 248,399,140 | 21,095,856 | 20,018 | weak | yes |
| 27 | 4 | N | 980437 | CHG086 | input | 223,083,607 | 13,951,728 | - | - | - |
| 28 | 4 | T | 980437 | CHG091 | H3K4Me3 | 254,777,628 | 12,340,2 57 | 7,007 | weak | yes |
| 29 | 4 | T | 980437 | CHG093 | H3K27Ac | 215,915,787 | 19,054,278 | 48,614 | weak | yes |
| 30 | 4 | T | 980437 | CHG090 | input | 214,007,053 | 18,743,433 | - | - | - |
| 31 | 5 | N | 980097 | CHG097 | H3K27Ac | 254,991,965 | 17,871,717 | 10,566 | weak | yes |
| 32 | 5 | N | 980097 | CHG094 | Input | 248,345,017 | 15,056,998 | - | - | - |
| 33 | 5 | T | 980097 | CHG101 | H3K27Ac | 254,857,885 | 16,050,861 | 81,607 | successful | yes |
| 34 | 5 | T | 980097 | CHG098 | Input | 235,148,448 | 16,412,565 | - | - | - |
| 35 | 6 | N | 990068 | CHG441 | H3K4Me3 | 25,942,766 | 18,661,944 | 9,040 | successful | yes |
| 36 | 6 | N | 990068 | CHG443 | H3K27Ac | 28,993,775 | 20,404,671 | 30,306 | successful | yes |
| 37 | 6 | N | 990068 | CHG444 | Input | 16,583,307 | 14,164,125 | - | - | - |
| 38 | 6 | T | 990068 | CHG437 | H3K4Me3 | 19,295,687 | 15,981,638 | 23,546 | successful | yes |
| 39 | 6 | T | 990068 | CHG439 | H3K27Ac | 30,394,067 | 26,279,884 | 84,958 | successful | yes |
| 40 | 6 | T | 990068 | CHG440 | Input | 54,957,058 | 46,535,339 | - | - | - |
| 41 | 7 | N | 200008 5 | CHG449 | H3K4Me3 | 22,207,074 | 17,120,624 | 13,421 | weak | yes |
| 42 | 7 | N | 2000085 | CHG451 | H3K27Ac | 31,752,518 | 26,505,029 | 93,432 | successful | yes |
| 43 | 7 | N | 2000085 | CHG452 | Input | 23,861,825 | 20,188,881 | - | - | - |
| 44 | 7 | T | 2000085 | CHG445 | H3K4Me3 | 27,386,842 | 17,898,292 | 16,274 | successful | yes |
| 45 | 7 | T | 2000085 | CHG447 | H3K27Ac | 37,833,126 | 29,893,873 | 67,464 | successful | yes |
| 46 | 7 | T | 2000085 | CHG448 | Input | 25,476,868 | 21,590,215 | - | - | - |
| 47 | 8 | N | 980401 | GCC005 | H3K4Me3 | 47,143,397 | 32,011,124 | 9,739 | weak | yes |
| 48 | 8 | N | 980401 | GCC006 | H3K4Me1 | 49,813,057 | 38,517,830 | 29,304 | successful | yes |
| 49 | 8 | N | 980401 | GCC007 | H3K27Ac | 49,333,955 | 34,378,734 | 104,483 | successful | yes |
| 50 | 8 | N | 980401 | GCC008 | Input | 48,654,609 | 39,027,473 | - | - | - |
| 51 | 8 | T | 980401 | GCC002 | H3K4Me1 | 46,014,858 | 35,781,553 | 5,374 | weak | yes |
| 52 | 8 | T | 980401 | GCC001 | H3K4Me3 | 40,037,248 | 16,724,980 | 11,773 | successful | yes |
| 53 | 8 | T | 980401 | GCC003 | H3K27Ac | 70,844,500 | 51,841,868 | 108,169 | successful | yes |
| 54 | 8 | T | 980401 | GCC004 | Input | 55,650,648 | 46,769,330 | - | - | - |
| 55 | 9 | N | 980447 | GCC013 | H3K4Me3 | 49,510,760 | 43,302,748 | 10,442 | successful | yes |
| 56 | 9 | N | 980447 | GCC014 | H3K4Me1 | 51,911,778 | 46,524,450 | 18,916 | weak | yes |
| 57 | 9 | N | 980447 | GCC015 | H3K27Ac | 43,725,655 | 38,581,698 | 147,189 | successful | yes |
| 58 | 9 | N | 980447 | GCC016 | Input | 43,722,729 | 36,570,838 | - | - | - |
| 59 | 9 | T | 980447 | GCC010 | H3K4Me1 | 51,224,701 | 40,643,956 | 7,959 | successful | yes |
| 60 | 9 | T | 980447 | GCC009 | H3K4Me3 | 41,895,137 | 28,002,598 | 9,325 | weak | yes |
| 61 | 9 | T | 980447 | GCC011 | H3K27Ac | 75,243,898 | 63,172,397 | 98,169 | successful | yes |
| 62 | 9 | T | 980447 | GCC012 | Input | 40,502,678 | 33,280,117 | - | - | - |
| 63 | 10 | N | 2001206 | GCC021 | H3K4Me3 | 42,094,067 | 35,485,202 | 12,682 | successful | yes |
| 64 | 10 | N | 2001206 | GCC022 | H3K4Me1 | 44,213,793 | 38,760,554 | 50,615 | weak | yes |
| 65 | 10 | N | 2001206 | GCC023 | H3K27Ac | 47,356,714 | 34,355,781 | 112,565 | successful | yes |
| 66 | 10 | N | 2001206 | GCC024 | Input | 58,885,884 | 49,927,340 | - | - | - |
| 67 | 10 | T | 2001206 | GCC017 | H3K4Me3 | 48,193,228 | 36,729,294 | 13,835 | successful | yes |
| 68 | 10 | T | 2001206 | GCC018 | H3K4Me1 | 43,730,845 | 35,480,758 | 44,504 | weak | yes |
| 69 | 10 | T | 2001206 | GCC019 | H3K27Ac | 52,518,766 | 42,398,517 | 111,758 | successful | yes |
| 70 | 10 | T | 2001206 | GCC020 | Input | 81,949,870 | 70,380,385 | - | - | - |
| 71 | 11 | N | 980436 | GCC029 | H3K4Me3 | 27,612,232 | 20,121,957 | 12,398 | weak | yes |
| 72 | 11 | N | 980436 | GCC030 | H3K4Me1 | 22,983,565 | 20,452,059 | 53,077 | weak | yes |
| 73 | 11 | N | 980436 | GCC031 | H3K27Ac | 23,061,305 | 15,315,483 | 104,880 | successful | yes |
| 74 | 11 | N | 980436 | GCC032 | Input | 24,411,542 | 21,182,579 | - | - | - |
| 75 | 11 | T | 980436 | GCC025 | H3K4Me3 | 31,564,679 | 24,866,375 | 8,625 | weak | yes |
| 76 | 11 | T | 980436 | GCC026 | H3K4Me1 | 51,645,661 | 38,028,800 | 58,456 | successful | yes |
| 77 | 11 | T | 980436 | GCC027 | H3K27Ac | 51,093,256 | 35,496,776 | 102,351 | successful | yes |
| 78 | 11 | T | 980436 | GCC028 | Input | 25,606,490 | 20,820,223 | - | - | - |
| 79 | 12 | N | 980417 | GCC037 | H3K4Me3 | 18,976,505 | 15,277,228 | 10,387 | successful | yes |
| 80 | 12 | N | 980417 | GCC039 | H3K27Ac | 30,443,642 | 25,447,390 | 70,910 | successful | yes |
| 81 | 12 | N | 980417 | GCC038 | H3K4Me1 | 22,127,416 | 18,537,610 | 109,119 | successful | yes |
| 82 | 12 | N | 980417 | GCC040 | Input | 33,758,416 | 28,242,473 | - | - | - |
| 83 | 12 | T | 980417 | GCC033 | H3K4Me3 | 42,615,610 | 27,972,601 | 10,260 | successful | yes |
| 84 | 12 | T | 980417 | GCC035 | H3K27Ac | 33,438,272 | 29,141,996 | 76,369 | successful | yes |
| 85 | 12 | T | 980417 | GCC034 | H3K4Me1 | 31,115,402 | 26,172,044 | 142,635 | weak | yes |
| 86 | 12 | T | 980417 | GCC036 | Input | 26,806,807 | 22,277,771 | - | - | - |
| 87 | 13 | N | 980319 | GCC075 | H3K4Me3 | 34,503,108 | 26,201,666 | 9,466 | successful | yes |
| 88 | 13 | N | 980319 | GCC076 | H3K4Me1 | 32,308,832 | 28,194,660 | 56,964 | weak | yes |
| 89 | 13 | N | 980319 | GCC077 | H3K27Ac | 28,534,828 | 24,595,902 | 73,073 | successful | yes |
| 90 | 13 | N | 980319 | GCC078 | Input | 31,533,287 | 26,147,884 | - | - | - |
| 91 | 13 | T | 980319 | GCC071 | H3K4Me3 | 31,707,599 | 22,793,5 55 | 14,049 | successful | yes |
| 92 | 13 | T | 980319 | GCC073 | H3K27Ac | 42,548,744 | 35,755,479 | 102,971 | successful | yes |
| 93 | 13 | T | 980319 | GCC072 | H3K4Me1 | 28,112,304 | 24,361,418 | 196,347 | weak | yes |
| 94 | 13 | T | 980319 | GCC074 | Input | 28,895,896 | 24,529,014 | - | - | - |
| 95 | 14 | N | 990275 | GCC088 | H3K4Me3 | 39,968,810 | 31,536,231 | 7,964 | successful | yes |
| 96 | 14 | N | 990275 | GCC089 | H3K27Ac | 52,738,627 | 22,089,449 | 70,246 | successful | yes |
| 97 | 14 | N | 990275 | GCC090 | Input | 33,342,252 | 21,049,309 | - | - | - |
| 98 | 14 | T | 990275 | GCC085 | H3K4Me3 | 26,399,904 | 14,795,436 | 25,423 | weak | yes |
| 99 | 14 | T | 990275 | GCC086 | H3K27Ac | 45,712,891 | 25,668,453 | 183,458 | successful | yes |
| 100 | 14 | T | 990275 | GCC087 | Input | 40,285,061 | 32,790,063 | - | - | - |
| 101 | 15 | N | 2000877 | GCC082 | H3K4Me3 | 52,151,546 | 22,229,998 | 11,368 | successful | yes |
| 102 | 15 | N | 2000877 | GCC083 | H3K27Ac | 45,775,899 | 41,027,897 | 61,175 | weak | yes |
| 103 | 15 | N | 2000877 | GCC084 | Input | 38,226,148 | 30,117,584 | - | - | - |
| 104 | 15 | T | 2000877 | GCC079 | H3K4Me3 | 49,368,282 | 24,022,463 | 9,837 | successful | yes |
| 105 | 15 | T | 2000877 | GCC080 | H3K27Ac | 38,621,705 | 33,990,267 | 41,048 | successful | yes |
| 106 | 15 | T | 2000877 | GCC081 | Input | 38,824,621 | 32,814,299 | - | - | - |
| 107 | 16 | N | 20020720 | GCC100 | H3K4Me3 | 58,679,413 | 34,278,884 | 9,901 | successful | yes |
| 108 | 16 | N | 20020720 | GCC101 | H3K27Ac | 43,532,496 | 37,750,9 17 | 65,167 | successful | yes |
| 109 | 16 | N | 20020720 | GCC102 | Input | 39,544,734 | 31,454,551 | - | - | - |
| 110 | 16 | T | 20020720 | GCC097 | H3K4Me3 | 57,599,648 | 16,022,427 | 12,922 | successful | yes |
| 111 | 16 | T | 20020720 | GCC098 | H3K27Ac | 35,400,105 | 29,507,542 | 74,115 | successful | yes |
| 112 | 16 | T | 20020720 | GCC099 | Input | 37,092,424 | 29,452,932 | - | - | - |
| 113 | 17 | N | 20021007 | GCC094 | H3K4Me3 | 56,788,147 | 18,217,449 | 16,073 | successful | yes |
| 114 | 17 | N | 20021007 | GCC095 | H3K27Ac | 40,488,514 | 33,372,754 | 122,851 | successful | yes |
| 115 | 17 | N | 20021007 | GCC096 | Input | 40,712,616 | 34,440,613 | - | - | - |
| 116 | 17 | T | 20021007 | GCC091 | H3K4Me3 | 33,903,211 | 27,230,052 | 7,843 | weak | yes |
| 117 | 17 | T | 20021007 | GCC092 | H3K27Ac | 50,268,912 | 19,156,361 | 98,104 | successful | yes |
| 118 | 17 | T | 20021007 | GCC093 | Input | 34,936,961 | 29,417,989 | - | - | - |
| 119 | CL1 | FU97 | FU97 | GCC043 | H3K27Ac | 30,087,131 | 22,566,178 | 21,867 | successful | yes |
| 120 | CL1 | FU97 | FU97 | GCC041 | H3K4Me3 | 26,986,288 | 23,243,556 | 26,562 | successful | yes |
| 121 | CL1 | FU97 | FU97 | GCC045 | Input | 33,566,067 | 23,430,741 | - | - | - |
| 122 | CL1 0 | RE RF-GC-1B | RERF-GC-1B | CHG374 | H3K27Ac | 39,882,8 20 | 19,500,590 | 11,201 | successful | yes |
| 123 | CL1 0 | RE RF-GC-1B | RERF-GC-1B | CHG371 | H3K4Me3 | 42,450,431 | 25,988,948 | 16,625 | successful | yes |
| 124 | CL10 | RE RF-GC-1B | RERF-GC-1B | CHG376 | Input | 21,437,700 | 16,948,709 | - | - | - |
| 125 | CL11 | SNU16 | SNU16 | CHG236 | H3K27Ac | 21,726,635 | 16,967,938 | 13,619 | successful | yes |
| 126 | CL11 | SNU16 | SNU16 | CHG233 | H3K4Me3 | 20,136,058 | 18,151,002 | 19,445 | successful | yes |
| 127 | CL11 | SNU16 | SNU16 | CHG232 | Input | 19,522,181 | 14,558,761 | - | - | - |
| 128 | CL12 | SNU1750 | SNU1750 | CHG230 | H3K27Ac | 18,716,777 | 15,805,037 | 15,074 | successful | yes |
| 129 | CL12 | SNU1750 | SNU1750 | CHG227 | H3K4Me3 | 16,655,044 | 14,883,880 | 18,130 | successful | yes |
| 130 | CL12 | SNU1750 | SNU1750 | CHG226 | Input | 19,602,424 | 13,575,272 | - | - | - |
| 131 | CL13 | YCC21 | YCC21 | CHG429 | H3K27Ac | 22,884,268 | 13,861,557 | 21,415 | successful | yes |
| 132 | CL13 | YCC21 | YCC21 | CHG427 | H3K4Me3 | 22,788,225 | 15,669,142 | 20,120 | successful | yes |
| 133 | CL13 | YCC21 | YCC21 | CHG431 | Input | 40,378,916 | 34,747,778 | - | - | - |
| 134 | CL13 | YCC22 | YCC22 | GCC063 | H3K27Ac | 33,314,935 | 23,877,905 | 11,774 | successful | yes |
| 135 | CL13 | YCC22 | YCC22 | GCC061 | H3K4Me3 | 27,410,298 | 24,163,717 | 25,417 | successful | yes |
| 136 | CL13 | YCC22 | YCC22 | GCC065 | Input | 26,685,5 96 | 18,976,555 | - | - | - |
| 137 | CL14 | YCC3 | YCC3 | GCC053 | H3K27Ac | 27,581,400 | 21,579,098 | 14,118 | successful | yes |
| 138 | CL14 | YCC3 | YCC3 | GCC051 | H3K4Me3 | 22,106,259 | 18,914,296 | 17,276 | successful | yes |
| 139 | CL14 | YCC3 | YCC3 | GCC055 | Input | 27,745,993 | 18,854,658 | - | - | - |
| 140 | CL15 | YCC7 | YCC7 | CHG424 | H3K27Ac | 38,599,550 | 22,445,268 | 32,770 | successful | yes |
| 141 | CL15 | YCC7 | YCC7 | CHG422 | H3K4Me3 | 19,594,480 | 14,546,474 | 22,521 | successful | yes |
| 142 | CL15 | YCC7 | YCC7 | CHG426 | Input | 24,527,190 | 21,748,808 | - | - | - |
| 143 | CL2 | HFE145 | HFE145 | CHG245 | H3K4Me3 | 24,122,708 | 19,760,850 | 18,492 | successful | yes |
| 144 | CL2 | HFE145 | HFE145 | CHG244 | Input | 22,447,791 | 17,960,470 | - | - | - |
| 145 | CL2 | HFE145 | HFE145 | HFE145 -EZH2-MJ-5246 | H3K4Me3 | 50,701,700 | 45,821,209 | 17,299 | weak | - |
| 146 | CL2 | HFE145 | HFE145 | HFE145 -input-MJ | Input | 36,885,332 | 36,157,452 | - | - | - |
| 147 | CL3 | Hs 1.Int | Hs 1.Int | HsInt-K4me3. merged | H3K4Me3 | 37,088,221 | 32,789,363 | 22,518 | successful | - |
| 148 | CL3 | Hs 1.Int | Hs 1.Int (replicate) | HsInt-G-K4me3. merged | H3K4Me3 | 30,617,105 | 27,713,302 | 20,298 | successful | - |
| 149 | CL3 | Hs 1.Int | Hs 1.Int | HsInt-input. merged | Input | 32,275,816 | 28,576,200 | - | - | - |
| 150 | CL4 | Hs 738.St/Int | Hs 738.St/Int | Hs738-K4me3. merged | H3K4Me3 | 37,945,394 | 33,334,651 | 150,552 | successful | - |
| 151 | CL4 | Hs 738.St/Int | Hs 738.St/Int | Hs738-K4me3. merged | Input | 32,275,816 | 24,581,922 | - | - | - |
| 152 | CL5 | IM95 | IM95 | CHG434 | H3K27Ac | 23,309,435 | 9,168,213 | 27,692 | successful | yes |
| 153 | CL5 | IM95 | IM95 | CHG432 | H3K4Me3 | 25,179,506 | 14,069,213 | 19,956 | successful | yes |
| 154 | CL5 | IM95 | IM95 | CHG436 | Input | 37,968,519 | 33,292,944 | - | - | - |
| 155 | CL6 | KATO3 | KATO3 | CHG242 | H3K27Ac | 24,559,532 | 17,356,721 | 28,730 | successful | yes |
| 156 | CL6 | KATO3 | KATO3 | CHG238 | Input | 20,527,352 | 14,593,025 | - | - | - |
| 157 | CL7 | MKN7 | MKN7 | CHG419 | H3K27Ac | 35,301,333 | 30,804,178 | 24,268 | successful | yes |
| 158 | CL7 | MKN7 | MKN7 | CHG417 | H3K4Me3 | 28,119,400 | 24,793,006 | 23,766 | successful | yes |
| 159 | CL7 | MKN7 | MKN7 | CHG421 | Input | 35,839,896 | 31,791,610 | - | - | - |
| 160 | CL8 | NCC59 | NCC59 | CHG218 | H3K27Ac | 22,973,156 | 19,828,610 | 14,937 | successful | yes |
| 161 | CL8 | NCC59 | NCC59 | CHG215 | H3K4Me3 | 15,642,441 | 13,907,147 | 12,410 | successful | yes |
| 162 | CL8 | NCC59 | NCC59 | CHG214 | Input | 17,926,188 | 13,139,789 | - | - | - |
| 163 | CL9 | OCUM1 | OCUM | CHG212 | H3K27Ac | 24,573,737 | 20,570,185 | 17,284 | successful | yes |
| 164 | CL9 | OCUM | OCUM | CHG209 | H3K4Me3 | 19,557,872 | 17,178,274 | 15,445 | successful | yes |
| 165 | CL9 | OCUM | OCUM | CHG208 | Input | 20,585,679 | 16,680,529 | - | - | - |

### Promoter Analysis

Promoter (H3K4Me3 hi/H3K4Me1 lo) regions were identified by calculating the H3K4Me3:H3K4Me1 ratio for all H3K4Me3 regions merged across normal and GC samples. We estimated the required sample size to achieve 80% power and 10% type I error (http://powerandsamplesize.com/) based on the average signals of top 100 differential promoters between tumor and normal samples. This result yielded a recommended sample size of 11 (average), which is met in our study (16 N/T). Regions with H3K4Me3:H3K4Me1 ratios <1 in both normal and GC samples were excluded from further analysis. For all analyses performed in this study, promoter regions were defined as genomic locations exhibiting H3K4me3 hi/me1 low signals, and for all subsequent analyses, it was only within this pre-defined H3K4me3 hi/me1 low subset that H3K4me3 signals were compared. H3K27ac data was used for correlative analysis. H3K4me3 data (fastqs) for colon carcinoma lines was downloaded from public databases - Hct116 and Caco2 from ENCODE and V503 and V400 from GSE36204. To compare promoter signals between GC and normal samples, we used the DESeq2 and edgeR bioconductor packages using a read count matrix of chipseq signals, adjusting for replicate information. Regions with fold changes greater than 1.5 (FDR 0.1) were selected as significantly different. The criteria of FC 1.5 and q<0.1 was based on previous literature comparing ChIP-seq profiles using DESeq2 and edgeR also using similar thresholds. Significantly altered promoters identified by DESeq2 overlapped almost completely with altered promoters found by edgeR. A regularized log transformation of the DESeq2 read counts was used to plot PCAs and heatmaps.

### Transcriptome Analysis

RNA-seq data was obtained from the European Genome-phenome Archive under Accession No: EGAS00001001128. Data was processed by first aligning to GENCODE v19 transcript annotations using TopHat v2.0.12. Cufflinks 2.2.0 was used to generate FPKM abundance measures. For identification of novel transcripts, Cufflinks was used without employing a reference transcript annotation. Transcripts were then merged across all GC and normal samples and compared against GENCODE annotations to identify novel transcripts using Cuffmerge 2.2.0. Deep-depth strand-specific RNA sequencing was also performed on 10 additional primary samples. Total RNA was extracted using the Qiagen RNeasy Mini kit, and RNA-seq libraries were constructed according to manufacturer's instructions using Illumina Stranded Total RNA Sample Prep Kit v2 (Illumina, San Diego, California, USA) Ribo-Zero Gold option (Epicentre, Madison, Wisconsin, USA), and 1ug total RNA. Sequencing was performed using the paired-end 101bp read option. TCGA datasets were downloaded from TCGA Data Portal (https://tcga-data.nci.nih.gov/tcga) in form of fastq files which were then aligned to GENCODE v19 transcript annotations using TopHat v2.0.12. To analyze promoter-associated RNA expression, RNA-seq reads from TCGA samples (tumors and normals) were mapped against the genomic locations of promoter regions originally defined by epigenomic profiling in the discovery samples, including all promoters, gained somatic promoters, and lost somatic promoters (see Fig. 1 in Main Text). RNA-seq reads mapping to these epigenome-defined promoter regions were then quantified, normalized by promoter length (kilobases) and by total library size, and fold changes in expression were computed between tumor and normal TCGA sample groups. Length of promoter loci was defined as the number of base pairs (bps) between the start and stop genomic coordinate of the H3K4me3 region as identified by the peak caller program CCAT v3.0.(190) Isoform level quantification for alternative promoter driven transcripts was performed using cufflinks (FPKM), Kallisto (TPM) and MISO (isoform centric analysis). Assigned counts for each isoform were normalized by DESeq2.

### DNA Methylation Analysis

Genomic DNA of gastric tumors and matched normal gastric tissues was extracted (QIAGEN) and processed for DNA methylation profiling using Illumina HumanMethylation450 BeadChips (HM450). Methylation β-values were calculated and background corrected using the *methylumi* R BioConductor package. Normalization was performed using the BMIQ method (wateRmelon package in R). CpG island locations were downloaded from the UCSC genome browser. Overlaps of at least 1 bp between promoter loci and CpG islands were identified using BEDTools intersect. For each group (all promoters, gained somatic promoters and lost somatic promoters), we identified probes overlapping the predicted promoter regions and calculated average beta value differences. A two-sample Wilcoxon test was performed.

### Survival Analysis

Kaplan-Meier survival analysis was used with overall survival as the outcome metric. Log-rank tests were used to assess the significance of the Kaplan-Meier analysis.

### Gene set enrichment Analysis

Gene set enrichment analysis was performed using MsigDB by computing the overlap of genes associated with somatic promoters against the C2 set of curated genes.

### Mass Spectrometry and Data Analysis

Peptide level mass spectrometry data for 90 colon and rectal cancer (CRC) samples and 60 normal colon epithelium samples were downloaded from the CPTAC portal generated by the Clinical Proteomic Tumor Analysis Consortium (NCI/NIH). (https://cptac-data-portal.georgetown.edu/cptac). Spectral counts were extracted using IDPicker's idQuery tool. Differentially expressed peptides were identified by fitting a linear model (limma R) on quantile normalized and log₂ transformed spectral counts. For GC cell line mass spectrometry, AGS, GES-1, SNU1750 and MKN1 cells were extracted with RIPA buffer supplemented with protease inhibitor. 150 µg protein extract of each biological quadruplicate (i.e. 4 replicates per cell line) were separated on a 12% NuPAGE Novel Bis-Tris precast gel (Thermo Scientific). For in-gel digestion, samples were separated into two fractions and reduced in 10 mM DTT for 1h at 56°C followed by alkylation with 55mM iodoacetamide (Sigma) for 45 min in the dark. Tryptic digests were performed in 50 mM ammonium bicarbonate buffer with 2 µg trypsin (Promega) at 37°C overnight. Peptides were desalted on StageTips and analysed by nanoflow liquid chromatography on an EASY-nLC 1200 system coupled to a Q Exactive HF mass spectrometer (Thermo Fisher Scientific). Peptides were separated on a C18-reversed phase column (25 cm long, 75 µm inner diameter) packed in-house with ReproSil-Pur C18-QAQ 1.9 µm resin (Dr Maisch). The column was mounted on an Easy Flex Nano Source and temperature controlled by a column oven (Sonation) at 40°C. A 225-min gradient from 2 to 40% acetonitrile in 0.5% formic acid at a flow of 225 nl/min was used. Spray voltage was set to 2.4 kV. The Q Exactive HF was operated with a TOP20 MS/MS spectra acquisition method per MS full scan. MS scans were conducted with 60,000 and MS/MS scans with 15,000 resolution. For data analysis, raw files were processed with MaxQuant version 1.5.2.8 against the UNIPROT annotated human protein database. Carbamidomethylation was set as a fixed modification while methionine oxidation and protein N-acetylation were considered as variable modifications. Search results were processed with MaxQuant filtered with a false discovery rate of 0.01. The match between run option and LFQ quantitation were activated. LFQ intensities were filtered for potential contaminants, reverse proteins and log₂ transformed. They were then imputed using open source software Perseus (0.5 width, 1.8 downshift) and fitted using linear models (limma R).

### 5' RACE and Gene Cloning

5' Rapid amplification of cDNA ends (5' RACE) was performed using the 5' RACE System for Rapid Amplification of cDNA Ends, Version 2(Invitrogen, 18374-058). Briefly, 2 µg of total RNA was used for each reverse transcription reaction with SuperScript™ II reverse transcriptase and gene-specific primer 1 for each gene. After cDNA synthesis, RNase mix (RNase H and RNase T1) was used to degrade the RNA. First strand cDNAs were then purified with S.N.A.P. columns, and tailed with dCTP and TdT. dC-tailed cDNAs were amplified using the abridged anchor primer and nested gene-specific primer 2 by Go Taq®Hot Start Polymerase (Promega, M5001). Subsequently, primary PCR products were reamplified with the abridged universal amplification primer (AUAP), and gene-specific primer 3. Gel electrophoresis was performed. PCR bands of interest were excised and purified for cloning with the TA Cloning Kit (Invitrogen, K2020). A minimum of 12 independent colonies were isolated, and purified plasmid DNA was sequenced bidirectionally on an ABI 3730 DNA analyzer (Applied Biosystems) (Table 2). Constructs for *MET* transcripts were generated by PCR amplification of full-length cDNAs encoding wild type and variant *MET* from KATOIII cells. Wild type and variant *RASA3* full-length transcripts were PCR amplified from NCC59 cells. cDNA fragments were cloned into the pCI-Puro-HA vector (modified from Promega's pCI-Neo vector, a gift from Wanjin Hong, Institute of Molecular and Cell Biology, Singapore). Plasmids were transiently transfected into cell lines using Lipofectamine 3000 (Thermo Scientific).

**Table 2: RACE Primers**

| **Gene** | **Gene specific primer 1** | **Gene specific primer 2** | **Gene specific primer 3** |
|---|---|---|---|
| **RASA3** | 5’GGAGTAGATACGC TCCGT3’ (SEQ ID NO: 1837) | 5'CACAGCCAGTGGC CGCTCAGGTA3' (SEQ ID NO: 1838) | 5'CTTCTCCACTGCCAGGATGTT3' (SEQ ID NO: 1839) |
| **MET** | 5'TAGGAGAATGTAC TGTAT 3' (SEQ ID NO: 1840) | 5'GGAGACACTGGAT GGGAGTC 3' (SEQ ID NO: 1841) | 5'CGAGAAACCACAACCTGCAT3' (SEQ ID NO: 1842) |

### Western Blotting

3x10⁵ HEK293 cells were seeded and transfected using Lipofectamine 3000 (Thermo Scientific). Cells were serum starved for 16 hours before addition of human HGF (R&D systems, 100 ng/ml) for 0, 15 and 30 minutes, and immediately harvested with cold Triton-XlOO Lysis Buffer (50 mM Tris pH 8.0, 150 mM NaCl, 1% Triton X-100) with protease and phosphatase inhibitors (Roche) on ice. Protein concentration was measured by Pierce BCA protein assay (Thermo Scientific). Cell lysates were heated at 95 oC for 10 min in SDS sample buffer and 20 µg of each cell lysate was loaded per well. Proteins were transferred to nitrocellulose membranes. Western blotting was performed by incubating membranes 4 hrs at room temperature with the following antibodies: Met & β-actin (Santa Cruz), p-MET (Y1234/1235 & Y1349), pSTAT3 (S727 & Y705), STAT3, ERK, p-ERK, Gab1, pGab1 (Y627) (Cell Signaling). Membranes were incubated in secondary antibodies at 1:3,000 for 1 hr at room temperature and developed with SuperSignal West Femto Maximum Sensitivity substrate (Thermo Scientific) using ChemiDocTM MP Imaging System (BIO-RAD). Western blot bands were quantified using Image Lab software (BIO-RAD). Experiments were repeated in triplicate.

### Cell Proliferation Assays

3x10³ GES1, SNU1967 and AGS cells were plated into 96-well plates in media with 10% fetal bovine serum and left overnight to attach. The next day (Day 0), cells were transiently transfected with wild-type and variant *RASA3* constructs using Lipofectamine 3000 (Thermo Scientific). The amount of the constructs was 40ng/well for AGS and 100ng/well for GES1 and SNU1967 cells. Cell proliferation was measured by the WST-8 assay (Cell Counting Kit-8, Dojindo) from 24 to 120 hours post-transfection. 10uL of WST-8 solution was added per well and the absorbance reading was measured at 450 nm after 2 hours of incubation in a humidified incubator.

### Transfection with RASA3 siRNAs

Two *RASA3* siRNAs were used to silence the *RASA3* SomT transcript in NCC24 cells (hs.Ri.RASA3.13.1 TriFECTa® Kit DsiRNA Duplex (Integrated DNA Technologies), and Silencer® Select Pre-Designed siRNA s355 (Life Technologies)). NCC24 cells were transfected either with the above two siRNAs or a non-targeting control (ON-TARGETplus Non-targeting pool, Dharmacon) at a final concentration of 100nM for 48 hours, subsequently followed by qPCR and western validation and migration/invasion assays.

### Migration and Invasion Assays

To determine cell migratory capacities, *RASA3* wild type and variant transfected AGS and GES1, SNU1967 and AGS, and siRNA treated NCC24 cells were tested using Corning Costar 6.5mm Transwell with 8.0µm Pore Polycarbonate Membrane Inserts (3422, Corning, NY, USA). 2.5 X10⁴ AGS cells and 2 X10⁴ GES1 cells, 3x10⁴ SNU1967 cells and 5x10⁴ NCC24 cells were suspended in 0.1 ml serum-free RPMI medium and added to the top of the Transwell insert. 0.6ml RPMI containing 10% FBS was added into the bottom well as a chemoattractant. After incubation for 24 h at 37°C in a 5% CO2 incubator, cells were fixed with 3.7% formaldehyde and permeabilized with 100% methanol. Non-migrated cells were scraped off with cotton swabs from the upper surface of the membrane. Migrated cells were stained with 0.5% crystal violet. The number of migrated cells were represented as the total area of migrated cells vs the area of transwell membrane calculated using ImageJ software. For cell invasion assays, the above Transwell inserts were coated with 0.1ml (300 µg/mL) Corning Matrigel matrix (354234, Corning, NY, USA) for 2 to 4h at 37°C before use. All subsequent steps were identical to the migration assay protocol.

### Measurement of RASA3 mRNA levels

Total RNA was extracted from three independent experiments using the Qiagen RNAeasy mini kit according to manufacturer's instructions. RNA was reverse transcribed using Improm-IITM Reverse Transcriptase (Promega). Real time PCR was performed in triplicate using Quantifast SYBR Green PCR kit (Qiagen) on an Applied Biosystems HT7900 Real Time PCR System. Fold change was calculated using the Delta Ct method and normalised to β-actin. Primer sequences are as follows. β-actin: F - 5' TCCCTGGAGAAGAGCTACG 3' (SEQ ID NO: 1843), R- 5' GTAGTTTCGTGGATGCCACA 3' (SEQ ID NO: 1844); *RASA3* SomT: F - 5' TTGTGAGTGGTTCAGCGGTA 3' (SEQ ID NO: 1845), R - 5' TCAAGCGAAACCATCTCTTCT 3' (SEQ ID NO: 1846).

### RAS-GTP Assay

GES1 cells were transfected with either RASA3 CanT, RASA3 SomT or empty vector for 48 hours. Cells were harvested for protein in FBS containing media or subjected to over-night serum starvation followed by serum stimulation for 30 minutes prior to harvest. Proteins were extracted using ice-cold lysis buffer (Active RAS Pull-down and Detection Kit) containing protease inhibitor cocktail (Nacalai Tesque). Active RAS fraction was obtained using the Active RAS Pull-down and Detection Kit (Thermo Fisher Scientific) according to manufacturer's instructions. Total RAS was measured in corresponding whole cell protein lysates. B-actin was used as a loading control. Protein concentrations were determined using the Pierce BCA protein assay (Thermo Scientific). SDS sample buffer was added to the lysates and boiled at 100°C for 5 minutes. Samples were loaded in each well of a 4-15% Mini-Protean TGX gel (Biorad) and transferred to a PVDF membrane using a semi-dry blotting system (Biorad). Membranes were probed with anti-RAS (1 in 200 dilution, supplied in Active RAS Pull-down and Detection Kit), or B-actin (1 in 5000 dilution, Sigma A5316) in 5% milk-PBST at 4°C over-night. Secondary anti-mouse antibody (LNA931, Amersham) was used at a dilution of 1 in 2000 for 1 hour at room temperature. Membranes were developed using Amersham ECL Prime Western Blotting Detection Reagent and imaged using a Chemidoc Imaging system (Biorad).

### Altered Peptide and Antigen Prediction

Altered peptides were defined as variant N-terminal protein sequences arising from somatic alterations in alternative promoter usage. The following filters were applied to select the pool of altered peptides - i) Fold change of at least 1.5 for alternate vs. canonical RNA-seq expression ii) Only one canonical and one alternate isoform per gene loci iii) Annotated transcripts are confirmed as protein coding by Gencode. Canonical promoters were defined as regions exhibiting unaltered H3K4me3 peaks. Random peptides from the human proteome were generated from amino acid sequences of Gencode coding transcripts. N-terminal peptide gains were identified as cases where the alternative transcript was associated with a different 5' region predicted to result in a different translated protein sequence compared to the canonical transcript. For each N terminal altered protein, we evaluated binding of 9-mer peptides using the NetMHCpan 2.8 using a strict threshold of IC<=50nm to identify strong MHC binders. N-terminal gained peptides were mapped against protein assembly data of the same gene to evaluate protein expression. Antigen predictions were performed against HLA types of 13 GC samples predicted using OptiType. OptiType was run using default parameters except BWA mem was used as an aligner for pre-filtering reads aligning to the Optitype provided reference sequences. 3 samples with poor coverage and unpaired reads with mismatches were omitted from analysis. Eleven HLA-A, HLA-B, and HLA-C allelic variants of increased prevalence in the South East Asian population (HLA-A*02:07/HLA-A*11:01/HLA-A*24:02/HLA-A*33:03/HLA-A*24:07, HLA-B*13:01/HLA-B*40:01/HLA-B*46:01, HLA-C*03:04/HLA-C*07:02/HLA-C*08:01) were obtained from the Allele Frequency Net Database (http://www.allelefrequencies.net).

### Association of cytolytic markers with alternative promoter usage

Local immune cytolytic activity was evaluated using the expression of Granzyme A (*GZMA*) and Perforin (*PRF1*). Tumor content was estimated using two algorithms - ASCAT(79) (aberrant cell fraction) and ESTIMATE (tumor purity). Expression data for the SG series was downloaded (GSE15460) and normalized using the robust multi-array average algorithm in the 'affy' R package and log₂ transformed. Affymetrix SNP Array 6.0 data for the SG series was downloaded from GSE31168 and GSE85466. Mutation frequencies for TCGA STAD samples were downloaded from the TCGA STAD publication data (https://tcga-data.nci.nih.gov/docs/publications/stad_2014/) using level 2 curated MAF files (QCv5_blacklist_Pass.aggregated.capture.tcga.uuid.curated.somatic.maf) filtered for "Missense" variant classification. Expression data for TCGA STAD samples (TPM) was computed using the kallisto algorithm. Raw SNP Array 6.0.CEL files for TCGA gastric cancers (STAD) were downloaded from the GDC data portal (https://gdc-portal.nci.nih.gov/). Access to this dataset was obtained using dbGaP credentials and an ID issued by eRA commons. Precomputed ESTIMATE scores for TCGA STAD were downloaded from http://bioinformatics.mdanderson.org/estimate/ and converted to tumor purity using the formula cos (0.6049872018+0.0001467884 × ESTIMATE score). Preprocessed expression data for the ACRG series was downloaded from GSE62254, and pre-computed ASCAT scores obtained from collaborators (JL). Expression of cytolytic markers was adjusted for missense mutation and tumor purity frequencies using a spline regression model.

### Peptides and Cells for Cytokine Assays

A set of peptides for 15 representative alternative promoters was purchased from GenScript (GenScript). Peptide sequences and composition of peptide pools for each alternative promoter are described in Table 3. Control peptide pools for human Actin were purchased from JPT (PM-ACTS, PepMix™ Human (Actin) JPT). Peripheral blood mononuclear cells (PBMCs) were obtained from 9 healthy volunteers of whom 8 PBMC samples were HLA-typed (Table 3).

**Table 3: HLA types of healthy PBMC donors**

| **Sample** | **HLA-A** | | **HLA-B** | | **HLA-C** | |
|---|---|---|---|---|---|---|
| Donor 1 | A*11:01 | A*24:02 | B*15:01 | B*51:01 | C*04:01 | C*14:02 |
| Donor 2 | A*11:01 | A*33:03 | B*40:01 | B*58:01 | C*03:02 | C*07:02 |
| Donor 3 | A*03:01 | A*33:03 | B*35:03 | B*38:01 | C*12:03 | C*12:03 |
| Donor 4 | A*02:07 | A*24:07 | B*15:02 | B*46:01 | C*01:02 | C*08:01 |
| Donor 5 | A*02:03 | A*11:01 | B*15:02 | B*51:01 | C*08:01 | C*14:02 |
| Donor 6 | A*02:01 | A*68:01 | B*15:13 | B*40:06 | C*08:01 | C*15:02 |
| Donor 7 | A*02:07 | A*33:03 | B*27:04 | B*58:01 | C*03:02 | C*12:02 |
| Donor 8 | A*02:03 | A*11:01 | B*38:02 | B*46:01 | C*01:02 | C*07:02 |
| Donor 9 | Not determined | | | | | |

### EpiMAX Assay

PBMCs were labelled with 1µM CFSE (Life Technologies, Thermo Fisher Scientific) and cultured at a density of 200,000 cells per well in complete culture medium (cRPMI comprising RPMI 1640 medium (Gibco, Thermo Fisher Scientific), 15mM HEPES (Gibco), 1% non-essential amino acid (Gibco), 1mM sodium pyruvate (Gibco), 1% penicillin/streptomycin (Gibco), 2mM L-glutamine (Gibco), 50µM β2-mercaptoethanol (Sigma, Merck), and 10% heat-inactivated FCS (Hyclone)) for 5 days. Individual peptide pools of each alternative promoter were added at the start of the culture at a concentration of 1µg/ml for each peptide. At the end of day 5, cells were stained with LIVE/DEAD® fixable near-IR dead cell stain kit (Life Technologies), and labelled with CD4-BUV737 (BD), CD8-PacificBlue (BD), CD3-PE (BioLegend), CD19-PE/TexasRed (Beckman), and CD56-APC (BD). Analysis of T cell proliferation by CFSE dilution was performed by flow cytometry using a LSRII (BD). In addition, magnetic bead-based cytokine multiplex analysis (human cytokine panel 1, Millipore, Merck) was performed on cell culture supernatants to measure secreted cytokine levels.

### IFN-γ Assay

To test the immunogenicity of the RASA3 WT and Variant protein sequences, CD14⁺ monocytes were isolated from a HLA-A*02:06 donor by positive selection using magnetic beads (Miltenyi, Germany). Dendritic cells were generated by GM-CSF (1000 IU/ml) and IL-4 (400 IU/ml), and further matured by TNF (10 ng/ml), IL-1b (10 ng/ml), IL-6 (10 ng/ml) (Miltenyi, Germany) and PGE2 (1 (µg/ml) (Stemcell Technologies, Canada) for 24 hours. The DCs were then primed with AGS cell lysates expressing WT *RASA3* or Variant *RASA3* for 24 hours, before being co-cultured with T cells from the same donor at the ratio of 1:5. After 5 days of co-culture with DC, T cells were isolated by positive selection using CD3 magnetic beads (Miltenyi, Germany) and co-cultured with AGS cells expressing either WT or Variant *RASA3* at the ratio of 20:1 for two days. Supernatants were harvested and IFN-γ release was measured by ELISA (R&D, USA).

### NanoString Analysis

Nanostring nCounter Reporter CodeSets were designed for 95 genes (83 upregulated in GC and 11 downregulated) and 5 housekeeping genes (*AGPAT1, CLTC, B2M, POL2RL* and *TBP* covering a broad expression range) on the SG series samples. For each gene, we designed 3 probes, targeting a) the 5' end of the alternate promoter location, b) the 5' end of the canonical promoter (defined by promoter regions of equal enrichment in both GC and normal samples OR the longest protein coding transcript) and c) a common downstream probe. Vendor-provided nCounter software (nSolver) was used for data analysis. Raw counts were normalized using the geometric mean of the internal positive control probes included in each CodeSet.

A separate NanoString assay was designed for 88 genes on the ACRG cohort. For each gene, we designed 3 probes, targeting a) the 5' end of the alternate promoter location, b) the 5' end of the canonical promoter (defined by promoter regions of equal enrichment in both GC and normal samples OR the longest protein coding transcript).

### Repeat Enrichment Analysis

Repetitive element families over-represented at regions exhibiting somatic promoter alterations were identified using RepeatMasker annotations from the UCSC Table Browser (GRCh37/hg19). "Unknown", "Simple_Repeat" and "Satellite" annotations were filtered from the repeat set. Repetitive elements were included only if they overlapped a promoter by a minimum of 50%. Enrichment of repetitive element families was assessed using a binomial test with Benjamini-Hochberg FDR correction and all promoter regions were used as the background.

### Functional Prediction Analysis

Genome wide and tissue specific functional scores were downloaded from GenoCanyon (http://genocanyon.med.yale.edu/GenoCanyon Downloads.html, Version 1.0.3) and GenoSkyline (http://genocanyon.med.yale.edu/GenoSkyline) respectively. Overlaps were calculated using bedtools IntersectBed and functional scores over each unannotated somatic promoter were computed.

### Transcription Factor Enrichment

Transcription factor binding sites for 237 TFs were obtained from the ReMap database, a public database of ENCODE and other public Chip-seq TFBS data sets. Overlaps were calculated and counted against the somatic promoter set. Relative enrichment scores were calculated as ratio of (#bases in state and overlap feature)/ (#bases in genome) and [(#bases overlap feature)/ (#bases in genome) X (#bases in state)/ (#bases in genome)].

### EZH2 Inhibition

IM95 were treated with GSK126 (Selleck, USA), a selective EZH2 inhibitor, at a concentration of 5uM. Cell proliferation was monitored in 96-well plates post-treatment with GSK126 using the CellTiter-Glo® Luminescent Cell Viability Assay (Promega) for three independent experiments. For RNA-seq analysis, total RNA was extracted using the Qiagen RNAeasy mini kit according to manufacturer's instructions. Cells were treated with GSK126 (Selleck, USA; dissolved in DMSO) at a concentration of 5uM. Control cells were treated with the same concentration of DMSO (0.1%). RNAseq differential analysis for promoter loci was carried out using edgeR on read counts mapping to H3K4me3 regions estimated using featureCounts. RNAseq gene level differential analysis was performed using cuffdiff2.2.1.

### Additional Information

**Accession codes:** Genomic data for this study has been deposited in the National Center for Biotechnology GEO database, under accession numbers GSE51776 and GSE75898.(https://www.ncbi.nlm.nih.gov/geo/query/acc.cgi?token=kfoxqeamzretpal&acc =GSE75898)

### Results

### Identifying Epigenomic Promoter Alterations in GC

Using NanoChIP-seq, we profiled three histone modification marks (H3K4me3, H3K27ac and H3K4me1) across 17 GCs, matched normal gastric mucosae (34 samples) and 13 GC cell lines, generating 110 epigenomic profiles (Tables 1 and 4 provide clinical and sequencing metrics) (Fig. 1a). Quality control of the Nano-ChIPseq data was performed using two independent methods: ChIP-enrichment at known promoters, and employing the ChIP-seq quality control and validation tool CHANCE (CHip-seq ANalytics and Confidence Estimation). Comparisons of Nano-ChIPseq read densities at 1,000 promoters associated with highly expressed protein-coding genes confirmed successful enrichment in all H3K27ac and H3K4me3 libraries. CHANCE analysis also revealed that the large majority (81%) of samples exhibited successful enrichment (Table 1). We have previously also shown that Nano-ChIP signals exhibit a good concordance with orthogonal ChIP-qPCR results.

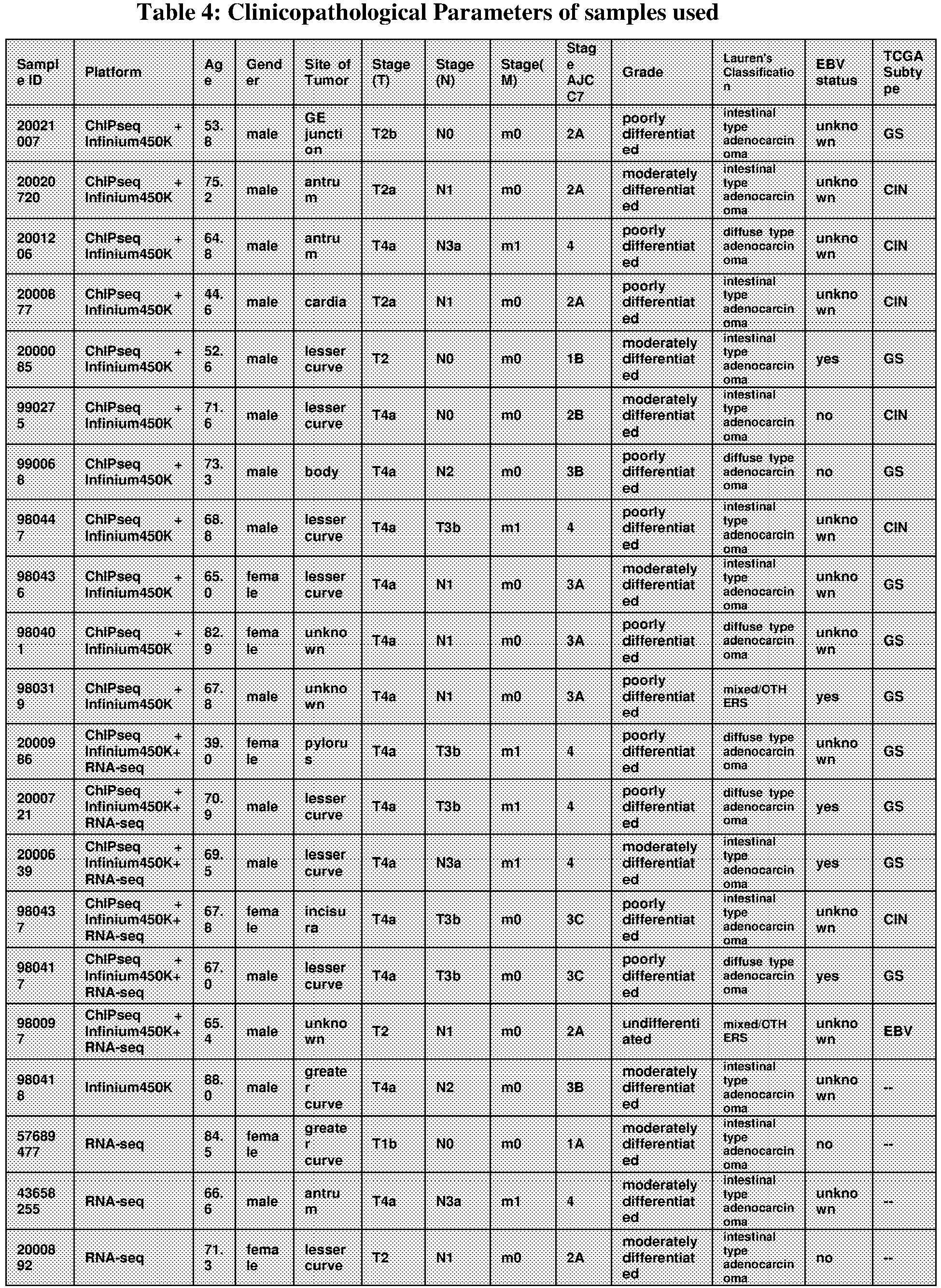

To enable accurate promoter identification, we integrated data from multiple histone modifications, selecting H3K4me3 regions simultaneously co-depleted for H3K4me1⁴² ("H3K4me3 hi/H3K4me1 lo regions"; Figure 7, Methods). Comparisons against data from external sources, including GENCODE reference transcripts, ENCODE chromatin-state models, and CAGE (CAP analysis gene expression) databases, validated the vast majority of H3K4me3 hi/H3K4me1 lo regions as true promoter elements (see section titled "Validation of H3K4me3 hi/H3K4me1 lo regions as true promoters" and Figure 7). Because primary gastric tissues comprise several different tissue types, including epithelial cells, immune cells, and stroma, we further confirmed that our promoter profiles were reflective of *bona-fide* gastric epithelia by comparisons against Epigenome Roadmap data for gastric and non-gastric tissues. Gastric tumor and matched normal promoter profiles exhibited the highest correlations to Roadmap gastric mucosae, and were distinct from other gastrointestinal tissues (small intestine, colon mucosa, colon sigmoid), stomach-associated muscle, skin, and blood (CD14) (Figure 8). Primary tissue promoter profiles also showed a significant overlap with promoter profiles of GC cell lines (87%), which are purely epithelial in origin, compared to gastrointestinal fibroblast lines (58-69%), and colon carcinoma lines (59-74%) (Figure 8).

In total, we mapped ∼23,000 promoter elements in the Nano-ChIPseq cohort. Visual exploration of these promoter elements identified three main promoter categories - unaltered promoters, promoters gained in tumors (gained somatic or tumor-specific promoters), and promoters present in normal gastric tissues but lost or decreased in GC (lost somatic or normal-specific promoters) (Fig. 1a-c). Representative examples of unaltered promoters included *RhoA* (Fig. 1a), while *CEACAM6,* an intracellular adhesion gene, exhibited somatic promoter gain at the *CEACAM6* transcription start site (TSS) in tumor samples and cell lines (Fig. 1b). Conversely, *ATP4A,* a parietal cell-associated H+/K+ ATPase with decreased expression in GC⁴³, exhibited somatic promoter loss (Fig. 1c). Both *CEACAM6* and *ATP4A* promoter alterations were correlated with increased and decreased *CEACAM6* and *ATP4A* gene expression in the same samples respectively (Fig. 1b and 1c).

Previous studies have established distinct molecular subtypes of GC. Due to limited sample sizes however, we elected in the current stay to identify promoter alterations ("somatic promoters") present in multiple GC tissues relative to control tissues irrespective of subtype. Focusing on recurrent alterations also has the benefit of reducing potential artefacts due to "private" epigenomic variation or individual sample-specific technical errors. Using two complementary read-count based algorithms commonly used for analysis of ChIP-seq data, we identified ∼2000 highly recurrent somatic promoters, of which 75% were gained in GCs (FC 1.5, q<0.1). Two-dimensional heat-map clustering and principal components analysis (PCA) plots based on somatic promoters confirmed a separation of GCs from normal samples based on promoter alterations (Fig. 1d and Figure 9). Somatic promoter H3K4me3 levels were also highly correlated with H3K27ac signals (r=0.91, P<0.001, Fig. 1e), commonly regarded as a marker of active regulatory activity. This correlation was observed across all somatic promoters (r=0.84, P<0.001, Fig. 1E), and also when gained somatic and lost somatic promoters were analyzed separately (r=0.78, P<0.001 for gained somatic; r=0.82, P<0.001 for lost somatic, Figure 9). Pathway analysis revealed that both gained somatic and lost somatic promoters were significantly associated with expression genesets previously reported to be up and downregulated in GC respectively (Fig. If). These included upregulated oncogenes (*MET, ABL2*), cell adhesion genes (*CEACAM6*) and claudin family members (*CLDN7, CLDN3*). 15-18% of somatic promoters mapped to non-coding RNAs (ncRNAs), including *HOTAIR* and *PVT1,* previously associated with GC (Table 5). Additional analyses at increasing thresholds of stringency (FC from 1.5-2 and FDR from 0.1-0.001) yielded similar results, supporting the robustness of this analysis (Figure 9). These results demonstrate that normal gastric epithelia and GCs can be distinguished on the basis of epigenomic promoter profiles.

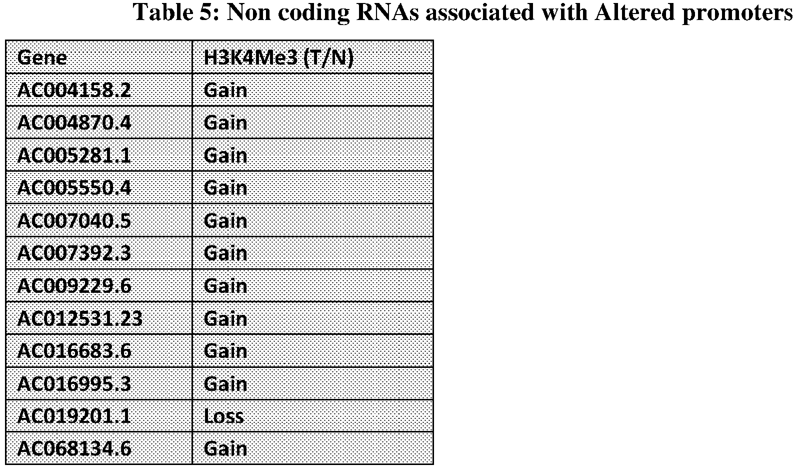

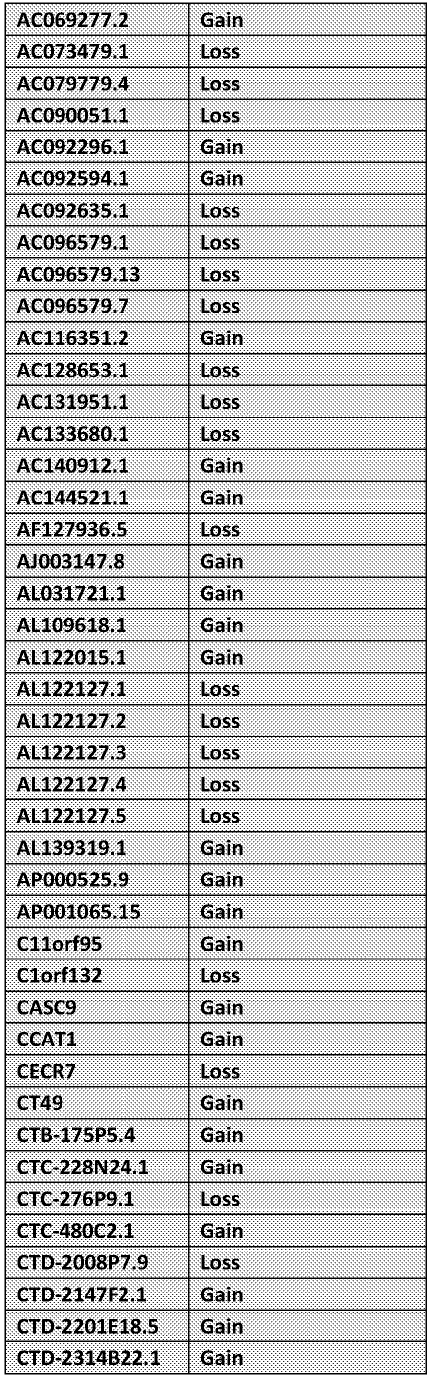

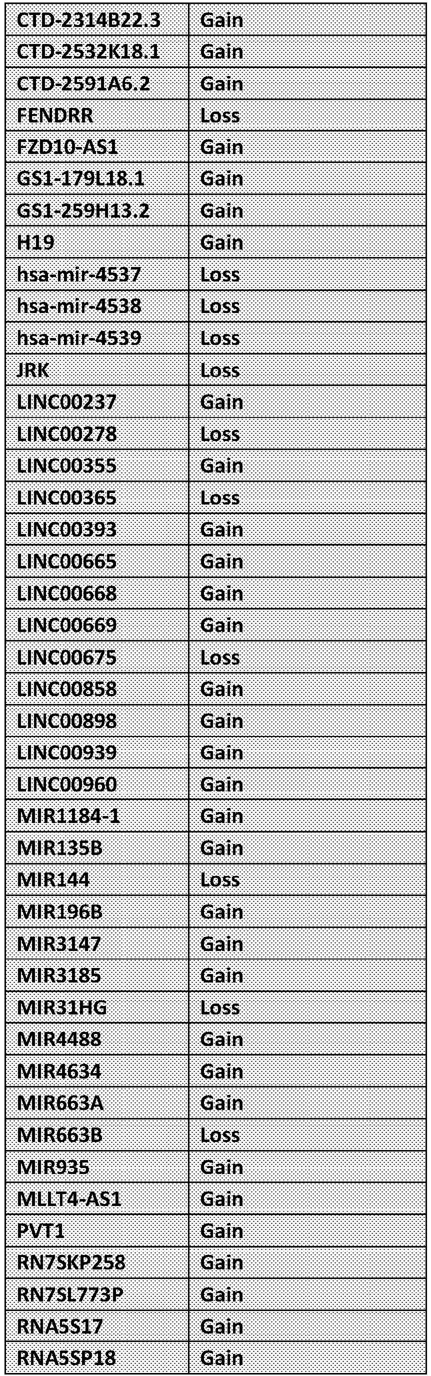

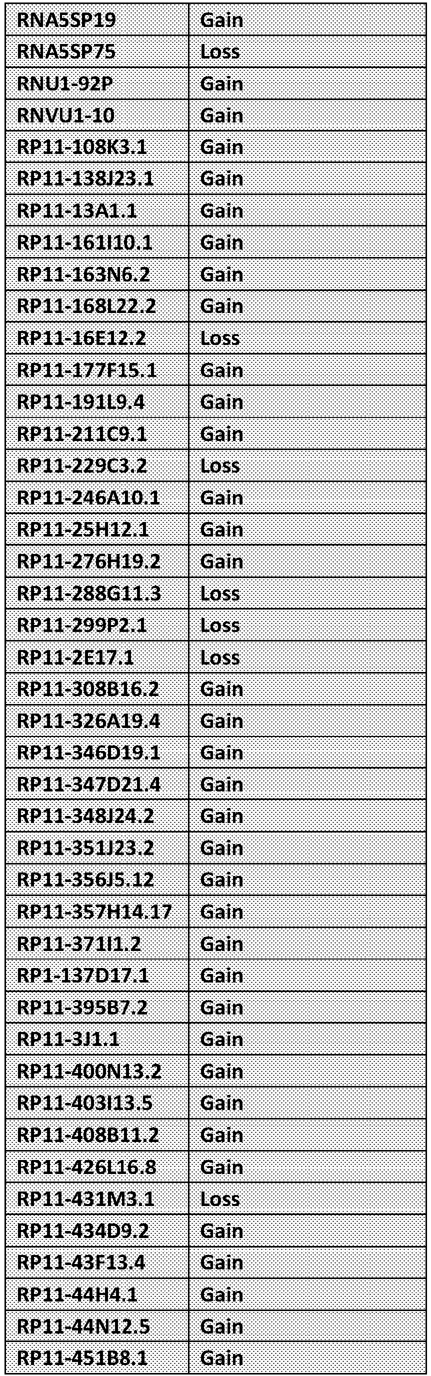

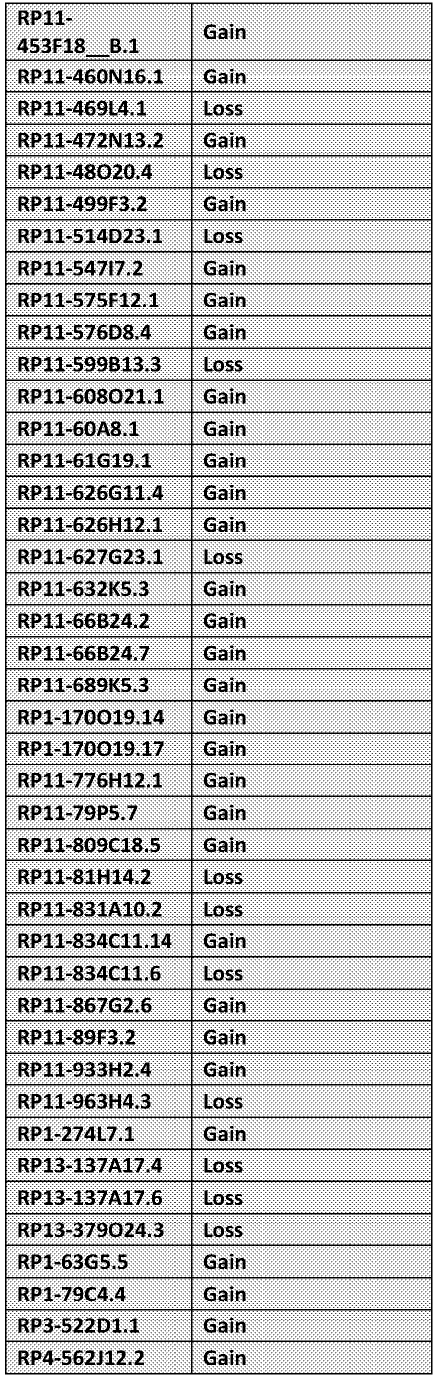

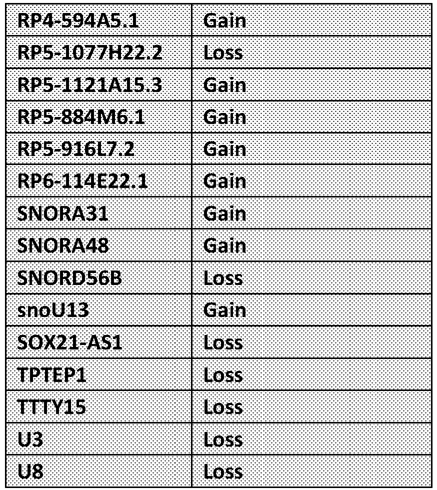

### Validation of H3K4me3 hi/H3K4me1 lo regions as true promoters

Four lines of evidence support the vast majority of H3K4me3 hi/H3K4me1 lo regions as true promoters. First, H3K4me3 hi/H3K4me1 lo regions were strongly enriched at genomic locations located 1kb upstream of known GENCODE transcription start sites (TSSs) (Figure 7). Second, at TSS regions, H3K4me3 signals exhibited a classical skewed bimodal intensity pattern, previously reported to be associated with promoters (Figure 7). Third, when overlapped with regions defined by the Epigenomic Roadmap (EpiRd) 15 state model, we observed significant enrichments of H3K4me3 hi/H3K4me1 lo regions at proximal promoter states (TSSs/Regions flanking transcription sites) in gastrointestinal tissues relative to other tissues (Figure 7). Fourth, CAGE (CAP analysis gene expression) is a specialized transcriptome sequencing method used to map gene promoters using 5' mRNA data. Integration with CAGE data from the FANTOM5 consortium revealed an 81% overlap of H3K4me3 hi/H3K4me1 lo regions with robust CAGE tag clusters. (Figure 7).

### Somatic Promoters in GC Exhibit Deregulation in Diverse Cancer Types

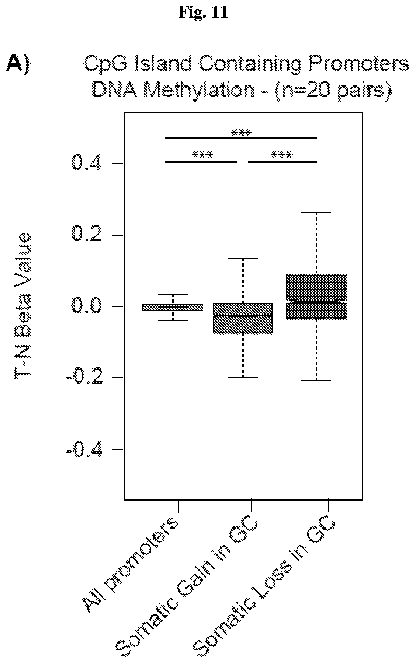

To explore relationships between epigenomic promoter alterations and gene expression, we analyzed RNA-seq data from the same discovery cohort (-106 million reads/sample), quantifying RNA-seq transcript reads mapping to the epigenome-guided promoter regions or directly downstream. Examining somatic promoter regions (Fig. 2A provides an illustrative example of a gained somatic promoter), we observed significantly increased expression at gained somatic promoters in GCs, and significantly decreased expression at lost somatic promoters, compared to either all promoters (P<0.001, Fig. 2B), or unaltered promoters (P<0.001, Figure 10). Among other types of epigenetic modifications, previous studies have also reported a reciprocal relationship between active regulatory regions and DNA methylation. Using Infinium 450K DNA methylation arrays, we identified 7,505 CpG sites overlapping somatic promoter regions (5,213 sites for gained somatic promoters, 2,292 sites for lost somatic promoters). Promoters gained in GC were significantly hypomethylated compared to all promoters, (P<0.001, Wilcoxon test) while promoters lost in GC were hypermethylated (P<0.001, Wilcoxon test) (Fig. 2b, bottom). As DNA methylation typically occurs in CpG rich regions,(56) we then repeated the analysis focusing only on CpG island bearing promoters (Methods and Materials). Similar to the original results, CpG island bearing promoters gained in GC were significantly hypomethylated compared to all CpG island bearing promoters, (P<0.001, Wilcoxon test) while CpG island bearing promoters lost in GC were hypermethylated (P<0.001, Wilcoxon test) (Figure 11).

To validate the somatic promoter alterations in a larger independent GC cohort and also to examine their behavior in other cancer types, we proceeded to query RNA-seq data of 354 GC samples from the TCGA consortium (n=321 GC, n=33 matched normals). To perform this analysis, RNA-seq reads from TCGA samples were mapped against the epigenome-guided somatic promoter regions defined by the discovery samples, and normalized to calculate fold change differences in expression in GC vs. normals (see Methods and Materials). Similar to the discovery series, we observed that TCGA GCs also exhibited significantly increased expression at gained somatic promoters, while lost somatic promoters exhibited decreased expression, relative to either all promoters (P<0.001, Fig. 2C) or unaltered promoters (P<0.001, Figure 10). We further tested the tissue-specificity of the GC somatic promoters by querying RNA-seq data from other tumor types, including colon, kidney renal clear cell carcinoma (ccRCC), and lung adenocarcinoma (LUAD) (Fig. 2d). Almost two-thirds (n=1231, 63%, FC=1.5) of GC somatic promoters were also differentially regulated in TCGA colon cancer samples and similarly, a significant proportion of GC somatic promoters were also associated with differential RNA-seq expression in TCGA ccRCC (n=939, 48%, FC=1.5) and LUAD samples (n=1059, 54%, FC=1.5) (Fig. 2D). This result suggests that many GC somatic promoters are also likely associated with deregulated promoter activity in other solid epithelial malignancies.

### Role of Alternative Promoters

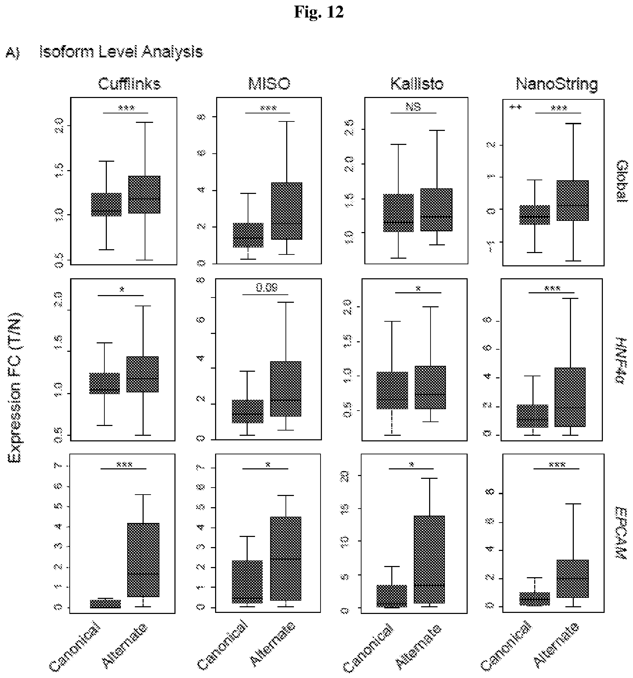

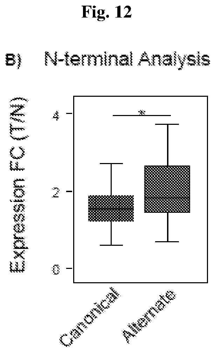

By comparing the somatic promoters against the reference Gencode database (V19), we discovered extensive use of alternative promoters (18%) in GCs, defined as situations where a common unaltered promoter is present in both normal tissues and tumors (canonical promoter) but a secondary tumor-specific promoter is engaged in the latter (alternative promoter). The remaining 82% of somatic promoters corresponded to single major isoforms or unannotated transcripts (see later). 57% of the alternative promoters occurred downstream of the canonical promoter. Using multiple RNA-seq analysis methods, we confirmed that transcript isoforms driven by alternative promoters are overexpressed in GCs to a significantly greater degree than canonical promoters in the same gene (Methods and Materials, Figure 12). For example, *HNF4α,* a transcription factor overexpressed in GC, is driven by two promoters (P1 and P2). At the *HNF4α* canonical promoter ("P2"), we observed equal promoter signals in GCs and normal tissues; however we also further observed gain of an additional promoter in GCs at a transcription start site 45kb downstream ("P1'). Similar *HNF4α* P1 promoter gains were also observed in GC cell lines (Fig. 3a), with RNA-seq analysis supporting *HNF4α* P1 isoform expression in GCs. Alternative promoter usage was also observed at the *EpCAM* gene, frequently used to identify circulating tumor cells, causing expression of *EpCAM* transcript ENST00000263735.4 (Fig. 3b). Notably, both the *HNF4α* and *EpCAM* alternative isoforms exhibited significantly greater cancer overexpression compared to their canonical isoforms (Figure 12). Other genes associated with tumor-specific alternative promoters, many reported for the first time, including *NKX6-3* (FC 1.83, q<0.05) and *GRIN2D* (FC 1.9, q<0.001). A complete list of GC tumor-specific promoters is provided (Table 6).

**Table 6: Alternative Promoters**

| **Loci** | **H3K4Me3 (T/N)** | **Type** | **Change protein in** | **Gene** |
|---|---|---|---|---|
| chr2:69900550-69901900 | Loss | Alternate | 1 | AAK1 |
| chr2:44058400-44060450 | Gain | Alternate | 1 | ABCG5 |
| chr1:179108750-179113100 | Gain | Alternate | 1 | ABL2 |
| chr1:6451200-6453300 | Gain | Alternate | 1 | ACOT7 |
| chr7:991700-995250 | Gain | Alternate | 1 | ADAP1 |
| chr11:69811750-69814800 | Gain | Alternate | 1 | ANO1 |
| chr19:50308050-50309350 | Gain | Alternate | 1 | AP2A1 |
| chr17:36620950-36622550 | Gain | Alternate | 1 | ARHGAP23 |
| chr2:10902450-10904150 | Gain | Alternate | 1 | ATP6V1C2 |
| chr7:70060000-70066050 | Gain | Alternate | 1 | AUTS2 |
| chr18:60804550-60807050 | Loss | Alternate | 1 | BCL2 |
| chr11:1463100-1464700 | Gain | Alternate | 1 | BRSK2 |
| chr4:2038150-2039400 | Gain | Alternate | 1 | C4orf48 |
| chr21:44482600-44484300 | Gain | Alternate | 1 | CBS |
| chr3:46988600-46990000 | Gain | Alternate | 1 | CCDC12 |
| chr16:28946800-28948350 | Gain | Alternate | 1 | CD19 |
| chr6:4836100-4837550 | Gain | Alternate | 1 | CDYL |
| chr6:118985250-118986450 | Loss | Alternate | 1 | CEP85L |
| chr9:124497650-124504300 | Gain | Alternate | 1 | DAB2IP |
| chr19:6474700-6477300 | Gain | Alternate | 1 | DENND1C |
| chr4:955250-957700 | Gain | Alternate | 1 | DGKQ |
| chr16:21059250-21060650 | Gain | Alternate | 1 | DNAH3 |
| chr7:35074250-35076850 | Gain | Alternate | 1 | DPY19L1 |
| chr6:56553350-56559100 | Gain | Alternate | 1 | DST |
| chr2:47595450-47602500 | Gain | Alternate | 1 | EPCAM |
| chrX:137860100-137861300 | Gain | Alternate | 1 | FGF13 |
| chr3:69283500-69286950 | Gain | Alternate | 1 | FRMD4B |
| chr7:99774000-99776200 | Gain | Alternate | 1 | GPC2 |
| chr10:25754300-25755900 | Gain | Alternate | 1 | GPR158 |
| chr11:123458150-123465950 | Gain | Alternate | 1 | GRAMD1B |
| chr20:43029650-43032200 | Gain | Alternate | 1 | HNF4A |
| chr17:46639600-46642950 | Gain | Alternate | 1 | HOXB3 |
| chr7:23506000-23515500 | Gain | Alternate | 1 | IGF2BP3 |
| chr1:38410700-38414500 | Loss | Alternate | 1 | INPP5B |
| chr19:17952000-17953950 | Gain | Alternate | 1 | JAK3 |
| chr14:24891600-24897600 | Loss | Alternate | 1 | KHNYN |
| chr18:21452050-21455250 | Gain | Alternate | 1 | LAMA3 |
| chr5:154091500-154095100 | Loss | Alternate | 1 | LARP1 |
| chr5:38605950-38609550 | Loss | Alternate | 1 | LIFR |
| chr16:1013250-1015550 | Gain | Alternate | 1 | LMF1 |
| chr19:49003900-49005550 | Gain | Alternate | 1 | LMTK3 |
| chr1:156896950-156898350 | Gain | Alternate | 1 | LRRC71 |
| chr1:156893100-156894550 | Gain | Alternate | 1 | LRRC71 |
| chr1:236045300-236047550 | Loss | Alternate | 1 | LYST |
| chr20:33134200-33135900 | Gain | Alternate | 1 | MAP1LC3A |
| chr7:130125100-130127800 | Gain | Alternate | 1 | MEST |
| chr7:116363550-116365500 | Gain | Alternate | 1 | MET |
| chr3:158448250-158451400 | Gain | Alternate | 1 | MFSD1 |
| chr1:1562700-1565700 | Gain | Alternate | 1 | MIB2 |
| chr14:102700300-102702150 | Gain | Alternate | 1 | MOK |
| chr17:60756900-60758850 | Gain | Alternate | 1 | MRC2 |
| chr8:144652950-144655550 | Gain | Alternate | 1 | MROH6 |
| chr7:100607850-100613600 | Gain | Alternate | 1 | MUC12 |
| chr11:76902300-76903800 | Gain | Alternate | 1 | MYO7A |
| chr1:24434350-24435800 | Gain | Alternate | 1 | MYOM3 |
| chr6:126136250-126140700 | Loss | Alternate | 1 | NCOA7 |
| chr2:233755200-233756650 | Gain | Alternate | 1 | NGEF |
| chr2:233791350-233792700 | Gain | Alternate | 1 | NGEF |
| chr17:26119900-26121850 | Gain | Alternate | 1 | NOS2 |
| chr1:200007500-200010950 | Gain | Alternate | 1 | NR5A2 |
| chr18:55099800-55108900 | Gain | Alternate | 1 | ONECUT2 |
| chr8:107629450-107632850 | Loss | Alternate | 1 | OXR1 |
| chr4:169575100-169577200 | Loss | Alternate | 1 | PALLD |
| chr19:18364400-18366800 | Loss | Alternate | 1 | PDE4C |
| chr4:111557000-111559350 | Gain | Alternate | 1 | PITX2 |
| chr8:145009000-145018500 | Gain | Alternate | 1 | PLEC |
| chr19:49370000-49372300 | Gain | Alternate | 1 | PLEKHA4 |
| chr11:16944700-16947800 | Gain | Alternate | 1 | PLEKHA7 |
| chr1:6530450-6535000 | Gain | Alternate | 1 | PLEKHG5 |
| chr5:74990850-74992350 | Gain | Alternate | 1 | POC5 |
| chr6:35359200-35364100 | Loss | Alternate | 1 | PPARD |
| chr19:49631500-49632100 | Gain | Alternate | 1 | PPFIA3 |
| chr22:22900650-22902550 | Gain | Alternate | 1 | PRAME |
| chr9:132458700-132461300 | Gain | Alternate | 1 | PRRX2 |
| chr9:139873000-139874300 | Gain | Alternate | 1 | PTGDS |
| chr1:29562850-29565950 | Gain | Alternate | 1 | PTPRU |
| chr17:2878500-2880550 | Gain | Alternate | 1 | RAP1GAP2 |
| chr9:134548500-134553400 | Loss | Alternate | 1 | RAPGEF1 |
| chr3:24851300-24854350 | Loss | Alternate | 1 | RARB |
| chr13:114769100-114771100 | Gain | Alternate | 1 | RASA3 |
| chr20:399750-402500 | Gain | Alternate | 1 | RBCK1 |
| chr19:14088450-14090950 | Gain | Alternate | 1 | RFX1 |
| chr4:3310150-3312100 | Gain | Alternate | 1 | RGS12 |
| chr8:74035400-74036300 | Loss | Alternate | 1 | SBSPON |
| chr21:38063750-38066650 | Loss | Alternate | 1 | SIM2 |
| chr19:19215350-19217300 | Gain | Alternate | 1 | SLC25A42 |
| chr7:103021250-103022850 | Loss | Alternate | 1 | SLC26A5 |
| chr12:40425950-40427700 | Loss | Alternate | 1 | SLC2A13 |
| chr12:20975550-20976900 | Gain | Alternate | 1 | SLCO1B3 |
| chr16:68418000-68421750 | Loss | Alternate | 1 | SMPD3 |
| chr4:186729400-186734150 | Loss | Alternate | 1 | SORBS2 |
| chr2:231206350-231208750 | Gain | Alternate | 1 | SP140L |
| chr7:87854350-87856200 | Gain | Alternate | 1 | SRI |
| chr3:17734300-17735900 | Gain | Alternate | 1 | TBC1D5 |
| chr8:67866500-67867950 | Gain | Alternate | 1 | TCF24 |
| chr6:10409250-10419650 | Gain | Alternate | 1 | TFAP2A |
| chr3:129512300-129514550 | Gain | Alternate | 1 | TMCC1 |
| chr18:20910450-20912050 | Gain | Alternate | 1 | TMEM241 |
| chr2:218874000-218875450 | Gain | Alternate | 1 | TNS1 |
| chr8:141017700-141019200 | Gain | Alternate | 1 | TRAPPC9 |
| chr4:8435700-8439650 | Loss | Alternate | 1 | TRMT44 |
| chr21:45844650-45846700 | Gain | Alternate | 1 | TRPM2 |
| chrX:107016000-107021000 | Loss | Alternate | 1 | TSC22D3 |
| chr2:3371900-3374350 | Gain | Alternate | 1 | TSSC1 |
| chr17:40784750-40786950 | Loss | Alternate | 1 | TUBG2 |
| chr16:1428050-1430700 | Gain | Alternate | 1 | UNKL |
| chr12:109507100-109508350 | Gain | Alternate | 1 | USP30 |
| chr20:50719850-50723350 | Gain | Alternate | 1 | ZFP64 |
| chr4:8128400-8130450 | Gain | Alternate | 0 | ABLIM2 |
| chr16:72660100-72662050 | Gain | Alternate | 0 | AC004158.2 |
| chr2:66801200-66811950 | Gain | Alternate | 0 | AC007392.3 |
| chr2:114081700-114084050 | Gain | Alternate | 0 | AC016745.3 |
| chr19:52104750-52106000 | Loss | Alternate | 0 | AC018755.16 |
| chr2:19504600-19506400 | Gain | Alternate | 0 | AC092594.1 |
| chr2:118899750-118901550 | Gain | Alternate | 0 | AC093901.1 |
| chr17:263900-267650 | Loss | Alternate | 0 | AC108004.3 |
| chr3:18734950-18736300 | Gain | Alternate | 0 | AC144521.1 |
| chr12:109568950-109570000 | Loss | Alternate | 0 | ACACB |
| chrX:23783150-23786000 | Gain | Alternate | 0 | ACOT9 |
| chr7:5601050-5603800 | Gain | Alternate | 0 | ACTB |
| chr7:15600650-15602200 | Gain | Alternate | 0 | AGMO |
| chr21:45336050-45337600 | Loss | Alternate | 0 | AGPAT3 |
| chr15:86232000-86236800 | Loss | Alternate | 0 | AKAP13 |
| chr9:112909300-112915400 | Loss | Alternate | 0 | AKAP2 |
| chr2:241496150-241498200 | Gain | Alternate | 0 | ANKMY1 |
| chr2:242127000-242129850 | Loss | Alternate | 0 | ANO7 |
| chr5:139972550-139973900 | Gain | Alternate | 0 | APBB3 |
| chr18:24443050-24445900 | Loss | Alternate | 0 | AQP4-AS1 |
| chr4:86395150-86399900 | Loss | Alternate | 0 | ARHGAP24 |
| chr19:47362700-47367650 | Gain | Alternate | 0 | ARHGAP35 |
| chr9:35672750-35677150 | Loss | Alternate | 0 | ARHGEF39 |
| chrX:100739600-100741600 | Gain | Alternate | 0 | ARMCX4 |
| chr9:120175650-120177900 | Loss | Alternate | 0 | ASTN2 |
| chr3:193270000-193274550 | Loss | Alternate | 0 | ATP13A4 |
| chr18:77102950-77104300 | Loss | Alternate | 0 | ATP9B |
| chr1:179486050-179487950 | Loss | Alternate | 0 | AXDND1 |
| chr4:102332100-102333250 | Gain | Alternate | 0 | BANK1 |
| chr1:94046300-94051100 | Loss | Alternate | 0 | BCAR3 |
| chr11:27686500-27687900 | Gain | Alternate | 0 | BDNF-AS |
| chr20:11897750-11902000 | Loss | Alternate | 0 | BTBD3 |
| chr11:63531650-63533550 | Gain | Alternate | 0 | Cllorf95 |
| chr19:30199050-30200500 | Gain | Alternate | 0 | C19orf12 |
| chr1:207991400-208001200 | Loss | Alternate | 0 | C1orf132 |
| chr6:109571700-109573350 | Gain | Alternate | 0 | C6orf183 |
| chr8:128305850-128307550 | Gain | Alternate | 0 | CASC8 |
| chr5:43409150-43412850 | Loss | Alternate | 0 | CCL28 |
| chr8:95245700-95247400 | Gain | Alternate | 0 | CDH17 |
| chr7:105603300-105604700 | Loss | Alternate | 0 | CDHR3 |
| chr7:90338500-90340500 | Loss | Alternate | 0 | CDK14 |
| chr7:29184550-29187650 | Gain | Alternate | 0 | CHN2 |
| chr15:79011600-79013200 | Gain | Alternate | 0 | CHRNB4 |
| chr7:139226300-139228850 | Gain | Alternate | 0 | CLEC2L |
| chr6:25164900-25167200 | Loss | Alternate | 0 | CMAHP |
| chr16:81684900-81687600 | Loss | Alternate | 0 | CMIP |
| chr6:37391200-37392800 | Gain | Alternate | 0 | CMTR1 |
| chr3:74662150-74664400 | Loss | Alternate | 0 | CNTN3 |
| chr11:111172600-111176650 | Loss | Alternate | 0 | COLCA1 |
| chr6:36722500-36725900 | Loss | Alternate | 0 | CPNE5 |
| chr11:85392850-85394650 | Loss | Alternate | 0 | CREBZF |
| chr16:21288600-21290700 | Gain | Alternate | 0 | CRYM |
| chr5:60597450-60601050 | Loss | Alternate | 0 | CTC-436P18.3 |
| chr15:45544050-45548600 | Loss | Alternate | 0 | CTD-2651B20.3 |
| chr20:110300-111350 | Gain | Alternate | 0 | DEFB126 |
| chr2:234326350-234331500 | Loss | Alternate | 0 | DGKD |
| chr1:223101350-223104800 | Loss | Alternate | 0 | DISP1 |
| chr11:111852050-111855050 | Loss | Alternate | 0 | DIXDC1 |
| chr13:50759600-50762100 | Gain | Alternate | 0 | DLEU1 |
| chr1:46954600-46956800 | Gain | Alternate | 0 | DMBX1 |
| chr16:30021900-30023950 | Gain | Alternate | 0 | DOC2A |
| chr6:56715250-56717500 | Gain | Alternate | 0 | DST |
| chr18:46894350-46895900 | Loss | Alternate | 0 | DYM |
| chr5:106838450-106842400 | Loss | Alternate | 0 | EFNA5 |
| chr4:111331750-111333350 | Gain | Alternate | 0 | ENPEP |
| chr14:74461400-74463450 | Loss | Alternate | 0 | ENTPD5 |
| chr19:55590850-55593800 | Gain | Alternate | 0 | EPS8L1 |
| chr5:172332450-172333000 | Loss | Alternate | 0 | ERGIC1 |
| chrl:17024500-17028900 | Gain | Alternate | 0 | ESPNP |
| chr1:216892850-216898200 | Loss | Alternate | 0 | ESRRG |
| chr1:217249050-217252200 | Loss | Alternate | 0 | ESRRG |
| chr6:36326200-36331550 | Gain | Alternate | 0 | ETV7 |
| chr12:124778800-124786100 | Loss | Alternate | 0 | FAM101A |
| chr17:47822200-47825200 | Loss | Alternate | 0 | FAM117A |
| chr4:187025100-187028650 | Loss | Alternate | 0 | FAM149A |
| chr1:178986050-178987900 | Loss | Alternate | 0 | FAM20B |
| chr7:102574000-102576900 | Loss | Alternate | 0 | FBXL13 |
| chr16:86529000-86534050 | Loss | Alternate | 0 | FENDRR |
| chr20:34192700-34196000 | Loss | Alternate | 0 | FER1L4 |
| chr8:124926550-124929550 | Gain | Alternate | 0 | FER1L6 |
| chr7:121942750-121947900 | Gain | Alternate | 0 | FEZF1 |
| chr12:32654200-32659150 | Loss | Alternate | 0 | FGD4 |
| chr16:86608950-86611800 | Gain | Alternate | 0 | FOXL1 |
| chr8:75230900-75235150 | Gain | Alternate | 0 | GDAP1 |
| chr7:100288750-100293000 | Gain | Alternate | 0 | GIGYF1 |
| chr11:58694450-58696550 | Loss | Alternate | 0 | GLYATL1 |
| chr5:89854500-89855350 | Loss | Alternate | 0 | GPR98 |
| chr2:165476750-165479250 | Gain | Alternate | 0 | GRB14 |
| chr9:140056700-140058300 | Gain | Alternate | 0 | GRIN1 |
| chr19:48900250-48904400 | Gain | Alternate | 0 | GRIN2D |
| chr9:104466750-104468450 | Gain | Alternate | 0 | GRIN3A |
| chr3:14642850-14644150 | Loss | Alternate | 0 | GRIP2 |
| chr11:2016000-2021350 | Gain | Alternate | 0 | H19 |
| chrX:152760450-152761150 | Gain | Alternate | 0 | HAUS7 |
| chr7:18534500-18539050 | Loss | Alternate | 0 | HDAC9 |
| chr15:83619150-83622750 | Loss | Alternate | 0 | HOMER2 |
| chr7:27159450-27164850 | Gain | Alternate | 0 | HOXA3 |
| chr7:27208400-27220700 | Gain | Alternate | 0 | HOXA9 |
| chr17:46678350-46683450 | Gain | Alternate | 0 | HOXB6 |
| chr17:46694850-46697150 | Gain | Alternate | 0 | HOXB8 |
| chr3:11178050-11179900 | Gain | Alternate | 0 | HRH1 |
| chr3:11195250-11198600 | Gain | Alternate | 0 | HRH1 |
| chr3:11265900-11269000 | Gain | Alternate | 0 | HRH1 |
| chr1:23543800-23544900 | Gain | Alternate | 0 | HTR1D |
| chrX:130711450-130713600 | Gain | Alternate | 0 | IGSF1 |
| chr17:38016450-38022250 | Loss | Alternate | 0 | IKZF3 |
| chr2:113619100-113622250 | Loss | Alternate | 0 | IL1B |
| chr4:143394250-143396200 | Gain | Alternate | 0 | INPP4B |
| chr19:2255550-2257400 | Loss | Alternate | 0 | JSRP1 |
| chr17:68071050-68073700 | Loss | Alternate | 0 | KCNJ16 |
| chr14:88788450-88791000 | Gain | Alternate | 0 | KCNK10 |
| chr4:56914350-56916700 | Gain | Alternate | 0 | KIAA1211 |
| chr10:24725650-24728200 | Loss | Alternate | 0 | KIAA1217 |
| chr11:33398050-33400750 | Gain | Alternate | 0 | KIAA1549L |
| chr15:31637200-31640250 | Loss | Alternate | 0 | KLF13 |
| chr19:55019200-55020400 | Gain | Alternate | 0 | LAIR2 |
| chr1:65991250-65992850 | Loss | Alternate | 0 | LEPR |
| chr5:78014050-78017100 | Loss | Alternate | 0 | LHFPL2 |
| chr12:113904650-113906650 | Gain | Alternate | 0 | LHX5 |
| chr22:30651400-30654850 | Gain | Alternate | 0 | LIF |
| chr20:21085550-21087550 | Gain | Alternate | 0 | LINC00237 |
| chr13:74234250-74236800 | Gain | Alternate | 0 | LINC00393 |
| chr3:8652200-8654000 | Gain | Alternate | 0 | LMCD1-AS1 |
| chr20:6031700-6033850 | Gain | Alternate | 0 | LRRN4 |
| chr3:116161150-116164900 | Gain | Alternate | 0 | LSAMP |
| chr11:1889150-1894600 | Loss | Alternate | 0 | LSP1 |
| chrX:149588950-149590100 | Gain | Alternate | 0 | MAMLD1 |
| chr1:27683050-27684600 | Loss | Alternate | 0 | MAP3K6 |
| chrX:20115700-20118300 | Loss | Alternate | 0 | MAP7D2 |
| chr3:150959500-150960300 | Gain | Alternate | 0 | MED12L |
| chr22:42148300-42150300 | Loss | Alternate | 0 | MEI1 |
| chr1:205537050-205540700 | Loss | Alternate | 0 | MFSD4 |
| chr1:22489600-22491100 | Gain | Alternate | 0 | MIR4418 |
| chr19:748150-750100 | Gain | Alternate | 0 | MISP |
| chr3:69914350-69917750 | Loss | Alternate | 0 | MITF |
| chr6:168215700-168217350 | Gain | Alternate | 0 | MLLT4-AS1 |
| chr19:1286150-1288700 | Gain | Alternate | 0 | MUM1 |
| chr19:50690700-50695700 | Gain | Alternate | 0 | MYH14 |
| chr17:73606350-73609450 | Gain | Alternate | 0 | MYO15B |
| chr17:31010250-31012000 | Gain | Alternate | 0 | MYO1D |
| chrl8:55888350-55892150 | Loss | Alternate | 0 | NEDD4L |
| chr2:131965200-131968600 | Gain | Alternate | 0 | NF1P8 |
| chr14:27147750-27148900 | Gain | Alternate | 0 | NOVA1-AS1 |
| chr11:108040050-108041550 | Loss | Alternate | 0 | NPAT |
| chr7:98248450-98250250 | Gain | Alternate | 0 | NPTX2 |
| chr15:76302650-76305350 | Loss | Alternate | 0 | NRG4 |
| chr9:132370500-132373750 | Gain | Alternate | 0 | NTMT1 |
| chr3:32118200-32120100 | Gain | Alternate | 0 | OSBPL10 |
| chr19:14171500-14173250 | Loss | Alternate | 0 | PALM3 |
| chr7:32107350-32111900 | Loss | Alternate | 0 | PDE1C |
| chr3:111450850-111453300 | Loss | Alternate | 0 | PHLDB2 |
| chr12:18395250-18399450 | Loss | Alternate | 0 | PIK3C2G |
| chr8:110534900-110536100 | Loss | Alternate | 0 | PKHD1L1 |
| chr20:8094750-8096650 | Gain | Alternate | 0 | PLCB1 |
| chr1:6544500-6545600 | Gain | Alternate | 0 | PLEKHG5 |
| chr22:41990400-41991450 | Gain | Alternate | 0 | PMM1 |
| chr6:31150550-31154950 | Loss | Alternate | 0 | POU5F1 |
| chr11:7626600-7631400 | Loss | Alternate | 0 | PPFIBP2 |
| chr2:182895050-182896750 | Gain | Alternate | 0 | PPP1R1C |
| chr8:143759850-143765700 | Loss | Alternate | 0 | PSCA |
| chr8:27237450-27239750 | Loss | Alternate | 0 | PTK2B |
| chr8:142384050-142385550 | Gain | Alternate | 0 | PTP4A3 |
| chr9:96767600-96770450 | Loss | Alternate | 0 | PTPDC1 |
| chr12:120661250-120664850 | Loss | Alternate | 0 | PXN |
| chr18:52384600-52386250 | Loss | Alternate | 0 | RAB27B |
| chr11:82706750-82709350 | Loss | Alternate | 0 | RAB30 |
| chr8:95485350-95488300 | Gain | Alternate | 0 | RAD54B |
| chr4:82964050-82966400 | Gain | Alternate | 0 | RASGEF1B |
| chr4:40512300-40518850 | Loss | Alternate | 0 | RBM47 |
| chr9:116225550-116228700 | Gain | Alternate | 0 | RGS3 |
| chr10:62758000-62762450 | Loss | Alternate | 0 | RHOBTB1 |
| chr8:104510350-104514700 | Gain | Alternate | 0 | RIMS2 |
| chr21:38379100-38379750 | Gain | Alternate | 0 | RIPPLY3 |
| chr8:61324800-61327100 | Gain | Alternate | 0 | RP11-163N6.2 |
| chr20:6301750-6304300 | Gain | Alternate | 0 | RP11-199O14.1 |
| chr3:187606800-187608950 | Gain | Alternate | 0 | RP11-30O15.1 |
| chr1:39191950-39194400 | Loss | Alternate | 0 | RP11-334L9.1 |
| chr11:112140350-112142500 | Gain | Alternate | 0 | RP11-356J5.12 |
| chr6:82809950-82812100 | Gain | Alternate | 0 | RP11-379B8.1 |
| chr14:39702300-39706400 | Loss | Alternate | 0 | RP11-407N17.3 |
| chr1:203394800-203398950 | Gain | Alternate | 0 | RP11-435P24.3 |
| chr9:72091300-72092650 | Gain | Alternate | 0 | RP11-470P21.2 |
| chr15:82161650-82163400 | Gain | Alternate | 0 | RP11-499F3.2 |
| chr4:88631250-88631950 | Gain | Alternate | 0 | RP11-742B18.1 |
| chr11:94372300-94374550 | Gain | Alternate | 0 | RP11-867G2.5 |
| chr3:131049650-131051500 | Gain | Alternate | 0 | RP11-933H2.4 |
| chr17:10746250-10749200 | Loss | Alternate | 0 | RP11-963H4.3 |
| chr6:85334900-85337050 | Gain | Alternate | 0 | RP1-90L14.1 |
| chr7:156735150-156736500 | Gain | Alternate | 0 | RP5-1121A15.3 |
| chr2:55236200-55238400 | Loss | Alternate | 0 | RTN4 |
| chr16:51186150-51187850 | Loss | Alternate | 0 | SALL1 |
| chr2:200326950-200329550 | Gain | Alternate | 0 | SATB2 |
| chr3:53031650-53034600 | Gain | Alternate | 0 | SFMBT1 |
| chr14:71849000-71850350 | Loss | Alternate | 0 | SIPA1L1 |
| chr1:232760700-232767700 | Gain | Alternate | 0 | SIPA1L2 |
| chr7:100448750-100451750 | Gain | Alternate | 0 | SLC12A9 |
| chr12:105344050-105348050 | Loss | Alternate | 0 | SLC41A2 |
| chr6:31843950-31847850 | Loss | Alternate | 0 | S LC44A4 |
| chr1:75840850-75842350 | Gain | Alternate | 0 | SLC44A5 |
| chr1:205637750-205639250 | Gain | Alternate | 0 | SLC45A3 |
| chr11:26985950-26987450 | Gain | Alternate | 0 | SLC5A12 |
| chr14:23622000-23623950 | Loss | Alternate | 0 | SLC7A8 |
| chr22:31459200-31461650 | Gain | Alternate | 0 | SMTN |
| chr20:10197250-10201300 | Gain | Alternate | 0 | SNAP25-AS1 |
| chr16:1842850-1844950 | Loss | Alternate | 0 | SPSB3 |
| chr11:4010850-4011700 | Loss | Alternate | 0 | STIM1 |
| chr8:99951150-99961750 | Gain | Alternate | 0 | STK3 |
| chr7:23761400-23764000 | Gain | Alternate | 0 | STK31 |
| chr1:110573450-110574700 | Loss | Alternate | 0 | STRIP1 |
| chr7:73131100-73134700 | Gain | Alternate | 0 | STX1A |
| chr20:46411750-46414250 | Gain | Alternate | 0 | SULF2 |
| chr12:79438650-79440250 | Gain | Alternate | 0 | SYT1 |
| chr15:57509850-57515600 | Loss | Alternate | 0 | TCF12 |
| chr12:110411050-110419200 | Gain | Alternate | 0 | TCHP |
| chr21:32640100-32641350 | Loss | Alternate | 0 | TIAM1 |
| chr19:3707600-3711250 | Loss | Alternate | 0 | TJP3 |
| chr10:102830000-102833650 | Loss | Alternate | 0 | TLX1NB |
| chr2:228241600-228244450 | Gain | Alternate | 0 | TM4SF20 |
| chr16:19427700-19435900 | Gain | Alternate | 0 | TMC5 |
| chr7:47490900-47493500 | Loss | Alternate | 0 | TNS3 |
| chr8:144436800-144438000 | Gain | Alternate | 0 | TOP1MT |
| chr13:45955000-45957700 | Gain | Alternate | 0 | TPT1-AS1 |
| chr17:3459750-3462900 | Loss | Alternate | 0 | TRPV3 |
| chr3:12522200-12524700 | Gain | Alternate | 0 | TSEN2 |
| chr22:46683150-46685350 | Loss | Alternate | 0 | TTC38 |
| chr6:133003800-133008900 | Gain | Alternate | 0 | VNN1 |
| chr15:53831700-53833550 | Gain | Alternate | 0 | WDR72 |
| chr11:102617350-102619450 | Gain | Alternate | 0 | WTAPP1 |
| chr11:68436350-68438200 | Gain | Alternate | 0 | Novel Gene |
| chr12:125226400-125228400 | Loss | Alternate | 0 | Novel Gene |
| chr12:89240400-89241750 | Gain | Alternate | 0 | Novel Gene |
| chr14:99752650-99754000 | Loss | Alternate | 0 | Novel Gene |
| chr18:76805850-76809250 | Gain | Alternate | 0 | Novel Gene |
| chr19:53560600-53562700 | Gain | Alternate | 0 | Novel Gene |
| chr2:45227500-45229600 | Gain | Alternate | 0 | Novel Gene |
| chr2:134784950-134786450 | Gain | Alternate | 0 | Novel Gene |
| chr2:176458500-176460750 | Gain | Alternate | 0 | Novel Gene |
| chr20:46600150-46603250 | Gain | Alternate | 0 | Novel Gene |
| chr4:10830100-10832350 | Gain | Alternate | 0 | Novel Gene |
| chr5:35404300-35405800 | Gain | Alternate | 0 | Novel Gene |
| chr5:42999400-43001150 | Gain | Alternate | 0 | Novel Gene |
| chr5:72496650-72498300 | Gain | Alternate | 0 | Novel Gene |
| chr1:204682350-204684550 | Loss | Alternate | 0 | Novel Gene |
| chr6:868400-871100 | Loss | Alternate | 0 | Novel Gene |
| chr1:220635500-220637400 | Gain | Alternate | 0 | Novel Gene |
| chr6:47146850-47150550 | Loss | Alternate | 0 | Novel Gene |
| chr6:160720200-160722150 | Gain | Alternate | 0 | Novel Gene |
| chr6:170474550-170475800 | Gain | Alternate | 0 | Novel Gene |
| chr1:242107250-242109450 | Gain | Alternate | 0 | Novel Gene |
| chr7:27274550-27276500 | Gain | Alternate | 0 | Novel Gene |
| chr9:17905350-17908250 | Loss | Alternate | 0 | Novel Gene |
| chr9:31848250-31849950 | Gain | Alternate | 0 | Novel Gene |
| chrX:56133300-56134800 | Gain | Alternate | 0 | Novel Gene |
| chrX:3466450-3468750 | Gain | Alternate | 0 | Novel Gene |
| chrX:6849150-6851300 | Gain | Alternate | 0 | Novel Gene |
| chr11:60941900-60945700 | Loss | Alternate | 0 | Novel Gene |
| chr11:71350450-71351500 | Gain | Alternate | 0 | Novel Gene |
| chr11:119775600-119779600 | Loss | Alternate | 0 | Novel Gene |
| chr5:82391600-82392950 | Gain | Alternate | 0 | XRCC4 |
| chr3:141107100-141108400 | Loss | Alternate | 0 | ZBTB38 |
| chr18:45660800-45664950 | Loss | Alternate | 0 | ZBTB7C |
| chr13:100619800-100623100 | Gain | Alternate | 0 | ZIC5 |
| chr2:180425300-180426950 | Loss | Alternate | 0 | ZNF385B |
| chr19:53539900-53541600 | Gain | Alternate | 0 | ZNF702P |

To explore the influence of alternative promoters on protein diversity, we identified 714 tumor-specific promoter alterations predicted to change N-terminal protein composition and also supported by both H3K4me3 and RNA-seq data. The vast majority of these alterations (>95%) were in-frame to that of the canonical protein. Of these, 47% (n=338) were predicted to cause gains of new N-terminal peptides in tumors (see Methods). To confirm protein-level expression of these N-terminal peptides in gastrointestinal cancer, we queried publically available peptide spectral data of 90 TCGA colorectal cancer (CRC) and 60 normal colon samples. CRC data was used for this analysis as large-scale proteomic data of primary GCs are not currently available, and because many GC somatic promoters are also observed in CRC (Fig. 2d). Among N-terminal peptides predicted to be gained in tumors, we confirmed protein expression of 33% (112/338) in the CRC data (Table 7), of which 51.8% were overexpressed in CRC samples relative to normal colon samples (FDR 10%). In a separate experiment, we further investigated if these N-terminal peptides also exhibit tumor overexpression in proteomic data from 3 GC cell lines and 1 normal gastric epithelial line (GES1) (Methods and Materials). Similar to the CRC data, 48% of the N-terminal peptides were overexpressed in the GC lines relative to normal GES1 gastric cells. Taken collectively, these analyses suggest that alternative promoters may contribute significantly towards proteomic diversity in gastrointestinal cancer.

**Table 7: Spectral Counts from CRC samples of N terminal peptides predicted to be gained in GC**

| **SEQ_ID_NO** | **Peptide** | **GeneId** | **SpectralCount** |
|---|---|---|---|
| SEQ ID NO: 1 | IDNSQVESGSLEDDWDFLPPKK | ENSG00000179218.9 | 2602 |
| SEQ ID NO: 2 | FYALSASFEPFSNK | ENSG00000179218.9 | 2047 |
| SEQ ID NO: 3 | EQFLDGDGWTSR | ENSG00000179218.9 | 1370 |
| SEQ ID NO: 4 | IKDPDASKPEDWDER | ENSG00000179218.9 | 805 |
| SEQ ID NO: 5 | GDVTAQIALQPALK | ENSG00000112096.12 | 601 |
| SEQ ID NO: 6 | GISLNPEQWSQLK | ENSG00000113387.7 | 536 |
| SEQ ID NO: 7 | AYHSFLVEPISCHAWNK | ENSG00000130429.8 | 497 |
| SEQ ID NO: 8 | IAVQPGTVGPQGR | ENSG00000134871.13 | 468 |
| SEQ ID NO: 9 | VLAQNSGFDLQETLVK | ENSG00000146731.6 | 435 |
| SEQ ID NO: 10 | CKDDEFTHLYTLIVRPDNTYEVK | ENSG00000179218.9 | 424 |
| SEQ ID NO: 11 | AKIDDPTDSKPEDWDKPEHIPDP DAK | ENSG00000179218.9 | 414 |
| SEQ ID NO: 12 | VHVIFNYK | ENSG00000179218.9 | 396 |
| SEQ ID NO: 13 | HEQNIDCGGGYVK | ENSG00000179218.9 | 361 |
| SEQ ID NO: 14 | LIDFGLAR | ENSG00000065534.14 | 359 |
| SEQ ID NO: 15 | TWKPTLVILR | ENSG00000130429.8 | 358 |
| SEQ ID NO: 16 | AIWNVINWENVTER | ENSG00000112096.12 | 353 |
| SEQ ID NO: 17 | IDDPTDSKPEDWDKPEHIPDPDA K | ENSG00000179218.9 | 323 |
| SEQ ID NO: 18 | NVRPDYLK | ENSG00000112096.12 | 320 |
| SEQ ID NO: 19 | NSVSQISVLSGGK | ENSG00000130429.8 | 317 |
| SEQ ID NO: 20 | DGNVLLHEMQIQHPTASLIAK | ENSG00000146731.6 | 314 |
| SEQ ID NO: 21 | AGATHVER | ENSG00000145016.9 | 311 |
| SEQ ID NO: 22 | LVALLNTLDR | ENSG00000119383.15 | 298 |
| SEQ ID NO: 23 | HHAAYVNNLNVTEEK | ENSG00000112096.12 | 296 |
| SEQ ID NO: 24 | FYGDEEKDKGLQTSQDAR | ENSG00000179218.9 | 290 |
| SEQ ID NO: 25 | KVHVIFNYK | ENSG00000179218.9 | 283 |
| SEQ ID NO: 26 | GPLPAAPPVAPER | ENSG00000115310.13 | 282 |
| SEQ ID NO: 27 | VLLSALER | ENSG00000100714.11 | 277 |
| SEQ ID NO: 28 | SVSIGYLLVK | ENSG00000134871.13 | 276 |
| SEQ ID NO: 29 | IQQEIAVQNPLVSER | ENSG00000167770.7 | 271 |
| SEQ ID NO: 30 | GELLEAIKR | ENSG00000112096.12 | 268 |
| SEQ ID NO: 31 | AHNQDLGLAGSCLAR | ENSG00000134871.13 | 265 |
| SEQ ID NO: 32 | YVVVTGITPTPLGEGK | ENSG00000100714.11 | 256 |
| SEQ ID NO: 33 | MEDLDQSPLVSSSDSPPRPQPAF K | ENSG00000115310.13 | 254 |
| SEQ ID NO: 34 | AAQAPSSFQLLYDLK | ENSG00000100714.11 | 253 |
| SEQ ID NO: 35 | LQAQLNELQAQLSQK | ENSG00000137497.13 | 250 |
| SEQ ID NO: 36 | ALQFLEEVK | ENSG00000146731.6 | 244 |
| SEQ ID NO: 37 | LLTSGYLQR | ENSG00000167770.7 | 242 |
| SEQ ID NO: 38 | GDLNDCFIPCTPK | ENSG00000100714.11 | 241 |
| SEQ ID NO: 39 | ASSEGGTAAGAGLDSLHK | ENSG00000130429.8 | 240 |
| SEQ ID NO: 40 | EAVTEILGIEPDREK | ENSG00000211460.7 | 236 |
| SEQ ID NO: 41 | EVEERPAPTPWGSK | ENSG00000130429.8 | 235 |
| SEQ ID NO: 42 | IITEGFEAAK | ENSG00000146731.6 | 235 |
| SEQ ID NO: 43 | YLNIFGESQPNPK | ENSG00000004864.9 | 234 |
| SEQ ID NO: 44 | LTAASVGVQGSGWGWLGFNK | ENSG00000112096.12 | 229 |
| SEQ ID NO: 45 | IAPLEEGTLPFNLAEAQR | ENSG00000004864.9 | 221 |
| SEQ ID NO: 46 | GQTLVVQFTVK | ENSG00000179218.9 | 220 |
| SEQ ID NO: 47 | AQLGVQAFADALLIIPK | ENSG00000146731.6 | 217 |
| SEQ ID NO: 48 | QVAPEKPVK | ENSG00000113387.7 | 217 |
| SEQ ID NO: 49 | VATAQDDITGDGTTSNVLIIGELL K | ENSG00000146731.6 | 215 |
| SEQ ID NO: 50 | GLLPQLLGVAPEK | ENSG00000004864.9 | 214 |
| SEQ ID NO: 51 | NAYVWTLK | ENSG00000130429.8 | 214 |
| SEQ ID NO: 52 | IYGADDIELLPEAQHK | ENSG00000100714.11 | 211 |
| SEQ ID NO: 53 | CHAIIDEQPLIFK | ENSG00000169756.12 | 210 |
| SEQ ID NO: 54 | KGISLNPEQWSQLK | ENSG00000113387.7 | 209 |
| SEQ ID NO: 55 | GIDPFSLDALSK | ENSG00000146731.6 | 207 |
| SEQ ID NO: 56 | LLQCYPPPEDAAVK | ENSG00000196961.8 | 207 |
| SEQ ID NO: 57 | GVPTGFILPIR | ENSG00000100714.11 | 204 |
| SEQ ID NO: 58 | IVTCGTDR | ENSG00000130429.8 | 204 |
| SEQ ID NO: 59 | TPVPSDIDISR | ENSG00000100714.11 | 203 |
| SEQ ID NO: 60 | YQEALAK | ENSG00000112096.12 | 198 |
| SEQ ID NO: 61 | VAWVSHDSTVCLADADKK | ENSG00000130429.8 | 197 |
| SEQ ID NO: 62 | LDIDPETITWQR | ENSG00000100714.11 | 194 |
| SEQ ID NO: 63 | IDNSQVESGSLEDDWDFLPPK | ENSG00000179218.9 | 192 |
| SEQ ID NO: 64 | LAILQVGNR | ENSG00000100714.11 | 192 |
| SEQ ID NO: 65 | AQAALAVNISAAR | ENSG00000146731.6 | 191 |
| SEQ ID NO: 66 | GALALAQAVQR | ENSG00000100714.11 | 189 |
| SEQ ID NO: 67 | TDPTTLTDEEINR | ENSG00000100714.11 | 189 |
| SEQ ID NO: 68 | LELSVLYK | ENSG00000167770.7 | 188 |
| SEQ ID NO: 69 | GLDGYQGPDGPR | ENSG00000134871.13 | 187 |
| SEQ ID NO: 70 | LSGLEQPQGALQTR | ENSG00000133316.11 | 184 |
| SEQ ID NO: 71 | SCQTALVEILDVIVR | ENSG00000067704.8 | 182 |
| SEQ ID NO: 72 | DDNMFQIGK | ENSG00000113387.7 | 181 |
| SEQ ID NO: 73 | EHNGQVTGIDWAPESNR | ENSG00000130429.8 | 179 |
| SEQ ID NO: 74 | KIKDPDASKPEDWDER | ENSG00000179218.9 | 178 |
| SEQ ID NO: 75 | MFGIPVVVAVNAFK | ENSG00000100714.11 | 178 |
| SEQ ID NO: 76 | FFEHFIEGGR | ENSG00000167770.7 | 177 |
| SEQ ID NO: 77 | IFHELTQTDK | ENSG00000100714.11 | 174 |
| SEQ ID NO: 78 | FINLFPETK | ENSG00000196961.8 | 172 |
| SEQ ID NO: 79 | FYGDEEKDK | ENSG00000179218.9 | 172 |
| SEQ ID NO: 80 | FNGGGHINHSIFWTNLSPNGGG EPK | ENSG00000112096.12 | 169 |
| SEQ ID NO: 81 | DPDASKPEDWDER | ENSG00000179218.9 | 168 |
| SEQ ID NO: 82 | LGSPDYGNSALLSLPGYRPTTR | ENSG00000137497.13 | 168 |
| SEQ ID NO: 83 | ASGDSARPVLLQVAESAYR | ENSG00000004864.9 | 167 |
| SEQ ID NO: 84 | TDTESELDLISR | ENSG00000100714.11 | 166 |
| SEQ ID NO: 85 | LDFVCSFLQK | ENSG00000137497.13 | 165 |
| SEQ ID NO: 86 | WIDETPPVDQPSR | ENSG00000119383.15 | 165 |
| SEQ ID NO: 87 | GLLGALTSTPYSPTQHLER | ENSG00000153310.14 | 164 |
| SEQ ID NO: 88 | KPEDWDEEMDGEWEPPVIQNP EYK | ENSG00000179218.9 | 162 |
| SEQ ID NO: 89 | FSDIQIR | ENSG00000100714.11 | 160 |
| SEQ ID NO: 90 | STSFNVQDLLPDHEYK | ENSG00000065534.14 | 160 |
| SEQ ID NO: 91 | GEQGFMGNTGPTGAVGDR | ENSG00000134871.13 | 159 |
| SEQ ID NO: 92 | QPSQGPTFGIK | ENSG00000100714.11 | 157 |
| SEQ ID NO: 93 | THLSLSHNPEQK | ENSG00000100714.11 | 157 |
| SEQ ID NO: 94 | APVPSTCSSTFPEELSPPSHQAK | ENSG00000137497.13 | 155 |
| SEQ ID NO: 95 | GEGGTTNPHIFPEGSEPK | ENSG00000167770.7 | 155 |
| SEQ ID NO: 96 | TALAEAELEYNPEHVSR | ENSG00000067704.8 | 155 |
| SEQ ID NO: 97 | FPLLKPSPK | ENSG00000067704.8 | 154 |
| SEQ ID NO: 98 | DQAANLMANR | ENSG00000198947.10 | 153 |
| SEQ ID NO: 99 | HLTAQVR | ENSG00000137497.13 | 153 |
| SEQ ID NO: 100 | FVLSSGK | ENSG00000179218.9 | 149 |
| SEQ ID NO: 101 | SSLPPVLGTESDATVK | ENSG00000065534.14 | 148 |
| SEQ ID NO: 102 | AWGAVVPLVGK | ENSG00000153310.14 | 146 |
| SEQ ID NO: 103 | IEGYPDPEVVWFK | ENSG00000065534.14 | 145 |
| SEQ ID NO: 104 | GKNVLINK | ENSG00000179218.9 | 144 |
| SEQ ID NO: 105 | GLQTSQDAR | ENSG00000179218.9 | 144 |
| SEQ ID NO: 106 | HTLTQIK | ENSG00000146731.6 | 144 |
| SEQ ID NO: 107 | VHAELADVLTEAVVDSILAIK | ENSG00000146731.6 | 144 |
| SEQ ID NO: 108 | YVIHTVGPIAYGEPSASQAAELR | ENSG00000133315.6 | 142 |
| SEQ ID NO: 109 | IQSSHNFQLESVNK | ENSG00000135052.12 | 141 |
| SEQ ID NO: 110 | QIDNPDYK | ENSG00000179218.9 | 140 |
| SEQ ID NO: 111 | DAEGILEDLQSYR | ENSG00000153310.14 | 139 |
| SEQ ID NO: 112 | YTAESSDTLCPR | ENSG00000067704.8 | 139 |
| SEQ ID NO: 113 | EESREPAPASPAPAGVEIR | ENSG00000113657.8 | 138 |
| SEQ ID NO: 114 | EMDRETLIDVAR | ENSG00000146731.6 | 138 |
| SEQ ID NO: 115 | NEVSFVIHNLPVLAK | ENSG00000086475.10 | 138 |
| SEQ ID NO: 116 | QVAPEKPVKK | ENSG00000113387.7 | 137 |
| SEQ ID NO: 117 | FLINLEGGDIR | ENSG00000067704.8 | 136 |
| SEQ ID NO: 118 | LSVNSVTAGDYSR | ENSG00000211460.7 | 135 |
| SEQ ID NO: 119 | QAQVNLTVVDKPDPPAGTPCAS DIR | ENSG00000065534.14 | 135 |
| SEQ ID NO: 120 | IFDDVSSGVSQLASK | ENSG00000101199.8 | 134 |
| SEQ ID NO: 121 | PDASKPEDWDER | ENSG00000179218.9 | 134 |
| SEQ ID NO: 122 | YGGAPQALTLK | ENSG00000196961.8 | 132 |
| SEQ ID NO: 123 | LVTPGETPSWTGSGFVR | ENSG00000172037.9 | 131 |
| SEQ ID NO: 124 | EQISDIDDAVR | ENSG00000113387.7 | 129 |
| SEQ ID NO: 125 | KPAAGLSAAPVPTAPAAGAPLM DFGNDFVPPAPR | ENSG00000115310.13 | 129 |
| SEQ ID NO: 126 | ATSSTQSLAR | ENSG00000137497.13 | 128 |
| SEQ ID NO: 127 | LLVPTQFVGAIIGK | ENSG00000136231.9 | 128 |
| SEQ ID NO: 128 | GELLEAIK | ENSG00000112096.12 | 126 |
| SEQ ID NO: 129 | FFQPTEMAAQDFFQR | ENSG00000196961.8 | 124 |
| SEQ ID NO: 130 | GSGSRPGIEGDTPR | ENSG00000113657.8 | 121 |
| SEQ ID NO: 131 | NAIDDGCVVPGAGAVEVAMAE ALIK | ENSG00000146731.6 | 121 |
| SEQ ID NO: 132 | AAAAAAVGPGAGGAGSAVPGG AGPCATVSVFPGAR | ENSG00000142453.7 | 120 |
| SEQ ID NO: 133 | DFLTPPLLSVR | ENSG00000196961.8 | 120 |
| SEQ ID NO: 134 | LFVVPADEAQAR | ENSG00000105223.14 | 120 |
| SEQ ID NO: 135 | WMIQYNNLNLK | ENSG00000100714.11 | 120 |
| SEQ ID NO: 136 | SLPISLVFLVPVR | ENSG00000169896.12 | 119 |
| SEQ ID NO: 137 | ALQVGCLLR | ENSG00000196961.8 | 118 |
| SEQ ID NO: 138 | ESFNPESYELDK | ENSG00000086475.10 | 118 |
| SEQ ID NO: 139 | TGWISTSSIWK | ENSG00000067704.8 | 118 |
| SEQ ID NO: 140 | EYAEDDNIYQQK | ENSG00000167770.7 | 117 |
| SEQ ID NO: 141 | TQIAICPNNHEVHIYEK | ENSG00000130429.8 | 117 |
| SEQ ID NO: 142 | SLEAQVAHADQQLR | ENSG00000137497.13 | 116 |
| SEQ ID NO: 143 | SVTLLIK | ENSG00000146731.6 | 116 |
| SEQ ID NO: 144 | IHFVPGWDCHGLPIEIK | ENSG00000067704.8 | 115 |
| SEQ ID NO: 145 | QQPDTELEIQQK | ENSG00000067704.8 | 115 |
| SEQ ID NO: 146 | KGEPVSAEDLGVSGALTVLMK | ENSG00000100714.11 | 114 |
| SEQ ID NO: 147 | LGIGMDTCVIPLR | ENSG00000086475.10 | 113 |
| SEQ ID NO: 148 | QPSWDPSPVSSTVPAPSPLSAAA VSPSK | ENSG00000115310.13 | 113 |
| SEQ ID NO: 149 | QISEGVEYIHK | ENSG00000065534.14 | 109 |
| SEQ ID NO: 150 | SEGGTAAGAGLDSLHK | ENSG00000130429.8 | 108 |
| SEQ ID NO: 151 | PTGFILPIR | ENSG00000100714.11 | 107 |
| SEQ ID NO: 152 | SQAGVSSGAPPGR | ENSG00000137497.13 | 107 |
| SEQ ID NO: 153 | VCGDSDKGFVVINQK | ENSG00000146731.6 | 107 |
| SEQ ID NO: 154 | LGIVQGIVGAR | ENSG00000172037.9 | 104 |
| SEQ ID NO: 155 | FLSLPEVR | ENSG00000106066.9 | 103 |
| SEQ ID NO: 156 | GLVLDHGAR | ENSG00000146731.6 | 102 |
| SEQ ID NO: 157 | LKNQVTQLK | ENSG00000100714.11 | 102 |
| SEQ ID NO: 158 | TSVQFQNFSPTVVHPGDLQTQL AVQTK | ENSG00000196961.8 | 102 |
| SEQ ID NO: 159 | EPPYGADVLR | ENSG00000067704.8 | 101 |
| SEQ ID NO: 160 | AAGPLLTDECR | ENSG00000133315.6 | 100 |
| SEQ ID NO: 161 | IIEVAPQVATQNVNPTPGATS | ENSG00000086475.10 | 100 |
| SEQ ID NO: 162 | LFSQGQDVSNK | ENSG00000130396.16 | 100 |
| SEQ ID NO: 163 | VSGPWEEADAEAVAR | ENSG00000090006.13 | 100 |
| SEQ ID NO: 164 | VTGTQPITCTWMK | ENSG00000065534.14 | 100 |
| SEQ ID NO: 165 | VLIDIR | ENSG00000113387.7 | 99 |
| SEQ ID NO: 166 | AVLEEGTDVVIK | ENSG00000067704.8 | 98 |
| SEQ ID NO: 167 | QFAEILHFTLR | ENSG00000153310.14 | 97 |
| SEQ ID NO: 168 | IVGAPMHDLLLWNNATVTTCHS K | ENSG00000100714.11 | 96 |
| SEQ ID NO: 169 | AYIQENLELVEK | ENSG00000100714.11 | 95 |
| SEQ ID NO: 170 | EIGLLSEEVELYGETK | ENSG00000100714.11 | 95 |
| SEQ ID NO: 171 | DSFLGSIPGK | ENSG00000067704.8 | 94 |
| SEQ ID NO: 172 | QLDALLEALK | ENSG00000172037.9 | 94 |
| SEQ ID NO: 173 | IIDEDFELTER | ENSG00000065534.14 | 93 |
| SEQ ID NO: 174 | DTINLLDQR | ENSG00000135052.12 | 92 |
| SEQ ID NO: 175 | VVQSLEQTAR | ENSG00000211460.7 | 92 |
| SEQ ID NO: 176 | DDSNLYINVK | ENSG00000100714.11 | 90 |
| SEQ ID NO: 177 | VSGQPQSVTASSDK | ENSG00000101199.8 | 90 |
| SEQ ID NO: 178 | EFCQQEVEPMCK | ENSG00000167770.7 | 89 |
| SEQ ID NO: 179 | AGNSLAASTAEETAGSAQGR | ENSG00000172037.9 | 88 |
| SEQ ID NO: 180 | EYWMDPEGEMKPGR | ENSG00000113387.7 | 88 |
| SEQ ID NO: 181 | LQSQLLSIEK | ENSG00000106976.14 | 88 |
| SEQ ID NO: 182 | AGESVELFGK | ENSG00000065534.14 | 86 |
| SEQ ID NO: 183 | NGEFFMSPNDFVTR | ENSG00000004864.9 | 86 |
| SEQ ID NO: 184 | VVVGAPQEIVAANQR | ENSG00000169896.12 | 86 |
| SEQ ID NO: 185 | SQAPLESSLDSLGDVFLDSGRK | ENSG00000137497.13 | 85 |
| SEQ ID NO: 186 | GCLELIK | ENSG00000100714.11 | 84 |
| SEQ ID NO: 187 | HSQTDQEPMCPVGMNK | ENSG00000134871.13 | 84 |
| SEQ ID NO: 188 | NPQVCGPGR | ENSG00000090006.13 | 83 |
| SEQ ID NO: 189 | SRGPGAPCQDVDECAR | ENSG00000090006.13 | 83 |
| SEQ ID NO: 190 | TKDEYLINSQTTEHIVK | ENSG00000067704.8 | 83 |
| SEQ ID NO: 191 | IATTTASAATAAAIGATPR | ENSG00000137497.13 | 82 |
| SEQ ID NO: 192 | LGHELQQAGLK | ENSG00000137497.13 | 82 |
| SEQ ID NO: 193 | TEVPPLLLILDR | ENSG00000136631.8 | 82 |
| SEQ ID NO: 194 | YGDEEKDK | ENSG00000179218.9 | 82 |
| SEQ ID NO: 195 | SESQGTAPAFK | ENSG00000065534.14 | 81 |
| SEQ ID NO: 196 | LPQEPGREQVVEDRPVGGR | ENSG00000135052.12 | 80 |
| SEQ ID NO: 197 | LPYGGQCRPCPCPEGPGSQR | ENSG00000172037.9 | 79 |
| SEQ ID NO: 198 | VYLLYRPGHYDILYK | ENSG00000167770.7 | 79 |
| SEQ ID NO: 199 | FQVATDALK | ENSG00000137497.13 | 78 |
| SEQ ID NO: 200 | LQEGQTLEFLVASVPK | ENSG00000172037.9 | 78 |
| SEQ ID NO: 201 | LQGAVCGVSSGPPPPR | ENSG00000011028.9 | 78 |
| SEQ ID NO: 202 | IQNVVTSFAPQR | ENSG00000172037.9 | 77 |
| SEQ ID NO: 203 | VSTLQNQR | ENSG00000169896.12 | 77 |
| SEQ ID NO: 204 | LSQLEEHLSQLQDNPPQEK | ENSG00000137497.13 | 76 |
| SEQ ID NO: 205 | SQAPLESSLDSLGDVFLDSGR | ENSG00000137497.13 | 76 |
| SEQ ID NO: 206 | AGPDLASCLDVDECR | ENSG00000090006.13 | 75 |
| SEQ ID NO: 207 | GTCHYYANK | ENSG00000134871.13 | 74 |
| SEQ ID NO: 208 | HKSETDTSLIR | ENSG00000146731.6 | 74 |
| SEQ ID NO: 209 | KQQNQELQEQLR | ENSG00000137497.13 | 74 |
| SEQ ID NO: 210 | SGDLYVLAADK | ENSG00000067704.8 | 74 |
| SEQ ID NO: 211 | AFGFSHLEALLDDSK | ENSG00000167770.7 | 73 |
| SEQ ID NO: 212 | EILTLLQGVHQGAGFQDIPK | ENSG00000211460.7 | 73 |
| SEQ ID NO: 213 | IQQCPGTETAEYQSLCPHGR | ENSG00000090006.13 | 73 |
| SEQ ID NO: 214 | KDPDASKPEDWDER | ENSG00000179218.9 | 73 |
| SEQ ID NO: 215 | SYWLSTTAPLPMMPVAEDEIKPY ISR | ENSG00000134871.13 | 73 |
| SEQ ID NO: 216 | VPQDVLQK | ENSG00000086475.10 | 73 |
| SEQ ID NO: 217 | DFGSFDKFK | ENSG00000112096.12 | 72 |
| SEQ ID NO: 218 | FIILSQEGSLCSVSIEK | ENSG00000065534.14 | 72 |
| SEQ ID NO: 219 | LAVATFAGIENK | ENSG00000004864.9 | 72 |
| SEQ ID NO: 220 | RLENAGSLK | ENSG00000065534.14 | 72 |
| SEQ ID NO: 221 | AAMPPQIIQFPEDQK | ENSG00000065534.14 | 71 |
| SEQ ID NO: 222 | EAQNLSAMEIR | ENSG00000067704.8 | 71 |
| SEQ ID NO: 223 | ILVAGDSMDSVK | ENSG00000196961.8 | 71 |
| SEQ ID NO: 224 | LVHSYPYDWR | ENSG00000067704.8 | 71 |
| SEQ ID NO: 225 | AEAGDAALSVAEWLR | ENSG00000186635.10 | 70 |
| SEQ ID NO: 226 | ELSNFYFSIIK | ENSG00000067704.8 | 70 |
| SEQ ID NO: 227 | AEAAAPYTVLAQSAPR | ENSG00000090006.13 | 69 |
| SEQ ID NO: 228 | GPGAPCQDVDECAR | ENSG00000090006.13 | 69 |
| SEQ ID NO: 229 | VSDFYDIEER | ENSG00000065534.14 | 69 |
| SEQ ID NO: 230 | NNDFYVTGESYAGK | ENSG00000106066.9 | 68 |
| SEQ ID NO: 231 | QPVVDTFDIR | ENSG00000142453.7 | 68 |
| SEQ ID NO: 232 | QQLQALSEPQPR | ENSG00000135052.12 | 68 |
| SEQ ID NO: 233 | APAEILNGKEISAQIR | ENSG00000100714.11 | 67 |
| SEQ ID NO: 234 | KLDVEEPDSANSSFYSTR | ENSG00000137497.13 | 67 |
| SEQ ID NO: 235 | QPPPDSSEEAPPATQNFIIPK | ENSG00000119383.15 | 67 |
| SEQ ID NO: 236 | SLADVDAILAR | ENSG00000172037.9 | 67 |
| SEQ ID NO: 237 | TGGSAQPETPYSGPGLLIDSLVLL PR | ENSG00000172037.9 | 67 |
| SEQ ID NO: 238 | CDLCQEVLADIGFVK | ENSG00000169756.12 | 66 |
| SEQ ID NO: 239 | FIAGTGCLVR | ENSG00000184207.8 | 66 |
| SEQ ID NO: 240 | HHAAYVNNLNVTEEKYQEALAK | ENSG00000112096.12 | 66 |
| SEQ ID NO: 241 | QGIVHLDLKPENIMCVNK | ENSG00000065534.14 | 66 |
| SEQ ID NO: 242 | TLGDQLSLLLGAR | ENSG00000011028.9 | 66 |
| SEQ ID NO: 243 | CTHWAEGGK | ENSG00000100714.11 | 65 |
| SEQ ID NO: 244 | FGLYLPLFKPSVSTSK | ENSG00000004864.9 | 65 |
| SEQ ID NO: 245 | GSCYPATGDLLVGR | ENSG00000172037.9 | 65 |
| SEQ ID NO: 246 | VMPLIIQGFK | ENSG00000086475.10 | 65 |
| SEQ ID NO: 247 | TPLWIGLAGEEGSR | ENSG00000011028.9 | 64 |
| SEQ ID NO: 248 | TQPDGTSVPGEPASPISQR | ENSG00000137497.13 | 64 |
| SEQ ID NO: 249 | VWGVPIPVFHHK | ENSG00000067704.8 | 64 |
| SEQ ID NO: 250 | ALLNVVDNAR | ENSG00000105223.14 | 63 |
| SEQ ID NO: 251 | GGTTNPHIFPEGSEPK | ENSG00000167770.7 | 63 |
| SEQ ID NO: 252 | YTVNFLEAK | ENSG00000142453.7 | 63 |
| SEQ ID NO: 253 | ATIQGVLR | ENSG00000196961.8 | 62 |
| SEQ ID NO: 254 | GPLGDQYQTVK | ENSG00000172037.9 | 62 |
| SEQ ID NO: 255 | VAAQVDGGAQVQQVLNIECLR | ENSG00000196961.8 | 62 |
| SEQ ID NO: 256 | FTPVVCGLR | ENSG00000090006.13 | 61 |
| SEQ ID NO: 257 | LFPNSLDQTDMHGDSEYNIMFG PDICGPGTK | ENSG00000179218.9 | 61 |
| SEQ ID NO: 258 | TILLSTTDPADFAVAEALEK | ENSG00000130396.16 | 61 |
| SEQ ID NO: 259 | LTYLGCASVNAPR | ENSG00000011454.12 | 60 |
| SEQ ID NO: 260 | SCYLSSLDLLLEHR | ENSG00000133315.6 | 60 |
| SEQ ID NO: 261 | VVATTQMQAADAR | ENSG00000166825.9 | 60 |
| SEQ ID NO: 262 | GVGGSQPPDIDKTELVEPTEYLV VHLK | ENSG00000166825.9 | 59 |
| SEQ ID NO: 263 | KEIHTVPDMGK | ENSG00000119383.15 | 59 |
| SEQ ID NO: 264 | LFTALFPFEK | ENSG00000169896.12 | 59 |
| SEQ ID NO: 265 | SLESALK | ENSG00000130429.8 | 59 |
| SEQ ID NO: 266 | VDDQIAIVFK | ENSG00000119383.15 | 59 |
| SEQ ID NO: 267 | VLDPAIPIPDPYSSR | ENSG00000172037.9 | 59 |
| SEQ ID NO: 268 | ATPFIECNGGR | ENSG00000134871.13 | 58 |
| SEQ ID NO: 269 | CSVCEAPAIAIAVHSQDVSIPHCP AGWR | ENSG00000134871.13 | 58 |
| SEQ ID NO: 270 | EAQVAHADQQLR | ENSG00000137497.13 | 58 |
| SEQ ID NO: 271 | EIILDDDECPLQIFR | ENSG00000130396.16 | 58 |
| SEQ ID NO: 272 | TPAAIPATPVAVSQPIR | ENSG00000130396.16 | 58 |
| SEQ ID NO: 273 | DLGFFGIYK | ENSG00000004864.9 | 57 |
| SEQ ID NO: 274 | EERPAPTPWGSK | ENSG00000130429.8 | 57 |
| SEQ ID NO: 275 | YVGFGNTPPPQK | ENSG00000101199.8 | 57 |
| SEQ ID NO: 276 | CLFQSPLFAK | ENSG00000142453.7 | 56 |
| SEQ ID NO: 277 | SETDTSLIR | ENSG00000146731.6 | 56 |
| SEQ ID NO: 278 | ILETWGELLSK | ENSG00000011454.12 | 54 |
| SEQ ID NO: 279 | YSGLCPHVVVLVATVR | ENSG00000100714.11 | 54 |
| SEQ ID NO: 280 | ENSLLFDPLSSSSSNK | ENSG00000166825.9 | 53 |
| SEQ ID NO: 281 | IKNEAEPEFASR | ENSG00000198947.10 | 53 |
| SEQ ID NO: 282 | VSAPDGPCPTGFER | ENSG00000090006.13 | 53 |
| SEQ ID NO: 283 | AQGIAQGAIR | ENSG00000172037.9 | 52 |
| SEQ ID NO: 284 | KVCGDSDKGFVVINQK | ENSG00000146731.6 | 52 |
| SEQ ID NO: 285 | LWSGYSLLYFEGQEK | ENSG00000134871.13 | 52 |
| SEQ ID NO: 286 | VPIWDQDIQFLPGSQK | ENSG00000133316.11 | 52 |
| SEQ ID NO: 287 | YLSYTLNPDLIR | ENSG00000166825.9 | 52 |
| SEQ ID NO: 288 | YVIGVGDAFR | ENSG00000169896.12 | 52 |
| SEQ ID NO: 289 | DLEVVEGSAAR | ENSG00000065534.14 | 51 |
| SEQ ID NO: 290 | FAVGSGSR | ENSG00000130429.8 | 50 |
| SEQ ID NO: 291 | GFGQSVVQLQGSR | ENSG00000169896.12 | 50 |
| SEQ ID NO: 292 | GLPGEVLGAQPGPR | ENSG00000134871.13 | 50 |
| SEQ ID NO: 293 | LAETLGR | ENSG00000169756.12 | 50 |
| SEQ ID NO: 294 | LPPKVESLESLYFTPIPAR | ENSG00000137497.13 | 50 |
| SEQ ID NO: 295 | PTDSKPEDWDKPEHIPDPDAK | ENSG00000179218.9 | 50 |
| SEQ ID NO: 296 | QLSLPQQEAQK | ENSG00000196961.8 | 50 |
| SEQ ID NO: 297 | DVTTFFSGK | ENSG00000101199.8 | 49 |
| SEQ ID NO: 298 | GQVEQANQELQELIQSVK | ENSG00000172037.9 | 49 |
| SEQ ID NO: 299 | IDDVLHTLTGAMSLLR | ENSG00000130396.16 | 49 |
| SEQ ID NO: 300 | LQLPNCIEDPVSPIVLR | ENSG00000169896.12 | 49 |
| SEQ ID NO: 301 | VESLESLYFTPIPAR | ENSG00000137497.13 | 49 |
| SEQ ID NO: 302 | FGDPLGYEDVIPEADREGVIR | ENSG00000169896.12 | 48 |
| SEQ ID NO: 303 | LEPNAQAQMYR | ENSG00000196961.8 | 48 |
| SEQ ID NO: 304 | DSLEDCVTIWGPEGR | ENSG00000011028.9 | 47 |
| SEQ ID NO: 305 | EAVTEILGIEPDR | ENSG00000211460.7 | 47 |
| SEQ ID NO: 306 | FQNLDKK | ENSG00000130429.8 | 47 |
| SEQ ID NO: 307 | GGECASPLPGLR | ENSG00000090006.13 | 47 |
| SEQ ID NO: 308 | IAVSKPSGPQPQADLQALLQSGA QVR | ENSG00000105223.14 | 47 |
| SEQ ID NO: 309 | VLELSIPASAEQIQHLAGAIAER | ENSG00000172037.9 | 47 |
| SEQ ID NO: 310 | AAPVPTAPAAGAPLMDFGNDFV PPAPR | ENSG00000115310.13 | 46 |
| SEQ ID NO: 311 | GGYTCVCPDGFLLDSSR | ENSG00000090006.13 | 46 |
| SEQ ID NO: 312 | VLLTRPGEGGTGLPGPPLITR | ENSG00000152894.10 | 46 |
| SEQ ID NO: 313 | ELQPQQQPR | ENSG00000130396.16 | 45 |
| SEQ ID NO: 314 | FCQLHSSGARPPAPAVPGLTR | ENSG00000090006.13 | 45 |
| SEQ ID NO: 315 | LAAGDQLLSVDGR | ENSG00000130396.16 | 45 |
| SEQ ID NO: 316 | SLTLDTWEPELLK | ENSG00000114331.8 | 45 |
| SEQ ID NO: 317 | EQVPGFTPR | ENSG00000100714.11 | 44 |
| SEQ ID NO: 318 | ETGVPIAGR | ENSG00000100714.11 | 44 |
| SEQ ID NO: 319 | KITIGQAPTEK | ENSG00000100714.11 | 44 |
| SEQ ID NO: 320 | FSTMPFLYCNPGDVCYYASR | ENSG00000134871.13 | 43 |
| SEQ ID NO: 321 | LLTIGDANGEIQR | ENSG00000142453.7 | 43 |
| SEQ ID NO: 322 | LQSQVISELDACK | ENSG00000132205.6 | 43 |
| SEQ ID NO: 323 | LTILAAR | ENSG00000065534.14 | 43 |
| SEQ ID NO: 324 | LVECLETVLNK | ENSG00000196961.8 | 43 |
| SEQ ID NO: 325 | SSPQFGVTLLTYELLQR | ENSG00000004864.9 | 43 |
| SEQ ID NO: 326 | YQCHEEGLVPSK | ENSG00000172037.9 | 43 |
| SEQ ID NO: 327 | GCQLCPPFGSEGFR | ENSG00000090006.13 | 42 |
| SEQ ID NO: 328 | KPGLEEAVESACAMR | ENSG00000067704.8 | 42 |
| SEQ ID NO: 329 | LVQCVDAFEEK | ENSG00000065534.14 | 42 |
| SEQ ID NO: 330 | QWFINITDIK | ENSG00000067704.8 | 42 |
| SEQ ID NO: 331 | SQLEAIFLR | ENSG00000105223.14 | 42 |
| SEQ ID NO: 332 | VLEGSELELAK | ENSG00000137497.13 | 42 |
| SEQ ID NO: 333 | VVQDLAAR | ENSG00000172037.9 | 42 |
| SEQ ID NO: 334 | AIMEFNPR | ENSG00000169896.12 | 41 |
| SEQ ID NO: 335 | ALAEGGSILSR | ENSG00000172037.9 | 41 |
| SEQ ID NO: 336 | EICPAGPGYHYSASDLR | ENSG00000090006.13 | 41 |
| SEQ ID NO: 337 | EQVVEDRPVGGR | ENSG00000135052.12 | 41 |
| SEQ ID NO: 338 | LYCNPGDVCYYASR | ENSG00000134871.13 | 41 |
| SEQ ID NO: 339 | TQDASGPELILPASIEFR | ENSG00000130396.16 | 41 |
| SEQ ID NO: 340 | YSEIEPSTEGEVIYR | ENSG00000172037.9 | 41 |
| SEQ ID NO: 341 | AWCVNCFACSTCNTK | ENSG00000169756.12 | 40 |
| SEQ ID NO: 342 | DDPTDSKPEDWDKPEHIPDPDA K | ENSG00000179218.9 | 40 |
| SEQ ID NO: 343 | IVQATTLLTMDK | ENSG00000130396.16 | 40 |
| SEQ ID NO: 344 | VDLSTSTDWK | ENSG00000133315.6 | 40 |
| SEQ ID NO: 345 | AQLLQQTR | ENSG00000213380.9 | 39 |
| SEQ ID NO: 346 | DVDECQLFR | ENSG00000090006.13 | 39 |
| SEQ ID NO: 347 | IEGYPDPEVVWFKDDQSIR | ENSG00000065534.14 | 39 |
| SEQ ID NO: 348 | LSSMAMISGLSGR | ENSG00000065534.14 | 39 |
| SEQ ID NO: 349 | NNGVLFENQLLQIGVK | ENSG00000196961.8 | 39 |
| SEQ ID NO: 350 | RADPAELR | ENSG00000004864.9 | 39 |
| SEQ ID NO: 351 | SAPASQASLR | ENSG00000137497.13 | 39 |
| SEQ ID NO: 352 | DWEQFEYK | ENSG00000137497.13 | 38 |
| SEQ ID NO: 353 | IQAELAVILK | ENSG00000137497.13 | 38 |
| SEQ ID NO: 354 | SNRDELELELAENRK | ENSG00000137497.13 | 38 |
| SEQ ID NO: 355 | TPVPEKVPPPKPATPDFR | ENSG00000065534.14 | 38 |
| SEQ ID NO: 356 | VSLEPHQGPGTPESK | ENSG00000137497.13 | 38 |
| SEQ ID NO: 357 | CTEPEDQLYYVK | ENSG00000106066.9 | 37 |
| SEQ ID NO: 358 | ECYFDTAAPDACDNILAR | ENSG00000090006.13 | 37 |
| SEQ ID NO: 359 | FGLGSVAGAVGATAVYPIDLVK | ENSG00000004864.9 | 37 |
| SEQ ID NO: 360 | GQEDAILSYEPVTR | ENSG00000082458.7 | 37 |
| SEQ ID NO: 361 | IMELEGR | ENSG00000135052.12 | 37 |
| SEQ ID NO: 362 | TCVSLAVSR | ENSG00000196961.8 | 37 |
| SEQ ID NO: 363 | TILTLTGVSTLGDVK | ENSG00000184207.8 | 37 |
| SEQ ID NO: 364 | VLQIVTNRDDVQGYAAK | ENSG00000196961.8 | 37 |
| SEQ ID NO: 365 | AFGFSHLEALLDDSKELQR | ENSG00000167770.7 | 36 |
| SEQ ID NO: 366 | AGPDSAGIALYSHEDVCVFK | ENSG00000142453.7 | 36 |
| SEQ ID NO: 367 | AQGVLAAQAR | ENSG00000172037.9 | 36 |
| SEQ ID NO: 368 | LPSFQQSCR | ENSG00000213380.9 | 36 |
| SEQ ID NO: 369 | MLSSFLSEDVFK | ENSG00000166825.9 | 36 |
| SEQ ID NO: 370 | DTEQTLYQVQER | ENSG00000172037.9 | 35 |
| SEQ ID NO: 371 | DVEVTKEEFVLAAQK | ENSG00000004864.9 | 35 |
| SEQ ID NO: 372 | INQLSEENGDLSFK | ENSG00000137497.13 | 35 |
| SEQ ID NO: 373 | LNIPATNVFANR | ENSG00000146733.9 | 35 |
| SEQ ID NO: 374 | SLVKPITQLLGR | ENSG00000169896.12 | 35 |
| SEQ ID NO: 375 | YLCEGTESPYQTGQLHPAIR | ENSG00000152894.10 | 35 |
| SEQ ID NO: 376 | ASMQPIQIAEGTGITTR | ENSG00000137497.13 | 34 |
| SEQ ID NO: 377 | IAGALGGLLTPLFLR | ENSG00000064545.10 | 34 |
| SEQ ID NO: 378 | LGASALDSIQEFR | ENSG00000032444.11 | 34 |
| SEQ ID NO: 379 | SGTIFDNFLITNDEAYAEEFGNET WGVTK | ENSG00000179218.9 | 34 |
| SEQ ID NO: 380 | TVLDLQSSLAGVSENLK | ENSG00000132205.6 | 34 |
| SEQ ID NO: 381 | AGPDLASCLDVDECRER | ENSG00000090006.13 | 33 |
| SEQ ID NO: 382 | EGGTAAGAGLDSLHK | ENSG00000130429.8 | 33 |
| SEQ ID NO: 383 | FYEFSQR | ENSG00000153310.14 | 33 |
| SEQ ID NO: 384 | GEWIKPGAIVIDCGINYVPDDK | ENSG00000100714.11 | 33 |
| SEQ ID NO: 385 | NDPYHPDHFNCANCGK | ENSG00000169756.12 | 33 |
| SEQ ID NO: 386 | SLEPHQGPGTPESK | ENSG00000137497.13 | 33 |
| SEQ ID NO: 387 | SLGEENFEVVK | ENSG00000132561.9 | 33 |
| SEQ ID NO: 388 | THIDTVINALK | ENSG00000196961.8 | 33 |
| SEQ ID NO: 389 | VHAELADVLTEAVVDSILAIKK | ENSG00000146731.6 | 33 |
| SEQ ID NO: 390 | VMQHQYQVSNLGQR | ENSG00000169896.12 | 33 |
| SEQ ID NO: 391 | ASFITPVPGGVGPMTVAMLMQ STVESAK | ENSG00000100714.11 | 32 |
| SEQ ID NO: 392 | FEHFIEGGR | ENSG00000167770.7 | 32 |
| SEQ ID NO: 393 | LQQAQLYPIAIFIKPK | ENSG00000082458.7 | 32 |
| SEQ ID NO: 394 | MTLADIER | ENSG00000004864.9 | 32 |
| SEQ ID NO: 395 | TVELLSGVVDQTK | ENSG00000004864.9 | 32 |
| SEQ ID NO: 396 | AMDYDLLLR | ENSG00000172037.9 | 31 |
| SEQ ID NO: 397 | DFGSFDK | ENSG00000112096.12 | 31 |
| SEQ ID NO: 398 | EPAVYFKEQFLDGDGWTSR | ENSG00000179218.9 | 31 |
| SEQ ID NO: 399 | FLINLEGGDIREESSYK | ENSG00000067704.8 | 31 |
| SEQ ID NO: 400 | GEWIKPGAIVIDCGINYVPDDKK PNGR | ENSG00000100714.11 | 31 |
| SEQ ID NO: 401 | HAVVVGR | ENSG00000100714.11 | 31 |
| SEQ ID NO: 402 | LEGDTFLLLIQSLK | ENSG00000104450.8 | 31 |
| SEQ ID NO: 403 | NTSVVDSEPVR | ENSG00000162614.14 | 31 |
| SEQ ID NO: 404 | PGTTDQVPR | ENSG00000113657.8 | 31 |
| SEQ ID NO: 405 | QLDQHLDLLK | ENSG00000172037.9 | 31 |
| SEQ ID NO: 406 | TVIVHGFTLGEK | ENSG00000067704.8 | 31 |
| SEQ ID NO: 407 | YAPDDIPNINSTCFK | ENSG00000130396.16 | 31 |
| SEQ ID NO: 408 | AADLLYAMCDR | ENSG00000196961.8 | 30 |
| SEQ ID NO: 409 | EMGEAFAADIPR | ENSG00000196961.8 | 30 |
| SEQ ID NO: 410 | IQGTLQPHAR | ENSG00000172037.9 | 30 |
| SEQ ID NO: 411 | LPIAVNGSLIYGVCAGK | ENSG00000059691.7 | 30 |
| SEQ ID NO: 412 | VNDDLISEFPHK | ENSG00000082458.7 | 30 |
| SEQ ID NO: 413 | DGGCSLPILR | ENSG00000090006.13 | 29 |
| SEQ ID NO: 414 | ENVDYIIQELR | ENSG00000136631.8 | 29 |
| SEQ ID NO: 415 | GAAVDEYFR | ENSG00000142453.7 | 29 |
| SEQ ID NO: 416 | GETAVPGAPEALR | ENSG00000184207.8 | 29 |
| SEQ ID NO: 417 | ILYSFATAFR | ENSG00000011454.12 | 29 |
| SEQ ID NO: 418 | NVFECNDQVVK | ENSG00000169896.12 | 29 |
| SEQ ID NO: 419 | STGSFVGELMYK | ENSG00000004864.9 | 29 |
| SEQ ID NO: 420 | TIRDLEVVEGSAAR | ENSG00000065534.14 | 29 |
| SEQ ID NO: 421 | TVFEALQAPACHENMVK | ENSG00000196961.8 | 29 |
| SEQ ID NO: 422 | VGLLQYGSTVK | ENSG00000132561.9 | 29 |
| SEQ ID NO: 423 | YVLSNQYRPDISPTER | ENSG00000130396.16 | 29 |
| SEQ ID NO: 424 | AEAELEYNPEHVSR | ENSG00000067704.8 | 28 |
| SEQ ID NO: 425 | ASPDLVPMGEWTAR | ENSG00000196961.8 | 28 |
| SEQ ID NO: 426 | CEACAPGHFGDPSRPGGR | ENSG00000172037.9 | 28 |
| SEQ ID NO: 427 | EDGYSDASGFGYCFR | ENSG00000090006.13 | 28 |
| SEQ ID NO: 428 | GDLIGVVEALTR | ENSG00000032444.11 | 28 |
| SEQ ID NO: 429 | LAILQVGNRDDSNLYINVK | ENSG00000100714.11 | 28 |
| SEQ ID NO: 430 | NDAGQAECSCQVTVDDAPASE NTK | ENSG00000065534.14 | 28 |
| SEQ ID NO: 431 | QNWFEAFEILDK | ENSG00000106066.9 | 28 |
| SEQ ID NO: 432 | SSEGLLATATVPLDLFK | ENSG00000157617.12 | 28 |
| SEQ ID NO: 433 | STTTIGLVQALGAHLYQNVFACV R | ENSG00000100714.11 | 28 |
| SEQ ID NO: 434 | VLVLEMFSGGDAAALER | ENSG00000172037.9 | 28 |
| SEQ ID NO: 435 | KQVAPEKPVK | ENSG00000113387.7 | 27 |
| SEQ ID NO: 436 | LQELEGTYEENER | ENSG00000172037.9 | 27 |
| SEQ ID NO: 437 | LVEQHGSDIWWTLPPEQLLPK | ENSG00000067704.8 | 27 |
| SEQ ID NO: 438 | NPTFMCLALHCIANVGSR | ENSG00000196961.8 | 27 |
| SEQ ID NO: 439 | SSDGRPDSGGTLR | ENSG00000130396.16 | 27 |
| SEQ ID NO: 440 | AAPQPLNLVSSVTLSK | ENSG00000114861.14 | 26 |
| SEQ ID NO: 441 | AVQAQGG ESQQEAQR | ENSG00000137497.13 | 26 |
| SEQ ID NO: 442 | DFLNQEGADPDSIEMVATR | ENSG00000172037.9 | 26 |
| SEQ ID NO: 443 | GQVLDVVER | ENSG00000172037.9 | 26 |
| SEQ ID NO: 444 | LALIQPSR | ENSG00000146733.9 | 26 |
| SEQ ID NO: 445 | LQQDVLQFQK | ENSG00000135052.12 | 26 |
| SEQ ID NO: 446 | LTFEELER | ENSG00000162614.14 | 26 |
| SEQ ID NO: 447 | QVTPLFIHFR | ENSG00000166825.9 | 26 |
| SEQ ID NO: 448 | SFNVQDLLPDHEYK | ENSG00000065534.14 | 26 |
| SEQ ID NO: 449 | SSCISQHVISEAK | ENSG00000090006.13 | 26 |
| SEQ ID NO: 450 | VLQIVTNR | ENSG00000196961.8 | 26 |
| SEQ ID NO: 451 | VVGDVAYDEAK | ENSG00000100714.11 | 26 |
| SEQ ID NO: 452 | ALQSGPPQSR | ENSG00000136231.9 | 25 |
| SEQ ID NO: 453 | ITIGQAPTEK | ENSG00000100714.11 | 25 |
| SEQ ID NO: 454 | KAQGVLAAQAR | ENSG00000172037.9 | 25 |
| SEQ ID NO: 455 | LKENLYPYLGPSTLR | ENSG00000136631.8 | 25 |
| SEQ ID NO: 456 | LPVTINK | ENSG00000196961.8 | 25 |
| SEQ ID NO: 457 | SILTAIPNDDPYFHITK | ENSG00000213380.9 | 25 |
| SEQ ID NO: 458 | SLGNVIHPDVVVNGGQDQSK | ENSG00000067704.8 | 25 |
| SEQ ID NO: 459 | AVQTSIATAYR | ENSG00000114331.8 | 24 |
| SEQ ID NO: 460 | DASKPEDWDER | ENSG00000179218.9 | 24 |
| SEQ ID NO: 461 | IPVSGPFLVK | ENSG00000136231.9 | 24 |
| SEQ ID NO: 462 | LLGPAGLTWER | ENSG00000138162.13 | 24 |
| SEQ ID NO: 463 | LPVEAFSAVFTK | ENSG00000032444.11 | 24 |
| SEQ ID NO: 464 | SEESTTVHSSPGATGTALFPTR | ENSG00000205277.5 | 24 |
| SEQ ID NO: 465 | SEESTTVHSSPGATGTALFPTR | ENSG00000205277.5 | 24 |
| SEQ ID NO: 466 | SEESTTVHSSPGATGTALFPTR | ENSG00000205277.5 | 24 |
| SEQ ID NO: 467 | TKVHAELADVLTEAVVDSILAIK | ENSG00000146731.6 | 24 |
| SEQ ID NO: 468 | YGEGHQAWIIGIVEK | ENSG00000086475.10 | 24 |
| SEQ ID NO: 469 | ADLYLEGK | ENSG00000067704.8 | 23 |
| SEQ ID NO: 470 | CLEEKNEILQGK | ENSG00000137497.13 | 23 |
| SEQ ID NO: 471 | FIFDCVSQEYGINPER | ENSG00000184207.8 | 23 |
| SEQ ID NO: 472 | IHGTEEGQQILK | ENSG00000137497.13 | 23 |
| SEQ ID NO: 473 | KIQTQLQR | ENSG00000166825.9 | 23 |
| SEQ ID NO: 474 | KVVGDVAYDEAK | ENSG00000100714.11 | 23 |
| SEQ ID NO: 475 | LDSISGNLQR | ENSG00000132205.6 | 23 |
| SEQ ID NO: 476 | LFEDLEFQQLER | ENSG00000019144.12 | 23 |
| SEQ ID NO: 477 | SLGNVIHPDVVVNGGQDQSKEP PYGADVLR | ENSG00000067704.8 | 23 |
| SEQ ID NO: 478 | TEVNSGFFYK | ENSG00000146731.6 | 23 |
| SEQ ID NO: 479 | TSAGTFPGSQPQAPASPVLPARP PPPPLPR | ENSG00000090006.13 | 23 |
| SEQ ID NO: 480 | VHSPQQVDFR | ENSG00000065534.14 | 23 |
| SEQ ID NO: 481 | VLTGNTIALVLGGGGAR | ENSG00000032444.11 | 23 |
| SEQ ID NO: 482 | VSALSVVR | ENSG00000004864.9 | 23 |
| SEQ ID NO: 483 | ASLENGVLLCDLINK | ENSG00000136153.15 | 22 |
| SEQ ID NO: 484 | ETLIDVAR | ENSG00000146731.6 | 22 |
| SEQ ID NO: 485 | FESKPQSQEVK | ENSG00000065534.14 | 22 |
| SEQ ID NO: 486 | GHLQIAACPNQDPLQGTTGLIPL LGIDVWEHAYYLQYK | ENSG00000112096.12 | 22 |
| SEQ ID NO: 487 | GICEALEDSDGRQDSPAGELPK | ENSG00000132561.9 | 22 |
| SEQ ID NO: 488 | GYLAPSGDLSLR | ENSG00000090006.13 | 22 |
| SEQ ID NO: 489 | LQSQLLSIEKEVEEYK | ENSG00000106976.14 | 22 |
| SEQ ID NO: 490 | SGQGSDRGSGSRPGIEGDTPR | ENSG00000113657.8 | 22 |
| SEQ ID NO: 491 | VAISTFQK | ENSG00000213380.9 | 22 |
| SEQ ID NO: 492 | GQDIFIIQTIPR | ENSG00000161542.12 | 21 |
| SEQ ID NO: 493 | ITLDAQDVLAHLVQMAFK | ENSG00000130396.16 | 21 |
| SEQ ID NO: 494 | RTEVPPLLLILDR | ENSG00000136631.8 | 21 |
| SEQ ID NO: 495 | SSPPVQFSLLHSK | ENSG00000196961.8 | 21 |
| SEQ ID NO: 496 | SSTGSPTSPLNAEK | ENSG00000065534.14 | 21 |
| SEQ ID NO: 497 | TKFPAEQYYR | ENSG00000211460.7 | 21 |
| SEQ ID NO: 498 | ANFWYQPSFHGVDLSALR | ENSG00000142453.7 | 20 |
| SEQ ID NO: 499 | DAQIAMMQQR | ENSG00000137497.13 | 20 |
| SEQ ID NO: 500 | EHGAFDAVK | ENSG00000100714.11 | 20 |
| SEQ ID NO: 501 | GLAQADGTLITCVDSGILR | ENSG00000133316.11 | 20 |
| SEQ ID NO: 502 | GLNCEQCQDFYR | ENSG00000172037.9 | 20 |
| SEQ ID NO: 503 | KVVATTQMQAADAR | ENSG00000166825.9 | 20 |
| SEQ ID NO: 504 | MKLTHSLQEELEK | ENSG00000151914.13 | 20 |
| SEQ ID NO: 505 | NIDVFNVEDQKR | ENSG00000135052.12 | 20 |
| SEQ ID NO: 506 | QASDKDDRPFQGEDVENSR | ENSG00000130396.16 | 20 |
| SEQ ID NO: 507 | SLDQTDMHGDSEYNIMFGPDIC GPGTK | ENSG00000179218.9 | 20 |
| SEQ ID NO: 508 | STIFHSSPDASGTTPSSAHSTTSG R | ENSG00000205277.5 | 20 |
| SEQ ID NO: 509 | STIFHSSPDASGTTPSSAHSTTSG R | ENSG00000205277.5 | 20 |
| SEQ ID NO: 510 | STIFHSSPDASGTTPSSAHSTTSG R | ENSG00000205277.5 | 20 |
| SEQ ID NO: 511 | STIFHSSPDASGTTPSSAHSTTSG R | ENSG00000205277.5 | 20 |
| SEQ ID NO: 512 | VCLHVQK | ENSG00000169896.12 | 20 |
| SEQ ID NO: 513 | VSQFLQVLETDLYR | ENSG00000213380.9 | 20 |
| SEQ ID NO: 514 | VSSTATTQDVIETLAEK | ENSG00000130396.16 | 20 |
| SEQ ID NO: 515 | YNTRPLGQEPPR | ENSG00000090006.13 | 20 |
| SEQ ID NO: 516 | ANHPMDAEVTK | ENSG00000196961.8 | 19 |
| SEQ ID NO: 517 | ASELGHSLNENVLKPAQEK | ENSG00000101199.8 | 19 |
| SEQ ID NO: 518 | AWVSHDSTVCLADADKK | ENSG00000130429.8 | 19 |
| SEQ ID NO: 519 | FSYDLSQCINQMK | ENSG00000135052.12 | 19 |
| SEQ ID NO: 520 | IYQFTAASPK | ENSG00000005020.8 | 19 |
| SEQ ID NO: 521 | KQDEPIDLFMIEIMEMK | ENSG00000146731.6 | 19 |
| SEQ ID NO: 522 | NIMAGLQQTNSEK | ENSG00000198947.10 | 19 |
| SEQ ID NO: 523 | RPDYLK | ENSG00000112096.12 | 19 |
| SEQ ID NO: 524 | SEESTTVHSSPVATATTPSPAR | ENSG00000205277.5 | 19 |
| SEQ ID NO: 525 | SEESTTVHSSPVATATTPSPAR | ENSG00000205277.5 | 19 |
| SEQ ID NO: 526 | SEESTTVHSSPVATATTPSPAR | ENSG00000205277.5 | 19 |
| SEQ ID NO: 527 | SEESTTVHSSPVATATTPSPAR | ENSG00000205277.5 | 19 |
| SEQ ID NO: 528 | THLTSLK | ENSG00000211460.7 | 19 |
| SEQ ID NO: 529 | AQEAEQLLR | ENSG00000172037.9 | 18 |
| SEQ ID NO: 530 | AQIINDAFNLASAHK | ENSG00000166825.9 | 18 |
| SEQ ID NO: 531 | DQLGGWFQSSLLTSVAAR | ENSG00000067704.8 | 18 |
| SEQ ID NO: 532 | GADDIELLPEAQHK | ENSG00000100714.11 | 18 |
| SEQ ID NO: 533 | GFSHLEALLDDSK | ENSG00000167770.7 | 18 |
| SEQ ID NO: 534 | GLLTDSPAATVLAEAR | ENSG00000019144.12 | 18 |
| SEQ ID NO: 535 | HSNFLGAYDSIR | ENSG00000172037.9 | 18 |
| SEQ ID NO: 536 | KNEFQGELEK | ENSG00000135052.12 | 18 |
| SEQ ID NO: 537 | SFLEEVLASGLHSR | ENSG00000136631.8 | 18 |
| SEQ ID NO: 538 | TEILGIEPDREK | ENSG00000211460.7 | 18 |
| SEQ ID NO: 539 | VILLDPSIIEAK | ENSG00000104450.8 | 18 |
| SEQ ID NO: 540 | AETVQAALEEAQR | ENSG00000172037.9 | 17 |
| SEQ ID NO: 541 | AFVENYPQFK | ENSG00000136631.8 | 17 |
| SEQ ID NO: 542 | DFISNLLK | ENSG00000065534.14 | 17 |
| SEQ ID NO: 543 | DGFFGLSISDR | ENSG00000172037.9 | 17 |
| SEQ ID NO: 544 | DHVFQVNNFEALK | ENSG00000169896.12 | 17 |
| SEQ ID NO: 545 | DPTDSKPEDWDKPEHIPDPDAK | ENSG00000179218.9 | 17 |
| SEQ ID NO: 546 | KIIELK | ENSG00000146731.6 | 17 |
| SEQ ID NO: 547 | LCCPVALAQDVTGALEDALAK | ENSG00000213380.9 | 17 |
| SEQ ID NO: 548 | PAIAHLIHSLNPVR | ENSG00000106066.9 | 17 |
| SEQ ID NO: 549 | PSSTPTTHFSASSTTLGR | ENSG00000205277.5 | 17 |
| SEQ ID NO: 550 | PSSTPTTHFSASSTTLGR | ENSG00000205277.5 | 17 |
| SEQ ID NO: 551 | PSSTPTTHFSASSTTLGR | ENSG00000205277.5 | 17 |
| SEQ ID NO: 552 | PSSTPTTHFSASSTTLGR | ENSG00000205277.5 | 17 |
| SEQ ID NO: 553 | PSSTPTTHFSASSTTLGR | ENSG00000205277.5 | 17 |
| SEQ ID NO: 554 | PSSTPTTHFSASSTTLGR | ENSG00000205277.5 | 17 |
| SEQ ID NO: 555 | PSSTPTTHFSASSTTLGR | ENSG00000205277.5 | 17 |
| SEQ ID NO: 556 | QFVTGIIDSLTISPK | ENSG00000132561.9 | 17 |
| SEQ ID NO: 557 | SEAVLQSPEFAIFR | ENSG00000198947.10 | 17 |
| SEQ ID NO: 558 | TTQGLTALLLSLK | ENSG00000136631.8 | 17 |
| SEQ ID NO: 559 | VPLSVQLKPEVSPTQDIR | ENSG00000125826.15 | 17 |
| SEQ ID NO: 560 | VTAIDFR | ENSG00000004864.9 | 17 |
| SEQ ID NO: 561 | YLIFPNPVCLEPGISYK | ENSG00000172037.9 | 17 |
| SEQ ID NO: 562 | YRLPNTLKPDSYR | ENSG00000166825.9 | 17 |
| SEQ ID NO: 563 | AFLLSLAALR | ENSG00000105223.14 | 16 |
| SEQ ID NO: 564 | DLAQYSSNDAVVETSLTK | ENSG00000114331.8 | 16 |
| SEQ ID NO: 565 | DRLPQEPGREQVVEDRPVGGR | ENSG00000135052.12 | 16 |
| SEQ ID NO: 566 | EAIQHPADEKLQEK | ENSG00000153310.14 | 16 |
| SEQ ID NO: 567 | EFQNNPNPR | ENSG00000169896.12 | 16 |
| SEQ ID NO: 568 | ELSAALQDKK | ENSG00000137497.13 | 16 |
| SEQ ID NO: 569 | ELSGSGLER | ENSG00000213380.9 | 16 |
| SEQ ID NO: 570 | ELWILNR | ENSG00000166825.9 | 16 |
| SEQ ID NO: 571 | FSTEYELQQLEQFKK | ENSG00000166825.9 | 16 |
| SEQ ID NO: 572 | GPALCGSQR | ENSG00000090006.13 | 16 |
| SEQ ID NO: 573 | GPLEPGPPKPGVPQEPGR | ENSG00000125826.15 | 16 |
| SEQ ID NO: 574 | GSLYQCDYSTGSCEPIR | ENSG00000169896.12 | 16 |
| SEQ ID NO: 575 | IQTQLQR | ENSG00000166825.9 | 16 |
| SEQ ID NO: 576 | KNSSIIGDYKQICSQLSER | ENSG00000011454.12 | 16 |
| SEQ ID NO: 577 | LEINFEELLK | ENSG00000162614.14 | 16 |
| SEQ ID NO: 578 | LIVPEPDVDFDAK | ENSG00000132205.6 | 16 |
| SEQ ID NO: 579 | LVGPEGFVVTEAGFGADIGMEK | ENSG00000100714.11 | 16 |
| SEQ ID NO: 580 | QEHCGCYTLLVENK | ENSG00000065534.14 | 16 |
| SEQ ID NO: 581 | RSQAGVSSGAPPGR | ENSG00000137497.13 | 16 |
| SEQ ID NO: 582 | SPGSTPTTHFPASSTTSGHSEK | ENSG00000205277.5 | 16 |
| SEQ ID NO: 583 | SPGSTPTTHFPASSTTSGHSEK | ENSG00000205277.5 | 16 |
| SEQ ID NO: 584 | SPGSTPTTHFPASSTTSGHSEK | ENSG00000205277.5 | 16 |
| SEQ ID NO: 585 | SPGSTPTTHFPASSTTSGHSEK | ENSG00000205277.5 | 16 |
| SEQ ID NO: 586 | VLSQIDVAQK | ENSG00000198947.10 | 16 |
| SEQ ID NO: 587 | YGGMFCNVEGAFESK | ENSG00000113657.8 | 16 |
| SEQ ID NO: 588 | ATVVVEATEPEPSGSIANPAASTS PSLSHR | ENSG00000131711.10 | 15 |
| SEQ ID NO: 589 | EMTADVIELK | ENSG00000067704.8 | 15 |
| SEQ ID NO: 590 | GEQGFMGNTGPTGAVGDRGPK | ENSG00000134871.13 | 15 |
| SEQ ID NO: 591 | LAEAELEYNPEHVSR | ENSG00000067704.8 | 15 |
| SEQ ID NO: 592 | LESEEDVSQAFLEAVAEEKPHVK PYFSK | ENSG00000065534.14 | 15 |
| SEQ ID NO: 593 | LMCELGNDVINR | ENSG00000114331.8 | 15 |
| SEQ ID NO: 594 | QQQDYWLIDVR | ENSG00000166825.9 | 15 |
| SEQ ID NO: 595 | SSEGGTAAGAGLDSLHK | ENSG00000130429.8 | 15 |
| SEQ ID NO: 596 | SYKPVFWSPSSR | ENSG00000067704.8 | 15 |
| SEQ ID NO: 597 | TAHLDEEVNKGDILVVATGQPE MVK | ENSG00000100714.11 | 15 |
| SEQ ID NO: 598 | TRPDGNCFYR | ENSG00000167770.7 | 15 |
| SEQ ID NO: 599 | TSGQCLCR | ENSG00000172037.9 | 15 |
| SEQ ID NO: 600 | AFCVANK | ENSG00000114331.8 | 14 |
| SEQ ID NO: 601 | AMISGLSGR | ENSG00000065534.14 | 14 |
| SEQ ID NO: 602 | AVESSKPLSNAQPSGPLKPVGN | ENSG00000065534.14 | 14 |
| SEQ ID NO: 603 | AYHSFLVEPISCHAWNKDR | ENSG00000130429.8 | 14 |
| SEQ ID NO: 604 | EGVVDIYNCVK | ENSG00000152894.10 | 14 |
| SEQ ID NO: 605 | GTWIHPEIDNPEYSPDPSIYAYD NFGVLGLDLWQVK | ENSG00000179218.9 | 14 |
| SEQ ID NO: 606 | HLTQAVCTVK | ENSG00000141447.12 | 14 |
| SEQ ID NO: 607 | ITISPLQELTLYNPER | ENSG00000136231.9 | 14 |
| SEQ ID NO: 608 | LACESASSTEVSGALK | ENSG00000169896.12 | 14 |
| SEQ ID NO: 609 | LDCTQCLQHPWLMK | ENSG00000065534.14 | 14 |
| SEQ ID NO: 610 | LDEEAENLVATVVPTHLAAAVPE VAVYLK | ENSG00000119383.15 | 14 |
| SEQ ID NO: 611 | LPEDDEPPARPPPPPPASVSPQA EPVWTPPAPAPAAPPSTPAAPK | ENSG00000115310.13 | 14 |
| SEQ ID NO: 612 | LPNTLKPDSYR | ENSG00000166825.9 | 14 |
| SEQ ID NO: 613 | LSSTQQSLAEK | ENSG00000082805.15 | 14 |
| SEQ ID NO: 614 | LVALETGIQK | ENSG00000019144.12 | 14 |
| SEQ ID NO: 615 | MHGGGPTVTAGLPLPK | ENSG00000100714.11 | 14 |
| SEQ ID NO: 616 | QQALELVVQEVSSVLR | ENSG00000157617.12 | 14 |
| SEQ ID NO: 617 | QSMAFSILNTPK | ENSG00000137497.13 | 14 |
| SEQ ID NO: 618 | SSNLLDLK | ENSG00000142453.7 | 14 |
| SEQ ID NO: 619 | VLQDQLK | ENSG00000135052.12 | 14 |
| SEQ ID NO: 620 | WVSHDSTVCLADADKK | ENSG00000130429.8 | 14 |
| SEQ ID NO: 621 | AAQLDGLEAR | ENSG00000172037.9 | 13 |
| SEQ ID NO: 622 | ANALASATCER | ENSG00000169756.12 | 13 |
| SEQ ID NO: 623 | ATDNEPSQFSEPR | ENSG00000132205.6 | 13 |
| SEQ ID NO: 624 | CGFSELYSWQR | ENSG00000067704.8 | 13 |
| SEQ ID NO: 625 | DLLQAAQDK | ENSG00000172037.9 | 13 |
| SEQ ID NO: 626 | EPAPASPAPAGVEIR | ENSG00000113657.8 | 13 |
| SEQ ID NO: 627 | EYELFEFR | ENSG00000136631.8 | 13 |
| SEQ ID NO: 628 | HKPGIVQETTFDLGGDIHSGTAL PTSK | ENSG00000130396.16 | 13 |
| SEQ ID NO: 629 | IWDLQGSEEPVFR | ENSG00000133316.11 | 13 |
| SEQ ID NO: 630 | LFGDVEASLGR | ENSG00000213380.9 | 13 |
| SEQ ID NO: 631 | LHTLGDNLLDPR | ENSG00000172037.9 | 13 |
| SEQ ID NO: 632 | RFSDIQIR | ENSG00000100714.11 | 13 |
| SEQ ID NO: 633 | SEVYGPMK | ENSG00000166825.9 | 13 |
| SEQ ID NO: 634 | SLSESAATR | ENSG00000159788.14 | 13 |
| SEQ ID NO: 635 | VTCVEMEPLAEYVVR | ENSG00000152894.10 | 13 |
| SEQ ID NO: 636 | YLFEEDNLLR | ENSG00000132561.9 | 13 |
| SEQ ID NO: 637 | AAECLDVDECHR | ENSG00000090006.13 | 12 |
| SEQ ID NO: 638 | AGMSSLKG | ENSG00000146731.6 | 12 |
| SEQ ID NO: 639 | ALASATCER | ENSG00000169756.12 | 12 |
| SEQ ID NO: 640 | CDSHDDPALGLVSGQCR | ENSG00000172037.9 | 12 |
| SEQ ID NO: 641 | DCSIALPYVCK | ENSG00000011028.9 | 12 |
| SEQ ID NO: 642 | DISLQGPGLAPEHCYIENLR | ENSG00000019144.12 | 12 |
| SEQ ID NO: 643 | FVLDHEDGLNLNEDLENFLQK | ENSG00000137497.13 | 12 |
| SEQ ID NO: 644 | GANQHATDEEGKDPLSIAVEAA NADIVTLLR | ENSG00000114331.8 | 12 |
| SEQ ID NO: 645 | GFSHLEALLDDSKELQR | ENSG00000167770.7 | 12 |
| SEQ ID NO: 646 | GSGVSNFAQLIVR | ENSG00000152894.10 | 12 |
| SEQ ID NO: 647 | IINDAFNLASAHK | ENSG00000166825.9 | 12 |
| SEQ ID NO: 648 | KVVQSLEQTAR | ENSG00000211460.7 | 12 |
| SEQ ID NO: 649 | QPAVEEPAEVTATVLASR | ENSG00000076662.5 | 12 |
| SEQ ID NO: 650 | QTQVLGLTQTCETLK | ENSG00000169896.12 | 12 |
| SEQ ID NO: 651 | RVEDAYILTCNVSLEYEK | ENSG00000146731.6 | 12 |
| SEQ ID NO: 652 | TLDFDALSVGQR | ENSG00000113657.8 | 12 |
| SEQ ID NO: 653 | VVNAMGK | ENSG00000169756.12 | 12 |
| SEQ ID NO: 654 | AKIDDPTDSKPEDWDKPEHIPD | ENSG00000179218.9 | 11 |
| SEQ ID NO: 655 | ALEQLLTELDDFLK | ENSG00000169129.10 | 11 |
| SEQ ID NO: 656 | ASKPEDWDER | ENSG00000179218.9 | 11 |
| SEQ ID NO: 657 | DLNQLFQQDSSSR | ENSG00000082805.15 | 11 |
| SEQ ID NO: 658 | ETPGRPPDPTGAPLPGPTGDPVK PTSLETPSAPLLSR | ENSG00000032444.11 | 11 |
| SEQ ID NO: 659 | GSACEEDVDECAQEPPPCGPGR | ENSG00000090006.13 | 11 |
| SEQ ID NO: 660 | KASSEGGTAAGAGLDSLHK | ENSG00000130429.8 | 11 |
| SEQ ID NO: 661 | LGFITNNSSK | ENSG00000184207.8 | 11 |
| SEQ ID NO: 662 | LPSHSDFLAELR | ENSG00000169896.12 | 11 |
| SEQ ID NO: 663 | LQDVHVAEGK | ENSG00000065534.14 | 11 |
| SEQ ID NO: 664 | LVTCTGYHQVR | ENSG00000133316.11 | 11 |
| SEQ ID NO: 665 | SIQLPTTVR | ENSG00000166825.9 | 11 |
| SEQ ID NO: 666 | VLSELGR | ENSG00000067704.8 | 11 |
| SEQ ID NO: 667 | WAPNENKFAVGSGSR | ENSG00000130429.8 | 11 |
| SEQ ID NO: 668 | AQELQQTGVLGAFESSFWHMQ EK | ENSG00000172037.9 | 10 |
| SEQ ID NO: 669 | ASAAAAAGGGATGHPGGGQGA ENPAGLK | ENSG00000104450.8 | 10 |
| SEQ ID NO: 670 | EAENFHEEDDVDVRPAR | ENSG00000162614.14 | 10 |
| SEQ ID NO: 671 | ERLPSHSDFLAELR | ENSG00000169896.12 | 10 |
| SEQ ID NO: 672 | EWSLESSPAQNWTPPQPR | ENSG00000101199.8 | 10 |
| SEQ ID NO: 673 | FYALSASFEPFSNKG | ENSG00000179218.9 | 10 |
| SEQ ID NO: 674 | GISLNPEQWSQLKEQISDIDDAV R | ENSG00000113387.7 | 10 |
| SEQ ID NO: 675 | HPLLVGHMPVMVAK | ENSG00000104728.11 | 10 |
| SEQ ID NO: 676 | IAHGNSSIIADR | ENSG00000100714.11 | 10 |
| SEQ ID NO: 677 | IYADSLKPNIPYK | ENSG00000130396.16 | 10 |
| SEQ ID NO: 678 | LAILDSQAGQIR | ENSG00000019144.12 | 10 |
| SEQ ID NO: 679 | NMVVDDDSPEMYK | ENSG00000162614.14 | 10 |
| SEQ ID NO: 680 | NRLDCTQCLQHPWLMK | ENSG00000065534.14 | 10 |
| SEQ ID NO: 681 | PVLLQVAESAYR | ENSG00000004864.9 | 10 |
| SEQ ID NO: 682 | QEPLGSDSEGVNCLAYDEAIMA QQDR | ENSG00000167770.7 | 10 |
| SEQ ID NO: 683 | QEVEELWIGLNDLK | ENSG00000011028.9 | 10 |
| SEQ ID NO: 684 | SFVIHNLPVLAK | ENSG00000086475.10 | 10 |
| SEQ ID NO: 685 | STTFHSSPR | ENSG00000205277.5 | 10 |
| SEQ ID NO: 686 | STTFHSSPR | ENSG00000205277.5 | 10 |
| SEQ ID NO: 687 | STTFHSSPR | ENSG00000205277.5 | 10 |
| SEQ ID NO: 688 | TAAGLMHTFNAHAATDITGFGIL GHAQNLAK | ENSG00000086475.10 | 10 |
| SEQ ID NO: 689 | TGAFGLR | ENSG00000172037.9 | 10 |
| SEQ ID NO: 690 | TSLTVVLLR | ENSG00000076662.5 | 10 |
| SEQ ID NO: 691 | VPPLLIYGPFGTGK | ENSG00000130589.12 | 10 |
| SEQ ID NO: 692 | VPSFAAGR | ENSG00000136231.9 | 10 |
| SEQ ID NO: 693 | VPVGDQPPDIEFQIR | ENSG00000106976.14 | 10 |
| SEQ ID NO: 694 | VYDPASPQR | ENSG00000133316.11 | 10 |
| SEQ ID NO: 695 | WFYIDFGGVKPMGSEPVPK | ENSG00000004864.9 | 10 |
| SEQ ID NO: 696 | WTPPAPAPAAPPSTPAAPK | ENSG00000115310.13 | 10 |
| SEQ ID NO: 697 | YDNQWFHGCTSTGR | ENSG00000011028.9 | 10 |
| SEQ ID NO: 698 | YFSYDCGADFPGVPLAPPR | ENSG00000172037.9 | 10 |
| SEQ ID NO: 699 | YGDEEKDKGLQTSQDAR | ENSG00000179218.9 | 10 |
| SEQ ID NO: 700 | YLETADYAIR | ENSG00000196961.8 | 10 |
| SEQ ID NO: 701 | AKQPDLAPGLTTIGASPTQTVTL VTQPVVTK | ENSG00000198947.10 | 9 |
| SEQ ID NO: 702 | ASPLLPANHVTMAK | ENSG00000067704.8 | 9 |
| SEQ ID NO: 703 | AVLELLQRPGNAR | ENSG00000105963.9 | 9 |
| SEQ ID NO: 704 | CFQVQGQEPQSR | ENSG00000011028.9 | 9 |
| SEQ ID NO: 705 | DKGLQTSQDAR | ENSG00000179218.9 | 9 |
| SEQ ID NO: 706 | DLTALSNMLPK | ENSG00000166825.9 | 9 |
| SEQ ID NO: 707 | DPFSLDALSK | ENSG00000146731.6 | 9 |
| SEQ ID NO: 708 | FGDPLGYEDVIPEADR | ENSG00000169896.12 | 9 |
| SEQ ID NO: 709 | FGLYLPLFK | ENSG00000004864.9 | 9 |
| SEQ ID NO: 710 | FSTEYELQQLEQFK | ENSG00000166825.9 | 9 |
| SEQ ID NO: 711 | GAVYLFHGTSGSGISPSHSQR | ENSG00000169896.12 | 9 |
| SEQ ID NO: 712 | HLCELLAQQF | ENSG00000196961.8 | 9 |
| SEQ ID NO: 713 | ILDQENLSSTALVK | ENSG00000169129.10 | 9 |
| SEQ ID NO: 714 | ISETTMLQSGMK | ENSG00000130396.16 | 9 |
| SEQ ID NO: 715 | ISYHGSCPQGLADSAWIPFR | ENSG00000011028.9 | 9 |
| SEQ ID NO: 716 | KQNWFEAFEILDK | ENSG00000106066.9 | 9 |
| SEQ ID NO: 717 | PISLVFLVPVR | ENSG00000169896.12 | 9 |
| SEQ ID NO: 718 | SKESSQVTSR | ENSG00000136631.8 | 9 |
| SEQ ID NO: 719 | SPPPCTYGR | ENSG00000090006.13 | 9 |
| SEQ ID NO: 720 | SQLNCLLLSGR | ENSG00000133316.11 | 9 |
| SEQ ID NO: 721 | TPLSAAAHTHPVYCVNVVGTQN AHNLITVSTDGK | ENSG00000158560.10 | 9 |
| SEQ ID NO: 722 | VNYDEENWR | ENSG00000166825.9 | 9 |
| SEQ ID NO: 723 | VSFVIHNLPVLAK | ENSG00000086475.10 | 9 |
| SEQ ID NO: 724 | VTLRPYLTPNDR | ENSG00000166825.9 | 9 |
| SEQ ID NO: 725 | WNVINWENVTER | ENSG00000112096.12 | 9 |
| SEQ ID NO: 726 | ADTDGGLIFR | ENSG00000163975.7 | 8 |
| SEQ ID NO: 727 | AGYTGLR | ENSG00000172037.9 | 8 |
| SEQ ID NO: 728 | AVESSKPLSNAQPSGPLKPVGNA K | ENSG00000065534.14 | 8 |
| SEQ ID NO: 729 | CSEGFVLAEDGRR | ENSG00000132561.9 | 8 |
| SEQ ID NO: 730 | DLMVLNDVYR | ENSG00000166825.9 | 8 |
| SEQ ID NO: 731 | FPAEQYYR | ENSG00000211460.7 | 8 |
| SEQ ID NO: 732 | FTGHCSCRPGVSGVR | ENSG00000172037.9 | 8 |
| SEQ ID NO: 733 | GDPGDTGAPGPVGMK | ENSG00000134871.13 | 8 |
| SEQ ID NO: 734 | GGPSLSSVLNELPSAATLR | ENSG00000167608.7 | 8 |
| SEQ ID NO: 735 | IKDPDASKPEDWDERAK | ENSG00000179218.9 | 8 |
| SEQ ID NO: 736 | ILCIGAVPGLQPR | ENSG00000110237.3 | 8 |
| SEQ ID NO: 737 | IQSDLTSHEISLEEMKK | ENSG00000198947.10 | 8 |
| SEQ ID NO: 738 | ITGHFYACQVAQR | ENSG00000136231.9 | 8 |
| SEQ ID NO: 739 | KVVGDVAYDEAKER | ENSG00000100714.11 | 8 |
| SEQ ID NO: 740 | LDTDILLGATCGLK | ENSG00000184207.8 | 8 |
| SEQ ID NO: 741 | LVSAVVEYGGK | ENSG00000136631.8 | 8 |
| SEQ ID NO: 742 | MLGVAAGMTHSNMANALASAT CER | ENSG00000169756.12 | 8 |
| SEQ ID NO: 743 | NIPNGLQEFLDPLCQR | ENSG00000130396.16 | 8 |
| SEQ ID NO: 744 | QADIIGKPSR | ENSG00000184207.8 | 8 |
| SEQ ID NO: 745 | QEISIMNCLHHPK | ENSG00000065534.14 | 8 |
| SEQ ID NO: 746 | QIVSEMLR | ENSG00000196961.8 | 8 |
| SEQ ID NO: 747 | RAEQLLQDAR | ENSG00000172037.9 | 8 |
| SEQ ID NO: 748 | RFENAPDSAK | ENSG00000082805.15 | 8 |
| SEQ ID NO: 749 | SGAPWFK | ENSG00000162614.14 | 8 |
| SEQ ID NO: 750 | SIVEHVASK | ENSG00000146733.9 | 8 |
| SEQ ID NO: 751 | SLVGLSQER | ENSG00000130396.16 | 8 |
| SEQ ID NO: 752 | TVNELQNLSSAEVVVPR | ENSG00000136231.9 | 8 |
| SEQ ID NO: 753 | VIAVVNK | ENSG00000130396.16 | 8 |
| SEQ ID NO: 754 | VSHSELR | ENSG00000146733.9 | 8 |
| SEQ ID NO: 755 | WSDGVGFSYHNFDR | ENSG00000011028.9 | 8 |
| SEQ ID NO: 756 | YGADDIELLPEAQHK | ENSG00000100714.11 | 8 |
| SEQ ID NO: 757 | AKPEASFQVWNK | ENSG00000073849.10 | 7 |
| SEQ ID NO: 758 | ALQLSNSPGASSAFLK | ENSG00000170776.15 | 7 |
| SEQ ID NO: 759 | ASSEGGTAAGAGLDSLHKNSVS QISVLSGGK | ENSG00000130429.8 | 7 |
| SEQ ID NO: 760 | AVEMAAQR | ENSG00000184207.8 | 7 |
| SEQ ID NO: 761 | AVLELLQR | ENSG00000105963.9 | 7 |
| SEQ ID NO: 762 | AYAQQLADWAR | ENSG00000165912.11 | 7 |
| SEQ ID NO: 763 | DHSAIPVINR | ENSG00000166825.9 | 7 |
| SEQ ID NO: 764 | DLRDPAVCR | ENSG00000172037.9 | 7 |
| SEQ ID NO: 765 | FGSCVPHTTRPR | ENSG00000082458.7 | 7 |
| SEQ ID NO: 766 | GPQYGTLEK | ENSG00000165912.11 | 7 |
| SEQ ID NO: 767 | HWDDVVCESR | ENSG00000172037.9 | 7 |
| SEQ ID NO: 768 | IVLYQTDASLTPWTVR | ENSG00000032444.11 | 7 |
| SEQ ID NO: 769 | KVHSPQQVDFR | ENSG00000065534.14 | 7 |
| SEQ ID NO: 770 | LCTDHGSQLVTITNR | ENSG00000011028.9 | 7 |
| SEQ ID NO: 771 | LDFLPDMMVEGR | ENSG00000048740.13 | 7 |
| SEQ ID NO: 772 | LEAVAEEKPHVKPYFSK | ENSG00000065534.14 | 7 |
| SEQ ID NO: 773 | LEVDAIVNAANSSLLGGGGVDG CIHR | ENSG00000133315.6 | 7 |
| SEQ ID NO: 774 | LLHEMQIQHPTASLIAK | ENSG00000146731.6 | 7 |
| SEQ ID NO: 775 | LLVEELPLR | ENSG00000198947.10 | 7 |
| SEQ ID NO: 776 | LMNSQLVTTEK | ENSG00000073849.10 | 7 |
| SEQ ID NO: 777 | LSNPPSAGPIVVHCSAGAGR | ENSG00000152894.10 | 7 |
| SEQ ID NO: 778 | LSPSSTETTTLPGSPTTPSLSEK | ENSG00000205277.5 | 7 |
| SEQ ID NO: 779 | LSPSSTETTTLPGSPTTPSLSEK | ENSG00000205277.5 | 7 |
| SEQ ID NO: 780 | LSPSSTETTTLPGSPTTPSLSEK | ENSG00000205277.5 | 7 |
| SEQ ID NO: 781 | LSPSSTETTTLPGSPTTPSLSEK | ENSG00000205277.5 | 7 |
| SEQ ID NO: 782 | MYLFYGNK | ENSG00000196961.8 | 7 |
| SEQ ID NO: 783 | PPLLLILDR | ENSG00000136631.8 | 7 |
| SEQ ID NO: 784 | PSLSLGTITDEEMK | ENSG00000137497.13 | 7 |
| SEQ ID NO: 785 | QCHECIEHIR | ENSG00000106066.9 | 7 |
| SEQ ID NO: 786 | QQNQELQEQLR | ENSG00000137497.13 | 7 |
| SEQ ID NO: 787 | SFAPILPHLAEEVFQHIPY | ENSG00000067704.8 | 7 |
| SEQ ID NO: 788 | SGLCPHVVVLVATVR | ENSG00000100714.11 | 7 |
| SEQ ID NO: 789 | SITILSTPEGTSAACK | ENSG00000136231.9 | 7 |
| SEQ ID NO: 790 | SLEGSDDAVLLQR | ENSG00000198947.10 | 7 |
| SEQ ID NO: 791 | SMDAETYVEGQR | ENSG00000130396.16 | 7 |
| SEQ ID NO: 792 | STTSGLVGESTPSR | ENSG00000205277.5 | 7 |
| SEQ ID NO: 793 | STTSGLVGESTPSR | ENSG00000205277.5 | 7 |
| SEQ ID NO: 794 | STTSGLVGESTPSR | ENSG00000205277.5 | 7 |
| SEQ ID NO: 795 | STTSGLVGESTPSR | ENSG00000205277.5 | 7 |
| SEQ ID NO: 796 | TQGSSTSWFGSNQSKPEFTVDLK | ENSG00000165322.13 | 7 |
| SEQ ID NO: 797 | VIMIVTDGRPQDSVAEVAAK | ENSG00000132561.9 | 7 |
| SEQ ID NO: 798 | VPPPKPATPDFR | ENSG00000065534.14 | 7 |
| SEQ ID NO: 799 | WGFCPIK | ENSG00000011028.9 | 7 |
| SEQ ID NO: 800 | YAVQVAEGMGYLESKR | ENSG00000061938.12 | 7 |
| SEQ ID NO: 801 | AAEEIGIKATHIKLPR | ENSG00000100714.11 | 6 |
| SEQ ID NO: 802 | AGDAVNVVVTGGK | ENSG00000132205.6 | 6 |
| SEQ ID NO: 803 | AGDTLSGTCLLIANK | ENSG00000142453.7 | 6 |
| SEQ ID NO: 804 | AGDTLSGTCLLIANKR | ENSG00000142453.7 | 6 |
| SEQ ID NO: 805 | AIDYEIQR | ENSG00000059691.7 | 6 |
| SEQ ID NO: 806 | ALEQALEK | ENSG00000166825.9 | 6 |
| SEQ ID NO: 807 | ALSSAGER | ENSG00000172037.9 | 6 |
| SEQ ID NO: 808 | CFLCDSR | ENSG00000172037.9 | 6 |
| SEQ ID NO: 809 | DAEEWVQQLK | ENSG00000005020.8 | 6 |
| SEQ ID NO: 810 | DDEFTH LYTLIVRPDNTYEVK | ENSG00000179218.9 | 6 |
| SEQ ID NO: 811 | DFGSFDKFKEK | ENSG00000112096.12 | 6 |
| SEQ ID NO: 812 | DGDVQAGANLSFNR | ENSG00000158560.10 | 6 |
| SEQ ID NO: 813 | EFASHLQQLQDALNELTEEHSK | ENSG00000137497.13 | 6 |
| SEQ ID NO: 814 | ETLPELPSVTR | ENSG00000059691.7 | 6 |
| SEQ ID NO: 815 | GAPMHDLLLWNNATVTTCHSK | ENSG00000100714.11 | 6 |
| SEQ ID NO: 816 | HKSDFGK | ENSG00000179218.9 | 6 |
| SEQ ID NO: 817 | IALETSLSK | ENSG00000076662.5 | 6 |
| SEQ ID NO: 818 | IGDFGLMR | ENSG00000061938.12 | 6 |
| SEQ ID NO: 819 | ILREEGPK | ENSG00000004864.9 | 6 |
| SEQ ID NO: 820 | KSEAPFTHK | ENSG00000162614.14 | 6 |
| SEQ ID NO: 821 | LCGDLVSCFQER | ENSG00000165912.11 | 6 |
| SEQ ID NO: 822 | LLDLLEGLTGQK | ENSG00000198947.10 | 6 |
| SEQ ID NO: 823 | LLEQSIQSAQETEK | ENSG00000198947.10 | 6 |
| SEQ ID NO: 824 | LQAEDCSIACLPR | ENSG00000152894.10 | 6 |
| SEQ ID NO: 825 | MNVVFAVK | ENSG00000136631.8 | 6 |
| SEQ ID NO: 826 | NPPAAYIQK | ENSG00000184922.9 | 6 |
| SEQ ID NO: 827 | NTSLNPQELQR | ENSG00000125826.15 | 6 |
| SEQ ID NO: 828 | NVLINKDIR | ENSG00000179218.9 | 6 |
| SEQ ID NO: 829 | PAETLKPMGN | ENSG00000065534.14 | 6 |
| SEQ ID NO: 830 | PAETLKPMGN | ENSG00000065534.14 | 6 |
| SEQ ID NO: 831 | PFSLDALSK | ENSG00000146731.6 | 6 |
| SEQ ID NO: 832 | PLLPANHVTMAK | ENSG00000067704.8 | 6 |
| SEQ ID NO: 833 | PSGYTCACDSGFR | ENSG00000090006.13 | 6 |
| SEQ ID NO: 834 | PSVVLSAAHTVAAR | ENSG00000032444.11 | 6 |
| SEQ ID NO: 835 | QASNGVLIR | ENSG00000166825.9 | 6 |
| SEQ ID NO: 836 | QGLELAADCHLSR | ENSG00000130396.16 | 6 |
| SEQ ID NO: 837 | QVEELLMAMEK | ENSG00000082805.15 | 6 |
| SEQ ID NO: 838 | QVEKEETNEIQVVNEEPQR | ENSG00000135052.12 | 6 |
| SEQ ID NO: 839 | RLEAEFPPHHSQSTFR | ENSG00000061938.12 | 6 |
| SEQ ID NO: 840 | SWDTNLIECNLDQELK | ENSG00000131711.10 | 6 |
| SEQ ID NO: 841 | TGEPCVAELTEENFQR | ENSG00000082805.15 | 6 |
| SEQ ID NO: 842 | VECEPSWQPFQGHCYR | ENSG00000011028.9 | 6 |
| SEQ ID NO: 843 | VRFTPVVCGLR | ENSG00000090006.13 | 6 |
| SEQ ID NO: 844 | VSLSQPR | ENSG00000090006.13 | 6 |
| SEQ ID NO: 845 | AAEGYTQFYYVDVLDGK | ENSG00000205277.5 | 5 |
| SEQ ID NO: 846 | AALEEVEGDVAELELK | ENSG00000114331.8 | 5 |
| SEQ ID NO: 847 | AEEFGNETWGVTK | ENSG00000179218.9 | 5 |
| SEQ ID NO: 848 | AFEDWLNDDLGSYQGAQGNR | ENSG00000101199.8 | 5 |
| SEQ ID NO: 849 | ATQEWLEK | ENSG00000137497.13 | 5 |
| SEQ ID NO: 850 | CSQFCTTGMDGGMSIWDVK | ENSG00000130429.8 | 5 |
| SEQ ID NO: 851 | DQLVIPDGQEEEQEAAGEGR | ENSG00000135052.12 | 5 |
| SEQ ID NO: 852 | EAQEAEAFALYHK | ENSG00000099991.12 | 5 |
| SEQ ID NO: 853 | EGNCSGCIQDCNR | ENSG00000104450.8 | 5 |
| SEQ ID NO: 854 | EGQIQSVVTYDLALDSGRPHSR | ENSG00000169896.12 | 5 |
| SEQ ID NO: 855 | EIDAALQKK | ENSG00000162614.14 | 5 |
| SEQ ID NO: 856 | ERFQNLDKK | ENSG00000130429.8 | 5 |
| SEQ ID NO: 857 | ETQPPDLPTTALGGCPSDWIQFL NK | ENSG00000011028.9 | 5 |
| SEQ ID NO: 858 | FREFLESQEDYDPCWSLQEK | ENSG00000101199.8 | 5 |
| SEQ ID NO: 859 | GGTAAGAGLDSLHK | ENSG00000130429.8 | 5 |
| SEQ ID NO: 860 | GLNPGTLNILVR | ENSG00000152894.10 | 5 |
| SEQ ID NO: 861 | GQLAPVFQR | ENSG00000213380.9 | 5 |
| SEQ ID NO: 862 | GSAASTCILTIESK | ENSG00000162614.14 | 5 |
| SEQ ID NO: 863 | ICGVEDAVSEMTR | ENSG00000146733.9 | 5 |
| SEQ ID NO: 864 | IITEGFEAAKEK | ENSG00000146731.6 | 5 |
| SEQ ID NO: 865 | ILKDIANR | ENSG00000067704.8 | 5 |
| SEQ ID NO: 866 | IQDLEHHLGLALNEVQAAK | ENSG00000011454.12 | 5 |
| SEQ ID NO: 867 | IVDAVIEQVK | ENSG00000170776.15 | 5 |
| SEQ ID NO: 868 | KVNVLQK | ENSG00000082805.15 | 5 |
| SEQ ID NO: 869 | LLLQCQVSSDPPATIIWTLNGK | ENSG00000065534.14 | 5 |
| SEQ ID NO: 870 | LSFEEMER | ENSG00000162614.14 | 5 |
| SEQ ID NO: 871 | LSPIPAVPASVPLQAWHPAK | ENSG00000104450.8 | 5 |
| SEQ ID NO: 872 | NQDNEDEWPLAEILSVK | ENSG00000172977.8 | 5 |
| SEQ ID NO: 873 | PTTLTDEEINR | ENSG00000100714.11 | 5 |
| SEQ ID NO: 874 | QIIEDQSGHYIWVPSPEKL | ENSG00000082458.7 | 5 |
| SEQ ID NO: 875 | QIQESEHMK | ENSG00000065534.14 | 5 |
| SEQ ID NO: 876 | RDFGSFDK | ENSG00000112096.12 | 5 |
| SEQ ID NO: 877 | RPQLEELITAAQNLK | ENSG00000198947.10 | 5 |
| SEQ ID NO: 878 | RPYWCISR | ENSG00000067704.8 | 5 |
| SEQ ID NO: 879 | SEESTASHSSQDATGTIVLPAR | ENSG00000205277.5 | 5 |
| SEQ ID NO: 880 | SEESTASHSSQDATGTIVLPAR | ENSG00000205277.5 | 5 |
| SEQ ID NO: 881 | SEESTASHSSQDATGTIVLPAR | ENSG00000205277.5 | 5 |
| SEQ ID NO: 882 | SEESTASHSSQDATGTIVLPAR | ENSG00000205277.5 | 5 |
| SEQ ID NO: 883 | SGTIFDNFLITNDEAY | ENSG00000179218.9 | 5 |
| SEQ ID NO: 884 | SQDADSPGSSGAPENLTFK | ENSG00000130396.16 | 5 |
| SEQ ID NO: 885 | TCYPLESRPSLSLGTITDEEMK | ENSG00000137497.13 | 5 |
| SEQ ID NO: 886 | TGLFTPDMAFETIVK | ENSG00000106976.14 | 5 |
| SEQ ID NO: 887 | VATEAEFSPEDSPSVR | ENSG00000155629.10 | 5 |
| SEQ ID NO: 888 | VPPPCDLGR | ENSG00000090006.13 | 5 |
| SEQ ID NO: 889 | VVSNFILQALQGEPLTVYGSGSQ TR | ENSG00000115652.10 | 5 |
| SEQ ID NO: 890 | AAIVFTDGR | ENSG00000132561.9 | 4 |
| SEQ ID NO: 891 | AGKGEVTFEDVK | ENSG00000004864.9 | 4 |
| SEQ ID NO: 892 | AIDLEIK | ENSG00000162614.14 | 4 |
| SEQ ID NO: 893 | AIEEELQEIASEPTNK | ENSG00000132561.9 | 4 |
| SEQ ID NO: 894 | ASFITPVPGGVGPMTVAMLMQ STVESAKR | ENSG00000100714.11 | 4 |
| SEQ ID NO: 895 | CAVVSSAGSLK | ENSG00000073849.10 | 4 |
| SEQ ID NO: 896 | CHYYANK | ENSG00000134871.13 | 4 |
| SEQ ID NO: 897 | CLTALPYICK | ENSG00000011028.9 | 4 |
| SEQ ID NO: 898 | DEELPTLLHFAAK | ENSG00000155629.10 | 4 |
| SEQ ID NO: 899 | DKVMPLIIQGFK | ENSG00000086475.10 | 4 |
| SEQ ID NO: 900 | DKVVALAEGR | ENSG00000101199.8 | 4 |
| SEQ ID NO: 901 | DQVFGSNLANLCQR | ENSG00000165322.13 | 4 |
| SEQ ID NO: 902 | DVFNVEDQKR | ENSG00000135052.12 | 4 |
| SEQ ID NO: 903 | EAELEYNPEHVSR | ENSG00000067704.8 | 4 |
| SEQ ID NO: 904 | EATDVIIIHSK | ENSG00000166825.9 | 4 |
| SEQ ID NO: 905 | EQYDVPQEWR | ENSG00000205277.5 | 4 |
| SEQ ID NO: 906 | ESPQDSAITR | ENSG00000011454.12 | 4 |
| SEQ ID NO: 907 | EVVLQWFTENSK | ENSG00000166825.9 | 4 |
| SEQ ID NO: 908 | EYFTFPASK | ENSG00000130396.16 | 4 |
| SEQ ID NO: 909 | FFDSACTMGAYHPLLYEK | ENSG00000073849.10 | 4 |
| SEQ ID NO: 910 | FGSFDKFK | ENSG00000112096.12 | 4 |
| SEQ ID NO: 911 | FIEAGQFNDNLYGTSIQSVR | ENSG00000082458.7 | 4 |
| SEQ ID NO: 912 | FIPGSALNGMVEMMDR | ENSG00000067704.8 | 4 |
| SEQ ID NO: 913 | GHLQIAACPNQD | ENSG00000112096.12 | 4 |
| SEQ ID NO: 914 | GSWQPVGDLLIDSLQDHLEK | ENSG00000198947.10 | 4 |
| SEQ ID NO: 915 | HVVPGVER | ENSG00000130589.12 | 4 |
| SEQ ID NO: 916 | IDYGTGHEAAFAAFLCCLCK | ENSG00000119383.15 | 4 |
| SEQ ID NO: 917 | IVGNGSEQQLQK | ENSG00000011454.12 | 4 |
| SEQ ID NO: 918 | KESEETIIQTDEDVPGPVPVK | ENSG00000152894.10 | 4 |
| SEQ ID NO: 919 | LEPAGPACPEGGR | ENSG00000213380.9 | 4 |
| SEQ ID NO: 920 | LETLTNQFSDSK | ENSG00000082805.15 | 4 |
| SEQ ID NO: 921 | LFSGSQVR | ENSG00000059691.7 | 4 |
| SEQ ID NO: 922 | LLEILK | ENSG00000082805.15 | 4 |
| SEQ ID NO: 923 | LLQQFPLDLEK | ENSG00000198947.10 | 4 |
| SEQ ID NO: 924 | LLTESVNSVIAQAPPVAQEALKK | ENSG00000198947.10 | 4 |
| SEQ ID NO: 925 | LPVEDKIR | ENSG00000100714.11 | 4 |
| SEQ ID NO: 926 | LPYGGQCR | ENSG00000172037.9 | 4 |
| SEQ ID NO: 927 | LSTAITLLPLEEGR | ENSG00000019144.12 | 4 |
| SEQ ID NO: 928 | LTASSTCGLNGPQPYCIVSHLQD EKK | ENSG00000172037.9 | 4 |
| SEQ ID NO: 929 | LVTPHGESEQIGVIPSK | ENSG00000082458.7 | 4 |
| SEQ ID NO: 930 | NAEVRPPFTYASLIR | ENSG00000114861.14 | 4 |
| SEQ ID NO: 931 | PAETLKPMGNAKPDENLK | ENSG00000065534.14 | 4 |
| SEQ ID NO: 932 | PGGAGPCATVSVFPGAR | ENSG00000142453.7 | 4 |
| SEQ ID NO: 933 | QELNTIASKPPR | ENSG00000169896.12 | 4 |
| SEQ ID NO: 934 | RFSTEYELQQLEQFKK | ENSG00000166825.9 | 4 |
| SEQ ID NO: 935 | RVPPPCAPGR | ENSG00000090006.13 | 4 |
| SEQ ID NO: 936 | SCHAGFGSPAGWDVPVGALIQR | ENSG00000163975.7 | 4 |
| SEQ ID NO: 937 | SFGHFPGPEFLDVEK | ENSG00000165322.13 | 4 |
| SEQ ID NO: 938 | SITEVGEALK | ENSG00000198947.10 | 4 |
| SEQ ID NO: 939 | SLQADTTNTDTALTTLEEALAEKE R | ENSG00000082805.15 | 4 |
| SEQ ID NO: 940 | SSNLLDLKNPFFR | ENSG00000142453.7 | 4 |
| SEQ ID NO: 941 | TGYAFVDCPDESWALK | ENSG00000136231.9 | 4 |
| SEQ ID NO: 942 | TQVTFFFPLDLSYR | ENSG00000169896.12 | 4 |
| SEQ ID NO: 943 | TSKDDLLLTDFEGALK | ENSG00000011454.12 | 4 |
| SEQ ID NO: 944 | TVTINTEQK | ENSG00000065534.14 | 4 |
| SEQ ID NO: 945 | VADLLQHINLMK | ENSG00000152894.10 | 4 |
| SEQ ID NO: 946 | VDANISVHHPGEPLGVR | ENSG00000059691.7 | 4 |
| SEQ ID NO: 947 | VMVGDLEDINEMIIK | ENSG00000198947.10 | 4 |
| SEQ ID NO: 948 | VVGDVAYDEAKER | ENSG00000100714.11 | 4 |
| SEQ ID NO: 949 | VYLLYR | ENSG00000167770.7 | 4 |
| SEQ ID NO: 950 | WANGLSEEKPLSVPR | ENSG00000064545.10 | 4 |
| SEQ ID NO: 951 | WAPNENK | ENSG00000130429.8 | 4 |
| SEQ ID NO: 952 | WCVLSTPEIQK | ENSG00000163975.7 | 4 |
| SEQ ID NO: 953 | WMDPEGEMKPGR | ENSG00000113387.7 | 4 |
| SEQ ID NO: 954 | WVLLQDILLK | ENSG00000198947.10 | 4 |
| SEQ ID NO: 955 | YEEQRPSLK | ENSG00000162614.14 | 4 |
| SEQ ID NO: 956 | YGLLNVTK | ENSG00000165322.13 | 4 |
| SEQ ID NO: 957 | YQHIGLVAMFR | ENSG00000169896.12 | 4 |
| SEQ ID NO: 958 | YVPAIAHLIHSLNPVR | ENSG00000106066.9 | 4 |
| SEQ ID NO: 959 | AAILQTEVDALR | ENSG00000082805.15 | 3 |
| SEQ ID NO: 960 | ADGGPEAGELPSIGEATAALALA GR | ENSG00000019144.12 | 3 |
| SEQ ID NO: 961 | AENYWWR | ENSG00000061938.12 | 3 |
| SEQ ID NO: 962 | AEQPPHLTPGIR | ENSG00000146733.9 | 3 |
| SEQ ID NO: 963 | AIEALSGK | ENSG00000136231.9 | 3 |
| SEQ ID NO: 964 | AIGNIELGIR | ENSG00000131711.10 | 3 |
| SEQ ID NO: 965 | AMNNSWHPECFR | ENSG00000169756.12 | 3 |
| SEQ ID NO: 966 | APNLSSGNVSLK | ENSG00000155629.10 | 3 |
| SEQ ID NO: 967 | AQVAHADQQLR | ENSG00000137497.13 | 3 |
| SEQ ID NO: 968 | AREHFGTVK | ENSG00000211460.7 | 3 |
| SEQ ID NO: 969 | ARFEQMAKAREE | ENSG00000162614.14 | 3 |
| SEQ ID NO: 970 | ASFANEDGQVSPGSLLLAGAIAG MPAASLVTPADVIK | ENSG00000004864.9 | 3 |
| SEQ ID NO: 971 | AVVVGFDPHFSYMK | ENSG00000184207.8 | 3 |
| SEQ ID NO: 972 | DDLLLTDFEGALK | ENSG00000011454.12 | 3 |
| SEQ ID NO: 973 | DNEETGFGSGTR | ENSG00000166825.9 | 3 |
| SEQ ID NO: 974 | DVDGLTSINAGK | ENSG00000100714.11 | 3 |
| SEQ ID NO: 975 | EAGIQPSLLCVR | ENSG00000163975.7 | 3 |
| SEQ ID NO: 976 | EDFNSKHMANQRALGK | ENSG00000172037.9 | 3 |
| SEQ ID NO: 977 | EEGDLGPVYGFQWR | ENSG00000176890.11 | 3 |
| SEQ ID NO: 978 | EELSSGDSLSPDPWK | ENSG00000130396.16 | 3 |
| SEQ ID NO: 979 | ELQKAVEEMK | ENSG00000198947.10 | 3 |
| SEQ ID NO: 980 | ENSMLREEMHRRFENAPDSAKT K | ENSG00000082805.15 | 3 |
| SEQ ID NO: 981 | EQISDIDDAVRK | ENSG00000113387.7 | 3 |
| SEQ ID NO: 982 | EVVDAGLVGLER | ENSG00000138162.13 | 3 |
| SEQ ID NO: 983 | FEALQAPACHENMVK | ENSG00000196961.8 | 3 |
| SEQ ID NO: 984 | FHLCSVATR | ENSG00000196961.8 | 3 |
| SEQ ID NO: 985 | FNLDTENAMTFQENAR | ENSG00000169896.12 | 3 |
| SEQ ID NO: 986 | FTEEIPLK | ENSG00000136231.9 | 3 |
| SEQ ID NO: 987 | GALTSTPYSPTQHLER | ENSG00000153310.14 | 3 |
| SEQ ID NO: 988 | GDEGPIGHQGPIGQEGAPGR | ENSG00000134871.13 | 3 |
| SEQ ID NO: 989 | GDSGQPLFLTPYIEAGK | ENSG00000106066.9 | 3 |
| SEQ ID NO: 990 | GEPVSAEDLGVSGALTVLM K | ENSG00000100714.11 | 3 |
| SEQ ID NO: 991 | GFSGIFPACHPCHACFGDWDR | ENSG00000172037.9 | 3 |
| SEQ ID NO: 992 | GIDTPQCHR | ENSG00000172037.9 | 3 |
| SEQ ID NO: 993 | GWDSSHEDDLPVYLAR | ENSG00000113657.8 | 3 |
| SEQ ID NO: 994 | HEQNIDCGGGYV | ENSG00000179218.9 | 3 |
| SEQ ID NO: 995 | HLNQGTDEDIYLLGK | ENSG00000073849.10 | 3 |
| SEQ ID NO: 996 | IAELQQR | ENSG00000137497.13 | 3 |
| SEQ ID NO: 997 | ILVVITDGEK | ENSG00000169896.12 | 3 |
| SEQ ID NO: 998 | INDAFNLASAHK | ENSG00000166825.9 | 3 |
| SEQ ID NO: 999 | INLPAPNPDHVGGYK | ENSG00000004864.9 | 3 |
| SEQ ID NO: 1000 | IQEILTQVK | ENSG00000136231.9 | 3 |
| SEQ ID NO: 1001 | IQPTTPSEPTAIK | ENSG00000198947.10 | 3 |
| SEQ ID NO: 1002 | ISPGSTEITTLPGSTTTPGLSEAST TFYSSPR | ENSG00000205277.5 | 3 |
| SEQ ID NO: 1003 | ISPGSTEITTLPGSTTTPGLSEAST TFYSSPR | ENSG00000205277.5 | 3 |
| SEQ ID NO: 1004 | ISPGSTEITTLPGSTTTPGLSEAST TFYSSPR | ENSG00000205277.5 | 3 |
| SEQ ID NO: 1005 | ISPGSTEITTLPGSTTTPGLSEAST TFYSSPR | ENSG00000205277.5 | 3 |
| SEQ ID NO: 1006 | ISSMERGLR | ENSG00000082805.15 | 3 |
| SEQ ID NO: 1007 | IVLDVGCGSGILSFFAAQAGAR | ENSG00000142453.7 | 3 |
| SEQ ID NO: 1008 | IYGADDIELLPEAQHKAEVYTK | ENSG00000100714.11 | 3 |
| SEQ ID NO: 1009 | KDVKLDK | ENSG00000170776.15 | 3 |
| SEQ ID NO: 1010 | KFQETEQTIQK | ENSG00000132205.6 | 3 |
| SEQ ID NO: 1011 | KFSYDLSQCINQMK | ENSG00000135052.12 | 3 |
| SEQ ID NO: 1012 | KLPAENGSSSAETLNAK | ENSG00000065534.14 | 3 |
| SEQ ID NO: 1013 | KLTELENELNTK | ENSG00000130396.16 | 3 |
| SEQ ID NO: 1014 | KQTENPK | ENSG00000198947.10 | 3 |
| SEQ ID NO: 1015 | KQVTPLFIHFR | ENSG00000166825.9 | 3 |
| SEQ ID NO: 1016 | KRVEDAYILTCNVSLEYEK | ENSG00000146731.6 | 3 |
| SEQ ID NO: 1017 | KVPFAWCAPESLK | ENSG00000061938.12 | 3 |
| SEQ ID NO: 1018 | LAGAPAPK | ENSG00000184207.8 | 3 |
| SEQ ID NO: 1019 | LHELYEKVFSRRADR | ENSG00000032444.11 | 3 |
| SEQ ID NO: 1020 | LLDPEDVDTTYPDKK | ENSG00000198947.10 | 3 |
| SEQ ID NO: 1021 | LLESLQENHFQEDEQFLGAVMP R | ENSG00000086475.10 | 3 |
| SEQ ID NO: 1022 | LLQVAVEDR | ENSG00000198947.10 | 3 |
| SEQ ID NO: 1023 | LLVSDIQTIQPSLNSVNEGGQK | ENSG00000198947.10 | 3 |
| SEQ ID NO: 1024 | LNLHSADWQR | ENSG00000198947.10 | 3 |
| SEQ ID NO: 1025 | LPAENGSSSAETLNAK | ENSG00000065534.14 | 3 |
| SEQ ID NO: 1026 | LPLEDADIIK | ENSG00000110237.3 | 3 |
| SEQ ID NO: 1027 | LPLQMALTELETLAEK | ENSG00000104728.11 | 3 |
| SEQ ID NO: 1028 | LPTEWNVLGTDQSLHDAGPR | ENSG00000170776.15 | 3 |
| SEQ ID NO: 1029 | LQEALSQLDFQWEK | ENSG00000198947.10 | 3 |
| SEQ ID NO: 1030 | LQEPSAQANCCDSEKNGDIGQQ IK | ENSG00000132205.6 | 3 |
| SEQ ID NO: 1031 | LQSQVISELDACKECTQGVQR | ENSG00000132205.6 | 3 |
| SEQ ID NO: 1032 | LYIGNLSENAAPSDLESIFK | ENSG00000136231.9 | 3 |
| SEQ ID NO: 1033 | MLESYLHAK | ENSG00000142453.7 | 3 |
| SEQ ID NO: 1034 | NLLLATR | ENSG00000061938.12 | 3 |
| SEQ ID NO: 1035 | NVLLHEMQIQHPTASLIAK | ENSG00000146731.6 | 3 |
| SEQ ID NO: 1036 | QKPCDLPLR | ENSG00000136231.9 | 3 |
| SEQ ID NO: 1037 | QPAAFIVTQYPLPNTVK | ENSG00000152894.10 | 3 |
| SEQ ID NO: 1038 | QQLGHIEAWAEK | ENSG00000130396.16 | 3 |
| SEQ ID NO: 1039 | QREEHYFCK | ENSG00000133315.6 | 3 |
| SEQ ID NO: 1040 | QVFHALEDELQK | ENSG00000151914.13 | 3 |
| SEQ ID NO: 1041 | QWMENPNNNPIHPNLR | ENSG00000166825.9 | 3 |
| SEQ ID NO: 1042 | SAQALVEQMVNEGVNADSIK | ENSG00000198947.10 | 3 |
| SEQ ID NO: 1043 | SATSVLVGEPTTSPISSGSTETTAL PGSTTTAGLSEK | ENSG00000205277.5 | 3 |
| SEQ ID NO: 1044 | SATSVLVGEPTTSPISSGSTETTAL PGSTTTAGLSEK | ENSG00000205277.5 | 3 |
| SEQ ID NO: 1045 | SATSVLVGEPTTSPISSGSTETTAL PGSTTTAGLSEK | ENSG00000205277.5 | 3 |
| SEQ ID NO: 1046 | SAVEGMPSNLDSEVAWGK | ENSG00000198947.10 | 3 |
| SEQ ID NO: 1047 | SEDSTIYDLLKDPVSLR | ENSG00000104728.11 | 3 |
| SEQ ID NO: 1048 | SLESALKDLK | ENSG00000130429.8 | 3 |
| SEQ ID NO: 1049 | SPNPALTFCVK | ENSG00000019144.12 | 3 |
| SEQ ID NO: 1050 | STTFYTSPR | ENSG00000205277.5 | 3 |
| SEQ ID NO: 1051 | STTFYTSPR | ENSG00000205277.5 | 3 |
| SEQ ID NO: 1052 | STTFYTSPR | ENSG00000205277.5 | 3 |
| SEQ ID NO: 1053 | STTFYTSPR | ENSG00000205277.5 | 3 |
| SEQ ID NO: 1054 | TCHYYANK | ENSG00000134871.13 | 3 |
| SEQ ID NO: 1055 | TCSECQELHWGDPGLQCHACDC DSR | ENSG00000172037.9 | 3 |
| SEQ ID NO: 1056 | TCYPLESR | ENSG00000137497.13 | 3 |
| SEQ ID NO: 1057 | TEFQLELPVK | ENSG00000169896.12 | 3 |
| SEQ ID NO: 1058 | TKEPVIMSTLETVR | ENSG00000198947.10 | 3 |
| SEQ ID NO: 1059 | TPLWIGLAGEEGSRR | ENSG00000011028.9 | 3 |
| SEQ ID NO: 1060 | TQSLNPAPFSPLTAQQMKPEKPS TLQRPQETVIR | ENSG00000130396.16 | 3 |
| SEQ ID NO: 1061 | TVGWNVPVGYLVESGR | ENSG00000163975.7 | 3 |
| SEQ ID NO: 1062 | VASSSSGNNFLSGSPASPMGDIL QTPQFQMR | ENSG00000137497.13 | 3 |
| SEQ ID NO: 1063 | VAWVSHDSTVCLADADK | ENSG00000130429.8 | 3 |
| SEQ ID NO: 1064 | VEQQPDYR | ENSG00000130396.16 | 3 |
| SEQ ID NO: 1065 | VIQEVSGLPSEGASEGNQYTPDA QR | ENSG00000169129.10 | 3 |
| SEQ ID NO: 1066 | VLDLLDPASGDLVIR | ENSG00000079616.8 | 3 |
| SEQ ID NO: 1067 | VLLHEMQIQHPTASLIAK | ENSG00000146731.6 | 3 |
| SEQ ID NO: 1068 | VMDKVTSDETR | ENSG00000138162.13 | 3 |
| SEQ ID NO: 1069 | VPRYELLLK | ENSG00000127084.13 | 3 |
| SEQ ID NO: 1070 | VQFGASHVFK | ENSG00000130396.16 | 3 |
| SEQ ID NO: 1071 | VSCIVSAAK | ENSG00000169129.10 | 3 |
| SEQ ID NO: 1072 | VTEILGIEPDREK | ENSG00000211460.7 | 3 |
| SEQ ID NO: 1073 | VVDALNQGLPR | ENSG00000079616.8 | 3 |
| SEQ ID NO: 1074 | WKTPAAIPATPVAVSQPIR | ENSG00000130396.16 | 3 |
| SEQ ID NO: 1075 | YLETADYAIREEIVLK | ENSG00000196961.8 | 3 |
| SEQ ID NO: 1076 | YLNWESDQPDNPSEENCGVIR | ENSG00000011028.9 | 3 |
| SEQ ID NO: 1077 | YVGFGNTPPPQKK | ENSG00000101199.8 | 3 |
| SEQ ID NO: 1078 | AAGNFATK | ENSG00000130396.16 | 2 |
| SEQ ID NO: 1079 | AEGERQPPPDSSEEAPPATQNFII PK | ENSG00000119383.15 | 2 |
| SEQ ID NO: 1080 | AGLVVEDALFETLPSDVR | ENSG00000171488.10 | 2 |
| SEQ ID NO: 1081 | AHCGDPVSLAAAGDGSPDIGPT GELSGSLK | ENSG00000127084.13 | 2 |
| SEQ ID NO: 1082 | AILQNHTDFKDK | ENSG00000142453.7 | 2 |
| SEQ ID NO: 1083 | AINVYGTSEPSQESELTTVGEKPE EPK | ENSG00000065534.14 | 2 |
| SEQ ID NO: 1084 | ALGEDQVAETSAMSDVLKDILK | ENSG00000157617.12 | 2 |
| SEQ ID NO: 1085 | ANIVMVLEIVSGGELFER | ENSG00000065534.14 | 2 |
| SEQ ID NO: 1086 | APEEQGLLPNGEPSQHSSAPQK | ENSG00000169129.10 | 2 |
| SEQ ID NO: 1087 | APGLGVLSPSGEER | ENSG00000065534.14 | 2 |
| SEQ ID NO: 1088 | AQDDVSEWASK | ENSG00000132561.9 | 2 |
| SEQ ID NO: 1089 | ASSISEEVAVGSIAATLK | ENSG00000170776.15 | 2 |
| SEQ ID NO: 1090 | ATLALDSVLTEEGK | ENSG00000170776.15 | 2 |
| SEQ ID NO: 1091 | AVGGDRQEAIQPGCIGGPKGLP GLPGPPGPTGAKGLRGIPGFAGA DGGP | ENSG00000134871.13 | 2 |
| SEQ ID NO: 1092 | AVGLVSTWTQR | ENSG00000127084.13 | 2 |
| SEQ ID NO: 1093 | AVSSADPR | ENSG00000138162.13 | 2 |
| SEQ ID NO: 1094 | AWHAFFTAAER | ENSG00000165912.11 | 2 |
| SEQ ID NO: 1095 | DCTQCLQHPWLMK | ENSG00000065534.14 | 2 |
| SEQ ID NO: 1096 | DEISDDAKDFISNLLK | ENSG00000065534.14 | 2 |
| SEQ ID NO: 1097 | DFGPASQHFLSTSVQGPWER | ENSG00000198947.10 | 2 |
| SEQ ID NO: 1098 | DFLDSLGFSTR | ENSG00000176890.11 | 2 |
| SEQ ID NO: 1099 | DGEWEPPVIQNPEYK | ENSG00000179218.9 | 2 |
| SEQ ID NO: 1100 | DTSPAPSGTTSAFVK | ENSG00000205277.5 | 2 |
| SEQ ID NO: 1101 | EAEDRARQEEERR | ENSG00000130396.16 | 2 |
| SEQ ID NO: 1102 | EAPYGAPR | ENSG00000090006.13 | 2 |
| SEQID NO: 1103 | ECAIYTNR | ENSG00000104450.8 | 2 |
| SEQ ID NO: 1104 | EGIVALRR | ENSG00000146731.6 | 2 |
| SEQ ID NO: 1105 | EGPYTVDAIQK | ENSG00000198947.10 | 2 |
| SEQ ID NO: 1106 | EKELQTIFDTLPPMR | ENSG00000198947.10 | 2 |
| SEQ ID NO: 1107 | ELEQQLQESAR | ENSG00000019144.12 | 2 |
| SEQ ID NO: 1108 | EQLDKIQSSHNFQLESVNK | ENSG00000135052.12 | 2 |
| SEQ ID NO: 1109 | EVTKEEFVLAAQK | ENSG00000004864.9 | 2 |
| SEQ ID NO: 1110 | EVVPGDSVNSLLSILDVITGHQHP QR | ENSG00000032444.11 | 2 |
| SEQ ID NO: 1111 | EYWMDPEGEMKPGRK | ENSG00000113387.7 | 2 |
| SEQ ID NO: 1112 | FGFSHLEALLDDSK | ENSG00000167770.7 | 2 |
| SEQ ID NO: 1113 | FGSQASQK | ENSG00000101199.8 | 2 |
| SEQ ID NO: 1114 | FHELTQTDK | ENSG00000100714.11 | 2 |
| SEQ ID NO: 1115 | FLDLGISIAENR | ENSG00000125826.15 | 2 |
| SEQ ID NO: 1116 | FLLDCGIR | ENSG00000065534.14 | 2 |
| SEQ ID NO: 1117 | FVDPSQDHALAK | ENSG00000130396.16 | 2 |
| SEQ ID NO: 1118 | FYGDEEK | ENSG00000179218.9 | 2 |
| SEQ ID NO: 1119 | GAWLGMNFNPK | ENSG00000011028.9 | 2 |
| SEQ ID NO: 1120 | GILVFQLK | ENSG00000130396.16 | 2 |
| SEQ ID NO: 1121 | GISLNPEQWSQL | ENSG00000113387.7 | 2 |
| SEQ ID NO: 1122 | GLYLPLFKPSVSTSK | ENSG00000004864.9 | 2 |
| SEQ ID NO: 1123 | GMEDLIPLVNR | ENSG00000106976.14 | 2 |
| SEQ ID NO: 1124 | GPIGHQGPIGQEGAPGR | ENSG00000134871.13 | 2 |
| SEQ ID NO: 1125 | GPNKHTLTQIK | ENSG00000146731.6 | 2 |
| SEQ ID NO: 1126 | GPTCNEFTGQCHCR | ENSG00000172037.9 | 2 |
| SEQ ID NO: 1127 | GSEGEPGIR | ENSG00000134871.13 | 2 |
| SEQ ID NO: 1128 | GTDVREPDDSPQGR | ENSG00000011028.9 | 2 |
| SEQ ID NO: 1129 | GWAGDSGPQGR | ENSG00000134871.13 | 2 |
| SEQ ID NO: 1130 | HAQEELPPPPPQKK | ENSG00000198947.10 | 2 |
| SEQ ID NO: 1131 | HSTVLENTDGK | ENSG00000163975.7 | 2 |
| SEQ ID NO: 1132 | IEELEEALR | ENSG00000082805.15 | 2 |
| SEQ ID NO: 1133 | IEGSGDQIDTYELSGGAR | ENSG00000106976.14 | 2 |
| SEQ ID NO: 1134 | IELHGKPIEVEHSVPK | ENSG00000136231.9 | 2 |
| SEQ ID NO: 1135 | IIDEDFELTERECIK | ENSG00000065534.14 | 2 |
| SEQ ID NO: 1136 | IKLIDFGLAR | ENSG00000065534.14 | 2 |
| SEQ ID NO: 1137 | ILDLLNEGSAR | ENSG00000079616.8 | 2 |
| SEQ ID NO: 1138 | ILMELDGPNWR | ENSG00000104450.8 | 2 |
| SEQ ID NO: 1139 | IPQAVVDVSSHLQK | ENSG00000171488.10 | 2 |
| SEQ ID NO: 1140 | IQAEQVDAVTLSGEDIYTAGK | ENSG00000163975.7 | 2 |
| SEQ ID NO: 1141 | IVIYVQQTTNK | ENSG00000011454.12 | 2 |
| SEQ ID NO: 1142 | IVSEFDYVEK | ENSG00000166825.9 | 2 |
| SEQ ID NO: 1143 | KADTLPR | ENSG00000049323.11 | 2 |
| SEQ ID NO: 1144 | KINQLSEENGDLSFK | ENSG00000137497.13 | 2 |
| SEQ ID NO: 1145 | KIQEILTQVK | ENSG00000136231.9 | 2 |
| SEQ ID NO: 1146 | KKLPAENGSSSAETLNAK | ENSG00000065534.14 | 2 |
| SEQ ID NO: 1147 | KLLLQCQVSSDPPATIIWTLNGK | ENSG00000065534.14 | 2 |
| SEQ ID NO: 1148 | KPAAGLSAAPVPTAPAAGAPL | ENSG00000115310.13 | 2 |
| SEQ ID NO: 1149 | KSPSSDSWTCADTSTER | ENSG00000101199.8 | 2 |
| SEQ ID NO: 1150 | KSSTGSPTSPLNAEK | ENSG00000065534.14 | 2 |
| SEQ ID NO: 1151 | LALLNEK | ENSG00000137497.13 | 2 |
| SEQ ID NO: 1152 | LDIDEK | ENSG00000130396.16 | 2 |
| SEQ ID NO: 1153 | LIAPLEGYTR | ENSG00000167608.7 | 2 |
| SEQ ID NO: 1154 | LKEEEEDKK | ENSG00000179218.9 | 2 |
| SEQ ID NO: 1155 | LKNQVTQLKEQVPGFTPR | ENSG00000100714.11 | 2 |
| SEQ ID NO: 1156 | LLDPQTNTEIANYPIYK | ENSG00000011454.12 | 2 |
| SEQ ID NO: 1157 | LLDRLPSFQQSCR | ENSG00000213380.9 | 2 |
| SEQ ID NO: 1158 | LLEAIKR | ENSG00000112096.12 | 2 |
| SEQ ID NO: 1159 | LLGFGSALLDNVDPNPENFVGA GIIQTK | ENSG00000196961.8 | 2 |
| SEQ ID NO: 1160 | LQAQLNELQAQLSQKEQAAEHY K | ENSG00000137497.13 | 2 |
| SEQ ID NO: 1161 | LQDVHVAEGKK | ENSG00000065534.14 | 2 |
| SEQ ID NO: 1162 | LQGEVLALEEER | ENSG00000019144.12 | 2 |
| SEQ ID NO: 1163 | LSALHLEVR | ENSG00000165912.11 | 2 |
| SEQ ID NO: 1164 | LSSQLVEHCQK | ENSG00000198947.10 | 2 |
| SEQ ID NO: 1165 | LSVMGCDVLK | ENSG00000163975.7 | 2 |
| SEQ ID NO: 1166 | LTAASVGVQGSGWGWLGFNKE R | ENSG00000112096.12 | 2 |
| SEQ ID NO: 1167 | LTDVAIGAPGEEDNR | ENSG00000169896.12 | 2 |
| SEQ ID NO: 1168 | LTHGVLHTK | ENSG00000105223.14 | 2 |
| SEQ ID NO: 1169 | LVTDPDSGLCSHYWGAIIR | ENSG00000130396.16 | 2 |
| SEQ ID NO: 1170 | MDPEGEMKPGR | ENSG00000113387.7 | 2 |
| SEQ ID NO: 1171 | MELLVK | ENSG00000145362.12 | 2 |
| SEQ ID NO: 1172 | MVSMMEGVIQK | ENSG00000130396.16 | 2 |
| SEQ ID NO: 1173 | MVVASSK | ENSG00000100714.11 | 2 |
| SEQ ID NO: 1174 | NDAGQAECSCQVTVDDAPASE NTKAPEMK | ENSG00000065534.14 | 2 |
| SEQ ID NO: 1175 | NILSEFQR | ENSG00000198947.10 | 2 |
| SEQ ID NO: 1176 | NLLEVSEVEQELACQNDHSSALQ NIK | ENSG00000136631.8 | 2 |
| SEQ ID NO: 1177 | NLVDSYMAIVNK | ENSG00000106976.14 | 2 |
| SEQ ID NO: 1178 | NVNVFFPHFK | ENSG00000151116.12 | 2 |
| SEQ ID NO: 1179 | PASAEQIQHLAGAIAER | ENSG00000172037.9 | 2 |
| SEQ ID NO: 1180 | PAVPASVPLQAWHPAK | ENSG00000104450.8 | 2 |
| SEQ ID NO: 1181 | PFSAIYFPCYAHVK | ENSG00000004864.9 | 2 |
| SEQ ID NO: 1182 | PGPVPAHSLCGHLVPK | ENSG00000172037.9 | 2 |
| SEQ ID NO: 1183 | PLQGTTGLIPLLGIDVWEHAYYL QYK | ENSG00000112096.12 | 2 |
| SEQ ID NO: 1184 | PNENKFAVGSGSR | ENSG00000130429.8 | 2 |
| SEQ ID NO: 1185 | PPVQFSLLHSK | ENSG00000196961.8 | 2 |
| SEQ ID NO: 1186 | QAPIGGDFPAVQK | ENSG00000198947.10 | 2 |
| SEQ ID NO: 1187 | QKLQDVHVAEGK | ENSG00000065534.14 | 2 |
| SEQ ID NO: 1188 | QLAAYIADKVDAAQMPQEAQK | ENSG00000198947.10 | 2 |
| SEQ ID NO: 1189 | QLSESSKLK | ENSG00000157617.12 | 2 |
| SEQ ID NO: 1190 | QQTANKVEIEK | ENSG00000011454.12 | 2 |
| SEQ ID NO: 1191 | QSSSSRDDNMFQIGK | ENSG00000113387.7 | 2 |
| SEQ ID NO: 1192 | QYTYGLVSCGLDR | ENSG00000004139.9 | 2 |
| SEQ ID NO: 1193 | RAGNSLAASTAEETAGSAQGR | ENSG00000172037.9 | 2 |
| SEQ ID NO: 1194 | REAPYGAPR | ENSG00000090006.13 | 2 |
| SEQ ID NO: 1195 | REPAPNAPGDIAAAFPAER | ENSG00000138162.13 | 2 |
| SEQ ID NO: 1196 | RGWDSSHEDDLPVYLAR | ENSG00000113657.8 | 2 |
| SEQ ID NO: 1197 | RLEEESAQLK | ENSG00000011454.12 | 2 |
| SEQ ID NO: 1198 | RQVEKEETNEIQVVNEEPQR | ENSG00000135052.12 | 2 |
| SEQ ID NO: 1199 | RSESQGTAPAFK | ENSG00000065534.14 | 2 |
| SEQ ID NO: 1200 | SCTEETHGFICQK | ENSG00000011028.9 | 2 |
| SEQ ID NO: 1201 | SDFGKFVLSSGK | ENSG00000179218.9 | 2 |
| SEQ ID NO: 1202 | SEYMEGNVR | ENSG00000166825.9 | 2 |
| SEQ ID NO: 1203 | SFAPILPHLAEEVFQHIPYIK | ENSG00000067704.8 | 2 |
| SEQ ID NO: 1204 | SKVPQETQSGGGSR | ENSG00000049323.11 | 2 |
| SEQ ID NO: 1205 | SPATTLSPASTTSSGVSEESTTSHS R | ENSG000002052 77.5 | 2 |
| SEQ ID NO: 1206 | SPATTLSPASTTSSGVSEESTTSHS R | ENSG00000205277.5 | 2 |
| SEQ ID NO: 1207 | SPATTLSPASTTSSGVSEESTTSHS RPGSTHTTAFPDSTTTPGLSR | ENSG00000205277.5 | 2 |
| SEQ ID NO: 1208 | SPATTLSPASTTSSGVSEESTTSHS RPGSTHTTAFPDSTTTPGLSR | ENSG00000205277.5 | 2 |
| SEQ ID NO: 1209 | SQDLQVIDLLTVGESR | ENSG00000169231.9 | 2 |
| SEQ ID NO: 1210 | SREPQAKPQLDLSIDSLDLSCEEG TPLSITSK | ENSG00000137497.13 | 2 |
| SEQ ID NO: 1211 | SRQELASGLPSPAATQELPVER | ENSG00000138162.13 | 2 |
| SEQ ID NO: 1212 | SSAAAGAPSR | ENSG00000049323.11 | 2 |
| SEQ ID NO: 1213 | SSPNVANQPPSPGGK | ENSG00000130396.16 | 2 |
| SEQ ID NO: 1214 | SSSEVLVLAETLDGVR | ENSG00000130589.12 | 2 |
| SEQ ID NO: 1215 | SVQEIAEQLLLENHPAR | ENSGO0000151914.13 | 2 |
| SEQ ID NO: 1216 | TCTGYHQVR | ENSG00000133316.11 | 2 |
| SEQ ID NO: 1217 | TGETSR | ENSG00000113387.7 | 2 |
| SEQ ID NO: 1218 | TIQNQLR | ENSG00000169896.12 | 2 |
| SEQ ID NO: 1219 | TLFSLMQYSEEFR | ENSG00000169896.12 | 2 |
| SEQ ID NO: 1220 | TPAPDGPR | ENSG00000032444.11 | 2 |
| SEQ ID NO: 1221 | TPGQIVSEK | ENSG00000059691. 7 | 2 |
| SEQ ID NO: 1222 | TPVPEK | ENSG00000065534.14 | 2 |
| SEQ ID NO: 1223 | TTLLDPDSCR | ENSG00000205277.5 | 2 |
| SEQ ID NO: 1224 | TTTESEVMK | ENSG00000100714.11 | 2 |
| SEQ ID NO: 1225 | TVLQIDCGLQLANDSVNR | ENSG00000104450.8 | 2 |
| SEQ ID NO: 1226 | VAQQPLSLVGCEVVPDPSPDHLY SFR | ENSG00000169129.10 | 2 |
| SEQ ID NO: 1227 | VHALNNVNK | ENSG00000198947.10 | 2 |
| SEQ ID NO: 1228 | VIVMPTTK | ENSG00000067704.8 | 2 |
| SEQ ID NO: 1229 | VLQEDLEQEQVR | ENSG00000198947.10 | 2 |
| SEQ ID NO: 1230 | VPAHAVVVR | ENSG00000163975.7 | 2 |
| SEQ ID NO: 1231 | WLNEVEFK | ENSG00000198947.10 | 2 |
| SEQ ID NO: 1232 | WTDGSIINFISWAPGK | ENSG00000011028.9 | 2 |
| SEQ ID NO: 1233 | WTDGSIINFISWAPGKPR | ENSG00000011028.9 | 2 |
| SEQ ID NO: 1234 | WVNAQFSK | ENSG00000198947.10 | 2 |
| SEQ ID NO: 1235 | YDNFGVLGLDLWQVK | ENSG00000179218.9 | 2 |
| SEQ ID NO: 1236 | YLLYRPGHYDILYK | ENSG00000167770.7 | 2 |
| SEQ ID NO: 1237 | YLSSLDLLLEHR | ENSG00000133315.6 | 2 |
| SEQ ID NO: 1238 | YLVHCLQSELNNYMPAFLDDPEE NSLQRPK | ENSG00000130396.16 | 2 |
| SEQ ID NO: 1239 | YRDPGVLPWGALEEEEEDGGR | ENSG00000167608.7 | 2 |
| SEQ ID NO: 1240 | AAAAAVGPGAGGAGSAVPGGA GPCATVSVFPGAR | ENSG00000142453.7 | 1 |
| SEQ ID NO: 1241 | AAAKVALTKRADPAELR | ENSG00000004864.9 | 1 |
| SEQ ID NO: 1242 | AAATEEPEVIPDPAK | ENSG00000152894.10 | 1 |
| SEQ ID NO: 1243 | AAEEPQQQK | ENSG00000167770.7 | 1 |
| SEQ ID NO: 1244 | AAGDGSPDIGPTGELSGSLKIPNR | ENSG00000127084.13 | 1 |
| SEQ ID NO: 1245 | AAGLQAEIGQVK | ENSG00000082805.15 | 1 |
| SEQ ID NO: 1246 | AASGVPR | ENSG00000155629.10 | 1 |
| SEQ ID NO: 1247 | ACGNMFGLMHGTCPETSGGLLI CLPR | ENSG00000086475.10 | 1 |
| SEQ ID NO: 1248 | ADSAVSQEQLR | ENSG00000165912.11 | 1 |
| SEQ ID NO: 1249 | AEEKPHVKPYFSK | ENSG00000065534.14 | 1 |
| SEQID NO: 1250 | AELEYNPEHVSR | ENSG00000067704.8 | 1 |
| SEQ ID NO: 1251 | AEQLLQDAR | ENSG00000172037.9 | 1 |
| SEQ ID NO: 1252 | AEYMRIQAQQQATKPSKEMS | ENSG00000017373.11 | 1 |
| SEQ ID NO: 1253 | AFCGLGTTGMWR | ENSG00000110237.3 | 1 |
| SEQ ID NO: 1254 | AFLEAVAEEKPHVKPYFSK | ENSG00000065534.14 | 1 |
| SEQ ID NO: 1255 | AHKQCALKLLR | ENSG00000141447.12 | 1 |
| SEQ ID NO: 1256 | ALMDLLQLTR | ENSG00000079616.8 | 1 |
| SEQ ID NO: 1257 | ALQDFEEPDK | ENSG00000061938.12 | 1 |
| SEQ ID NO: 1258 | ALQFLEEVKVSR | ENSG00000146731.6 | 1 |
| SEQ ID NO: 1259 | ALQHMAAMSSAQIVSATAIHNK LGLPGIPRPT | ENSG00000187079.10 | 1 |
| SEQ ID NO: 1260 | AMAYETLEQYGK | ENSG00000104450.8 | 1 |
| SEQ ID NO: 1261 | AMLAAVLEQELPALAENLHQEQ K | ENSG00000142733.10 | 1 |
| SEQ ID NO: 1262 | AMLAAVLEQELPALAENLHQEQ K | ENSG00000142733.10 | 1 |
| SEQ ID NO: 1263 | ANGITMYAVGVGKAIEEELQEIA SEPTNK | ENSG00000132561.9 | 1 |
| SEQ ID NO: 1264 | APAPDVPGCSR | ENSG00000172037.9 | 1 |
| SEQ ID NO: 1265 | APILPHLAEEVFQHIPYIK | ENSG00000067704.8 | 1 |
| SEQ ID NO: 1266 | AQALLADVDTLLFDCDGVL WR | ENSG00000184207.8 | 1 |
| SEQ ID NO: 1267 | AQNSGFDLQETLVK | ENSG00000146731.6 | 1 |
| SEQ ID NO: 1268 | ARFEQMAK | ENSG00000162614.14 | 1 |
| SEQ ID NO: 1269 | ARPEAYQVPASYQPDEEER | ENSG00000125826.15 | 1 |
| SEQ ID NO: 1270 | ARTSAGVGAWGAAAVGRTAGV R | ENSG00000133315.6 | 1 |
| SEQ ID NO: 1271 | ASIPLKELEQFNSDIQK | ENSG00000198947.10 | 1 |
| SEQ ID NO: 1272 | ATSCFPRPMTPRDR | ENSG00000137497.13 | 1 |
| SEQ ID NO: 1273 | AVTSVSGPGEHLR | ENSG00000169231.9 | 1 |
| SEQ ID NO: 1274 | CAEVVSGK | ENSG00000067704.8 | 1 |
| SEQ ID NO: 1275 | CFGLLLSPGK | ENSG00000011454.12 | 1 |
| SEQ ID NO: 1276 | CGDSDKGFVVINQK | ENSG00000146731.6 | 1 |
| SEQ ID NO: 1277 | CGGLSCNGAAATADLALGR | ENSG00000172037.9 | 1 |
| SEQ ID NO: 1278 | CLCPPDFAGK | ENSG00000090006.13 | 1 |
| SEQ ID NO: 1279 | CLQHPWLMK | ENSG00000065534.14 | 1 |
| SEQ ID NO: 1280 | CLVENAGDVAFVR | ENSG00000163975.7 | 1 |
| SEQ ID NO: 1281 | CSGNIDPM DPDACDPHTGQCLR | ENSG00000172037.9 | 1 |
| SEQ ID NO: 1282 | CTEGPIDLVFVIDGSK | ENSG00000132561.9 | 1 |
| SEQ ID NO: 1283 | CTQCLQHPWLMK | ENSG00000065534.14 | 1 |
| SEQ ID NO: 1284 | CVRWAPNENK | ENSG00000130429.8 | 1 |
| SEQ ID NO: 1285 | DALLEALK | ENSG00000172037.9 | 1 |
| SEQ ID NO: 1286 | DCCFEISAPDKR | ENSG00000005020.8 | 1 |
| SEQ ID NO: 1287 | DDRTGTGTLSVFGMQARYSLR | ENSG00000176890.11 | 1 |
| SEQ ID NO: 1288 | DEDFELTERECIK | ENSG00000065534.14 | 1 |
| SEQ ID NO: 1289 | DISLQGPGLAPE | ENSG00000019144.12 | 1 |
| SEQ ID NO: 1290 | DITAALAAER | ENSG00000106976.14 | 1 |
| SEQ ID NO: 1291 | DLNVISSLLK | ENSG00000225485.3 | 1 |
| SEQ ID NO: 1292 | DQREPLPPAPAENEMK | ENSG00000104728.11 | 1 |
| SEQ ID NO: 1293 | DQSPLVSSSDSPPRPQPAFK | ENSG00000115310.13 | 1 |
| SEQ ID NO: 1294 | DRRGSGKPR | ENSG00000130396.16 | 1 |
| SEQ ID NO: 1295 | DSSHAFTLDELR | ENSG00000163975.7 | 1 |
| SEQ ID NO: 1296 | DWDSPYSHDLDTSADSVGNACR | ENSG00000105223.14 | 1 |
| SEQ ID NO: 1297 | EAEQLLRGPLGDQYQTVK | ENSG00000172037.9 | 1 |
| SEQ ID NO: 1298 | EAEVQTWLQQIGFSK | ENSG00000004139.9 | 1 |
| SEQ ID NO: 1299 | EDTVQSVK | ENSG00000106066.9 | 1 |
| SEQ ID NO: 1300 | EEAEQVLGQAR | ENSG00000198947.10 | 1 |
| SEQ ID NO: 1301 | EGIVALR | ENSG00000146731.6 | 1 |
| SEQ ID NO: 1302 | EGTEAEPLPLR | ENSG00000142733.10 | 1 |
| SEQ ID NO: 1303 | EGTEAEPLPLR | ENSG00000142733.10 | 1 |
| SEQ ID NO: 1304 | EGTPGIFQK | ENSG00000205277.5 | 1 |
| SEQ ID NO: 1305 | EGVIQNFK | ENSG00000130396.16 | 1 |
| SEQ ID NO: 1306 | EIDAALQK | ENSG00000162614.14 | 1 |
| SEQ ID NO: 1307 | EIHTVPDMGKWKR | ENSG00000119383.15 | 1 |
| SEQ ID NO: 1308 | EKLTAASVGVQGSGWGWLGFN K | ENSG00000112096.12 | 1 |
| SEQ ID NO: 1309 | ELEAKMLAQKAEEKENHCPTML R | ENSG00000079616.8 | 1 |
| SEQ ID NO: 1310 | ELEEKDGDVQAGANLSFNR | ENSG00000158560.10 | 1 |
| SEQ ID NO: 1311 | ELETLTTNYQWLCTR | ENSG00000198947.10 | 1 |
| SEQ ID NO: 1312 | ELLLSGPPEVAAPDTPYLHVDSA AQR | ENSG00000138162.13 | 1 |
| SEQ ID NO: 1313 | ELQDGIGQR | ENSG00000198947.10 | 1 |
| SEQ ID NO: 1314 | EMSKKAPSEISRK | ENSG00000198947.10 | 1 |
| SEQ ID NO: 1315 | ENIRQEISIMNCLHHPK | ENSG00000065534.14 | 1 |
| SEQ ID NO: 1316 | EPMKAPLCGEGDQPGGFESQEK | ENSG00000138162.13 | 1 |
| SEQ ID NO: 1317 | EPYAREMLAISFISAVNR | ENSG00000225485.3 | 1 |
| SEQ ID NO: 1318 | ERARKFSGSGLAMGLGSASASA WRR | ENSG00000082458.7 | 1 |
| SEQ ID NO: 1319 | ERARKFSGSGLAMGLGSASASA WRR | ENSG00000082458.7 | 1 |
| SEQ ID NO: 1320 | ERVLSLSQALATEASQWHR | ENSG00000105559.7 | 1 |
| SEQ ID NO: 1321 | ESGRGSSTPPGPIAALGMPDTGP GSSSLGK | ENSG00000127084.13 | 1 |
| SEQ ID NO: 1322 | ESGSLEDDWDFLPPKK | ENSG00000179218.9 | 1 |
| SEQ ID NO: 1323 | EVARNVFECNDQVVK | ENSG00000169896.12 | 1 |
| SEQ ID NO: 1324 | EVPEEGPGAPAR | ENSG00000186635.10 | 1 |
| SEQ ID NO: 1325 | EYQEDLALR | ENSG00000125826.15 | 1 |
| SEQ ID NO: 1326 | FAGDSLK | ENSG00000151914.13 | 1 |
| SEQ ID NO: 1327 | FGPGDQVR | ENSG00000114331.8 | 1 |
| SEQ ID NO: 1328 | FGVLGLDLWQVK | ENSG00000179218.9 | 1 |
| SEQ ID NO: 1329 | FKDNPTVVVEDLR | ENSG00000114331.8 | 1 |
| SEQ ID NO: 1330 | FNGAPTANFQQDVGTK | ENSG00000073849.10 | 1 |
| SEQ ID NO: 1331 | FNHPAEAKWMK | ENSG00000019144.12 | 1 |
| SEQ ID NO: 1332 | FNRALNCMNLPPDK | ENSG00000184922.9 | 1 |
| SEQ ID NO: 1333 | FRLAEDGKR | ENSG00000132561.9 | 1 |
| SEQ ID NO: 1334 | FSAEALR | ENSG00000073849.10 | 1 |
| SEQ ID NO: 1335 | FSPEVPGQK | ENSG00000131711.10 | 1 |
| SEQ ID NO: 1336 | FTDFEEVR | ENSG00000106976.14 | 1 |
| SEQ ID NO: 1337 | FVPIIGIAMPLSSR | ENSG00000151835.9 | 1 |
| SEQ ID NO: 1338 | FWPAIDDGLRR | ENSG00000105223.14 | 1 |
| SEQ ID NO: 1339 | FWVVDQTHFYLGSANMDWR | ENSG00000105223.14 | 1 |
| SEQ ID NO: 1340 | GAAVDEYFRQPVVDTFDIR | ENSG00000142453.7 | 1 |
| SEQ ID NO: 1341 | GAFHRPVLGGFR | ENSG00000165912.11 | 1 |
| SEQ ID NO: 1342 | GAGLAWGVHDCQLCSER | ENSG00000090006.13 | 1 |
| SEQ ID NO: 1343 | GAPISAYQIVVEELHPHRT | ENSG00000152894.10 | 1 |
| SEQ ID NO: 1344 | GATGHPGGGQGAENPAGLKSQ GNELFR | ENSG00000104450.8 | 1 |
| SEQ ID NO: 1345 | GCLELIKETGVPIAGR | ENSGO0000100714.11 | 1 |
| SEQ ID NO: 1346 | GCPQEDSDIAFLIDGSGSIIPHDF R | ENSG00000169896.12 | 1 |
| SEQ ID NO: 1347 | GDEGPIGHQGPIGQEGAPGRPG SPGLPGMPGR | ENSG00000134871.13 | 1 |
| SEQ ID NO: 1348 | GDKGERGAPGVTGPK | ENSG00000134871.13 | 1 |
| SEQ ID NO: 1349 | GDNVLINTFSGLLK | ENSG00000142733.10 | 1 |
| SEQ ID NO: 1350 | GDNVLINTFSGLLK | ENSG00000142733.10 | 1 |
| SEQ ID NO: 1351 | GDTGNPGAPGTPGTKGWAGDS GPQGRP | ENSG00000134871.13 | 1 |
| SEQ ID NO: 1352 | GEFAIDGYSVR | ENSG00000005020.8 | 1 |
| SEQ ID NO: 1353 | GEGLYADPYGLLHEGR | ENSG00000017373.11 | 1 |
| SEQ ID NO: 1354 | GEIAPLKENVSHVNDLAR | ENSG00000198947.1D | 1 |
| SEQ ID NO: 1355 | GEWKPRQIDNPDYK | ENSG00000179218.9 | 1 |
| SEQ ID NO: 1356 | GGCVALATGSAMGLWEVK | ENSG00000011028.9 | 1 |
| SEQID NO: 1357 | GGHDIILAAFDNFK | ENSG00000184922.9 | 1 |
| SEQ ID NO: 1358 | GGSQPPDIDKTELVEPTEYLVVHL K | ENSG00000166825.9 | 1 |
| SEQ ID NO: 1359 | GGVSAVPGFR | ENSG00000134871.13 | 1 |
| SEQID NO: 1360 | GHLQIAACPNQDPLQGTTGLIPL LGIDVWEHAY | ENSG00000112096.12 | 1 |
| SEQ ID NO: 1361 | GHPDRLPLQMALTELETLAEK | ENSG0000O104728.11 | 1 |
| SEQ ID NO: 1362 | GKEAGEVR | ENSG00000169896.12 | 1 |
| SEQ ID NO: 1363 | GKNVLINKDIR | ENSG00000179218.9 | 1 |
| SEQ ID NO: 1364 | GLCFLFGSNLR | ENSG00000169896.12 | 1 |
| SEQ ID NO: 1365 | GLEEAVESACAMR | ENSG00000067704.8 | 1 |
| SEQ ID NO: 1366 | GLGKYICQKCHAIIDEQPL | ENSG00000169756.12 | 1 |
| SEQ ID NO: 1367 | GNCFCYGHASECAPAPGAPAHA EGMVHGACICK | ENSG00000172037.9 | 1 |
| SEQ ID NO: 1368 | GPAPARPKMLVISGGDGYEDFRL SSGGGSSS | ENSG00000110237.3 | 1 |
| SEQ ID NO: 1369 | GPGAGSALDDGRR | ENSG00000196961.8 | 1 |
| SEQ ID NO: 1370 | GPPSSVPK | ENSG00000184922.9 | 1 |
| SEQ ID NO: 1371 | GQLQDELEKGER | ENSG00000082805.15 | 1 |
| SEQ ID NO: 1372 | GQTPEAGADKRSPRRASAAAAA GGGATGHPGG | ENSG00000104450.8 | 1 |
| SEQ ID NO: 1373 | GREPASCEDLCGGGVGADGGGS DR | ENSG00000065534.14 | 1 |
| SEQ ID NO: 1374 | GRISVSLQEEASGGSLAAPAR | ENSG00000032444.11 | 1 |
| SEQ ID NO: 1375 | GSDGMDAVRSAPTLIR | ENSG00000150672.12 | 1 |
| SEQ ID NO: 1376 | GSRPGIEGDTPR | ENSG00000113657.8 | 1 |
| SEQ ID NO: 1377 | GTISFFEIDGR | ENSG00000172977.8 | 1 |
| SEQ ID NO: 1378 | GTWIHPEIDNPEYSPD | ENSG00000179218.9 | 1 |
| SEQ ID NO: 1379 | GVTDTLAQIR | ENSG00000017373.11 | 1 |
| SEQ ID NO: 1380 | GWDCHGLPIEIK | ENSG00000067704.8 | 1 |
| SEQ ID NO: 1381 | HCELCRPFFYR | ENSG00000172037.9 | 1 |
| SEQ ID NO: 1382 | HFQIDYDEDGNCSLIISDVCGDD DAK | ENSG00000065534.14 | 1 |
| SEQ ID NO: 1383 | HGGLSLVQTTDYIYPIVDDPYM MGR | ENSG00000086475.10 | 1 |
| SEQ ID NO: 1384 | HLDTLHNFVSR | ENSG00000151914.13 | 1 |
| SEQ ID NO: 1385 | HLNPGLQLYR | ENSG00000114331.8 | 1 |
| SEQ ID NO: 1386 | HTEILEILEIPQLMDTCVR | ENSG00000213380.9 | 1 |
| SEQ ID NO: 1387 | HTLTQIKDAVR | ENSG00000146731.6 | 1 |
| SEQ ID NO: 1388 | IAALNASSTIEDDHEGSFK | ENSG00000099991.12 | 1 |
| SEQ ID NO: 1389 | IAEIQAR | ENSG00000152894.10 | 1 |
| SEQ ID NO: 1390 | IDALREELMEGMDR | ENSG00000132205.6 | 1 |
| SEQ ID NO: 1391 | IFEEQPCLRK | ENSG00000099991.12 | 1 |
| SEQ ID NO: 1392 | IFLTEQPLEGLEK | ENSG00000198947.10 | 1 |
| SEQ ID NO: 1393 | IFSAYIK | ENSG00000130429.8 | 1 |
| SEQ ID NO: 1394 | IIDRIHGTEEGQQILK | ENSG00000137497.13 | 1 |
| SEQ ID NO: 1395 | ILHKGEELAK | ENSG00000169129.10 | 1 |
| SEQ ID NO: 1396 | INELENGGEILNETRSFHHK | ENSG00000059691. 7 | 1 |
| SEQ ID NO: 1397 | IPASAEQIQHLAGAIAER | ENSG00000172037.9 | 1 |
| SEQ ID NO: 1398 | IQGTLQPH | ENSG00000172037.9 | 1 |
| SEQ ID NO: 1399 | IQNQWDEVQEHLQNR | ENSG00000198947.10 | 1 |
| SEQ ID NO: 1400 | IQNVVTSFAPQRRAAWWQSEN GIPA | ENSG00000172037.9 | 1 |
| SEQ ID NO: 1401 | IRQKVDDCERCR | ENSG00000011454.12 | 1 |
| SEQ ID NO: 1402 | ITEQEKLK | ENSG00000151914.13 | 1 |
| SEQ ID NO: 1403 | ITSVSTGNLCTEEQTPPPRPEAYPI PTQTYTR | ENSG00000130396.16 | 1 |
| SEQ ID NO: 1404 | IVLGGTTVHNTK | ENSG00000136631.8 | 1 |
| SEQ ID NO: 1405 | IVTTHIR | ENSG00000106976.14 | 1 |
| SEQ ID NO: 1406 | KDAEGILEDLQSYR | ENSG00000153310.14 | 1 |
| SEQ ID NO: 1407 | KDVEVTKEEFVLAAQK | ENSG00000004864.9 | 1 |
| SEQ ID NO: 1408 | KEADMQQK | ENSG00000158560.10 | 1 |
| SEQ ID NO: 1409 | KHPSSPECLVSAQK | ENSG00000137497.13 | 1 |
| SEQ ID NO: 1410 | KIQNHIQTLK | ENSG00000198947.10 | 1 |
| SEQ ID NO: 1411 | KISEESGETAKRR | ENSG00000099991.12 | 1 |
| SEQ ID NO: 1412 | KIYAVEASTMAQHAEVLVK | ENSG00000142453.7 | 1 |
| SEQ ID NO: 1413 | KKEELNAVR | ENSG00000198947.10 | 1 |
| SEQ ID NO: 1414 | KKGPGAGSALDDGR | ENSG00000196961.8 | 1 |
| SEQ ID NO: 1415 | KLMQIR | ENSG00000151914.13 | 1 |
| SEQ ID NO: 1416 | KLSSQLVEHCQK | ENSG00000198947.10 | 1 |
| SEQ ID NO: 1417 | KLTFEYR | ENSG00000119383.15 | 1 |
| SEQ ID NO: 1418 | KMEEEPLGPDLEDLKR | ENSG00000198947.10 | 1 |
| SEQ ID NO: 1419 | KMSGTVSK | ENSG00000136631.8 | 1 |
| SEQ ID NO: 1420 | KQVAPEKPVKK | ENSG00000113387.7 | 1 |
| SEQ ID NO: 1421 | KSSTGSPTSPLNAEKLESEEDVSQ AF | ENSG00000065534.14 | 1 |
| SEQ ID NO: 1422 | KTRPDGNCFYR | ENSG00000167770.7 | 1 |
| SEQ ID NO: 1423 | KVSTLQNQR | ENSG00000169896.12 | 1 |
| SEQ ID NO: 1424 | LAGEEEALR | ENSG00000125826.15 | 1 |
| SEQ ID NO: 1425 | LCDNIVSESESTTAR | ENSG00000170776.15 | 1 |
| SEQ ID NO: 1426 | LCIEHVEEHGLDIDGIYR | ENSG00000165322.13 | 1 |
| SEQ ID NO: 1427 | LCQFEEAKQDCDQALQLADGNV K | ENSG00000104450.8 | 1 |
| SEQ ID NO: 1428 | LDAWEEAQVEFMASHGNDAAR | ENSG00000105963.9 | 1 |
| SEQ ID NO: 1429 | LDEDLTTLGQMSK | ENSG00000110237.3 | 1 |
| SEQ ID NO: 1430 | LDLFEISQPTEDLEFHGVMR | ENSG00000130396.16 | 1 |
| SEQ ID NO: 1431 | LEAIKR | ENSG00000112096.12 | 1 |
| SEQ ID NO: 1432 | LEMLQQIANR | ENSG00000151914.13 | 1 |
| SEQ ID NO: 1433 | LESEEDVSQAFLEAVAEEKPHVK | ENSG00000065534.14 | 1 |
| SEQ ID NO: 1434 | LESEEDVSQAFLEAVAEEKPHVK PY | ENSG00000065534.14 | 1 |
| SEQ ID NO: 1435 | LETMARNEVIADINCK | ENSG00000141447.12 | 1 |
| SEQ ID NO: 1436 | LEYNVDAANGIVMEGYLFK | ENSG00000114331.8 | 1 |
| SEQ ID NO: 1437 | LFPNSLDQTDMHGDSEYNIMFG PDICGPGTKK | ENSG00000179218.9 | 1 |
| SEQ ID NO: 1438 | LGCTMSMR | ENSG00000059691. 7 | 1 |
| SEQ ID NO: 1439 | LGIEKTDPTTLTDEEINR | ENSG00000100714.11 | 1 |
| SEQ ID NO: 1440 | LGIVNVDEAVLHFK | ENSG00000155629.10 | 1 |
| SEQ ID NO: 1441 | LGYTPLIVACHYGNVK | ENSG00000145362.12 | 1 |
| SEQ ID NO: 1442 | LHEMQIQHPTASLIAK | ENSG00000146731.6 | 1 |
| SEQ ID NO: 1443 | LHYNELGAK | ENSG00000198947.10 | 1 |
| SEQ ID NO: 1444 | LKAVQAQGG ESQQEAQR | ENSG00000137497.13 | 1 |
| SEQ ID NO: 1445 | LKEDMKKIVAVPLNEQK | ENSG00000138640.10 | 1 |
| SEQ ID NO: 1446 | LKEEEEDKKR | ENSG00000179218.9 | 1 |
| SEQ ID NO: 1447 | LKELNDWLTK | ENSG00000198947.10 | 1 |
| SEQ ID NO: 1448 | LKLSFEEMER | ENSG00000162614.14 | 1 |
| SEQ ID NO: 1449 | LKLTFEELER | ENSG00000162614.14 | 1 |
| SEQ ID NO: 1450 | LKPEIQCVSAK | ENSG00000163975.7 | 1 |
| SEQ ID NO: 1451 | LLEATPTDSCGYFR | ENSG00000142733.10 | 1 |
| SEQ ID NO: 1452 | LLEATPTDSCGYFR | ENSG00000142733.10 | 1 |
| SEQ ID NO: 1453 | LLKGESALQR | ENSG00000114331.8 | 1 |
| SEQ ID NO: 1454 | LLNEGQR | ENSG00000163975.7 | 1 |
| SEQ ID NO: 1455 | LNGFQLENFTLK | ENSG00000136231.9 | 1 |
| SEQ ID NO: 1456 | LNKILK | ENSG00000067704.8 | 1 |
| SEQ ID NO: 1457 | LNREVAESPRPR | ENSG00000019144.12 | 1 |
| SEQ ID NO: 1458 | LPPSSPQKLADVAAPPGGPPPPH SPYSGPPSR | ENSG00000017373.11 | 1 |
| SEQ ID NO: 1459 | LQDAFSAIGQNADLDLPQIAVVG GQSAGK | ENSG00000106976.14 | 1 |
| SEQ ID NO: 1460 | LQELEGTYEENERALESK | ENSG00000172037.9 | 1 |
| SEQ ID NO: 1461 | LQQQCDDYGSSYLGVIELIGEK | ENSG00000132205.6 | 1 |
| SEQ ID NO: 1462 | LSAHTHTLSLTDINELVCGAPGD APCATSPCGGAGCR | ENSG00000172037.9 | 1 |
| SEQ ID NO: 1463 | LSFEEMERQRR | ENSG00000162614.14 | 1 |
| SEQ ID NO: 1464 | LSGWLAQQEDAHR | ENSG00000032444.11 | 1 |
| SEQ ID NO: 1465 | LSHFEYVKNEDLEK | ENSG00000061938.12 | 1 |
| SEQ ID NO: 1466 | LSIPQLSVTDYEIM | ENSG00000198947.10 | 1 |
| SEQ ID NO: 1467 | LSIPQLSVTDYEIMEQR | ENSG00000198947.10 | 1 |
| SEQ ID NO: 1468 | LSPAYS LGS LTGAS PCQS PCVQR | ENSG00000019144.12 | 1 |
| SEQ ID NO: 1469 | LSSGGGSSSETVGR | ENSG00000110237.3 | 1 |
| SEQ ID NO: 1470 | LTEEQCLFSAWLSEKEDAVNK | ENSG00000198947.10 | 1 |
| SEQ ID NO: 1471 | LVAAGGLDAVLYWCR | ENSG00000004139.9 | 1 |
| SEQ ID NO: 1472 | LVEFSAFLEQQR | ENSG00000187079.10 | 1 |
| SEQ ID NO: 1473 | LVPSVNGVR | ENSG00000100714.11 | 1 |
| SEQ ID NO: 1474 | LVTPHGESEQIGVIPSKK | ENSG00000082458.7 | 1 |
| SEQ ID NO: 1475 | LVVTQEDVELAYQEAMMNMAR LNRTAAGLMH | ENSG00000086475.10 | 1 |
| SEQ ID NO: 1476 | MAAAEAGGDDAR | ENSG00000184207.8 | 1 |
| SEQ ID NO: 1477 | MAVWEAEQLGGLQR | ENSG00000130589.12 | 1 |
| SEQ ID NO: 1478 | MEALENR | ENSG00000132561.9 | 1 |
| SEQ ID NO: 1479 | MEFDEKELRR | ENSG00000106976.14 | 1 |
| SEQ ID NO: 1480 | MESGRGSSTPPGPIAALGMPDT GPG | ENSG00000127084.13 | 1 |
| SEQ ID NO: 1481 | MESGRGSSTPPGPIAALGMPDT GPGSSSLGK | ENSG00000127084.13 | 1 |
| SEQ ID NO: 1482 | MESQLK | ENSG00000082805.15 | 1 |
| SEQ ID NO: 1483 | MGMSFGLESGK | ENSG00000114126.13 | 1 |
| SEQ ID NO: 1484 | MGNAAGSAEQPAGPAAPPPK | ENSG00000184922.9 | 1 |
| SEQ ID NO: 1485 | MIISTPQRLTSSGSVLIGSPYTPAP AMVTQTHIA | ENSG00000114126.13 | 1 |
| SEQ ID NO: 1486 | MILTNPEGR | ENSG00000152894.10 | 1 |
| SEQ ID NO: 1487 | MKAAKSGTKDGLEK | ENSG00000074964.12 | 1 |
| SEQ ID NO: 1488 | MLEDLGFKDLTLQPR | ENSG00000125826.15 | 1 |
| SEQ ID NO: 1489 | MNSLTLNR | ENSG00000213380.9 | 1 |
| SEQ ID NO: 1490 | MSDKSDLKAELER | ENSG00000158560.10 | 1 |
| SEQ ID NO: 1491 | MSGSSGGAAAPAASSGPAAAAS AAGSGCGGGA | ENSG00000038382.13 | 1 |
| SEQ ID NO: 1492 | MSKSLGNVIHP | ENSG00000067704.8 | 1 |
| SEQ ID NO: 1493 | MVSTSATDEPR | ENSG00000032444.11 | 1 |
| SEQ ID NO: 1494 | NANSSPVASTTPSASATTNPASA TTLDQSKA | ENSG00000166825.9 | 1 |
| SEQ ID NO: 1495 | NATLVNEADKLR | ENSG00000166825.9 | 1 |
| SEQ ID NO: 1496 | NAVLEHMEELQEQVALLTER | ENSG00000184922.9 | 1 |
| SEQ ID NO: 1497 | NDKSYWLSTTAPLPMMPVAEDE IKPYISR | ENSG00000134871.13 | 1 |
| SEQ ID NO: 1498 | NFVKEAEEISSNRR | ENSG00000213380.9 | 1 |
| SEQ ID NO: 1499 | NILVSDMEMNEQQE | ENSG00000011028.9 | 1 |
| SEQ ID NO: 1500 | NLAATLQDIETK | ENSG00000019144.12 | 1 |
| SEQ ID NO: 1501 | NLEELYLVGSLSHDISR | ENSG00000171488.10 | 1 |
| SEQ ID NO: 1502 | NLLEVSEVEQELACQNDHSSALQ NIKR | ENSG00000136631.8 | 1 |
| SEQ ID NO: 1503 | NLVGSGSEIQFLSEAQDDPQKR | ENSG00000115652.10 | 1 |
| SEQ ID NO: 1504 | NRTEAEVKR | ENSG00000169129.10 | 1 |
| SEQ ID NO: 1505 | NSLSVLSPK | ENSG00000171488.10 | 1 |
| SEQ ID NO: 1506 | NTSAASTAQLVEATEELRR | ENSG00000172037.9 | 1 |
| SEQ ID NO: 1507 | NVQVFLISGGFR | ENSG00000146733.9 | 1 |
| SEQ ID NO: 1508 | NYPSSLCALCVGDEQGR | ENSG00000163975.7 | 1 |
| SEQ ID NO: 1509 | PCPCPEGPGSQR | ENSG00000172037.9 | 1 |
| SEQ ID NO: 1510 | PCQDVDECAR | ENSG00000090006.13 | 1 |
| SEQ ID NO: 1511 | PDENLKSASKEELKK | ENSG00000065534.14 | 1 |
| SEQ ID NO: 1512 | PEAYQVPASYQPDEEERAR | ENSG00000125826.15 | 1 |
| SEQ ID NO: 1513 | PEGEMKPGR | ENSG00000113387.7 | 1 |
| SEQ ID NO: 1514 | PETPYSGPGLLIDSLVLLPR | ENSG00000172037.9 | 1 |
| SEQ ID NO: 1515 | PEVVWFK | ENSG00000065534.14 | 1 |
| SEQ ID NO: 1516 | PGAGAVEVAMAEALIK | ENSG00000146731.6 | 1 |
| SEQ ID NO: 1517 | PGEMGPQGPPGEPGFRGAPGK | ENSG00000134871.13 | 1 |
| SEQ ID NO: 1518 | PGETPSWTGSGFVR | ENSG00000172037.9 | 1 |
| SEQ ID NO: 1519 | PGFHGQAAR | ENSG00000172037.9 | 1 |
| SEQ ID NO: 1520 | PGHVGQMGPVGAPGRPGPPGP PGPK | ENSG00000134871.13 | 1 |
| SEQ ID NO: 1521 | PILPHLAEEVFQHIPYIK | ENSG00000067704.8 | 1 |
| SEQ ID NO: 1522 | PKIDDVLHTLTGAMSLLRR | ENSG00000130396.16 | 1 |
| SEQ ID NO: 1523 | PKMLVISGGDGYEDFR | ENSG00000110237.3 | 1 |
| SEQ ID NO: 1524 | PPDIDKTELVEPTEYLVVHLK | ENSG00000166825.9 | 1 |
| SEQ ID NO: 1525 | PPKPATPDFR | ENSG00000065534.14 | 1 |
| SEQ ID NO: 1526 | PPVIQNPEYK | ENSG00000179218.9 | 1 |
| SEQ ID NO: 1527 | PPVLGTESDATVK | ENSG00000065534.14 | 1 |
| SEQ ID NO: 1528 | PQLLGVAPEK | ENSG00000004864.9 | 1 |
| SEQ ID NO: 1529 | PRMSAQEQLERMR | ENSG00000105559.7 | 1 |
| SEQ ID NO: 1530 | PSGPATAEDPGRRPVLPQR | ENSG00000132205.6 | 1 |
| SEQ ID NO: 1531 | PTPRPVPMKRHIFR | ENSG00000186635.10 | 1 |
| SEQ ID NO: 1532 | PVAGSELPR | ENSG00000176890.11 | 1 |
| SEQ ID NO: 1533 | PYWCISR | ENSG00000067704.8 | 1 |
| SEQ ID NO: 1534 | QAASPLEPK | ENSG00000137497.13 | 1 |
| SEQ ID NO: 1535 | QAEEVNTEWEK | ENSG00000198947.10 | 1 |
| SEQ ID NO: 1536 | QAEGLSEDGAAMAVEPTQIQLS K | ENSG00000198947.10 | 1 |
| SEQ ID NO: 1537 | QAPSSFQLLYDLK | ENSG00000100714.11 | 1 |
| SEQ ID NO: 1538 | QAQLEKELSAALQDKK | ENSG00000137497.13 | 1 |
| SEQ ID NO: 1539 | QAQVNLTVVDKPD | ENSG00000065534.14 | 1 |
| SEQ ID NO: 1540 | QDCDQALQLADGNVK | ENSG00000104450.8 | 1 |
| SEQ ID NO: 1541 | QEMVIEVKAIGGKK | ENSG00000110237.3 | 1 |
| SEQ ID NO: 1542 | QETPPPRSPPVANSGSTGFSRRG SGRGGGPTP | ENSG00000105559.7 | 1 |
| SEQ ID NO: 1543 | QGPMTQAINR | ENSG00000170776.15 | 1 |
| SEQ ID NO: 1544 | QHEVEEATNILTATR | ENSG00000114331.8 | 1 |
| SEQ ID NO: 1545 | QIASLTGLVQSALLR | ENSG00000017373.11 | 1 |
| SEQ ID NO: 1546 | QICSQLSER | ENSG00000011454.12 | 1 |
| SEQ ID NO: 1547 | QKASGDSAR | ENSG00000004864.9 | 1 |
| SEQ ID NO: 1548 | QKMEEEKRRTEEER | ENSG00000162614.14 | 1 |
| SEQ ID NO: 1549 | QLELACETQEEVDSWK | ENSG00000106976.14 | 1 |
| SEQ ID NO: 1550 | QLNETGGPVLVSAPISPEEQDKL ENK | ENSG00000198947.10 | 1 |
| SEQ ID NO: 1551 | QLPKPNQDTMQILFR | ENSG00000165322.13 | 1 |
| SEQ ID NO: 1552 | QLQTLAPK | ENSG00000105223.14 | 1 |
| SEQ ID NO: 1553 | QNGDSAYLYLLSAR | ENSG00000125826.15 | 1 |
| SEQ ID NO: 1554 | QPDVEEILSK | ENSG00000198947.10 | 1 |
| SEQ ID NO: 1555 | QQNLAVSESPVTPSALAELLDLLD SR | ENSG00000059691. 7 | 1 |
| SEQ ID NO: 1556 | QQQMHIVDMLSK | ENSG00000130396.16 | 1 |
| SEQ ID NO: 1557 | QSSHNFQLESVNK | ENSG00000135052.12 | 1 |
| SEQ ID NO: 1558 | QTLLAESEALTSYSHR | ENSG00000167608.7 | 1 |
| SEQ ID NO: 1559 | QTSVADLLASFNDQSTSDYLVVY LR | ENSG00000167770.7 | 1 |
| SEQ ID NO: 1560 | QVFGQTTIHQHIPFNWDSEFVQ LHFGK | ENSG00000004864.9 | 1 |
| SEQ ID NO: 1561 | QVVQDLLK | ENSG00000141447.12 | 1 |
| SEQ ID NO: 1562 | RASAAAAAGGGATGHPGGGQG AENPAGLK | ENSG00000104450.8 | 1 |
| SEQ ID NO: 1563 | RCDLCAPGYYGFGPTGCQACQC SHEGALSSLCEK | ENSG00000172037.9 | 1 |
| SEQ ID NO: 1564 | RCEQVQPGYFR | ENSG00000172037.9 | 1 |
| SEQ ID NO: 1565 | RDNEVDGQDYHFVVSR | ENSG00000082458.7 | 1 |
| SEQ ID NO: 1566 | RDPSSNDINGGMEPTPSTVSTPS PSADLLGLR | ENSG00000196961.8 | 1 |
| SEQ ID NO: 1567 | REMAAASAAAISGAGR | ENSG00000079616.8 | 1 |
| SEQ ID NO: 1568 | RETLFTLDDQALGPELTAPAPEPP AEEPR | ENSG00000213380.9 | 1 |
| SEQ ID NO: 1569 | RFSTEYELQQLEQFK | ENSG00000166825.9 | 1 |
| SEQ ID NO: 1570 | RGSDELTVPRYR | ENSG00000017373.11 | 1 |
| SEQ ID NO: 1571 | RIEGSGDQIDTYELSGGAR | ENSG00000106976.14 | 1 |
| SEQ ID NO: 1572 | RKEEEEAEDK | ENSG00000179218.9 | 1 |
| SEQ ID NO: 1573 | RLDIDEKPLVVQLNWNKDDR | ENSG00000130396.16 | 1 |
| SEQ ID NO: 1574 | RPPEPEKAPPAAPTRPSALELK | ENSG00000184922.9 | 1 |
| SEQ ID NO: 1575 | RPRPQGRSVSEPR | ENSG00000125744.7 | 1 |
| SEQ ID NO: 1576 | RQAEGLSEDGAAMAVEPTQIQL SK | ENSG00000198947.10 | 1 |
| SEQ ID NO: 1577 | RRKVPPSGSGGSELSNGEAGEAY R | ENSG00000110237.3 | 1 |
| SEQ ID NO: 1578 | RSLELQTRTEEEKK | ENSG00000127084.13 | 1 |
| SEQ ID NO: 1579 | RSSYLLAITTERSK | ENSG00000225485.3 | 1 |
| SEQ ID NO: 1580 | RVAAQVDGGAQVQQVLNIECLR | ENSG00000196961.8 | 1 |
| SEQ ID NO: 1581 | SAEESDRLR | ENSG00000130396.16 | 1 |
| SEQ ID NO: 1582 | SCDCDPMGSQDGGR | ENSG00000172037.9 | 1 |
| SEQ ID NO: 1583 | SDVLETVVLINPSDEAVSTEVR | ENSG00000131711.10 | 1 |
| SEQ ID NO: 1584 | SEDYELLCPNGAR | ENSG00000163975.7 | 1 |
| SEQ ID NO: 1585 | SFGSSLMESEVNLDR | ENSG00000198947.10 | 1 |
| SEQ ID NO: 1586 | SGHDQVVELLLERGAPLLAR | ENSG00000145362.12 | 1 |
| SEQ ID NO: 1587 | SGLTSLHLAAQEDKVNVADILTK | ENSG00000145362.12 | 1 |
| SEQ ID NO: 1588 | SGRPSCLYSAARPSGSYR | ENSG00000124831.14 | 1 |
| SEQ ID NO: 1589 | SGTIFDNFLITNDEA | ENSG00000179218.9 | 1 |
| SEQ ID NO: 1590 | SGTLALVEPLVASLDPGR | ENSG00000004139.9 | 1 |
| SEQ ID NO: 1591 | SKIVGAPMHDLLLWNNATVTTC HSK | ENSG00000100714.11 | 1 |
| SEQ ID NO: 1592 | SKPEDWDER | ENSG00000179218.9 | 1 |
| SEQ ID NO: 1593 | SLEGSDDAVLLQRRLDNMNFKW SELR | ENSG00000198947.10 | 1 |
| SEQ ID NO: 1594 | SLNPEQWSQLK | ENSG00000113387.7 | 1 |
| SEQ ID NO: 1595 | SLSDPSRRGELAGPGFEGPGGEP IREV | ENSG00000110237.3 | 1 |
| SEQ ID NO: 1596 | SNRDELELELAENR | ENSG00000137497.13 | 1 |
| SEQ ID NO: 1597 | SPARPQPGEGPGGPGGPPEVSR | ENSG00000105559.7 | 1 |
| SEQ ID NO: 1598 | SPARPQPGEGPGGPGGPPEVSR | ENSG00000105559.7 | 1 |
| SEQ ID NO: 1599 | SPDTTLSPASTTSSGVSEESTTSHS R | ENSG00000205277.5 | 1 |
| SEQ ID NO: 1600 | SPDTTLSPASTTSSGVSEESTTSHS R | ENSG00000205277.5 | 1 |
| SEQ ID NO: 1601 | SPDTTLSPASTTSSGVSEESTTSHS R | ENSG00000205277.5 | 1 |
| SEQ ID NO: 1602 | SPFPSQHLEAPEDK | ENSG00000198947.10 | 1 |
| SEQ ID NO: 1603 | SPGPPQVDGTPTMSLERPPR | ENSG00000155629.10 | 1 |
| SEQ ID NO: 1604 | SPTTTLSPASMTSLGVGEESTTSR | ENSG00000205277.5 | 1 |
| SEQ ID NO: 1605 | SPTTTLSPASMTSLGVGEESTTSR | ENSG00000205277.5 | 1 |
| SEQ ID NO: 1606 | SPTTTLSPASMTSLGVGEESTTSR | ENSG00000205277.5 | 1 |
| SEQ ID NO: 1607 | SPTTTLSPASMTSLGVGEESTTSR | ENSG00000205277.5 | 1 |
| SEQ ID NO: 1608 | SQAYADYIGFILTLNEGVK | ENSG00000119383.15 | 1 |
| SEQ ID NO: 1609 | SQMNCNLGTCQLQR | ENSG00000205277.5 | 1 |
| SEQ ID NO: 1610 | SRQELNTIASKPPR | ENSG00000169896.12 | 1 |
| SEQ ID NO: 1611 | SSHVTIDTLK | ENSG00000163975.7 | 1 |
| SEQ ID NO: 1612 | SSQNDSPGDASEGPEYLAIGNLD PRGR | ENSG00000145016.9 | 1 |
| SEQ ID NO: 1613 | STEYELQQLEQFKK | ENSG00000166825.9 | 1 |
| SEQ ID NO: 1614 | STSFNVQDLLPDHEYKFR | ENSG00000065534.14 | 1 |
| SEQ ID NO: 1615 | SVEQEVVQSQLNHCVNLYK | ENSG00000198947.10 | 1 |
| SEQ ID NO: 1616 | SVYTMPLANHR | ENSG00000090006.13 | 1 |
| SEQ ID NO: 1617 | SWAEDEKQKAETVQAALEEAQR | ENSG00000172037.9 | 1 |
| SEQ ID NO: 1618 | SWCSGHLHLRCPR | ENSG00000032444.11 | 1 |
| SEQ ID NO: 1619 | SYVDTGGVSR | ENSG00000184922.9 | 1 |
| SEQ ID NO: 1620 | SYVITGSWNPK | ENSG00000011454.12 | 1 |
| SEQ ID NO: 1621 | TAIWEDQNLR | ENSG00000205277.5 | 1 |
| SEQ ID NO: 1622 | TALLTAGDIYLLSTFR | ENSG00000169231.9 | 1 |
| SEQ ID NO: 1623 | TEALMDAQKEDFNSK | ENSG00000172037.9 | 1 |
| SEQ ID NO: 1624 | TEFCLHDGPPYANGDPHVGHAL NK | ENSG00000067704.8 | 1 |
| SEQ ID NO: 1625 | TESSGGWQNR | ENSG00000011028.9 | 1 |
| SEQ ID NO: 1626 | THIESSGHGVDTCLHVVLSSKVC R | ENSG00000019144.12 | 1 |
| SEQ ID NO: 1627 | TKVHAELADVLTEAVVDSILAIKK | ENSG00000146731.6 | 1 |
| SEQ ID NO: 1628 | TLEIALEQKKEECLK | ENSG00000082805.15 | 1 |
| SEQ ID NO: 1629 | TLNATGEEIIQQSSK | ENSG00000198947.10 | 1 |
| SEQ ID NO: 1630 | TLPSMVHR | ENSG00000101199.8 | 1 |
| SEQ ID NO: 1631 | TMNGDMR | ENSG00000120549.11 | 1 |
| SEQ ID NO: 1632 | TNHIGWVQEFLNEENR | ENSG00000184922.9 | 1 |
| SEQ ID NO: 1633 | TNIQLPACLR | ENSG00000213380.9 | 1 |
| SEQ ID NO: 1634 | TPDELQK | ENSG00000198947.10 | 1 |
| SEQ ID NO: 1635 | TPLERDDLHESVFR | ENSG00000151914.13 | 1 |
| SEQ ID NO: 1636 | TSGNQDEILVIR | ENSG00000106976.14 | 1 |
| SEQ ID NO: 1637 | TTLSPASSTSPGLQGESTAFQTHP ASTHTTPSPPSTATAPVEESTTYH R | ENSG00000205277.5 | 1 |
| SEQ ID NO: 1638 | TTLSPASSTSPGLQGESTAFQTHP ASTHTTPSPPSTATAPVEESTTYH R | ENSG00000205277.5 | 1 |
| SEQ ID NO: 1639 | TTLSPASSTSPGLQGESTAFQTHP ASTHTTPSPPSTATAPVEESTTYH R | ENSG00000205277.5 | 1 |
| SEQ ID NO: 1640 | TTQGLTALLLSLKK | ENSG00000136631.8 | 1 |
| SEQ ID NO: 1641 | TTQIINITMTK | ENSG00000137497.13 | 1 |
| SEQ ID NO: 1642 | TWVQQSETK | ENSG00000198947.10 | 1 |
| SEQ ID NO: 1643 | VAIGPSVLNAAR | ENSG00000067704.8 | 1 |
| SEQ ID NO: 1644 | VAYIPDEMAAQQNPLQQPR | ENSG00000136231.9 | 1 |
| SEQ ID NO: 1645 | VDSDMNDAYLGYAAAIILR | ENSG00000169896.12 | 1 |
| SEQ ID NO: 1646 | VEDAYILTCNVSLEYEK | ENSG00000146731.6 | 1 |
| SEQ ID NO: 1647 | VGAPMHDLLLWNNATVTTCHS K | ENSG00000100714.11 | 1 |
| SEQ ID NO: 1648 | VHLFDIITQYR | ENSG00000213380.9 | 1 |
| SEQ ID NO: 1649 | VIECFNVESR | ENSG00000104728.11 | 1 |
| SEQ ID NO: 1650 | VLGHFEKPLFLELCR | ENSG00000032444.11 | 1 |
| SEQ ID NO: 1651 | VLMDLQNQK | ENSG00000198947.10 | 1 |
| SEQ ID NO: 1652 | VLTTSPSR | ENSG00000019144.12 | 1 |
| SEQ ID NO: 1653 | VMLPPGAQHSDEK | ENSG00000130396.16 | 1 |
| SEQ ID NO: 1654 | VNFRPRYVTRYKTVTQLEWRCCP GFRGGDCQEGPK | ENSG00000132205.6 | 1 |
| SEQ ID NO: 1655 | VPDMAEIQSR | ENSG00000032444.11 | 1 |
| SEQ ID NO: 1656 | VQLLSQYDNEK | ENSG00000184922.9 | 1 |
| SEQ ID NO: 1657 | VSRASSPEGRHLPSPQLGTK | ENSG00000105559.7 | 1 |
| SEQ ID NO: 1658 | VTCTGYHQVR | ENSG00000133316.11 | 1 |
| SEQ ID NO: 1659 | VTEFDAAR | ENSG00000136631.8 | 1 |
| SEQ ID NO: 1660 | VVQEENQHMQMTIQALQDELR | ENSG00000082805.15 | 1 |
| SEQ ID NO: 1661 | VYLDLTPVK | ENSG00000169129.10 | 1 |
| SEQ ID NO: 1662 | WCATSDPEQHK | ENSG00000163975.7 | 1 |
| SEQ ID NO: 1663 | WFSIQNNQLVYQK | ENSG00000114331.8 | 1 |
| SEQ ID NO: 1664 | WIEFCQLLSER | ENSG00000198947.10 | 1 |
| SEQ ID NO: 1665 | WYQNPDYNFFNNYK | ENSG00000073849.10 | 1 |
| SEQ ID NO: 1666 | YADSLKPNIPYK | ENSG00000130396.16 | 1 |
| SEQ ID NO: 1667 | YENHSATAESSR | ENSG00000152894.10 | 1 |
| SEQ ID NO: 1668 | YLITATLTPER | ENSG00000132205.6 | 1 |
| SEQ ID NO: 1669 | YLQQPGCLLVGTNMDNR | ENSG00000184207.8 | 1 |
| SEQ ID NO: 1670 | YLRELSGSGLER | ENSG00000213380.9 | 1 |
| SEQ ID NO: 1671 | YLSASEYGSSVDGHPEVPETK | ENSG00000169129.10 | 1 |
| SEQ ID NO: 1672 | YNASSQQQR | ENSG00000165322.13 | 1 |
| SEQ ID NO: 1673 | YQETMSAIR | ENSG00000198947.10 | 1 |
| SEQ ID NO: 1674 | YSFWLTTIPEQSFQGSPSADTLK | ENSG00000134871.13 | 1 |
| SEQ ID NO: 1675 | YTKQGFGNLPICMAK | ENSG00000100714.11 | 1 |
| SEQ ID NO: 1676 | YVPAIAHLIHSLN | ENSG00000106066.9 | 1 |
| SEQ ID NO: 1677 | AAECLDVDECHRVPPPCDLGR | ENSG00000090006.13 | 0 |
| SEQ ID NO: 1678 | AEGGKRPAR | ENSG00000104450.8 | 0 |
| SEQ ID NO: 1679 | AEPVWTPPAPAPAPAAPPSTPAAP K | ENSG00000115310.13 | 0 |
| SEQ ID NO: 1680 | AFLCPLICHNGGVCVKPDR | ENSG00000090006.13 | 0 |
| SEQ ID NO: 1681 | AHLIHSLNPVR | ENSG00000106066.9 | 0 |
| SEQ ID NO: 1682 | AIAHLIHSLNPVR | ENSG00000106066.9 | 0 |
| SEQ ID NO: 1683 | AIWNVINW | ENSG00000112096.12 | 0 |
| SEQ ID NO: 1684 | AIWNVINWENV | ENSG00000112096.12 | 0 |
| SEQ ID NO: 1685 | ANGITMYAVGVGK | ENSG00000132561.9 | 0 |
| SEQ ID NO: 1686 | AQPVPFVPQVLGVMIGAGVAVV VTAVLILLVVRR | ENSG00000032444.11 | 0 |
| SEQ ID NO: 1687 | ARILTAAR | ENSG00000004139.9 | 0 |
| SEQ ID NO: 1688 | AVGPGAGGAGSAVPGGAGPCA TVSVFPGAR | ENSG00000142453.7 | 0 |
| SEQ ID NO: 1689 | AYDNFGVLGLDLWQVK | ENSG00000179218.9 | 0 |
| SEQ ID NO: 1690 | CVCPAGFR | ENSG00000090006.13 | 0 |
| SEQ ID NO: 1691 | CVHGPTGSR | ENSG00000090006.13 | 0 |
| SEQ ID NO: 1692 | CVPPRTSAGTFPGSQPQAPASPV LPAR | ENSG00000090006.13 | 0 |
| SEQ ID NO: 1693 | DHPSSHSAQPPR | ENSG00000138162.13 | 0 |
| SEQ ID NO: 1694 | DKERLQAMMTHLHVKSTEPK | ENSG00000114861.14 | 0 |
| SEQ ID NO: 1695 | DLDNAEEKADALNK | ENSG00000011454.12 | 0 |
| SEQ ID NO: 1696 | DLYSALIQFFQIFPEYK | ENSG00000106066.9 | 0 |
| SEQ ID NO: 1697 | DPASDKLLGPAGLTWERNLPGA GVGKEMAGVPPTLR | ENSG00000138162.13 | 0 |
| SEQ ID NO: 1698 | DSAVMDDSVVIPSHQVSTLAK | ENSG00000145362.12 | 0 |
| SEQ ID NO: 1699 | DSSTPYQEIAAVPSAGR | ENSG00000138162.13 | 0 |
| SEQ ID NO: 1700 | DWDSPYSHDLDT | ENSG00000105223.14 | 0 |
| SEQ ID NO: 1701 | DWDSPYSHDLDTS | ENSG00000105223.14 | 0 |
| SEQ ID NO: 1702 | EDLDQSPLVSSSDSPPRPQPAFK | ENSG00000115310.13 | 0 |
| SEQ ID NO: 1703 | EESREPAPASPAPA | ENSG00000113657.8 | 0 |
| SEQ ID NO: 1704 | ELSSKGVK | ENSG00000176890.11 | 0 |
| SEQ ID NO: 1705 | EMELRRQALEEERR | ENSG00000019144.12 | 0 |
| SEQ ID NO: 1706 | ENGTVPK | ENSG00000165322.13 | 0 |
| SEQ ID NO: 1707 | ENKEVVLQWFTENSK | ENSG00000166825.9 | 0 |
| SEQ ID NO: 1708 | EVAESPRPR | ENSG00000019144.12 | 0 |
| SEQ ID NO: 1709 | FILDNLK | ENSG00000151835.9 | 0 |
| SEQ ID NO: 1710 | FLEAVAEEKPHVKPYFSK | ENSG00000065534.14 | 0 |
| SEQ ID NO: 1711 | FPIEGGQKDPK | ENSG00000107957.12 | 0 |
| SEQ ID NO: 1712 | FSTEYELQQLEQFKKDNEETGFG SGTR | ENSG00000166825.9 | 0 |
| SEQ ID NO: 1713 | FWPAIDDGLR | ENSG00000105223.14 | 0 |
| SEQ ID NO: 1714 | FYIDFGGVKPMGSEPVPKSR | ENSG00000004864.9 | 0 |
| SEQ ID NO: 1715 | GADLIEEAASRIVDAVIEQVKAAG ALLTEGE | ENSG00000170776.15 | 0 |
| SEQ ID NO: 1716 | GADYAEPTWNLK | ENSG00000166825.9 | 0 |
| SEQ ID NO: 1717 | GDEEKDKGLQTSQDAR | ENSG00000179218.9 | 0 |
| SEQ ID NO: 1718 | GDILQTPQFQMR | ENSG00000137497.13 | 0 |
| SEQ ID NO: 1719 | GDNLPQYR | ENSG00000205277.5 | 0 |
| SEQ ID NO: 1720 | GNEAVASR | ENSG00000135052.12 | 0 |
| SEQ ID NO: 1721 | GPNKHTLTQIKDAVR | ENSG00000146731.6 | 0 |
| SEQ ID NO: 1722 | GQGPMFLDADFVAFTNHFK | ENSG00000198947.10 | 0 |
| SEQ ID NO: 1723 | GTATPELHTATDYR | ENSG00000170776.15 | 0 |
| SEQ ID NO: 1724 | GWAGDSGPQGRPGVFGLPGEK | ENSG00000134871.13 | 0 |
| SEQ ID NO: 1725 | GYLAPSGDLSLRR | ENSG00000090006.13 | 0 |
| SEQ ID NO: 1726 | HAEQQALR | ENSG00000142453.7 | 0 |
| SEQ ID NO: 1727 | IEDPSLLNSR | ENSG00000032444.11 | 0 |
| SEQ ID NO: 1728 | IFMEEVPGGSLSSLLRS | ENSG00000142733.10 | 0 |
| SEQ ID NO: 1729 | IFMEEVPGGSLSSLLRS | ENSG00000142733.10 | 0 |
| SEQ ID NO: 1730 | IIEVAPQVATQNVNPTPGAT | ENSG00000086475.10 | 0 |
| SEQ ID NO: 1731 | ILNSDQTTCR | ENSG00000132561.9 | 0 |
| SEQ ID NO: 1732 | ISCWGHSEPSMR | ENSG00000105223.14 | 0 |
| SEQ ID NO: 1733 | IVVHSVENMNFR | ENSG00000184922.9 | 0 |
| SEQ ID NO: 1734 | KAVAHMK | ENSG00000132561.9 | 0 |
| SEQ ID NO: 1735 | KDITAALAAER | ENSG00000106976.14 | 0 |
| SEQ ID NO: 1736 | KDNEETGFGSGTR | ENSG00000166825.9 | 0 |
| SEQ ID NO: 1737 | KHQGHFLLGTLSR | ENSG00000061938.12 | 0 |
| SEQ ID NO: 1738 | KIAEIQARR | ENSG00000152894.10 | 0 |
| SEQ ID NO: 1739 | KKEADMQQK | ENSG00000158560.10 | 0 |
| SEQ ID NO: 1740 | KLFGGPGSRR | ENSG00000110237.3 | 0 |
| SEQ ID NO: 1741 | KPAAGLSAAPVPTAPAAGAP | ENSG00000115310.13 | 0 |
| SEQ ID NO: 1742 | KSSTGSPTSPLNAEKLESEEDVSQ A | ENSG00000065534.14 | 0 |
| SEQ ID NO: 1743 | KVVATTQMQAADARK | ENSG00000166825.9 | 0 |
| SEQ ID NO: 1744 | LADSDQASKVQQQK | ENSG00000137497.13 | 0 |
| SEQ ID NO: 1745 | LAYVSCVR | ENSG00000032444.11 | 0 |
| SEQ ID NO: 1746 | LGIVQGIVGARNTSAASTAQLVE ATEELRREIG | ENSG00000172037.9 | 0 |
| SEQ ID NO: 1747 | LHYNELGAKVTERKQQ | ENSG00000198947.10 | 0 |
| SEQ ID NO: 1748 | LIEVGPSGAQFLGK | ENSG00000145362.12 | 0 |
| SEQ ID NO: 1749 | LKQTNLQWIK | ENSG00000198947.10 | 0 |
| SEQ ID NO: 1750 | LKTVFYR | ENSG00000104728.11 | 0 |
| SEQ ID NO: 1751 | LLISCWGHSEPSMR | ENSG00000105223.14 | 0 |
| SEQ ID NO: 1752 | LMFDRSEVYGPMK | ENSG00000166825.9 | 0 |
| SEQ ID NO: 1753 | LMLEWQFQK | ENSG00000130396.16 | 0 |
| SEQ ID NO: 1754 | LPAAPPVAPER | ENSG00000115310.13 | 0 |
| SEQ ID NO: 1755 | LPPVLGTESDATVK | ENSG00000065534.14 | 0 |
| SEQ ID NO: 1756 | LPQEPGR | ENSG00000135052.12 | 0 |
| SEQ ID NO: 1757 | LQGQDSERVRAWQR | ENSG00000165912.11 | 0 |
| SEQ ID NO: 1758 | LSRKGGHER | ENSG00000019144.12 | 0 |
| SEQ ID NO: 1759 | LTELENELNTK | ENSG00000130396.16 | 0 |
| SEQ ID NO: 1760 | LTGKAEGGK | ENSG00000104450.8 | 0 |
| SEQ ID NO: 1761 | LWEAVKRR | ENSG00000061938.12 | 0 |
| SEQ ID NO: 1762 | LWHLDPDTEYEIR | ENSG00000152894.10 | 0 |
| SEQ ID NO: 1763 | LYGVVLTPPMK | ENSG00000061938.12 | 0 |
| SEQ ID NO: 1764 | MELEEVTRLLNLKDK | ENSG00000104450.8 | 0 |
| SEQ ID NO: 1765 | MIEDSGPGMKVLL | ENSG00000136631.8 | 0 |
| SEQ ID NO: 1766 | MPVAGSELPR | ENSG00000176890.11 | 0 |
| SEQ ID NO: 1767 | NFVLVLSPGALDK | ENSG00000004139.9 | 0 |
| SEQ ID NO: 1768 | NIMFGPDICGPGTK | ENSG00000179218.9 | 0 |
| SEQ ID NO: 1769 | NITIIVEDPIAESCNDKAKLRGPL | ENSG00000145016.9 | 0 |
| SEQ ID NO: 1770 | NPKAEVARAQAALAVNISAARG LQDVLRTNLGPK | ENSG00000146731.6 | 0 |
| SEQ ID NO: 1771 | NQVTQLK | ENSG00000100714.11 | 0 |
| SEQ ID NO: 1772 | NVINWENVTER | ENSG00000112096.12 | 0 |
| SEQ ID NO: 1773 | PGHYDILYK | ENSG00000167770.7 | 0 |
| SEQ ID NO: 1774 | PGSPGLPGMPGR | ENSG00000134871.13 | 0 |
| SEQ ID NO: 1775 | PLEEGLNKAIHYFR | ENSG00000115652.10 | 0 |
| SEQ ID NO: 1776 | PLSTRVPR | ENSG00000132561.9 | 0 |
| SEQ ID NO: 1777 | PSAGFLPTHR | ENSG00000090006.13 | 0 |
| SEQ ID NO: 1778 | PSGPQPQADLQALLQSGAQVR | ENSG00000105223.14 | 0 |
| SEQ ID NO: 1779 | PSSSGSTGTKLSPARSTTSGLVGE STPSR | ENSG00000205277.5 | 0 |
| SEQ ID NO: 1780 | PSSSGSTGTKLSPARSTTSGLVGE STPSR | ENSG00000205277.5 | 0 |
| SEQ ID NO: 1781 | QGYILNSDQTTCR | ENSG00000132561.9 | 0 |
| SEQ ID NO: 1782 | QVFEELWK | ENSG00000059691.7 | 0 |
| SEQ ID NO: 1783 | QVKPKTVSEEERKV | ENSG00000065534.14 | 0 |
| SEQ ID NO: 1784 | QYISKMIEDSGPGMK | ENSG00000136631.8 | 0 |
| SEQ ID NO: 1785 | QYMPWEAALSSLSYFK | ENSG00000166825.9 | 0 |
| SEQ ID NO: 1786 | RADVLAFPSSGFTDLAEIVSR | ENSG00000032444.11 | 0 |
| SEQ ID NO: 1787 | RAVAAQPGRKR | ENSG00000172977.8 | 0 |
| SEQ ID NO: 1788 | RDEGSQDQTGSLSRARPSSR | ENSG00000110237.3 | 0 |
| SEQ ID NO: 1789 | RDPEVGKDELSKPSSDAESR | ENSG00000138162.13 | 0 |
| SEQ ID NO: 1790 | RMQSSADLIIQEFMDLRTR | ENSG00000151914.13 | 0 |
| SEQ ID NO: 1791 | SASFEPFSNK | ENSG00000179218.9 | 0 |
| SEQ ID NO: 1792 | SDQIGLPDFNAGAMENWGLVT YR | ENSG00000166825.9 | 0 |
| SEQ ID NO: 1793 | SFACQCPEGHVLR | ENSG00000132561.9 | 0 |
| SEQ ID NO: 1794 | SFLKLILQVEKWQEECEEGEGRTI IHCLNGGGR | ENSG00000152894.10 | 0 |
| SEQ ID NO: 1795 | SFPAAQIPIAVEEPGSSSRESVSK AGMPVSADAAK | ENSG00000138162.13 | 0 |
| SEQ ID NO: 1796 | SFTQGEGAR | ENSG00000132561.9 | 0 |
| SEQ ID NO: 1797 | SFTQGEGARPLSTR | ENSG00000132561.9 | 0 |
| SEQ ID NO: 1798 | SHTLSHASYLR | ENSG00000145362.12 | 0 |
| SEQ ID NO: 1799 | SLEQLQK | ENSG00000137497.13 | 0 |
| SEQ ID NO: 1800 | SPHTTLSPAGSTTR | ENSG00000205277.5 | 0 |
| SEQ ID NO: 1801 | SPHTTLSPAGSTTR | ENSG00000205277.5 | 0 |
| SEQ ID NO: 1802 | SPHTTLSPAGSTTR | ENSG00000205277.5 | 0 |
| SEQ ID NO: 1803 | SPHTTLSPAGSTTR | ENSG00000205277.5 | 0 |
| SEQ ID NO: 1804 | SQTLIDLNR | ENSG00000059691.7 | 0 |
| SEQ ID NO: 1805 | SSHNFQLESVNK | ENSG00000135052.12 | 0 |
| SEQ ID NO: 1806 | STCAPSPQR | ENSG00000138162.13 | 0 |
| SEQ ID NO: 1807 | STTFYSSPR | ENSG00000205277.5 | 0 |
| SEQ ID NO: 1808 | STTFYSSPR | ENSG00000205277.5 | 0 |
| SEQ ID NO: 1809 | STTFYSSPR | ENSG00000205277.5 | 0 |
| SEQ ID NO: 1810 | STTFYSSPR | ENSG00000205277.5 | 0 |
| SEQ ID NO: 1811 | STTFYSSPR | ENSG00000205277.5 | 0 |
| SEQ ID NO: 1812 | STTFYSSPR | ENSG00000205277.5 | 0 |
| SEQ ID NO: 1813 | STTFYSSPR | ENSG00000205277.5 | 0 |
| SEQ ID NO: 1814 | STTFYSSPR | ENSG00000205277.5 | 0 |
| SEQ ID NO: 1815 | STTFYSSPR | ENSG00000205277.5 | 0 |
| SEQ ID NO: 1816 | TATAGAISELTESRLR | ENSG00000128487.12 | 0 |
| SEQ ID NO: 1817 | TEVAIGPSVLNAAR | ENSG00000067704.8 | 0 |
| SEQ ID NO: 1818 | TGDPQETLRR | ENSG00000137497.13 | 0 |
| SEQ ID NO: 1819 | THLSLSHNPEQKGVPTGFILPIRDI R | ENSG00000100714.11 | 0 |
| SEQ ID NO: 1820 | THTATGIR | ENSG00000169896.12 | 0 |
| SEQ ID NO: 1821 | TLATQLNQQK | ENSG00000151914.13 | 0 |
| SEQ ID NO: 1822 | TPVPEKVPPPKPATPDF | ENSG00000065534.14 | 0 |
| SEQ ID NO: 1823 | TVQQPTVQHR | ENSG00000132561.9 | 0 |
| SEQ ID NO: 1824 | TYQGFWNPPLAPR | ENSG00000152894.10 | 0 |
| SEQ ID NO: 1825 | VLCGDAGLLRGLADGLVQAGVG TEALLTPLVGRLARL | ENSG00000142733.10 | 0 |
| SEQ ID NO: 1826 | VLCGDAGLLRGLADGLVQAGVG TEALLTPLVGRLARL | ENSG00000142733.10 | 0 |
| SEQ ID NO: 1827 | VNYDEENWRK | ENSG00000166825.9 | 0 |
| SEQ ID NO: 1828 | VPEGFTCR | ENSG00000090006.13 | 0 |
| SEQ ID NO: 1829 | WSELRKKSLNIR | ENSG00000198947.10 | 0 |
| SEQ ID NO: 1830 | WSSRGSGGWGVYRSPSFGAGE GLLR | ENSG00000110237.3 | 0 |
| SEQ ID NO: 1831 | WYQPSFHGVDLSALR | ENSG00000142453.7 | 0 |
| SEQ ID NO: 1832 | YCNPGDVCYYASR | ENSG00000134871.13 | 0 |
| SEQ ID NO: 1833 | YGNLGHVNIGAIQEPLAFILPK | ENSG00000213380.9 | 0 |
| SEQ ID NO: 1834 | YITISGNR | ENSG00000151914.13 | 0 |
| SEQ ID NO: 1835 | YLSYTLNPDLIRK | ENSG00000166825.9 | 0 |
| SEQ ID NO: 1836 | YMVTER | ENSG00000105223.14 | 0 |

To examine possible functions of somatic promoters on cancer development, we focused on *RASA3,* a RAS GTPase-activating protein required for G_{αi}-induced inhibition of mitogen-activated protein kinases. In both GCs (50%) and GC lines, we observed gain of promoter activity at an intronic region 127kb downstream apart from the canonical *RASA3* TSS (Fig. 3c, top, Figure 10). RNA-seq and 5' RACE analysis confirmed expression of this shorter *RASA3* isoform (Fig. 3c, bottom), and expression of this shorter *RASA3* isoform was also observed in TCGA RNA-seq data (Fig. 3c). Compared to the canonical full-length *RASA3* protein (CanT), the shorter 31 kDa *RASA3* somatic isoform (SomT) is predicted to lack the N-terminal RasGAP domain (Fig. 3d). Consistent with these predictions, transection of *RASA3* CanT into GES1 normal gastric epithelial cells induced lower levels of active GTP-bound RAS compared to either empty vector or *RASA3* SomT transfected cells, indicating that RASA3 CanT has higher RASGAP activity (Figure 13).

To address functions of *RASA3* SomT, we transfected the *RASA3* CanT and SomT isoforms into SNU1967 GC cells. Compared to untransfected cells, transfection of *RASA3* SomT into SNU1967 cells significantly stimulated migration (P<0.01) and invasion (P<0.01) while *RASA3* CanT significantly suppressed invasion (P<0.001) (Fig. 3E, Figure 13). Similarly, transfection of *RASA3* SomT into GES1 cells significantly stimulated migration (p<0.01, Fig. 3e) and invasion (P<0.01, Figure 13) while *RASA3* CanT did not. When tested on *KRAS* mutated AGS GC cells that are innately highly migratory, expression of *RASA3* CanT potently suppressed migration while *RASA3* SomT exhibited significantly less attenuation (P<0.01, Figure 13). These results suggest that tumor-specific use of *RASA3* SomT is likely to increase GC cell migration and invasion. Notably, *RASA3* CanT and SomT transfections did not alter SNU1967, GES1 or AGS cellular proliferation rates (Figure 13). To confirm that these observations are not due to non-physiological *in vitro* expression levels, we then examined NCC24 GC cells, which normally express high endogenous levels of *RASA3* SomT and minimal *RASA3* CanT (Figure 13). Silencing of endogenous *RASA3* SomT using two independent siRNA constructs significantly inhibited NCC24 migration and invasion (P<0.01-0.001) (Figure 13), consistent with *RASA3* SomT playing a role in promoting cancer migration and invasion.

In an earlier study, we reported a transcript isoform of the *MET* receptor tyrosine kinase, driven by an internal alternative promoter, which has been independently confirmed in other cancer types. However, functional implications of this *MET* variant remain unclear. RNA-seq and 5' RACE analysis confirmed transcript expression of this shorter isoform, predicted to harbor a truncated SEMA domain (Figure 14). To assess functional differences between wild type (WT) and variant (Var) MET, we performed transient transfections of MET(WT) and MET(Var) into HEK293 cells. In both untreated and HGF-treated conditions, MET-Var transfected cells exhibited significantly higher levels of p-Gab1 (Y627), a key mediator of MET signaling (e.g. 2.48-3.95 fold comparing MET-Var vs MET-WT, P=0.003 (untreated), P<0.05 (T15 and T30).(66) In addition, in HGF-untreated samples, cells transfected with MET-Var also exhibited higher p-ERK1/2 levels (2.74 fold) and also higher p-STAT3 (Y705)(67-70) levels (1.80 fold) compared to MET-WT (P=0.023 and P=0.026 for p-ERK and p-STAT3 (Y705) respectively). These results suggest that expression of the MET Var isoform may promote MET-downstream signaling kinetics in a manner important for GC tumorigenesis.

### Somatic Promoters Correlate with Tumor Immunity

Cancer immunoediting is a process where developing tumors sculpt their immunogenic and antigenic profile to evade host immune surveillance. Mechanisms of cancer immunoediting are diverse, including upregulation of immune checkpoint inhibitors such as *PD-L1.* To explore potential contributions of somatic promoters to tumor immunity, we identified somatic promoter-associated N-terminal peptides with high predicted affinity binding to GC specific MHC Class I HLA alleles (Table 8 and 9), which are required for antigen presentation to CD8+ cytotoxic T cells (IC50 ≤ 50nM, Fig. 4a). Analysis of recurrent somatic promoter-associated peptides using the NetMHCpan-2.8 algorithm revealed a significant enrichment in high-affinity MHC I binding compared to multiple control peptide populations, including canonical GC peptides (average 36% vs 24%; P<0.01), randomly selected peptides (P<0.001), and C-terminal peptides (P<0.01) (Fig. 4B shows HLA-A, B, and C combined, Figure 15A depicts data for HLA-A only). The majority of high affinity somatic promoter-associated peptides corresponded to situations where the somatic transcript lacking the N-terminal peptide is overexpressed in tumors relative to normal tissues (78% lost; 76/97 high-affinity peptides, Fig. 4C). Notably, because transcripts driven by the N-terminal lacking somatic TSSs are also overexpressed in tumors to a significantly greater degree than transcripts driven by the canonical TSS (P<0.05, Wilcoxon one sided test) (Figure 12), such a scenario would be predicted to result in relative depletion of these N-terminal immunogenic peptides in tumors. Interestingly, an analogous N-terminal analysis using RNA-seq data alone (in the absence of epigenomic data) revealed that epigenome-guided N-terminal peptides exhibited significantly higher predicted immunogenicity scores compared to RNA-seq-only identified peptides (36.10% vs 27% for MHC presentation, P=0.02, Fisher Test), suggesting that epigenome-guided promoter identification can provide complementary value to RNA-seq-only guided analyses (Figure 15).

**Table 8: HLA prediction of GC samples**

| **Sample** | **A1** | **A2** | **B1** | **B2** | **C1** | **C2** |
|---|---|---|---|---|---|---|
| 2000639 | A*33:03 | A*24:02 | B*58:01 | B*40:01 | C*03:02 | C*03:67 |
| 2000721 | A*11:01 | A*11:01 | B*46:01 | B*15:01 | C*01:02 | C*04:01 |
| 2000986 | A*24:02 | A*11:01 | B*40:01 | B*38:02 | C*07:02 | C*15:02 |
| 980437 | A*33:03 | A*02:07 | B*40:01 | B*39:01 | C*07:02 | C*04:01 |
| 990068 | A*02:03 | A*11:01 | B*51:01 | B*55:02 | C*08:01 | C*14:02 |
| 2000085 | A*24:07 | A*34:01 | B*15:21 | B*15:21 | C*04:03 | C*04:03 |
| 980401 | A*33:03 | A*11:01 | B*58:01 | B*40:01 | C*03:02 | C*07:02 |
| 980447 | A*11:01 | A*11:01 | B*38:02 | B*27:04 | C*12:02 | C*07:02 |
| 2001206 | A*02:07 | A*24:02 | B*46:01 | B*40:06 | C*01:02 | C*08:01 |
| 980436 | A*02:03 | A *02:07 | B*46:01 | B*46:01 | C*01:02 | C*01:02 |
| 980417 | A*33:03 | A*11:01 | B*58:01 | B*46:01 | C*03:02 | C*01:02 |
| 980319 | A*33:03 | A*11:02 | B*58:01 | B*27:04 | C*03:02 | C*12:02 |
| 20021007 | A*24:10 | A*24:02 | B*15:27 | B*40:01 | C*03:04 | C*04:01 |

**Table 9: Recurrent N terminal sequences with high affinity to MHC Class I**

| SEQ ID NO. | Gene | N terminal sequence | High Affinity HLA |
|---|---|---|---|
| SEQ ID NO: 1847 | ENSG00000007171.12 | MACPWKFLFKTKFHQYA MNGEKDINNNVEKAPCAT SSPVTQDDLQYHNLSKQQ NESPQPLVETGKKSPESLVK LDATPLSSPRHVRIKNWGS GMTFQDTLHHKAKGILTCR SKSCLGSIMTPKSLTRGPRD KPTPPDELLPQAIEFVNQYY GSFKEAKIEEHLARVEAVTK EIETTGTYQLTGDELIFATK QAWRNAPRCIGRIQWSNL QVFDARSCSTARE | A*02:03,A*02:07,A*11:0 1,A*11:02,A*24:10,A*34: 01,B*15:01,B*15:21,B*1 5:27,B*27:04,B*39:01,B* 40:01,B*46:01, B*58:01,C *03:02,C*12:02 |
| SEQ ID NO: 1848 | ENSG00000011028.9 | MGPGRPAPAPWPRHLLRC VLLLGCLHLGRPGAPGDAA LPEPNVFLIFSHGLQGCLEA QGGQVRVTPACNTSLPAQ RWKWVSRNRLFNLGTMQ CLGTGWPGTNTTASLGMY ECDREALNLRWHCRTLGD QLSLLLGARTSNISKPGTLE RGDQTRSGQWRIYGSEED LCALPYHEVYTIQGNSHGK PCTIPFKYDNQWFHGCTST GREDGHLWCATTQDYGK DERWGFCPIKSNDCETFW DKDQLTDSCYQFNFQSTLS WREAWASCEQQGADLLSI TEIHEQTYINGLLTGYSSTL WIGLNDLDTSGGWQWSD NSPLKYLNWESDQPDNPS EENCGVIRTESSGGWQNR DCSIALPYVCKKKPNATAEP TPPDRWANVKVECEPSW QPFQGHCYRLQAEKRSW QESKKACLRGGGDLVSIHS MAELEFITKQIKQEVEELWI | A*02:03,A*11:01,A*11:0 2,A*24:02,A*24:07,A*24: 10,A*33:03,B*15:01,B*1 5:27,B*38:02,B*39:01, B* 40:01, B*40:06,B*51:01,B *58:01,C*03:02,C*03:04, C*12:02,C*14:02 |
| | | GLNDLKLQMNFEWSDGSL VSFTHWHPFEPNNFRDSLE DCVTIWGPEGRWNDSPC NQSLPSICKKAGQLSQGAA EEDHGCRKGWTWHSPSC YWLGEDQVTYSEARRLCT DHGSQLVTITNRFEQAFVS SLIYNWEGEYFWTALQDL NSTGSFFWLSGDEVMYTH WNRDQPGYSRGGCVALA TGSAMGLWEVKNCTSFRA RYICRQSLGTPVTPELPGPD PTPSLTGSCPQGWASDTKL RYCYKVFSSERLQDKKSWV QAQGACQELGAQLLSLASY EEEHFVANMLNKIFGESEP EIHEQHWFWIGLNRRDPR GGQSWRWSDGVGFSYHN FDRSRHDDDDIRGCAVLDL ASLQWVAMQCDTQLDWI CKIPRGTDVREPDDSPQGR REWLRFQEAEYKFFEHHST WAQAQRICTWFQAELTSV HSQAELDFLSHNLQKFSRA QEQHWWIGLHTSESDGRF RWTDGSIINFISWAPGKPR PVGKDKKCVYMTASRED WGDQRCLTALPYICKRSNV TKETQPPDLPTTALGGCPS DWIQFLNKCFQVQGQEPQ SRVKWSEAQFSCEQQEAQ LVTITNPLEQAFITASLPNV TFDLWIGLHASQRDFQWV EQEPLMYANWAPGEPSG PSPAPSGNKPTSCAVVLHS PSAHFTGRWDDRSCTEET HGFICQKGTDPSLSPSPAAL PPAPGTELSYLNGTFRLLQK PLRWHDALLLCESRNASLA YVPDPYTQAFLTQAARGLR TPLWIGLAGEEGSRRYSW VSEEPLNYVGWQDGEPQ QPGGCTYVDVDGAWRTT SCDTKLQGAVCGVSSGPPP PRRISYHGSCPQGLADSA WIPFREHCYSFHMELLLGH KEARQRCQRAGGAVLSILD EMENVFVWEHLQSYEGQS RGAWLGMNFNPKGGTLV | |
| | | WQDNTAVNYSNWGPPGL GPSMLSHNSCYWIQSNSG LWRPGACTNITMGVVCKL PRAEQSSFSPSALPENPAAL VVVLMAVLLLLALLTAALIL YRRRQSIERGAFEGARYSR SSSSPTEATEKNILVSDME MNEQQE | |
| SEQ ID NO: 1849 | ENSG00000020256.15 | MNASSEGESFAGSVQIPG GTTVLVELTPDIHICGICKQ QFNNLDAFVAHKQSGCQL TGTSAAAPSTVQFVSEETV PATQTQTTTRTITSETQTIT VSAPEFVFEHGYQTY | A*02:03,B*15:01,C*03:0 2,C*03:04 |
| SEQ ID NO: 1850 | ENSG00000032389.8 | MEDDAPVIYGLEFQARALT PQTAETDAIRFLVGTQSLKY DNQIHIIDFDDENNIINKNV LLHQAGEIWHISASPADRG VLTTCYNRRDIIESFGILPVA QSPTIVFVNTLHQVFFRGQ VAASDSKVLTCAAVWR | A*02:03,A*24:07,A*24:1 0,A*33:03,B*15:01,B*15: 21,B*15:27,B*38:02,B*3 9:01,B*40:01,B*40:06,B* 46:01,B*51:01,B*55:02,B *58:01,C*01:02,C*03:02, C*03:04,C*03:67,C*04:0 1,C*08:01,C*12:02,C*14: 02,C*15:02 |
| SEQ ID NO: 1851 | ENSG00000037042.8 | MLEAILGGGGLPVEGRGST EFEAFRLILFGSEDSVLPSPL LYKMAHMGSDGGVLPVH YATILFSL | A*02:03,A*11:01,A*11:0 2,A*24:02,A*24:07,A*24: 10,B*40:01,B*40:06,B*5 1:01,C*01:02,C*04:03,C* 08:01,C*14:02 |
| SEQ ID NO: 1852 | ENSG00000053747.11 | MAAAARPRGRALGPVLPP TPLLLLVLRVLPACGATARD PGAAAGLSLHPTYFNLAEA ARIWATATCGERGPGEGR PQPELYCKLVGGPTAPGSG HTIQGQFCDYCNSEDPRKA HPVTNAIDGSERWWQSPP LSSGTQYNRVNLTLDLGQL FHVAYILIKFANSPRPDLWV LERSVDFGSTYSPWQYFAH SKVDCLKEFGREANMAVT RDDDVLCVTEYSRIVPLEN GEVVVSLINGRPGAKNFTF SHTLREFTKATNIRLRFLRT NTLLGHLISKAQRDPTVTR RYYYSIKDISIGGQCVCNGH AEVCNINNPEKLFRCECQH HTCGETCDRCCTGYNQRR WRPAAWEQSHECEACNC HGHASNCYYDPDVERQQA SLNTQGIYAGGGVCINCQH | A*02:03,A*11:01,A*11:0 2,A*24:02,A*24:07,A*24: 10,A*33:03,B*15:01,B*3 9:01,B*40:01,B*55:02,B* 58:01,C*03:02,C*03:04,C *03:67,C*07:02,C*12:02, C*14:02,C*15:02 |
| | | NTAGVNCEQCAKGYYRPY GVPVDAPDGCIPCSCDPEH ADGCEQGSGRCHCKPNFH GDNCEKCAIGYYNFPFCLRI PIFPVSTPSSEDPVAGDIKG CDCNLEGVLPEICDAHGRC LCRPGVEGPRCDTCRSGFY SFPICQACWCSALGSYQM PCSSVTGQCECRPGVTGQ RCDRCLSGAYDFPHCQGSS SACDPAGTINSNLGYCQCK LHVEGPTCSRCKLLYWNLD KENPSGCSECKCHKAGTVS GTGECRQGDGDCHCKSHV GGDSCDTCEDGYFALEKSN YFGCQGCQCDIGGALSSM CSGPSGVCQCREHVVGKV CQRPENNYYFPDLHHMKY EIEDGSTPNGRDLRFGFDP LAFPEFSWRGYAQMTSVQ NDVRITLNVGKSSGSLFRVI LRYVNPGTEAVSGHITIYPS WGAAQSKEIIFLPSKEPAFV TVPGNGFADPFSITPGIWV ACIKAEGVLLDYLVLLPRDY YEASVLQLPVTEPCAYAGP PQENCLLYQHLPVTRFPCT LACEARHFLLDGEPRPVAV RQPTPAHPVMVDLSGREV ELHLRLRIPQVGHYVVVVE YSTEAAQLFVVDVNVKSSG SVLAGQVNIYSCNYSVLCR SAVIDHMSRIAMYELLADA DIQLKGHMARFLLHQVCII PIEEFSAEYVRPQVHCIASY GRFVNQSATCVSLAHETPP TALILDVLSGRPFPHLPQQS SPSVDVLPGVTLKAPQNQ VTLRGRVPHLGRYVFVIHF YQAAHPTFPAQVSVDGG WPRAGSFHASFCPHVLGC RDQVIAEGQIEFDISEPEVA ATVKVPEGKSLVLVRVLVV PAENYDYQILHKKSMDKSL EFITNCGKNSFYLDPQTASR FCKNSARSLVAFYHKGALP CECHPTGATGPHCSPEGG QCPCQPNVIGRQCTRCAT GHYGFPRCKPCSCGRRLCE | |
| | | EMTGQCRCPPRTVRPQCE VCETHSFSFHPMAGCEGC NCSRRGTIEAAMPECDRDS GQCRCKPRITGRQCDRCAS GFYRFPECVPCNCNRDGTE PGVCDPGTGACLCKENVE GTECNVCREGSFHLDPANL KGCTSCFCFGVNNQCHSS HKRRTKFVDMLGWHLETA DRVDIPVSFNPGSNSMVA DLQELPATIHSASWVAPTS YLGDKVSSYGGYLTYQAKS FGLPGDMVLLEKKPDVQLT GQHMSIIYEETNTPRPDRL HHGRVHVVEGNFRHASSR APVSREELMTVLSRLADVRI QGLYFTETQRLTLSEVGLEE ASDTGSGRIALAVEICACPP AYAGDSC | |
| SEQ ID NO: 1853 | ENSG00000059145.14 | MPSVSKAAAAALSGSPPQ TEKPTHYRYLKEFRTEQCPL FSQHKCAQHRPFTCFHWH FLNQRRRRPLRRRDGTFNY SPDVYCSKYNEATGVCPDG DECPYLHRTTGDTERKYHL RYYKTGTCIHETDARGHCV KNGLHCAFAHGPLDLRPPV CDVRELQAQEALQNGQLG GGEGVPDLQPGVLASQA MIEKILSEDPRWQDANFVL GSYKTEQCPKPPRLCRQGY ACPHYHNSRDRRRNPRRF QYRSTPCPSVKHGDEWGE PSRCDGGDGCQYCHSRTE QQFHPESTKCNDMRQTGY CPRGPFCAFAHVEKSLGM VNEWGCHDLHLTSPSSTG SGQPGNAKRRDSPAEGGP RGSEQDSKQNHLAVFAAV HPPAPSVSSSVASSLASSAG SGSSSPTALPAPPARALPLG PASSTVEAVLGSALDLHLS NVNIASLEKDLEEQDGHDL GAAGPRSLAGSAPVAIPGS LPRAPSLHSPSSASTSPLGS LSQPLPGPVGSSA | A*02:03,A*24:10,A*33:0 3,B*15:01,B*39:01,B*40: 01,B*58:01,C*03:02,C*0 3:04,C*15:02 |
| SEQ ID NO: 1854 | ENSG00000060656.15 | MARAQALVLALTFQLCAPE TETPAAGCTFEEASDPAVP CEYSQAQYDDFQWEQVRI | A*02:03,A*11:01,A*11:0 2,A*24:02,A*24:10,A*33: 03,A*34:01,B*15:01,B*15:27,B*38:02,B*39:01,B* 40:01,B*55:02,B*58:01,C *03:02,C*03:04,C*07:02, C*12:02,C*14:02,C*15:02 |
| | | HPGTRAPADLPHGSYLMV NTSQHAPGQRAHVIFQSLS ENDTHCVQFSYFLYSRDGH SPGTLGVYVRVNGGPLGS AVWNMTGSHGRQWHQA ELAVSTFWPNEYQVLFEALI SPDRRGYMGLDDILLLSYP CAKAPHFSRLGDVEVNAG QNASFQCMAAGRAAEAE RFLLQRQSGALVPAAGVR HISHRRFLATFPLAAVSRAE QDLYRCVSQAPRGAGVSN FAELIVKEPPTPIAPPQLLRA GPTYLIIQLNTNSIIGDGPIV RKEIEYRMARGPWAEVHA VSLQTYKLWHLDPDTEYEI SVLLTRPGDGGTGRPGPPL ISRTKCAEPMRAPKGLAFA EIQARQLTLQWEPLGYNVT RCHTYTVSLCYHYTLGSSH NQTIRECVKTEQGVSRYTIK NLLPYRNVHVRLVLTNPEG RKEGKEVTFQTDEDVPSGI AAESLTFTPLEDMIFLKWEE PQEPNGLITQYEISYQSIESS DPAVNVPGPRRTISKLRNE TYHVFSNLHPGTTYLFSVR ARTGKGFGQAALTEITTNIS APSFDYADMPSPLGESENT ITVLLRPAQGRGAPISVYQV IVEEERARRLRREPGGQDC FPVPLTFEAALARGLVHYF GAELAASSLPEAMPFTVGD NQTYRGFWNPPLEPRKAY LIYFQAASHLKGETRLNCIRI ARKAACKESKRPLEVSQRS EEMGLILGICAGGLAVLILLL GAIIVIIRKGKPVNMTKATV NYRQEKTHMMSAVDRSFT DQSTLQEDERLGLSFMDT HGYSTRGDQRSGGVTEAS SLLGGSPRRPCGRKGSPYH TGQLHPAVRVADLLQHIN QMKTAEGYGFKQEYESFFE GWDATKKKDKVKGSRQEP MPAYDRHRVKLHPMLGD PNADYINANYIDGYHRSNH FIATQGPKPEMVYDFWR MVWQEHCSSIVMITKLVE | |
| | | VGRVKCSRYWPEDSDTYG DIKIMLVKTETLAEYVVRTF ALERRGYSARHEVRQFHFT AWPEHGVPYHATGLLAFIR RVKASTPPDAGPIVIHCSA GTGRTGCYIVLDVMLDMA ECEGVVDIYNCVKTLCSRR VNMIQTEEQYIFIHDAILEA CLCGETTIPVSEFKATYKEM IRIDPQSNSSQLREEFQTLN SVTPPLDVEECSIALLPRNR DKNRSMDVLPPDRCLPFLI STDGDSNNYINAALTDSYT RSAAFIVTLHPLQSTTPDF WRLVYDYGCTSIVMLNQL NQSNSAWPCLQYWPEPG RQQYGLMEVEFMSGTAD EDLVARVFRVQNISRLQEG HLLVRHFQFLRWSAYRDTP DSKKAFLHLLAEVDKWQA ESGDGRTIVHCLNGGGRS GTFCACATVLEMIRCHNLV DVFFAAKTLRNYKPNMVE TMDQYHFCYDVALEYLEGL ESR | |
| SEQ ID NO: 1855 | ENSG00000066248.10 | METRESEDLEKTRRKSASD QWNTDNEPAKVKPELLPE KEETSQADQDIQDKEPHC HIPIKRNSIFNRSIRRKSKAK ARDNPERNASCLADSQDN GKSVNEPLTLNIPWSRMPP CRT | A*02:03,A*11:01,A*11:0 1,A*11:02,A*11:02,A*24: 02,A*24:10,A*33:03,A*3 3:03,A*34:01,B*15:01,B* 15:21,B*15:27,B*39:01,B *40:01,B*46:01, B*58:01, C*03:02,C*03:04,C*03:6 7,C*12:02,C*14:02 |
| SEQ ID NO: 1856 | ENSG00000077092.14 | MTTSGHACPVPAVNGHM THYPATPYPLLFPPVIGGLS LPPLHGLHGHPPPSGCSTP SPATIETQS | A*24:02,A*24:07,A*24:1 0,A*34:01,B*15:01,B*15: 21,B*15:27,B*46:01,B*5 1:01,B*55:02,C*01:02,C* 03:02,C*04:01,C*07:02,C *12:02,C*14:02 |
| SEQ ID NO: 1857 | ENSG00000079308.12 | MTRLSWCFSCVIRWGKYL FSCLLPLRFCLRSQPEDLEA PKTHRFKVKTFKKVKPCGIC RQVITQEGCTCKVCSFSCH RKCQAKVAAPCVPPSNHE LVPITTENAPKNVVDKGEG ASRGGNTRKSLEDNGSTRV TPSVQPHLQPIRN | A*02:03,A*02:07,B*27:0 4,B*39:01,B*46:01,C*01: 02,C*03:02,C*03:04,C*0 3:67,C*08:01,C*14:02 |
| SEQ ID NO: 1858 | ENSG00000080823.17 | MKNYKAIGKIGEGTFSEVM KMQSLRDGNYYACKQMK | A*02:03,A*33:03,B*40:0 1,C*03:02,C*14:02 |
| | | QRFESIEQVNNLREIQALRR LNPHPNILMLHEVVFDRKS GSLALICELMDMNIYELIRG RRYPLSEKKIMHYMYQLCK SLDHIHRNGIFHRDVKPENI LIKQDVLKLGD | |
| SEQ ID NO: 1859 | ENSG00000097021.15 | MARPGLIHSAPGLPDTCAL LQPPAASAAAAPS | A*02:03 |
| SEQ ID NO: 1860 | ENSG00000100441.5 | MPTWGARPASPDRFAVSA EAENKVREQQPHVERIFSV GVSVLPKDCPDNPHIWLQ LEGPKENASRAKEYLKGLCS PELQDEIHYPPKLHCIFLGA QGFFLDCLAWSTSAHLVPR APGSLMISGLTEAFVMAQS RVEELAERLSWDFTPGPSS GASQCTGVLRDFSALLQSP GDAHREALLQLPLAVQEEL LSLVQEASSGQGPGALAS WEGRSSALLGAQCQGVRA PPSDGRESLDTGSMGPGD CRGARGDTYAVEKEGGKQ GGPREMDWGWKELPGEE AWEREVALRPQSVGGGAR ESAPLKGKALGKEEIALGG GGFCVHREPPGAHGSCHR AAQSRGASLLQRLHNGNA SPPRVPSPPPAPEPPWHC GDRGDCGDRGDVGDRGD KQQGMARGRGPQWKRG ARGGNLVTGTQRFKEALQ DPFTLCLANVPGQPDLRHI VIDGSNVAMVHGLQHYFS SRGIAIAVQYFWDRGHRDI TVFVPQWRFSKDAKVRES HFLQKLYSLSLLSLTPSRVM DGKRISSYDDRFMVKLAEE TDGIIVSNDQFRDLAEESEK W | A*02:03,A*02:07,A*11:0 1,A*11:02,A*24:02,A*24: 07,A*24:10,A*33:03, B*1 5:01,B*15:21,B*15:27,B* 40:01,B*40:06,B*55:02,B *58:01,C*03:02,C*03:04, C*03:67,C*04:01,C*04:0 3,C*07:02,C*08:01,C*14: 02,C*15:02 |
| SEQ ID NO: 1861 | ENSG00000103056.7 | MVLYTTPFPNSCLSALHCV SWALIFPCYWLVDRLAASF IPTTYEKRQRADDPCCLQLL CTALFTPIYLALLVASLPFAF LGFLFWSPLQSARRPYIYSR LEDKGLAGGAALLSEWKG TGPGKSFCFATANVCLLPD SLARVNNLFNTQARAKEIG QRIRNGAARPQIKIYIDSPT NTSISAASFSSLVSPQGGD | A*02:03,A*02:07,A*11:0 1,A*11:02,A*24:02,A*24: 07,A*24:10,B*15:01,B*1 5:21,B*15:27,B*27:04,B* 38:02,B*39:01,B*40:01,B *40:06,B*46:01,B*51:01, B*55:02,B*58:01,C*01:0 2,C*03:02,C*03:04,C*03: 67,C*04:01,C*04:03,C*0 7:02,C*08:01,C*12:02,C*15:02 |
| | | GVARAVPGSIKRTASVEYK GDGGRHPGDEAANGPAS GDPVDSSSPEDACIVRIGG EEGGRPPEADDPVPGGQA RNGAGGGPRGQTPNHNQ QDGDSGSLGSPSASRESLV KGRAGPDTSASGEPGANS KLLYKASVVKKAAARRRRH PDEAFDHEVSAFFPANLDF LCLQEVFDKRAATKLKEQL HGYFEYILYDVGVYGCQGC CSFKCLNSGLLFASRYPI | |
| SEQ ID NO: 1862 | ENSG00000103227.14 | MLGAGLIKIRGDRCWRDL TCMDFHYETQPMPNPVA YYLHHSPWWFHRFETLSN HFIELLVPFFLFLGRRACIIH GVLQILFQAVLIVSGNLSFL NWLTMVPSLACFDDATLG FLFPSGPGSLKDRVLQMQ RDIRGARPEPRFGSVVRRA ANVSLGVLLAWLSVPVVLN LLSSRQVMNTHFNSLHIVN TYGAFGSITKERAEVILQGT ASSNASAPDAMWEDYEFK CKPGDPSRRPCLISPYHYRL DWLMWFAAFQTYEHND WIIHLAGKLLASDAEALSLL AHNPFAGRPPPRWVRGE HYRYKFSRPGGRHAAEGK WWVRKRIGAYFPPLS | A*02:03,A*11:01,A*11:0 2,A*24:02,A*24:07,A*24: 10,A*33:03,B*15:01,B*3 8:02,B*40:01,B*58:01,C* 03:02,C*03:04,C*07:02,C *14:02,C*15:02 |
| SEQ ID NO: 1863 | ENSG00000105559.7 | MEGSRPRSSLSLASSASTIS SLSSLSPKKPTRAVNKIHAF GKRGNALRRDPNLPVHIR GWLHKQDSSGLRLWKRR WFVLSGHCLFYYKDSREES VLGSVLLPSYNIRPDGPGA PRGRRFTFTAEHPGMRTY VLAADTLEDLRGWLRALG RASRAEGDDYGQPRSPAR PQPGEGPGGPGGPPEVSR GEEGRISESPEVTRLSRGRG RPRLLTPSPTTDLHSGLQM RRARSPDLFTPLSRPPSPLS LPRPRSAPARRPPAPSGDT APPARPHTPLSRIDVRPPLD WGPQRQTLSRPPTPRRGP PSEAGGGKPPRSPQHWSQ EPRTQAHSGSPTYLQLPPR PPGTRASMVLLPGPPLEST | A*02:03,A*11:01,A*11:0 2,A*24:10,A*33:03,B*39: 01,B*40:01,B*58:01,C*0 3:02,C*03:04,C*14:02 |
| | | FHQSLETDTLLTKLCGQDR LLRRLQEEIDQKQEEKEQLE AALELTRQQLGQATREAG APGRAWGRQRLLQDRLVS VRATLCHLTQERERVWDT YSGLEQELGTLRETLEYLLH LGSPQDRVSAQQQLWMV EDTLAGLGGPQKPPPHTEP DSPSPVLQGEESSERESLPE SLELSSPRSPETDWGRPPG GDKDLASPHLGLGSPRVSR ASSPEGRHLPSPQLGTKAP VARPRMSAQEQLERMRR NQECGRPFPRPTSPRLLTL GRTLSPARRQPDVEQRPV VGHSGAQKWLRSSGSWSS PRNTTPYLPTSEGHRERVLS LSQALATEASQWHRMMT GGNLDSQGDPLPGVPLPP SDPTRQETPPPRSPPVANS GSTGFSRRGSGRGGGPTP WGPAWDAGIAPPVLPQD EGAWPLRVTLLQSSF | |
| SEQ ID NO: 1864 | ENSG00000105639.14 | | A*02:03,A*11:01,A*11:0 2,A*24:02,A*24:07,A*24: 10,A*33:03,B*15:01,B*3 9:01,B*40:01,B*55:02,B* 58:01,C*03:02,C*03:04,C *07:02,C*14:02 |
| | | | |
| SEQ ID NO: 1865 | ENSG00000105650.17 | MQAPVPHSQRRESFLYRS DSDYELSPKAMSRNSSVAS DLHGEDMIVTPFAQVLASL RTVRSNVAALARQQCLGA AKQGPVGN | A*02:03,B*15:01,B*39:0 1,B*40:01,C*03:02,C*03: 04,C*15:02 |
| SEQ ID NO: 1866 | ENSG00000105963.9 | MAKERRRAVLELLQRPGN ARCADCGAPDPDWASYTL GVFICLSCSGIHRNIPQVSK VKSVRLDAWEEAQVEFMA SHGNDAARARFESKVPSFY YRPTP | A*02:03,A*24:10,B*15:0 1,C*03:02,C*03:04 |
| SEQ ID NO: 1867 | ENSG00000105976.10 | MKAPAVLAPGILVLLFTLV QRSNGECKEALAKSEMNV NMKYQLPNFTAETPIQNVI LHEHHIFLGATNYIYVLNEE | A*02:03,A*11:01,A*11:0 2,A*24:02,A*24:07,A*24: 10,A*33:03,A*34:01,B*1 5:01,B*15:27,B*39:01,B*40:01,B*58:01,C*03:02,C *03:04,C*03:67,C*07:02, C*12:02,C*14:02,C*15:02 |
| | | DLQKVAEYKTGPVLEHPDC FPCQDCSSKANLSGGVWK DNINMALVVDTYYDDQLIS CGSVNRGTCQRHVFPHNH TADIQSEVHCIFSPQIEEPS QCPDCVVSALGAKVLSSVK DRFINFFVGNTINSSYFPDH PLHSISVRRLKETKDGFMFL TDQSYIDVLPEFRDSYPIKY VHAFESNNFIYFLTVQRETL DAQTFHTRIIRFCSINSGLH SYMEMPLECILTEKRKKRST KKEVFNILQAAYVSKPGAQ LARQIGASLNDDILFGVFA QSKPDSAEPMDRSAMCAF PIKYVNDFFNKIVNKNNVR CLQHFYGPNHEHCFNRTLL RNSSGCEARRDEYRTEFTT ALQRVDLFMGQFSEVLLTS ISTFIKGDLTIANLGTSEGRF MQVVVSRSGPSTPHVNFL LDSHPVSPEVIVEHTLNQN GYTLVITGKKITKIPLNGLGC RHFQSCSQCLSAPPFVQCG WCHDKCVRSEECLSGTWT QQICLPAIYKVFPNSAPLEG GTRLTICGWDFGFRRNNK FDLKKTRVLLGNESCTLTLS ESTMNTLKCTVGPAMNKH FNMSIIISNGHGTTQYSTFS YVDPVITSISPKYGPMAGG TLLTLTGNYLNSGNSRHISI GGKTCTLKSVSNSILECYTP AQTISTEFAVKLKIDLANRE TSIFSYREDPIVYEIHPTKSFI SGGSTITGVGKNLNSVSVP RMVINVHEAGRNFTVACQ HRSNSEIICCTTPSLQQLNL QLPLKTKAFFMLDGILSKYF DLIYVHNPVFKPFEKPVMIS MGNENVLEIKGNDIDPEA VKGEVLKVGNKSCENIHLH SEAVLCTVPNDLLKLNSELN IEWKQAISSTVLGKVIVQP DQNFTGLIAGVVSISTALLL LLGFFLWLKKRKQIKDLGSE LVRYDARVHTPHLDRLVSA RSVSPTTEMVSNESVDYRA TFPEDQFPNSSQNGSCRQ | |
| | | VQYPLTDMSPILTSGDSDIS SPLLQNTVHIDLSALNPELV QAVQHVVIGPSSLIVHFNE VIGRGHFGCVYHGTLLDN DGKKIHCAVKSLNRITDIGE VSQFLTEGIIMKDFSHPNVL SLLGICLRSEGSPLVVLPYM KHGDLRNFIRNETHNPTVK DLIGFGLQVAKGMKYLASK KFVHRDLAARNCMLDEKF TVKVADFGLARDMYDKEY YSVHNKTGAKLPVKWMAL ESLQTQKFTTKSDVWSFGV LLWELMTRGAPPYPDVNT FDITVYLLQGRRLLQPEYCP DPLYEVMLKCWHPKAEM RPSFSELVSRISAIFSTFIGEH YVHVNATYVNVKCVAPYP SLLSSEDNADDEVDTRPAS FWETS | |
| SEQ ID NO: 1868 | ENSG00000107317.7 | MATHHTLWMGLALLGVL GDLQAAPEAQVSVQPNFQ QD | A*02:03,B*15:01,C*03:0 2,C*03:04,C*12:02 |
| SEQ ID NO: 1869 | ENSG00000111700.8 | MDQHQHLNKTAESASSEK KKTRRCNGFK | A*11:01,A*11:02 |
| SEQ ID NO: 1870 | ENSG00000111860.9 | | A*02:03,A*11:01,A*11:0 2,A*24:02,A*24:07,A*24: 10,A*33:03,B*15:01,B*1 5:27,B*39:01,B*40:01,C* 03:02,C*03:04,C*14:02 |
| | | | |
| SEQ ID NO: 1871 | ENSG00000111912.14 | XEKTCSSLEREPHFSLLTMR GQRLPLDIQIFYCARPDEEP FVKIITVEEAKRRKSTCSYYE DEDEEVLPVLRPHSALLEN MHIEQLARRLPARVQGYP WRLAYSTLEHGTSLKTLYRK SASLDSPVLLVIKDMDNQIF GAYATHPFKFSDHYYGTGE TFLYTFSPHFKVFKWSGEN SYFINGDISSLELGGGGGRF GLWLDADLYHGRSNSCST FNNDILSKKEDFIVQDLEV WAFD | A*02:03,A*11:01,A*11:0 2,A*24:02,A*24:07,A*24: 10,A*33:03,B*15:01,B*1 5:27,B*40:01,B*55:02,C* 03:02,C*03:04,C*03:67,C *12:02,C*14:02,C*15:02 |
| SEQ ID NO: 1872 | ENSG00000112033.9 | MEQPQEEAPEVREEEEKEE VAEAEGAPELNGGPQHAL PSSSYTDLSRSSSPPSLLDQL QMGCDGASCGSLNMECR VCGDKASGFHYGVHACEG CKGFFRRTIRMKLEYEKCER SCKIQKKNRNKCQYCRFQK CLALGMSHNAIRFGRMPE AEKRKLVAGLTANEGSQYN PQVADLKAFSKHIYNAYLK NFNMTKKKARSILTGKASH TAPFVIHDIETLWQAEKGL VWKQLVNGLPPYKEISVHV FYRCQCTTVETVRELTEFAK SIPSFSSLFLNDQVTLLKYG VHEAIFAMLASIVNKDGLL VANGSGFVTREFLRSLRKP | A*02:03,A*02:07,A*11:0 1,A*11:02,A*24:02,A*24: 07,A*24:10,A*33:03,A*3 4:01,B*15:01,B*15:21,B* 15:27,B*27:04,B*38:02,B *39:01,B*40:01,B*40:06, B*46:01,B*51:01,B*55:0 2,B*58:01,C*01:02,C*03: 02,C*03:04,C*04:01,C*0 4:03,C*07:02,C*08:01,C* 12:02,C*15:02 |
| | | FSDIIEPKFEFAVKFNALELD DSDLALFIAAIILCGDRPGL MNVPRVEAIQDTILRALEF HLQANHPDAQYLFP | |
| SEQ ID NO: 1873 | ENSG00000113594.5 | MMDIYVCLKRPSWMVDN KRMRTASNFQWLLSTFILL YLMNQVNSQKKGAPHDLK CVTNNLQVWNCSWKAPS GTGRGTDYEVCIENRSRSC YQLEKTSIKIPALSHGDYEITI NSLHDFGSSTSKFTLNEQN VSLIPDTPEILNLSADFSTST LYLKWNDRGSVFPHRSNVI WEIKVLRKESMELVKLVTH NTTLNGKDTLHHWSWAS DMPLECAIHFVEIRCYIDNL HFSGLEEWSDWSPVKNIS WIPDSQTKVFPQDKVILVG SDITFCCVSQEKVLSALIGH TNCPLIHLDGENVAIKIRNIS VSASSGTNVVFTTEDNIFG TVIFAGYPPDTPQQLNCET HDLKEIICSWNPGRVTALV GPRATSYTLVESFSGKYVRL KRAEAPTNESYQLLFQMLP NQEIYNFTLNAHNPLGRSQ STILVNITEKVYPHTPTSFKV KDINSTAVKLSWHLPGNFA KINFLCEIEIKKSNSVQEQR NVTIKGVENSSYLVALDKL NPYTLYTFRIRCSTETFWK WSKWSNKKQHLTTEASPS KGPDTWREWSSDGKNLIIY WKPLPINEANGKILSYNVS CSSDEETQSLSEIPDPQHKA EIRLDKNDYIISVVAKNSVG SSPPSKIASMEIPNDDLKIE QVVGMGKGILLTWHYDP NMTCDYVIKWCNSSRSEP CLMDWRKVPSNSTETVIES DEFRPGIRYNFFLYGCRNQ GYQLLRSMIGYIEELAPIVA PNFTVEDTSADSILVKWED IPVEELRGFLRGYLFYFGKG ERDTSKMRVLESGRSDIKV KNITDISQKTLRIADLQGKT SYHLVLRAYTDGGVGPEKS MYVVTKENSVGLIIAILIPVA VAVIVGVVTSILCYRKREWI | A*02:03,A*11:01,A*11:0 2,A*24:02,A*24:07,A*24: 10,A*33:03,A*34:01,B*1 5:01,B*39:01,B*40:01,B* 58:01,C*03:02,C*03:04,C *03:67,C*12:02,C*14:02, C*15:02 |
| | | KETFYPDIPNPENCKALQF QKSVCEGSSALKTLEMNPC TPNNVEVLETRSAFPKIEDT EIISPVAERPEDRSDAEPEN HVVVSYCPPIIEEEIPNPAA DEAGGTAQVIYIDVQSMY QPQAKPEEEQENDPVGGA GYKPQMHLPINSTVEDIAA EEDLDKTAGYRPQANVNT WNLVSPDSPRSIDSNSEIVS FGSPCSINSRQFLIPPKDED SPKSNGGGWSFTNFFQNK PND | |
| SEQ ID NO: 1874 | ENSG00000114541.10 | MASVFMCGVEDLLFSGSR FVWNLTVSTLRRWYTERLR ACHQVLRTWCGLQDVYQ MTEGRHCQVHLLDDRRLE LLVQPKLLARELLDLVASHF NLKEKEYFGITFIDDTGQQ NWLQLDHRVLDHDLPKKP GPTILHFAVRFYIESISFLKD KTTVELFFLNAKACVHKGQ IEVESETIFKLAAFILQEAKG DYTSDENARKDLKTLPAFP TKTLQEHPSLAYCEDRVIEH YLKIKGLTRGQAVVQY | A*02:03,A*11:01,A*11:0 2,A*24:10,A*33:03,A*34: 01,B*40:01,B*58:01,C*0 7:02,C*12:02,C*14:02 |
| SEQ ID NO: 1875 | ENSG00000115977.14 | MKKFFDSRREQGGSGLGS GSSGGGGSTSGLGSGYIGR VFGIGRQQVTVDEVLAEG GFAIVFLVRTSNGMKCALK RMFVNNEHDLQVCKREIQI MRDLSGHKNIVGYIDSSIN NVSSGDVWEVLILMDFCR GGQVVNLMNQRLQTGFT ENEVLQIFCDTCEAVARLH QCKTPIIHRDLKVENILLHD RGHYVLCDFGSATNKFQN PQTEGVNAVEDEIKKYTTL SYRAPEMVNLYSGKIITTKA DIWALGCLLYKLCYFTLPFG ESQVAICDGNFTIPDNSRYS QDMHCLIRYMLEPDPDKR PDIYQVSYFSFKLLKKECPIP NVQNSPIPAKLPEPVKASE AAAKKTQPKARLTDPIPTTE TSIAPRQRPKAGQTQPNP GILPIQPALTPRKRATVQPP PQAAGSSNQPGLLASVPQ PKPQAPPSQPLPQTQAKQ | A*02:03,A*11:01,A*11:0 2,A*24:02,A*24:07,A*24: 10,B*15:01,B*39:01,B*4 0:01,C*03:02,C*12:02,C* 14:02 |
| | | PQAPPTPQQTPSTQAQGL PAQAQATPQHQQQLFLK QQQQQQQPPPAQQQPA GTFYQQQQAQTQQFQAV HPATQKPAIAQFPVVSQG GSQQQLMQNFYQQQQQ QQQQQQQQQLATALHQ QQLMTQQAALQQKPTMA AGQQPQPQPAAAPQPAP AQEPAIQAPVRQQPKVQT TPPPAVQGQKVGSLTPPSS PKTQRAGHRRILSDVTHSA VFGVPASKSTQLLQAAAAE AELLDPGRQTLQ | |
| SEQ ID NO: 1876 | ENSG00000116833.9 | MSSNSDTGDLQESLKHGLT PIGAGLPDRHGSPIPARGR LV | A*02:03 |
| SEQ ID NO: 1877 | ENSG00000118855.14 | MDAGKLARHPTDTGSERA VPALAEIRPWWAPPLRPQ | C*03:02,C*03:04,C*14:0 2 |
| SEQ ID NO: 1878 | ENSG00000119547.5 | MKAAYTAYRCLTKDLEGCA MNPELTMESLGTLHGPAG GGSGGGGGGGGGGGG GPGHEQELLASPSPHHAG RGAAGSLRGPPPPPTAHQ ELGTAAAAAAAASRSAMV TSMASILDGGDYRPELSIPL HHAMSMSCDSSPPGMG MSNTYTTLTPLQPLPPISTV SDKFHHPHPHHHPHHHH HHHHQRLSGNVSGSFTLM RDERGLPAMNNLYSPYKE MPGMSQSLSPLAATPLGN GLGGLHNAQQSLPNYGPP GHDKMLSPNFDAHHTAM LTRGEQHLSRGLGTPPAA MMSHLNGLHHPGHTQSH GPVLAPSRERPPSSSSGSQ VATSGQLEEINTKEVAQRIT AELKRYSIPQAIFAQRVLCR SQGTLSDLLRNPKPWSKLK SGRETFRRMWKWLQEPEF QRMSALRLAA | A*02:03,A*11:01,A*11:0 2,A*24:10,A*33:03,B*15: 01,B*15:27,B*39:01,B*5 8:01,C*03:02,C*03:04,C* 07:02,C*14:02 |
| SEQ ID NO: 1879 | ENSG00000125826.15 | | A*02:03,A*02:07,A*11:0 1,A*11:02,A*24:10,A*33: 03,B*40:01,C*03:02,C*0 3:04 |
| | | | |
| SEQ ID NO: 1880 | ENSG00000129116.13 | MSALASRSAPAMQSSGSF NYARPKQFIAAQNLGPAS GHGTPASSPSSSSLPSPMS PTPRQFGRAPVPPFAQPF GAEPEAPWGSSSPSPPPPP PPVFSPTAAFPVPDVFPLPP PPPPLPSPGQASHCSSPAT RFGHSQTPAAFLSALLPSQ PPPAAVNALGLPKGVTPA GFPKKASRTARIASDEEIQG TKDAVIQDLERKLRFKEDLL NNGQPRLTYEERMARRLL GADSATVFNIQEPEEETAN QEYKVSSCEQRLISEIEYRLE RSPVDESGDEVQYGDVPV ENGMAPFFEMKLKHYKIFE GMPVTFTCRVAGNPKPKIY WFKDGKQISPKSDHYTIQR DLDGTCSLHTTASTLDDDG NYTIMAANPQGRISCTGRL MVQAVNQRGRSPRSPSG HPHVRRPRSRSRDSGDEN EPIQERFFRPHFLQAPGDLT VQEGKLCRMDCKVSGLPT PDLSWQLDGKPVRPDSAH KMLVRENGVHSLIIEPVTSR DAGIYTCIATNRAGQNSFS LELVVAAKE | A*02:03,A*11:01,A*11:0 2,A*24:02,A*24:10,A*33: 03,B*15:01,B*39:01,B*4 0:01,B*58:01,C*03:02,C* 03:04 |
| SEQ ID NO: 1881 | ENSG00000129682.9 | MSGKVTKPKEEKDASKVLD DAPPGTQEYIMLRQDSIQS AELKKKESPFRAKCHEIFCC PLKQVHHKENTEPEEPQLK GIVTKLYSRQGYHLQLQAD GTIDGTKDEDSTYTLFNLIP VGLRVVAIQGVQTKLYLA | A*02:03,A*02:07,A*24:1 0,A*34:01,B*27:04,B*38: 02,B*39:01,B*46:01,B*5 5:02,C*03:02,C*07:02,C* 08:01,C*15:02 |
| SEQ ID NO: 1882 | ENSG00000131374.10 | MYHSLSETRHPLQPEEQEV GIDPLSSYSNKSGGDSNKN GRRTSSTLDSEGTFNSYRKE WEELFVNNNYLATIRQKGI NGQLRSSRFRSICWKLFLC VLPQDKSQWISRIEELRAW YSNIKEIHITNPRKVVGQQ DL | A*02:03,A*24:02,A*24:0 7,A*24:10,A*33:03,B*27: 04,B*51:01,C*07:02,C*1 5:02 |
| SEQ ID NO: 1883 | ENSG00000131620.13 | MWEASGMEERALEELAM EETALDPLLAEAAGAVDGE GAPPGGPSAQAATMRVN EKYSTLPAEDRSVHIINICAI EDIGYLPSEGTLLNSLSVDP DAECKYGLYFRDGRRKVDY ILVYHHKRPSGNRTLVRRV QHSDTPSGARSVKQDHPL PGKGASLDAGSGEPP | A*02:03,A*24:10,A*33:0 3,B*38:02,B*40:01,C*01: 02 |
| SEQ ID NO: 1884 | ENSG00000132005.4 | MATQAYTELQAAPPPSQP PQAPPQAQPQPPPPPPPA APQPPQPPTAAATPQPQY VTELQSPQPQAQPPGGQK QYVTELPAVPAPSQPTGAP TPSPAPQQYIVVTVSEGAM RASETVSEASPGSTASQTG VPTQVVQQVQGTQQRLL VQTSVQAKPGHVSPLQLT NIQVPQQALPTQRLVVQS AAPGSKGGQVSLTVHGTQ QVHSPPEQSPVQANSSSSK TAGAPTGTVPQQLQVHGV QQSVPVTQERSVVQATPQ APKPGPVQPLTVQGLQPV HVAQEVQQLQQVPVPHV YSSQVQYVEGGDASYTASA IRSSTYSYPETPLYTQTASTS YYEAAGTATQVSTPATSQA VASSGS | B*15:01,B*58:01,C*03:0 2,C*03:04,C*03:67,C*12: 02,C*14:02 |
| SEQ ID NO: 1885 | ENSG00000132359.9 | MFGRKRSVSFGGFGWIDK TMLASLKVKKQELANSSDA TLPDRPLSPPLTAPPTMKSS EFFEMLEKMQGIKLEEQKP GPQKNKDDYIPYPSIDEVV | A*02:03,A*11:01,A*11:0 2,A*34:01,B*40:01,C*03: 02,C*03:04,C*14:02,C*1 5:02 |
| | | EKGGPYPQVILPQFGGYWI EDPENVGTPTSLGSSICEEE EEDNLSPNTFGYKLECKGE ARAYRRHFLGKDHLNFYCT GSSLGNLILSVKCEEAEGIEY LRVILRSKLKTVHERIPLAGL SKLPSVPQIAKAFCDDAVG LRFNPVLYPKASQ | |
| SEQ ID NO: 1886 | ENSG00000134490.9 | MCVRRSLVGLTFCTCYLAS YLTNKYVLSVLKFTYPTLFQ GWQTLIGGLLLHVSWKLG WVEINSSSRSHVLVWLPAS VLFVGIIYAGSRALSRLAIPV FLTLHNVAEVIICGYQKCFQ KEKTSPAKICSALLLLAAAG CLPFNDSQFNPDGYFWAII HLLCVGAYKILQKSQKPSAL SDIDQQYLNYIFSVVLLAFA SHPTGDLFSVLDFPFLYFYR FHGSCCASGFLGFFLMFST VKLKNLLAPGQCAAWIFFA KIITAGLSILLFDAILTSATTG CLLLGALGEALLVFSERKSS | A*02:03,A*11:01,A*11:0 2,A*24:02,A*24:07,A*24: 10,A*33:03,B*15:01,B*1 5:27,B*58:01,C*03:02,C* 03:04,C*12:02 |
| SEQ ID NO: 1887 | ENSG00000135093.8 | MLSSRAEAAMTAADRAIQ RFLRTGAAVRYKVMKNW GVIGGIAAALAAGIYVIWG PITERKKRRKGLVPGLVNL GNTCFMNSLLQGLSACPA FIRWLEEFTSQYSRDQKEP PSHQYLSLTLLHLLKALSCQ EVTDDEVLDASCLLDVLRM YRWQISSFEEQDAHELFHV ITSSLEDERDRQPRVTHLFD VHSLEQQSEITPKQITCRTR GSPHPTSNHWKSQHPFHG RLTSN | A*02:03,A*02:07,A*11:0 1,A*11:02,A*24:02,A*24: 07,A*24:10,B*15:21,B*2 7:04,B*38:02,B*39:01,B* 40:01,B*51:01,B*58:01,C *03:02,C*07:02,C*14:02, C*15:02 |
| SEQ ID NO: 1888 | ENSG00000136231.9 | MNKLYIGNLSENAAPSDLE SIFKDAKIPVSGPFLVKTGY AFVDCPDESWALKAIEALS GKIELHGKPIEVEHSVPKRQ RIRKLQIRNIPPHLQWEVLD SLLVQYGVVESCEQVNTDS ETAVVNVTYSSKDQARQA LDKLNGFQLENFTLKVAYIP DEMAAQQNPLQQPRGRR GLGQRGSSRQGSPGSVSK QKPCDLPLRLLVPTQFVGAI IGKEGATIRNITKQTQSKID VHRKENAGAAEKSITILSTP | A*02:03,A*11:01,A*11:0 2,A*24:10,A*33:03,A*34: 01,B*15:01,B*15:27,C*0 3:02,C*03:04,C*14:02 |
| | | EGTSAACKSILEIMHKEAQ DIKFTEEIPLKILAHNNFVG RLIGKEGRNLKKIEQDTDTK ITISPLQELTLYNPERTITVK GNVETCAKAEEEIMKKIRE SYENDIASMNLQAHLIPGL NLNALGLFPPTSGMPPPTS GPPSAMTPPYPQFEQSETE TVHLFIPALSVGAIIGKQGQ HIKQLSRFAGASIKIAPAEA PDAKVRMVIITGPPEAQFK AQGRIYGKIKEENFVSPKEE VKLEAHIRVPSFAAGRVIGK GGKTVNELQNLSSAEVVVP RDQTPDENDQVVVKITGH FYACQVAQRKIQEILTQVK QHQQQKALQSGPPQSRRK | |
| SEQ ID NO: 1889 | ENSG00000136848.12 | MEPDSLLDQDDSYESPQE RPGSRRSLPGSLSEKSPSM EPSAATPFRVTGFLSRRLKG SIKRTKSQPKLDRNHSFRHI | A*02:03 |
| SEQ ID NO: 1890 | ENSG00000137203.6 | MLWKLTDNIKYEDCEDRH DGTSNGTARLPQLGTVGQ SPYTSAPPLSHTPNADFQP PYFPPPYQPIYPQSQDPYS HVNDPYSLNPLHAQPQPQ HPGWPGQRQSQESGLLHT HRGLPHQLSGLDPRRDYRR HEDLLHGPHALSSGLGDLSI HSLPHAIEEVPHVEDPGINI PDQTVIKKGPVSLSKSNSN AVSAIPINKDNLFGGVVNP NEVFCSVPGRLSLLSSTSK | A*02:03,A*11:01,A*11:0 2,A*24:02,A*24:10,A*33: 03,B*39:01,C*14:02 |
| SEQ ID NO: 1891 | ENSG00000137474.15 | MVILQQGDHVWMDLRLG QEFDVPIGAVVKLCDSGQV QVVDDEDNEHWISPQNA THIKPMHPTSVHGVEDMI RLGDLNEAGILRNLLIRYRD HLIYTYTGSILVAVNPYQLLS IYSPEHIRQYTNKKIGEMPP HIFAIADNCYFNMKRNSRD QCCIISGESGAGKTESTKLIL QFLAAISGQHSWIEQQVLE ATPILEAFGNAKTIRNDNSS RFGKYIDIHFNKRGAIEGAK IEQYLLEKSRVCRQALDERN YHVFYCMLEGMSEDQKKK LGLGQASDYNYLAMGNCI TCEGRVDSQEYANIRSAM | A*02:03,A*11:01,A*11:0 2,A*24:02,A*24:07,A*24: 10,A*33:03,B*15:01,B*3 9:01,B*40:01,B*55:02,B* 58:01,C*03:02,C*03:04,C *03:67,C*07:02,C*12:02, C*14:02,C*15:02 |
| | | KVLMFTDTENWEISKLLAA ILHLGNLQYEARTFENLDA CEVLFSPSLATAASLLEVNP PDLMSCLTSRTLITRGETVS TPLSREQALDVRDAFVKGI YGRLFVWIVDKINAAIYKPP SQDVKNSRRSIGLLDIFGFE NFAVNSFEQLCINFANEHL QQFFVRHVFKLEQEEYDLE SIDWLHIEFTDNQDALDMI ANKPMNIISLIDEESKFPKG TDTTMLHKLNSQHKLNAN YIPPKNNHETQFGINHFAG IVYYETQGFLEKNRDTLHG DIIQLVHSSRNKFIKQIFQA DVAMGAETRKRSPTLSSQF KRSLELLMRTLGACQPFFV RCIKPNEFKKPMLFDRHLC VRQLRYSGMMETIRIRRAG YPIRYSFVEFVERYRVLLPG VKPAYKQGDLRGTCQRMA EAVLGTHDDWQIGKTKIFL KDHHDMLLEVERDKAITD RVILLQKVIRGFKDRSNFLK LKNAATLIQRHWRGHNCR KNYGLMRLGFLRLQALHRS RKLHQQYRLARQRIIQFQA RCRAYLVRKAFRHRLWAVL TVQAYARGMIARRLHQRL RAEYLWRLEAEKMRLAEEE KLRKEMSAKKAKEEAERKH QERLAQLAREDAERELKEK EAARRKKELLEQMERARH EPVNHSDMVDKMFGFLG TSGGLPGQEGQAPSGFED LERGRREMVEEDLDAALPL PDEDEEDLSEYKFAKFAATY FQGTTTHSYTRRPLKQPLLY HDDEGDQLAALAVWITILR FMGDLPEPKYHTAMSDGS EKIPVMTKIYETLGKKTYKR ELQALQGEGEAQLPEGQK KSSVRHKLVHLTLKKKSKLT EEVTKRLHDGESTVQGNS MLEDRPTSNLEKLHFIIGNG ILRPALRDEIYCQISKQLTH NPSKSSYARGWILVSLCVG CFAPSEKFVKYLRNFIHGGP PGYAPYCEERLRRTFVNGT | |
| | | RTQPPSWLELQATKSKKPI MLPVTFMDGTTKTLLTDSA TTAKELCNALADKISLKDRF GFSLYIALFD | |
| SEQ ID NO: 1892 | ENSG00000138075.7 | MGDLSSLTPGGSMGLQV NRGSQSSLEGAPATAPEPH SLGILHASYSVSHRVRPW WDITSCRQQWTRQILKDV SLYVESGQIMCILGSSGSGK TTLLDAMSGRLGRAGTFLG EVYVNGRALRREQFQDCFS YVLQSDTLLSSLTVRETLHY TALLAIRRGNPGSFQKKVE AVMAELSLSHVADRLIGNY SLGGISTGERRRVSIAAQLL QDPKVMLFDEPTTGLDCM TANQIVVLLVELARRNRIVV LTIHQPRSELFQLFDKIAILS FGELIFCGTPAEMLDFFND CGYPCPEHSNPFDFY | A*02:03,A*02:07,A*11:0 1,A*11:02,A*24:02,A*24: 07,A*24:10,A*33:03,A*3 4:01,B*15:01,B*15:21,B* 15:27,B*27:04,B*38:02,B *39:01,B*40:01,B*40:06, B*46:01,B*55:02,B*58:0 1,C*03:02,C*03:04,C*03: 67,C*04:01,C*04:03,C*0 7:02,C*08:01,C*12:02,C* 14:02,C*15:02 |
| SEQ ID NO: 1893 | ENSG00000142185.12 | MEPSALRKAGSEQEEGFE GLPRRVTDLGMVSNLRRS NSSLFKSWRLQCPFGNND KQESLSSWIPENIKKKECVY FVESSKLSDAGKVVCQCGY THEQHLEEATKPHTFQGT QWDPKKHVQEMPTDAFG DIVFTGLSQKVKKYVRVSQ DTPSSVIYHLMTQHWGLD VPNLLISVTGGAKNFNMKP RLKSIFRRGLVKVAQTTGA WIITGGSHTGVMKQVGEA VRDFSLSSSYKEGELITIGVA TWGTVHRREGLIHPTGSFP AEYILDEDGQGNLTCLDSN HSHFILVDDGTHGQYGVEI PLRTRLEKFISEQTKERGGV AIKIPIVCVVLEGGPGTLHTI DNATTNGTPCVVVEGSGR VADVIAQVANLPVSDITISLI QQKLSVFFQEMFETFTESRI VEWTKKIQDIVRRRQLLTV FREGKDGQQDVDVAILQA LLKASRSQDHFGHENWDH QLKLAVAWNRVDIARSEIF MDEWQWKPSDLHPTMT AALISNKPEFVKLFLENGVQ LKEFVTWDTLLYLYENLDPS CLFHSKLQMHHVAQVLRE | A*02:03,A*11:01,A*11:0 2,A*24:02,A*24:07,A*24: 10,A*33:03,A*34:01,B*1 5:01,B*15:27,B*39:01,B* 40:01,B*58:01,C*03:02,C *03:04,C*12:02,C*14:02, C*15:02 |
| | | LLGDFTQPLYPRPRHNDRL RLLLPVPHVKLNVQGVSLR SLYKRSSGHVTFTMDPIRD LLIWAIVQNRRELAGIIWA QSQDCIAAALACSKILKELS KEEEDTDSSEEMLALAEEY EHRAIGVFTECYRKDEERA QKLLTRVSEAWGKTTCLQL ALEAKDMKFVSHGGIQAFL TKVWWGQLSVDNGLWR VTLCMLAFPLLLTGLISFREK RLQDVGTPAARARAFFTAP VVVFHLNILSYFAFLCLFAY VLMVDFQPVPSWCECAIY LWLFSLVCEEMRQLFYDPD ECGLMKKAALYFSDFWNK LDVGAILLFVAGLTCRLIPA TLYPGRVILSLDFILFCLRLM HIFTISKTLGPKIIIVKRMMK DVFFFLFLLAVWVVSFGVA KQAILIHNERRVDWLFRGA VYHSYLTIFGQIPGYIDGVN FNPEHCSPNGTDPYKPKCP ESDATQQRPAFPEWLTVLL LCLYLLFTNILLLNLLIAMFN YTFQQVQEHTDQIWKFQR HDLIEEYHGRPAAPPPFILL SHLQLFIKRVVLKTPAKRHK QLKNKLEKNEEAALLSWEI YLKENYLQNRQFQQKQRP EQKIEDISNKVDAMVDLLD LDPLKRSGSMEQRLASLEE QVAQTAQALHWIVRTLRA SGFSSEADVPTLASQKAAE EPDAEPGGRKKTEEPGDSY HVNARHLLYPNCPVTRFPV PNEKVPWETEFLIYDPPFYT AERKDAAAMDPMGENP MGRTGLRGRGSLSCFGPN HTLYPMVTRWRRNEDGAI CRKSIKKMLEVLVVKLPLSE HWALPGGSREPGEMLPRK LKRILRQEHWPSFENLLKC GMEVYKGYMDDPRNTDN AWIETVAVSVHFQDQNDV ELNRLNSNLHACDSGASIR WQVVDRRIPLYANHKTLL QKAAAEFGAHY | |
| SEQ ID NO: 1894 | ENSG00000142235.4 | MRQVLWLCNVCVTARETR | A*02:03,A*33:03,B*15:01,B*39:01,B*40:01,C*03: 02,C*03:04 |
| | | HHLHLPAILDKMPAPGALI LLAAVSASGCLASPAHPDG FALGRAPLAPPYAVVLISCS GLLAFIFLLLTCLCCKRGDV GFKEFENPEGEDCSGEYTP PAEETSSSQSLPDVYILPLAE VSLPMPAPQPSHSDMTTP LGLSRQHLSYLQEIGSGWF GKVILGEIFSDYTPAQVVVK ELRASAGPLEQRKFISEAQP YRSLQHPNVLQCLGLCVET LPFLLIMEFCQLGDLKRYLR AQRPPEGLSPELPPRDLRTL QRMGLEIARGLAHLHSHN YV | |
| SEQ ID NO: 1895 | ENSG00000142661.14 | MTLPHSLGGAGDPRPPQA MEVHRLEHRQEEEQKEER QHSLRMGSSVRRRTFRSSE EEHEFSAADYALAAALALT ASSELSWEAQLRRQTSAVE LEERGQKRVGFGNDWERT EIAFLQTHRLLRQRRDWKT LRRRTEEKVQEAKELRELCY GRGPWFWIPLRSHAVWE HTTVLLTCTVQASPPPQVT WYKNDTRIDPRLFRAGKYR ITNNYGLLSLEIRRCAIEDSA TYTVRVKNAHGQASSFAK VLVRTYLGKDAGFDSEIFKR STFGPSVEFTSVLKPVFARE KEPFSLSCLFSEDVLDAESIQ WFRDGSLLRSSRRRKILYTD RQASLKVSCTYKEDEGLYM VRVPSPFGPREQSTYVLVR DAEAENPGAPGSPLNVRCL DVNRDCLILTWAPPSDTRG NPITAYTIERCQGESGEWIA CHEAPGGTCRCPIQGLVEG QSYRFRVRAISRVGSSVPSK ASELVVMGDHDAARRKTE IPFDLGNKITISTDAFEDTVT IPSPPTNVHASEIREAYVVL AWEEPSPRDRAPLTYSLEK SVIGSGTWEAISSESPVRSP RFAVLDLEKKKSYVFRVRA MNQYGLSDPSEPSEPIALR GPPATLPPPAQVQAFRDT QTSVSLTWDPVKDPELLGY YIYSRKVGTSEWQTVNNKP | A*02:03,A*11:01,A*11:0 2,A*24:02,A*24:07,A*24: 10,A*33:03,B*15:01,B*1 5:27,B*39:01,B*40:01,B* 58:01,C*03:02,C*03:04,C *03:67,C*07:02,C*08:01, C*12:02,C*14:02 |
| | | IQGTRFTVPGLRTGKEYEFC VRSVSEAGVGESSAATEPIR VKQALATPSAPYGFALLNC GKNEMVIGWKPPKRRGG GKILGYFLDQHDSEELDWH AVNQQPIPTRVCKVSDLHE GHFYEFRARAANWAGVG ELSAPSSLFECKEWTMPQP GPPYDVRASEVRATSLVLQ WEPPLYMGAGPVTGYHVS FQEEGSEQWKPVTPGPISG THLRVSDLQPGKSYVFQVQ AMNSAGLGQPSMPTDPV LLEDKPGAHEIEVGVDEEG FIYLAFEAPEAPDSSEFQWS KDYKGPLDPQRVKIEDKVN KSKVILKEPGLEDLGTYSVIV TDADEDISASHTLTEEELEK LKKLSHEIRNPVIKLISGWNI DILERGEVRLWLEVEKLSPA AELHLIFNNKEIFSSPNRKIN FDREKGLVEVIIQNLSEEDK GSYTAQLQDGKAKNQITLT LVDDDFDKLLRKADAKRRD WKRKQGPYFERPLQWKVT EDCQVQLTCKVTNTKKETR FQWFFQRAEMPDGQYDP ETGTGLLCIEELSKKDKGIYR AMVSDDRGEDDTILDLTG DALDAIFTELGRIGALSATP LKIQGTEEGIRIFSKVKYYNV EYMKTTWFHKDKRLESGD RIRTGTTLDEIWLHILDPKD SDKGKYTLEIAAGKEVRQLS TDLSGQAFEDAMAEHQRL KTLAIIEKNRAKVVRGLPDV ATIMEDKTLCLTCIVSGDPT PEISWLKNDQPVTFLDRYR MEVRGTEVTITIEKVNSEDS GRYGVFVKNKYGSETGQV TISVFKHGDEPKELKSM | |
| SEQ ID NO: 1896 | ENSG00000143669.9 | MSTDSNSLAREFLTDVNRL CNAVVQRVEAREEEEEETH MATLGQYLVHGRGFLLLTK LNSIIDQALTCREELLTLLLSL LPLVWKIPVQEEKATDFNL PLSADIILTKEKNSSSQRST QEKLHLEGSALSSQVSAKV NVFRKSRRQRKITHRYSVR | A*02:03,A*11:01,A*11:0 2,A*24:02,A*24:07,A*24: 10,A*33:03,A*34:01,B*1 5:01,B*15:27,B*39:01,B* 40:01,B*55:02,B*58:01,C *03:02,C*03:04,C*03:67, C*07:02,C*12:02,C*14:0 2,C*15:02 |
| | | DARKTQLSTSDSEANSDEK GIAMNKHRRPHLLHHFLTS FPKQDHPKAKLDRLATKEQ TPPDAMALENSREIIPRQG SNTDILSEPAALSVISNMN NSPFDLCHVLLSLLEKVCKF DVTLNHNSPLAASVVPTLT EFLAGFGDCCSLSDNLESR VVSAGWTEEPVALIQRML FRTVLHLLSVDVSTAEMM PENLRKNLTELLRAALKIRIC LEKQPDPFAPRQKKTLQEV QEDFVFSKYRHRALLLPELL EGVLQILICCLQSAASNPFY FSQAMDLVQEFIQHHGFN LFETAVLQMEWLVLRDGV PPEASEHLKALINSVMKIM STVKKVKSEQLHHSMCTRK RHRRCEYSHFMHHHRDLS GLLVSAFKNQVSKNPFEET ADGDVYYPERCCCIAVCAH QCLRLLQQASLSSTCVQILS GVHNIGICCCMDPKSVIIPL LHAFKLPALKNFQQHILNIL NKLILDQLGGAEISPKIKKA ACNICTVDSDQLAQLEETL QGNLCDAELSSSLSSPSYRF QGILPSSGSEDLLWKWDAL KAYQNFVFEEDRLHSIQIA NHICNLIQKGNIVVQWKLY NYIFNPVLQRGVELAHHCQ HLSVTSAQSHVCSHHNQC LPQDVLQIYVKTLPILLKSRV IRDLFLSCNGVSQIIELNCLN GIRSHSLKAFETLIISLGEQQ KDASVPDIDGIDIEQKELSS VHVGTSFHHQQAYSDSPQ SLSKFYAGLKEAYPKRRKTV NQDVHINTINLFLCVAFLCV SKEAESDRESANDSEDTSG YDSTASEPLSHMLPCISLES LVLPSPEHMHQAADIWS MCRWIYMLSSVFQKQFYR LGGFRVCHKLIFMIIQKLFR SHKEEQGKKEGDTSVNEN QDLNRISQPKRTMKEDLLS LAIKSDPIPSELGSLKKSADS LGKLELQHISSINVEEVSAT EAAPEEAKLFTSQESETSLQ | |
| | | SIRLLEALLAICLHGARTSQ QKMELELPNQNLSVESILFE MRDHLSQSKVIETQLAKPL FDALLRVALGNYSADFEHN DAMTEKSHQSAEELSSQP GDFSEEAEDSQCCSFKLLVE EEGYEADSESNPEDGETQD DGVDLKSETEGFSASSSPN DLLENLTQGEIIYPEICMLEL NLLSASKAKLDVLAHVFESF LKIIRQKEKNVFLLMQQGT VKNLLGGFLSILTQDDSDF QACQRVLVDLLVSLMSSRT CSEELTLLLRIFLEKSPCTKIL LLGILKIIESDTTMSPSQYLT FPLLHAPNLSNGVSSQKYP GILNSKAMGLLRRARVSRS KKEADRESFPHRLLSSWHI APVHLPLLGQNCWPHLSE GFSVSLWFNVECIHEAEST TEKGKKIKKRNKSLILPDSSF DGTESDRPEGAEYINPGER LIEEGCIHIISLGSKALMIQV WADPHNATLIFRVCMDSN DDMKAVLLAQVESQENIFL PSKWQHLVLTYLQQPQGK RRIHGKISIWVSGQRKPDV TLDFMLPRKTSLSSDSNKTF CMIGHCLSSQEEFLQLAGK WDLGNLLLFNGAKVGSQE AFYLYACGPNHTSVMPCK YGKPVNDYSKYINKEILRCE QIRELFMTKKDVDIGLLIESL SVVYTTYCPAQYTIYEPVIRL KGQMKTQLSQRPFSSKEV QSILLEPHHLKNLQPTEYKT IQGILHEIGGTGIFVFLFARV VELSSCEETQALALRVILSLI KYNQQRVHELENCNGLSM IHQVLIKQKCIVGFYILKTLL EGCCGEDIIYMNENGEFKL DVDSNAIIQDVKLLEELLLD WKIWSKAEQGVWETLLAA LEVLIRADHHQMFNIKQL LKAQVVHHFLLTCQVLQEY KEGQLTPMPREVCRSFVKII AEVLGSPPDLELLTIIFNFLL AVHPPTNTYVCHNPTNFYF SLHIDGKIFQEKVRSIMYLR | |
| | | HSSSGGRSLMSPGFMVISP SGFTASPYEGENSSNIIPQQ MAAHMLRSRSLPAFPTSSL LTQSQKLTGSLGCSIDRLQ NIADTYVATQSKKQNSLGS SDTLKKGKEDAFISSCESAK TVCEMEAVLSAQVSVSDV PKGVLGFPVVKADHKQLG AEPRSEDDSPGDESCPRRP DYLKGLASFQRSHSTIASLG LAFPSQNGSAAVGRWPSL VDRNTDDWENFAYSLGYE PNYNRTASAHSVTEDCLVP ICCGLYELLSGVLLILPDVLL EDVMDKLIQADTLLVLVNH PSPAIQQGVIKLLDAYFARA SKEQKDKFLKNRGFSLLAN QLYLHRGTQELLECFIEMFF GRHIGLDEEFDLEDVRNM GLFQKWSVIPILGLIETSLYD NILLHNALLLLLQILNSCSKV ADMLLDNGLLYVLCNTVA ALNGLEKNIPMSEYKLLAC DIQQLFIAVTIHACSSSGSQ YFRVIEDLIVMLGYLQNSK NKRTQNMAVALQLRVLQ AAMEFIRTTANHDSENLTD SLQSPSAPHHAVVQKRKSI AGPRKFPLAQTESLLMKM RSVANDELHVMMQRRMS QENPSQATETELAQRLQRL TVLAVNRIIYQEFNSDIIDIL RTPENVTQSKTSVFQTEISE ENIHHEQSSVFNPFQKEIFT YLVEGFKVSIGSSKASGSKQ QWTKILWSCKETFRMQLG RLLVHILSPAHAAQERKQIF EIVHEPNHQEILRDCLSPSL QHGAKLVLYLSELIHNHQG ELTEEELGTAELLMNALKLC GHKCIPPSASTKADLIKMIK EEQKKYETEEGVNKAAWQ KTVNNNQQSLFQRLDSKS KDISKIAADITQAVSLSQGN ERKKVIQHIRGMYKVDLSA SRHWQELIQQLTHDRAV WYDPIYYPTSWQLDPTEG PNRERRRLQRCYLTIPNKYL LRDRQKSEDVVKPPLSYLFE | |
| | | DKTHSSFSSTVKDKAASESI RVNRRCISVAPSRETAGELL LGKCGMYFVEDNASDTVE SSSLQGELEPASFSWTYEEI KEVHKRWWQLRDNAVEIF LTNGRTLLLAFDNTKVRDD VYHNILTNNLPNLLEYGNIT ALTNLWYTGQITNFEYLTH LNKHAGRSFNDLMQYPVF PFILADYVSETLDLNDLLIYR NLSKPIAVQYKEKEDRYVD TYKYLEEEYRKGAREDDPM PPVQPYHYGSHYSNSGTVL HFLVRMPPFTKMFLAYQD QSFDIPDRTFHSTNTTWRL SSFESMTDVKELIPEFFYLPE FLVNREGFDFGVRQNGER VNHVNLPPWARNDPRLFI LIHRQALESDYVSQNICQW IDLVFGYKQKGKASVQAIN VFHPATYFGMDVSAVEDP VQRRALETMIKTYGQTPR QLFHMAHVSRPGAKLNIE GELPAAVGLLVQFAFRETR EQVKEITYPSPLSWIKGLK WGEYVGSPSAPVPVVCFS QPHGERFGSLQALPTRAIC GLSRNFCLLMTYSKEQGVR SMNSTDIQWSAILSWGYA DNILRLKSKQSEPPVNFIQS SQQYQVTSCAWVPDSCQL FTGSKCGVITAYTNRFTSST PSEIEMETQIHLYGHTEEIT SLFVCKPYSILISVSRDGTCII WDLNRLCYVQSLAGHKSP VTAVSASETSGDIATVCDS AGGGSDLRLWTVNGDLV GHVHCREIICSVAFSNQPE GVSINVIAGGLENGIVRLW STWDLKPVREITFPKSNKPI ISLTFSCDGHHLYTANSDGT VIAWCRKDQQRLKQPMFY SFLSSYAAG | |
| SEQ ID NO: 1897 | ENSG00000143882.5 | MSEFWLISAPGDKENLQAL ERMNTVTSKSNLSYNTKFA IPDFKVGTLDSLVGLSDELG KLDTFAESLIRRMAQSVVE VMEDSKGKVQEHLLANGV DLTSFVTHFEWD | A*02:03,A*11:01,A*11:0 2,A*33:03,B*58:01,C*03: 02,C*03:04 |
| SEQ ID NO: 1898 | ENSG00000145214.9 | MAAAAEPGARAWLGGGS PRPGSPACSPVLGSGGRAR PGPGPGPGPERAGVRAPG PAAAPGHSFRKVTLTKPTF CHLCSDFIWGLAGFLCDVC NFMSHEKCLKHVRIPCTSV APSLVRVPVAHCFGPRGLH KRKFCAVCRKVLEAPALHC EVCELHLHPDCVPFACSDC RQCHQDGHQDHDTHHH HWREGNLPSGARCEVCRK TCGSSDVLAGVRCEWCGV QAHSLCSAALAPECGFGRL RSLVLPPACVRLLPGGFSKT QSFRIVEAAEPGEGGDGA DGSAAVGPGRETQATPES GKQTLKIFDGDDAVRRSQF RLVTVSRLAGAEEVLEAALR AHHIPEDPGHLELCRLPPSS QACDAWAGGKAGSAVISE EGRSPGSGEATPEAWVIRA LPRAQEVLKIYPGWLKVGV AYVSVRVTPKSTARSVVLE VLPLLGRQAESPESFQLVEV AMGCRHVQRTMLMDEQ PLLDRLQDIRQMSVRQVS QTRFYVAESRDVAPHVSLF VGGLPPGLSPEEYSSLLHEA GATKATVVSVSHIYSSQGA VVLDVACFAEAERLYMLLK DMAVRGRLLTALVLPDLLH AKLPPDSCPLLVFVNPKSG GLKGRDLLCSFRKLLNPHQ VFDLTNGGPLPGLHLFSQV PCFRVLVCGGDGTVGWVL GALEETRYRLACPEPSVAIL PLGTGNDLGRVLRWGAGY SGEDPFSVLLSVDEADAVL MDRWTILLDAHEAGSAEN DTADAEP | A*02:03,A*11:01,A*11:0 2,A*33:03,B*15:01,B*39: 01,B*40:01,C*03:02,C*0 3:04 |
| SEQ ID NO: 1899 | ENSG00000151025.9 | MGAMAYPLLLCLLLAQLGL GAVGASRDPQGRPDSPRE RTPKGKPHAQQPGRASAS DSSAPWSRSTDGTILAQKL AEEVPMDVASYLYTGDSH QLKRANCSGRYELAGLPGK WPALASAHPSLHRALDTLT HATNFLNVMLQSNKSREQ NLQDDLDWYQALVWSLLE | A*02:03,A*02:07,A*11:0 1,A*11:02,A*24:02,A*24: 07,A*24:10,A*33:03,B*1 5:01,B*39:01,B*40:01,B* 55:02,B*58:01,C*03:02,C *03:04,C*03:67,C*07:02, C*12:02,C*14:02 |
| | | GEPSISRAAITFSTDSLSAPA PQVFLQATREESRILLQDLS SSAPHLANATLETEWFHGL RRKWRPHLHRRGPNQGP RGLGHSWRRKDGLGGDKS HFKWSPPYLECENGSYKPG WLVTLSSAIYGLQPNLVPEF RGVMKVDINLQKVDIDQC SSDGWFSGTHKCHLNNSE CMPIKGLGFVLGAYECICK AGFYHPGVLPVNNFRRRG PDQHISGSTKDVSEEAYVC LPCREGCPFCADDSPCFVQ EDKYLRLAIISFQALCMLLD FVSMLVVYHFRKAKSIRAS GLILLETILFGSLLLYFPVVILY FEPSTFRCILLRWARLLGFA TVYGTVTLKLHRVLKVFLSR TAQRIPYMTGGRVMRML AVILLVVFWFLIGWTSSVC QNLEKQISLIGQGKTSDHLI FNMCLIDRWDYMTAVAEF LFLLWGVYLCYAVRTVPSA FHEPRYMAVAVHNELIISAI FHTIRFVLASRLQSDWML MLYFAHTHLTVTVTIGLLLI PKFSHSSNNPRDDIATEAY EDELDMGRSGSYLNSSINS AWSEHSLDPEDIRDELKKL YAQLEIYKRKKMITNNPHL QKKRCSKKGLGRSIMRRIT EIPETVSRQCSKEDKEGAD HGTAKGTALIRKNPPESSG NTGKSKEETLKNRVFSLKKS HSTYDHVRDQTEESSSLPT ESQEEETTENSTLESLSGKK LTQKLKEDSEAESTESVPLV CKSASAHNLSSEKKTGHPR TSMLQKSLSVIASAKEKTLG LAGKTQTAGVEERTKSQKP LPKDKETNRNHSNSDNTET KDPAPQNSNPAEEPRKPQ KSGIMKQQRVNPTTANSD LNPGTTQMKDNFDIGEVC PWEVYDLTPGPVPSESKV QKHVSIVASEMEKNPTFSL KEKSHHKPKAAEVCQQSN QKRIDKAEVCLWESQGQSI LEDEKLLISKTPVLPERAKEE | |
| | | NGGQPRAANVCAGQSEEL PPKAVASKTENENLNQIGH QEKKTSSSEENVRGSYNSS NNFQQPLTSRAEVCPWEF ETPAQPNAGRSVALPASSA LSANKIAGPRKEEIWDSFK V | |
| SEQ ID NO: 1900 | ENSG00000151229.8 | MSRKASENVEYTLRSLSSL MGERRRKQPEPDAASAAG ECSLLAAAESSTSLQSAGA GGGGVGDLERAARRQFQ QDETPAFVYVVAVFSALGG FLFGYDTGVVSGAMLLLKR QLSLDALWQELLVSSTVGA AAVSALAGGALNGVFGRR AAILLASALFTAGSAVLAAA NNKETLLAGRLVVGLGIGIA SMTVPVYIAEVSPPNLRGR LVTINTLFITGGQFFASVVD GAFSYLQKDGW | A*02:03,A*02:07,A*11:0 1,A*11:02,A*24:10,A*34: 01,B*15:01,B*15:21,B*1 5:27,B*27:04,B*40:01,B* 40:06,B*46:01,B*55:02,B *58:01,C*01:02,C*03:02, C*03:04,C*03:67,C*04:0 1,C*04:03,C*08:01,C*12: 02,C*15:02 |
| SEQ ID NO: 1901 | ENSG00000151914.13 | MAGYLSPAAYLYVEEQEYL QAYEDVLERYKDERDKVQ KKTFTKWINQHLMKVRKH VNDLYEDLRDGHNLISLLEV LSGDTLPREKGRMRFHRL QNVQIALDYLKRRQVKLVN IRNDDITDGNPKLTLGLIWT IILHFQISDIHVTGESEDMS AKERLLLWTQQATEGYAGI RCENFTTCWRDGKLFNAII HKYRPDLIDMNTVAVQSN LANLEHAFYVAEKIGVIRLL DPEDVDVSSPDEKSVITYVS SLYDAFPKVPEGGEGIGAN DVEVKWIEYQNMVNYLIQ WIRHHVTTMSERTFPNNP VELKALYNQYLQFKETEIPP KETEKSKIKRLYKLLEIWIEF GRIKLLQGYHPNDIEKEWG KLIIAMLEREKALRPEVERL EMLQQIANRVQRDSVICE DKLILAGNALQSDSKRLESG VQFQNEAEIAGYILECENLL RQHVIDVQILIDGKYYQAD QLVQRVAKLRDEIMALRN ECSSVYSKGRILTTEQTKLM ISGITQSLNSGFAQTLHPSL TSGLTQSLTPSLTSSSMTSG LSSGMTSRLTPSVTPAYTP | A*02:03,A*11:01,A*11:0 2,A*24:02,A*24:07,A*24: 10,A*33:03,A*34:01,B*1 5:01,B*15:27,B*39:01,B* 40:01,B*55:02,B*58:01,C *03:02,C*03:04,C*07:02, C*12:02,C*14:02,C*15:02 |
| | | GFPSGLVPNFSSGVEPNSL QTLKLMQIRKPLLKSSLLDQ NLTEEEINMKFVQDLLNW VDEMQVQLDRTEWGSDL PSVESHLENHKNVHRAIEE FESSLKEAKISEIQMTAPLKL TYAEKLHRLESQYAKLLNTS RNQERHLDTLHNFVSRAT NELIWLNEKEEEEVAYDWS ERNTNIARKKDYHAELMRE LDQKEENIKSVQEIAEQLLL ENHPARLTIEAYRAAMQT QWSWILQLCQCVEQHIKE NTAYFEFFNDAKEATDYLR NLKDAIQRKYSCDRSSSIHK LEDLVQESMEEKEELLQYK STIANLMGKAKTIIQLKPRN SDCPLKTSIPIKAICDYRQIEI TIYKDDECVLANNSHRAK WKVISPTGNEAMVPSVCF TVPPPNKEAVDLANRIEQQ YQNVLTLWHESHINMKSV VSWHYLINEIDRIRASNVAS IKTMLPGEHQQVLSNLQSR FEDFLEDSQESQVFSGSDIT QLEKEVNVCKQYYQELLKS AEREEQEESVYNLYISEVRN IRLRLENCEDRLIRQIRTPLE RDDLHESVFRITEQEKLKKE LERLKDDLGTITNKCEEFFS QAAASSSVPTLRSELNVVL QNMNQVYSMSSTYIDKLK TVNLVLKNTQAAEALVKLY ETKLCEEEAVIADKNNIENLI STLKQWRSEVDEKRQVFH ALEDELQKAKAISDEMFKT YKERDLDFDWHKEKADQL VERWQNVHVQIDNRLRDL EGIGKSLKYYRDTYHPLDD WIQQVETTQRKIQENQPE NSKTLATQLNQQKMLVSEI EMKQSKMDECQKYAEQYS ATVKDYELQTMTYRAMVD SQQKSPVKRRRMQSSADLI IQEFMDLRTRYTALVTLMT QYIKFAGDSLKRLEEEEKSL EEEKKEHVEKAKELQKWVS NISKTLKDAEKAGKPPFSK QKISSEEISTKKEQLSEALQT | |
| | | IQLFLAKHGDKMTDEERNE LEKQVKTLQESYNLLFSESL KQLQESQTSGDVKVEEKLD KVIAGTIDQTTGEVLSVFQ AVLRGLIDYDTGIRLLETQL MISGLISPELRKCFDLKDAK SHGLIDEQILCQLKELSKAK EIISAASPTTIPVLDALAQS MITESMAIKVLEILLSTGSLV IPATGEQLTLQKAFQQNLV SSALFSKVLERQNMCKDLI DPCTSEKVSLIDMVQRSTL QENTGMWLLPVRPQEGG RITLKCGRNISILRAAHEGLI DRETMFRLLSAQLLSGGLI NSNSGQRMTVEEAVREGV IDRDTASSILTYQVQTGGII QSNPAKRLTVDEAVQCDLI TSSSALLVLEAQRGYVGLI WPHSGEIFPTSSSLQQELIT NELAYKILNGRQKIAALYIP ESSQVIGLDAAKQLGIIDNN TASILKNITLPDKMPDLGDL EACKNARRWLSFCKFQPST VHDYRQEEDVFDGEEPVT TQTSEETKKLFLSYLMINSY MDANTGQRLLLYDGDLDE AVGMLLEGCHAEFDGNTA IKECLDVLSSSGVFLNNASG REKDECTATPSSFNKCHCG EPEHEETPENRKCAIDEEFN EMRNTVINSEFSQSGKLAS TISIDPKVNSSPSVCVPSLIS YLTQTELADISMLRSDSENI LTNYENQSRVETNERANEC SHSKNIQNFPSDLIENPIMK SKMSKFCGVNETENEDNT NRDSPIFDYSPRLSALLSHD KLMHSQGSFNDTHTPESN GNKCEAPALSFSDKTMLSG QRIGEKFQDQFLGIAAINIS LPGEQYGQKSLNMISSNP QVQYHNDKYISNTSGEDEK THPGFQQMPEDKEDESEIE EYSCAVTPGGDTDNAIVSL TCATPLLDETISASDYETSLL NDQQNNTGTDTDSDDDF YDTPLFEDDDHDSLLLDGD DRDCLHPEDYDTLQEEND | |
| | | ETASPADVFYDVSKENENS MVPQGAPVGSLSVKNKAH CLQDFLMDVEKDELDSGE KIHLNPVGSDKVNGQSLET GSERECTNILEGDESDSLTD YDIVGGKESFTASLKFDDSG SWRGRKEEYVTGQEFHSD TDHLDSMQSEESYGDYIYD SNDQDDDDDGIDEEGG GIRDENGKPRCQNVAEDM DIQLCASILNENSDENENIN TMILLDKMHSCSSLEKQQR VNVVQLASPSENNLVTEKS NLPEYTTEIAGKSKENLLNH EMVLKDVLPPIIKDTESEKT FGPASISHDNNNISSTSELG TDLANTKVKLIQGSELPELT DSVKGKDEYFKNMTPKVD SSLDHIICTEPDLIGKPAEES HLSLIASVTDKDPQGNGSD LIKGRDGKSDILIEDETSIQK MYLGEGEVLVEGLVEEENR HLKLLPGKNTRDSFKLINSQ FPFPQITNNEELNQKGSLK KATVTLKDEPNNLQIIVSKS PVQFENLEEIFDTSVSKEIS DDITSDITSWEGNTHFEESF TDGPEKELDLFTYLKHCAK NIKAKDVAKPNEDVPSHVL ITAPPMKEHLQLGVNNTKE KSTSTQKDSPLNDMIQSN DLCSKESISGGGTEISQFTP ESIEATLSILSRKHVEDVGK NDFLQSERCANGLGNDNS SNTLNTDYSFLEINNKKERI EQQLPKEQALSPRSQEKEV QIPELSQVFVEDVKDILKSR LKEGHMNPQEVEEPSACA DTKILIQNLIKRITTSQLVNE ASTVPSDSQMSDSSGVSP MTNSSELKPESRDDPFCIG NLKSELLLNILKQDQHSQKI TGVFELMRELTHMEYDLEK RGITSKVLPLQLENIFYKLLA DGYSEKIEHVGDFNQKACS TSEMMEEKPHILGDIKSKE GNYYSPNLETVKEIGLESST VWASTLPRDEKLKDLCNDF PSHLECTSGSKEMASGDSS | |
| | | TEQFSSELQQCLQHTEKM HEYLTLLQDMKPPLDNQES LDNNLEALKNQLRQLETFE LGLAPIAVILRKDMKLAEEF LKSLPSDFPRGHVEELSISH QSLKTAFSSLSNVSSERTKQ IMLAIDSEMSKLAVSHEEFL HKLKSFSDWVSEKSKSVKD IEIVNVQDSEYVKKRLEFLK NVLKDLGHTKMQLETTAF DVQFFISEYAQDLSPNQSK QLLRLLNTTQKCFLDVQES VTTQVERLETQLHLEQDLD DQKIVAERQQEYKEKLQGI CDLLTQTENRLIGHQEAFM IGDGTVELKKYQSKQEELQ KDMQGSAQALAEVVKNTE NFLKENGEKLSQEDKALIE QKLNEAKIKCEQLNLKAEQ SKKELDKVVTTAIKEETEKV AAVKQLEESKTKIENLLDW LSNVDKDSERAGTKHKQVI EQNGTHFQEGDGKSAIGE EDEVNGNLLETDVDGQVG TTQENLNQQYQKVKAQHE KIISQHQAVIIATQSAQVLL EKQGQYLSPEEKEKLQKN MKELKVHYETALAESEKKM KLTHSLQEELEKFDADYTEF EHWLQQSEQELENLEAGA DDINGLMTKLKRQKSFSED VISHKGDLRYITISGNRVLE AAKSCSKRDGGKVDTSAT HREVQRKLDHATDRFRSLY SKCNVLGNNLKDLVDKYQ HYEDASCGLLAGLQACEAT ASKHLSEPIAVDPKNLQRQ LEETKALQGQISSQQVAVE KLKKTAEVLLDARGSLLPAK NDIQKTLDDIVGRYEDLSKS VNERNEKLQITLTRSLSVQD GLDEMLDWMGNVESSLK EQDVGTGYCRSSEQYKCH E | |
| SEQ ID NO: 1902 | ENSG00000152359.10 | MSSDEEKYSLPVVQNDSSR GSSVSSNLQEEYEELLHYAI VTPNIEPCASQSSHPKGEL VPDVRISTIHDILHSQGNNS EVRETAIEVGKGCDFHISSH | A*02:03,A*11:01,A*11:0 2,A*24:02,A*24:10,A*33: 03,A*34:01,B*39:01,B*4 0:01,B*55:02,C*03:02,C* 03:04,C*12:02 |
| | | SKTDESSPVLSPRKPSHPV MDFFSSHLLADSSSPATNS SHTDAHEILVSDFLVSDENL QKMENVLDLWSSGLKTNII SELSKWRLNFIDWHRME MRKEKEKHAAHLKQLCNQ INELKELQKTFEISIGRKDEV ISSLSHAIGKQKEKIELMRTF FHWRIGHVRARQDVYEGK LADQYYQRTLLKKVWKVW RSVVQKQWKDVVERACQ ARAEEVCIQISNDYEAKVA MLSGALENAKAEIQRMQH EKEHFEDSMKKAFMRGVC ALNLEAMTIFQNRNDAGI DSTNNKKEEYGPGVQGKE HSAHLDPSAPPMPLPVTSP LLPSPPAAVGGASATAVPS AASMTSTRAASASSVHVP VSALGAGSAATAASEEMY VPRVVTSAQQKAGRTITAR ITGRCDFASKNRISSSLAIM GVSPPMSSVVVEKHHPVT VQTIPQATAAKYPRTIHPES STSASRSLGTRSAHTQSLTS VHSIKVVD | |
| SEQ ID NO: 1903 | ENSG00000153046.13 | MASEELYEVERIVDKRKNK KGKTEYLVRWKGYDSEDD TWEPEQHLVNCEEYIHDF NRRHTEKQKESTLTRTNRT SPNNARKQISRSTNSNFSK TSPKALVIGKDHESKNSQLF AASQKFRKNTAPSLSSRKN | A*02:03,A*11:01,A*11:0 2,A*33:03,B*15:01,C*03: 02,C*07:02,C*15:02 |
| SEQ ID NO: 1904 | ENSG00000154556.13 | MSYYQRPFSPSAYSLPASL NSSIVMQHGTSLDSTDTYP QHAQSLDGTTSSSIPLYRSS EEEKRVTVIKAPHYPGIGPV DESGIPTAIRTTVDRPKDW YKTMFKQIHMVHKPDDDT DMYNTPYTYNAGLYNPPY SAQSHPAAKTQTYRPLSKS HSDNSPNAFKDASSPVPPP HVPPPVPPLRPRDRSSTEK HDWDPPDRKVDTRKFRSE PRSIFEYEPGKSSILQHERPA SLYQSSIDRSLERPMSSAS MASDFRKRRKSEPAVGPP RGLGDQSASRTSPGRVDLP GSSTTLTKSFTSSSPSSPSRA | A*02:03,A*11:01,A*11:0 2,A*24:10,A*33:03,B*15: 01,B*15:27,B*39:01,B*5 8:01,C*03:02,C*03:04,C* 07:02,C*12:02,C*14:02,C *15:02 |
| | | KGGDDSKICPSLCSYSGLN GNPSSELDYCSTYRQHLDV PRDSPRAISFKNGWQMAR QNAEIWSSTEETVSPKIKSR SCDDLLNDDCDSFPDPKVK SESMGSLLCEEDSKESCPM AWGSPYVPEVRSNGRSRIR HRSARNAPGFLKMYKKM HRINRKDLMNSEVICSVKS RILQYESEQQHKDLLRAWS QCSTEEVPRDMVPTRISEF EKLIQKSKSMPNLGDDMLS PVTLEPPQNGLCPKRRFSIE YLLEEENQSGPPARGRRGC QSNALVPIHIEVTSDEQPR AHVEFSDSDQDGVVSDHS DYIHLEGSSFCSESDFDHFS FTSSESFYGSSHHHHHHHH HHHRHLISSCKGRCPASYT RFTTMLKHERARHENTEEP RRQEMDPGLSKLAFLVSPV PFRRKKNSAPKKQTEKAKC KASVFEALDSALKDICDQIK AEKKRGSLPDNSILHRLISEL LPDVPERNSSLRALRRSPLH QPLHPLPPDGAIHCPPYQN DCGRMPRSASFQDVDTAN SSCHHQDRGGAL | |
| SEQ ID NO: 1905 | ENSG00000155275.14 | MAEVGRTGISYPGALLPQG FWAAVEVWLERPQVANK RLCGARLEARWSAALPCAE ARGPGTSAGSEQKERGPG PGQGSPGGGPGPRSLSGP EQGTACCELEEAQGQCQQ EEAQREAASVPLRDSGHP GHAEGREGDFPAADLDSL WEDFSQSLARGNSELLAFL TSSGAGSQPEAQRELDVVL RTVIPKTSPHCPLTTPRREIV VQDVLNGTITFLPLEEDDE GNLKVKMSNVYQIQLSHS KEEWFISVLIFCPERWHSD GIVYPKPTWLGEELLAKLAK WSVENKKSDFKSTLSLISIM KYSKAYQELKEKYKEMVKV WPEVTDPEKFVYEDVAIAA YLLILWEEERAERRLTARQS FVDLGCGNGLLVHILSSEG HPGRGIDVRRRKIWDMYG | A*02:03,A*11:01,A*11:0 2,A*24:02,A*24:10,A*33: 03,B*15:01,B*15:27,B*3 9:01,B*40:01,B*55:02,B* 58:01,C*03:02,C*14:02,C *15:02 |
| | | PQTQLEEDAITPNDKTLFP DVDWLIGNHSDELTPWIP VIAARSSYNCRFFVLPCCFF DFIGRYSRRQSKKTQYREYL DFIKEVGFTCGFHVDEDCL RIPSTKRVCLVGKSRTYPSS REASVDEKRTQYIKSRRGC PVSPPGWELSPSPRWVAA GSAGHCDGQQALDARVG CVTRAWAAEHGAGPQAE GPWLPGFHPREKAERVRN CAALPRDFIDQVVLQVANL LLGGKQLNTRSSRNGSLKT WNGGESLSLAEVANELDT ETLRRLKRECGGLQTLLRNS HQVFQVVNGRVHIRDWR EETLWKTKQPEAKQRLLSE ACKTRLCWFFMHHPDGC ALSTDCCPFAHGPAELRPP RTTPRKKIS | |
| SEQ ID NO: 1906 | ENSG00000155506.12 | MATQVEPLLPGGATLLQA EEHGGLVRKKPPPAPEGKG EPGPNDVRGGEPDGSARR PRPPCAKPHKEGTGQQER ESPRPLQLPGAEGPAISDG EEGGGEPGAGGGAAGAA GAGRRDFVEAPPPKVNPW TKNALPPVLTTVNGQ | A*02:03 |
| SEQ ID NO: 1907 | ENSG00000157514.12 | MNTEMYQTPMEVAVYQL HNFSISFFSSLLGGDVVSVK LD | A*02:03,A*24:02,A*24:0 7,A*24:10,B*15:01,C*03: 02,C*03:04,C*03:67,C*1 2:02,C*15:02 |
| SEQ ID NO: 1908 | ENSG00000158321.11 | MDGPTRGHGLRKKRRSRS QRDRERRSRGGLGAGAAG GGGAGRTRALSLASSSGSD KEDNGKPPSSAPSRPRPPR RKRRESTSAEEDIIDGFAMT SFVTFEALEKDVALKPQER VEKRQTPLTKKKREALTNG LSFHSKKSRLSHPHHYSSDR ENDRNLCQHLGKRKKMPK ALRQLKPGQNSCRDSDSES ASGESKGFHRSSSRERLSDS SAPSSLGTGYFCDSDSDQE EKASDASSEKLFNTVIVNKD PELGVGTLPEHDSQDAGPI VPKISGLERSQEKSQDCCKE PIFEPVVLKDPCPQVAQPIP QPQTEPQLRAPSPDPDLV | A*02:03,A*24:10,B*15:0 1,B*15:27,B*39:01,B*58: 01,C*03:02,C*03:04,C*0 3:67,C*12:02,C*14:02,C* 15:02 |
| | | QRTEAPPQPPPLSTQPPQ GPPEAQLQPAPQPQVQRP PRPQSPTQLLHQNLPPVQ AHPSAQSLSQPLSAYNSSSL SLNSLSSSRSSTPAKTQPAP PHISHHPSASPFPLSLPNHS PLHSFTPTLQPPAHSHHPN MFAPPTALPPPPPLT | |
| SEQ ID NO: 1909 | ENSG00000158486.9 | MGATGRLELTLAAPPHPG PAFQRSKARETQGEEEGSE MQIAKSDSIHHMSHSQGQ PELPPLPASANEEPSGLYQT VMSHSFYPPLMQRTSWTL AAPFKEQHHHRGPSDSIA NNYSLMAQDLKLKDLLKVY QPATISVPRDRTGQGLPSS GNRSSSEPMRKKTKFSSRN KEDSTRIKLAFKTSIFSPMK KEVKTSLTFPGSRPMSPEQ QLDVMLQQEMEMESKEK KPSESDLERYYYYLTNGIRK DMIAPEEGEVMVRISKLIS NTLLTSPFLEPLMVVLVQE KENDYYCSLMKSIVDYILM DPMERKRLFIESIPRLFPQR VIRAPVPWHSVYRSAKKW NEEHLHTVNPMMLRLKEL WFAEFRDLRFVRTAEILAG KLPLQPQEFWDVIQKHCLE AHQTLLNKWIPTCAQLFTS RKEHWIHFAPKSNYDSSRN IEEYFASVASFMSLQLRELV IKSLEDLVSLFMIHKDGNDF KEPYQEMKFFIPQLIMIKLE VSEPIIVFNPSFDGCWELIR DSFLEIIKNSNGIPKLKYIPLK FSFTAAAADRQCVKAAEP GEPSMHAAATAMAELKGY NLLLGTVNAEEKLVSDFLIQ TFKVFQKNQVGPCKYLNV YKKYVDLLDNTAEQNIAAF LKENHDIDDFVTKINAIKKR RNEIASMNITVPLAMFCLD ATALNHDLCERAQNLKDH LIQFQVDVNRDTNTSICNQ YSHIADKVSEVPANTKELVS LIEFLKKSSAVTVFKLRRQLR DASERLEFLMDYADLPYQI EDIFDNSRNLLLHKRDQAE | A*02:03,A*02:07,A*11:0 1,A*11:02,A*24:02,A*24: 07,A*24:10,A*33:03,A*3 4:01,B*15:01,B*15:21,B* 15:27,B*27:04,B*38:02,B *39:01,B*40:01,B*40:06, B*46:01,B*51:01,B*55:0 2,B*58:01,C*01:02,C*03: 02,C*03:04,C*03:67,C*0 4:01,C*04:03,C*07:02,C* 08:01,C*12:02,C*14:02,C *15:02 |
| | | MDLIKRCSEFELRLEGYHRE LESFRKREVMTTEEMKHN VEKLNELSKNLNRAFAEFEL INKEEELLEKEKSTYPLLQA MLKNKVPYEQLWSTAYEF SIKSEEWMNGPLFLLNAEQ IAEEIGNMWRTTYKLIKTLS DVPAPRRLAENVKIKIDKFK QYIPILSISCNPGMKDRHW QQISEIVGYEIKPTETTCLSN MLEFGFGKFVEKLEPIGAA ASKEYSLEKNLDRMKLDW VNVTFSFVKYRDTDTNILC AIDDIQMLLDDHVIKTQTM CGSPFIKPIEAECRKWEEKLI RIQDNLDAWLKCQATWLY LEPIFSSEDIIAQMPEEGRK FGIVDSYWKSLMSQAVKD NRILVAADQPRMAEKLQE ANFLLEDIQKGLNDYLEKKR LFFPRFFFLSNDELLEILSETK DPLRVQPHLKKCFEGIAKLE FTDNLEIVGMISSEKETVPFI QKIYPANAKGMVEKWLQ QVEQMMLASMREVIGLGI EAYVKVPRNHWVLQWPG QVVICVSSIFWTQEVSQAL AENTLLDFLKKSNDQIAQIV QLVRGKLSSGARLTLGALT VIDVHARDVVAKLSEDRVS DLNDFQWISQLRYYWVAK DVQVQIITTEALYGYEYLGN SPRLVITPLTDRCYRTLMGA LKLNLGGAPEGPAGTGKTE TTKDLAKALAKQCVVFNCS DGLDYKAMGKFFKGLAQA GAWACFDEFNRIEVEVLSV VAQQILSIQQAIIRKLKTFIF EGTELSLNPTCAVFIT | |
| SEQ ID NO: 1910 | ENSG00000159263.11 | MKEKSKNAAKTRREKENG EFYELAKLLPLPSAITSQLDK ASIIRLTTSYLKMRAVFPEG LGDA | A*02:03,A*24:02,A*24:0 7,A*24:10,A*34:01,B*15: 01,B*15:21,B*15:27,B*3 8:02,B*39:01,B*40:01,B* 40:06,B*51:01,B*55:02,C *14:02,C*15:02 |
| SEQ ID NO: 1911 | ENSG00000159788.14 | MFRAGEASKRPLPGPSPPR VRSVEVARGRAGYGFTLSG QAPCVLSCVMRGSPADFV GLRAGDQILAVNEINVKKA | A*02:03,A*11:01,A*11:0 2,A*24:10,A*33:03,A*34: 01,B*15:01,B*40:01,B*5 5:02,C*15:02 |
| | | SHEDVVKLIGKCSGVLHMV IAEGVGRFESCSSDEEGGLY EGKGWLKPKLDSKALGINR AERVVEEMQSGGIFNMIF ENPSLCASNSEPLKLKQRSL SESAATRFDVGHESINNPN PNMLSKEEISKVIHDDSVFS IGLESHDDFALDASILNVA MIVGYLGSIELPSTSSNLES DSLQAIRGCMRRLRAEQKI HSLVTMKIMHDCVQLSTD KAGVVAEYPAEKLAFSAVC PDDRRFFGLVTMQTNDD GSLAQEEEGALRTSCHVF MVDPDLFNHKIHQGIARR FGFECTADPDTNGCLEFPA SSLPVLQFISVLYRDMGELI EGMRARAFLDGDADAHQ NNSTSSNSDSGIGNFHQEE KSNRVLVVD | |
| SEQ ID NO: 1912 | ENSG00000160200.13 | | A*02:03,A*11:01,A*11:0 2,A*24:10,A*33:03,B*15: 01,B*38:02,B*39:01,B*4 0:01,B*58:01,C*03:02,C* 03:04,C*07:02,C*14:02 |
| | | | |
| SEQ ID NO: 1913 | ENSG00000160799.7 | MQDGRKGGAYAGKMEAT TAGVGRLEEEALRRKERLK ALREKTG | A*02:03 |
| SEQ ID NO: 1914 | ENSG00000160838.9 | MSSEQSAPGASPRAPRPG TQKSSGAVTKKGERAAKEK PATVLPPVGEEEPKSPEEY QCSGVLETDFAELCTRWG YTDFPKVVNRPRPHPPFVP SASLSEKATLDDPRLSGSCS LNSLESKYVFFRPTIQVELE QEDSKSVKEIYIRGWKVEE RILGVFSKCLPPLTQLQAIN LWKVGLTDKTLTTFIELLPL CSSTLRKVSLEGNPLPEQSY HKL | A*02:03,A*11:01,A*11:0 2,A*24:02,A*24:07,A*24: 10,B*40:01,B*55:02,C*0 1:02,C*03:02,C*04:01,C* 04:03,C*07:02,C*15:02 |
| SEQ ID NO: 1915 | ENSG00000164093.11 | METNCRKLVSACVQLGVQ PAAVECLFSKDSEIKKVEFT DSPESRKEAASSKFFPRQH | A*11:01,A*11:02,A*33:0 3 |
| SEQ ID NO: 1916 | ENSG00000164764.10 | MRTLWMALCALSRLWPG AQAGCAEAGRCCPGRDPA CFARGWRLDRVYGTCFCD QACRFTGDCCFDYDRACP ARPCFVGEWSPWSGCAD QCKPTTRVRRRSVQQEPQ NGGAPCPPLEERAGCLEYS TPQGQDCGHTYVPAFITTS AFNKERTRQATSPHWSTH TEDAGYCMEFKTESLTPHC ALENWPLTRWMQYLREG YTVCVDCQPPAMNSVSLR CSGDGLDSDGNQTLHWQ AIGNPRCQGTWKKVRRVD QCSCPAVHSFIFI | A*11:01,A*11:02,A*24:1 0,A*33:03,B*55:02,C*03: 02,C*03:04 |
| SEQ ID NO: 1917 | ENSG00000164830.13 | MDYLTTFTEKSGRLLRGTA NRLLGFGGGGEARQVRFE DYLREPAQGDLGCGSPPH RPPAPSSPEGP | A*33:03 |
| SEQ ID NO: 1918 | ENSG00000166689.10 | MAAATVGRDTLPEHWSY GVCRDGRVFFINDQLRCTT WLHPRTGEPVNSGHMIRS DLPRGWEE | A*33:03 |
| SEQ ID NO: 1919 | ENSG00000167157.9 | MDSAAAAFALDKPALGPG PPPPPPALGPGDCAQARK NFSVSHLLDLEEVAAAGRL AARPGARAEAREGAAREP SGGSSGSEAAPQ | A*11:01,A*11:02,C*03:0 2,C*03:04,C*03:67 |
| SEQ ID NO: 1920 | ENSG00000167632.10 | MSVPDYMQCAEDHQTLL VVVQPVGIVSEENFFRIYKR | A*02:03,A*02:07,A*11:0 1,A*11:02,A*24:02,A*24:07,A*24:10,A*33:03,B*1 5:01,B*15:27,B*39:01,B* 40:01,B*55:02,B*58:01,C *03:02,C*03:04,C*03:67, C*07:02,C*12:02,C*14:0 2,C*15:02 |
| | | ICSVSQISVRDSQRVLYIRYR HHYPPENNEWGDFQTHR KVVGLITITDCFSAKDWPQ TFEKFHVQKEIYGSTLYDSR LFVFGLQGEIVEQPRTDVA FYPNYEDCQTVEKRIEDFIE SLFIVLESKRLDRATDKSGD KIPLLCVPFEKKDFVGLDTD SRHYKKRCQGRMRKHVG DLCLQAGMLQDSLVHYH MSVELLRSVNDFLWLGAA LEGLCSASVIYHYPGGTGG KSGARRFQGSTLPAEAANR HRPGALTTNGINPDTSTEI GRAKNCLSPEDIIDKYKEAIS YYSKYKNAGVIELEACIKAV RVLAIQKRSMEASEFLQNA VYINLRQLSEEEKIQRYSILS ELYELIGFHRKSAFFKRVAA MQCVAPSIAEPGWRACYK LLLETLPGYSLSLDPKDFSR GTHRGWAAVQMRLLHEL VYASRRMGNPALSVRHLSF LLQTMLDFLSDQEKKDVA QSLENYTSKCPGTMEPIAL PGGLTLPPVPFTKLPIVRHV KLLNLPASLRPHKMKSLLG QNVSTKSPFIYSPIIAHNRG EERNKKIDFQWVQGDVCE VQLMVYNPMPFELRVEN MGLLTSGVEFESLPAALSLP AESGLYPVTLVGVPQTTGTI TVNGYHTTVFGVFSDCLLD NLPGIKTSGSTVEVIPALPR LQISTSLPRSAHSLQPSSGD EISTNVSVQLYNGESQQLII KLENIGMEPLEKLEVTSKVL TTKEKLYGDFLSWKLEETLA QFPLQPGKVATFTINIKVKL DFSCQENLLQDLSDDGISV SGFPLSSPFRQVVRPRVEG KPVNPPESNKAGDYSHVKT LEAVLNFKYSGGPGHTEGY YRNLSLGLHVEVEPSVFFTR VSTLPATSTRQCHLLLDVF NSTEHELTVSTRSSEALILH AGECQRMAIQVDKFNFES FPESPGEKGQFANPKQLEE ERREARGLEIHSKLGICWRI | |
| | | PSLKRSGEASVEGLLNQLVL EHLQLAPLQWDVLVDGQP CDREAVAACQVGDPVRLE VRLTNRSPRSVGPFALTVV PFQDHQNGVHNYDLHDT VSFVGSSTFYLDAVQPSGQ SACLGALLFLYTGDFFLHIRF HEDSTSKELPPSWFCLPSV HVCALEAQA | |
| SEQ ID NO: 1921 | ENSG00000170615.10 | MDHAEENEILAATQRYYVE RPIFSHPVLQERLHTKDKVP DSIADKLKQAFTCTPKKIRN IIYMFLPITKWLPAYKFKEY VLGDLVSGISTGVLQLPQG LAFAMLAAVPPIFGLYSSFY PVIMYCFLGTSRHISIGPFA VISLMIGGVAVRLVPDDIVI PGGVNATNGTEARDALRV KVAMSVTLLSGIIQFCLGVC RFGFVAIYLTEPLVRGFTTA AAVHVFTSMLKYLFGVKTK RYSGIFSVVYSTVAVLQNV KNLNVCSLGVGLMVFGLLL GGKEFNERFKEKLPAPIPLE FFAVVMGTGISAGFNLKES YNVDVVGTLPLGLLPPANP DTSLFHLVYVDAIAIAIVGFS VTISMAKTLANKHGYQVD GNQELIALGLCNSIGSLFQT FSISCSLSRSLVQEGTGGKT QLAGCLASLMILLVILATGF LFESLPQAVLSAIVIVNLKG MFMQFSDLPFFWRTSKIEL TIWLTTFVSSLFLGLDYGLIT AVIIALLTVIYRTQS | A*02:03,A*02:07,A*11:0 1,A*11:02,A*24:02,A*24: 07,A*24:10,A*33:03,A*3 4:01,B*15:01,B*15:21,B* 15:27,B*27:04,B*38:02,B *39:01,B*40:01,B*40:06, B*46:01,B*51:01,B*55:0 2,B*58:01,C*01:02,C*03: 02,C*03:04,C*03:67,C*0 4:01,C*04:03,C*08:01,C* 12:02,C*14:02,C*15:02 |
| SEQ ID NO: 1922 | ENSG00000171680.16 | MHYDGHVRFDLPPQGSVL ARNVSTRSCPPRTSPAVDL EEEEEEESSVDGKGDRKSTG LKLSKKKARRRHTDDPSKE CFTLKFDLNVDIETEIVPAM KKKSLGEVLLPVFERKGIAL GKVDIYLDQSNTPLSLTFEA YRFGGHYLRVKAPAKPGDE GKVEQGMKDSKSLSLPILR PAGTGPPALERVDAQSRRE SLDILAPGRRRKNMSEFLG EASIPGQEPPTPSSCSLPSG SSGSTNTGDSWKNRAASR FSGFFSSGPSTSAFGREVDK | A*02:03,A*02:07,A*11:0 1,A*11:02,A*24:10,A*33: 03,B*15:01,B*39:01,B*4 0:01,B*58:01,C*03:02,C* 03:04,C*07:02,C*12:02,C *14:02,C*15:02 |
| | | MEQLEGKLHTYSLFGLPRL PRGLRFDHDSWEEEYDED EDEDNACLRLEDSWRELID GHEKLTRRQCHQQEAVW ELLHTEASYIRKLRVIINLFLC CLLNLQESGLLCEVEAERLF SNIPEIAQLHRRLWASVMA PVLEKARRTRALLQPGDFL KGFKMFGSLFKPYIRYCME EEGCMEYMRGLLRDNDLF RAYITWAEKHPQCQRLKLS DMLAKPHQRLTKYPLLLKS VLRKTEEPRAKEAVVAMIG SVERFIHHVNACMRQRQE RQRLAAVVSRIDAYEVVES SSDEVDKLLKEFLHLDLTAPI PGASPEETRQLLLEGSLRM KEGKDSKMDVYCFLFTDLL LVTKAVKKAERTRVIRPPLL VDKIVCRELRDPGSFLLIYLN EFHSAVGAYTFQASGQALC RGWVDTIYNAQNQLQQL RAQEPPGSQQPLQSLEEEE DEQEEEEEEEEEEEGEDS GTSAASSPTIMRKSSGSPD SQHCASDGSTETLAMVVV EPGDTLSSPEFDSGPFSSQS DETSLSTTASSATPTSELLPL GPVDGRSCSMDSAYGTLS PTSLQDFVAPGPMAELVP RAPESPRVPSPPPSPRLRRR TPVQLLSCPPHLLKSKSEAS LLQLLAGAGTHGTPSAPSR SLSELCLAVPAPGIRTQGSP QEAGPSWDCRGAPSPGSG PGLVGCLAGEPAGSHRKRC GDLPSGASPRVQPEPPPGV SAQHRKLTLAQLYRIRTTLL LNSTLTASEV | |
| SEQ ID NO: 1923 | ENSG00000171791.10 | MAHAGRTGYDNREIVMK YIHYKLSQRGYEWDAGDV GAAPPGAAPAPGIFSSQPG HTPHPAASRDPVARTSPLQ TPAAPGAAAGPALSPVPPV VHLTLRQAGDDFSRRYRRD FAEMSSQLHLTPFTARGRF ATVVEELFRDGVNWGRIV AFFEFGGVMCVESVNREM SPLVDNIALWMTEYLNRHL | A*02:03,A*11:01,A*11:0 2,A*24:02,A*24:07,A*24: 10,A*33:03,A*34:01,B*1 5:21,B*27:04,B*40:01,B* 40:06,B*46:01,B*55:02,B *58:01,C*01:02,C*03:02, C*04:01,C*04:03,C*14:02 |
| | | HTWIQDNGGWDAFVELY GPS | |
| SEQ ID NO: 1924 | ENSG00000172765.12 | MKRGTSLHSRRGKPEAPK GSPQINRKSGQEMTAVM QSGRPRSSSTTDAPTSSAM MEIACAAAAAAAACLPGE EGTAE | A*02:03,A*33:03,C*03:0 2,C*03:04 |
| SEQ ID NO: 1925 | ENSG00000174672.11 | MTSTGKDGGAQHAQYVG PYRLEKTLGKGQTGLVKLG VHCVTCQKVAIKIVNREKLS ESVLMKVEREIAILKLIEHPH VLKLHDVYENKKYLYLVLEH VSGGELFDYLVKKGRLTPK EARKFFRQIISALDFCHSHSI CHRDLKPENLLLDEKNNIRI ADFGMASLQVGDSLLETSC GSPHYACPEVIRGEKYDGR KADVWSCGVILFALLVGAL PFDDDNLRQLLEKVKRGVF HMPHFIPPDCQSLLRGMIE VDAARRLTLEHIQKHIWYI GGKNEPEPEQPIPRKVQIR SLPSLEDIDPDVLDSMHSL GCFRDRNKLLQDLLSEEEN QEKMIYFLLLDRKERYPSQE DEDLPPRNEIDPPRKRVDS PMLNRHGKRRPERKSMEV LSVTDGGSPVPARRAIEMA QHGQSKAMFSKSLDIAEA HPQFSKEDRSRSISGASSGL STSPLSSPRVTPHPSPRGSP LPTPKGTPVHTPKESPAGT PNPTPPSSPSVGGVPWRA RLNSIKNSFLGSPRFHRRKL QVPTPEEMSNLTPESSPEL AKKSWFGNFISLEKEEQIFV VIKDKPLSSIKADIVHAFLSI PSLSHSVISQTSFRAEYKAT GGPAVFQKPVKFQVDITYT EGGEAQKENGIYSVTFTLLS GPSRRFKRVVETIQAQLLST HDPPAAQHLSEPPPPAPGL SWGAGLKGQKVATSYESSL | A*02:03,A*11:01,A*11:0 2,A*24:02,A*24:10,A*33: 03,B*40:01,C*03:02,C*0 3:04,C*14:02 |
| SEQ ID NO: 1926 | ENSG00000177380.9 | MMCEVMPTISEDGRRGSA LGPDEAGGELERLMVTML TERERLLETLREAQDGLAT AQLRLRELGHEKDSLQRQL SIALPQEFAALTKELNLCRE QLLEREEEIAELKAERNNTR | A*02:03,A*11:01,A*11:0 2,A*24:10,A*33:03,B*15: 01,B*39:01,B*40:01,B*5 8:01,C*03:02,C*03:04,C* 03:67,C*12:02 |
| | | LLLEHLECLVSRHERSLRMT VVKRQAQSPGGVSSEVEV LKALKSLFEHHKALDEKVRE RLRMALERVAVLEEELELS NQETLNLREQLSRRRSGLE EPGKDGDGQTLANGLGPG GDSNRRTAELEEALERQRA EVCQLRERLAVLCRQMSQ LEEELGTAHRELGKAEEAN SKLQRDLKEALAQREDME ERITTLEKRYLSAQREATSL HDANDKLENELASKESLYR QSEEKSRQLAEWLDDAKQ KLQQTLQKAETLPEIEAQLA QRVAALNKAEERHGNFEE RLRQLEAQLEEKNQELQRA RQREKMNDDHNKRLSETV DKLLSESNERLQLHLKERM GALEEKNSLSEEIANMKKL QDELLLNKEQLLAEMERM QMEIDQLRGRPPSSYSRSL PGSALELRYSQAPTLPSGA HLDPYVAGSGRAGKRGR WSGVKEEPSKDWERSAPA GSIPPPFPGELDGSDEEEAE GMFGAELLSPSGQADVQT LAIMLQEQLEAINKEIKLIQE EKETTEQRAEELESRVSSSG LDSLGRYRSSCSLPPSLTTST LASPSPPSSGHSTPRLAPPS PAREGTDKANHVPKEEAG APRGEGPAIPGDTPPPTPR SARLERMTQALALQAGSLE DGGPPRGSEGTPDSLHKA PKKKSIKSSIGRLFGKKEKG RMGPPGRDSSSLAGTPSD ETLATDPLGLAKLTGPGDK DRRNKRKHELLEEACRQGL PFAAWDGPTVVSWLELW VGMPAWYVAACRANVKS GAIMANLSDTEIQREIGISN PLHRLKLRLAIQEMVSLTSP SAPASSRTSTGNVWMTHE EMESLTATTKPILAYGDMN HEWVGNDWLPSLGLPQY RSYFMESLVDARMLDHLN KKELRGQLKMVDSFHRVSL HYGIMCLKRLNYDRKDLER RREESQTQIRDVMVWSNE | |
| | | RVMGWVSGLGLKEFATNL TESGVHGALLALDETFDYS DLALLLQIPTQNAQARQLL EKEFSNLISLGTDRRLDEDS AKSFSRSPSWRKMFREKDL RGVTPDSAEMLPPNFRSA AAGALGSPGLPLRKLQPEG QTSGSSRADGVSVRTYSC | |
| SEQ ID NO: 1927 | ENSG00000177455.7 | | A*02:03,A*11:01,A*11:0 2,A*24:10,B*39:01,B*40: 01,B*58:01,C*03:02,C*0 3:04,C*12:02,C*14:02,C* 15:02 |
| SEQ ID NO: 1928 | ENSG00000178209.10 | MVAGMLMPRDQLRAIYE VLFREGVMVAKKDRRPRSL HPHVPGVTNLQVMRAMA SLRARGLVRETFAWCHFY WYLTNEGIAHLRQYLHLPP EIVPASLQRVRRPVAMVM PARRTPHVQAVQGPLGSP PKRGPLPTEEQRVYRRKEL EEVSPETPVVPATTQRTLA RPGPEPAPAT | A*02:03,A*11:01,A*11:0 2,A*24:02,A*24:10,A*33: 03,A*34:01,B*55:02,C*0 3:02,C*03:04 |
| SEQ ID NO: 1929 | ENSG00000181035.9 | | A*02:03,A*11:01,A*11:0 2,A*24:02,A*24:07,A*24: 10,A*33:03,B*15:01,B*3 9:01,B*40:01,C*03:02,C* 03:04,C*03:67,C*12:02,C *14:02 |
| SEQ ID NO: 1930 | ENSG00000185404.12 | MAGGGSDLSTRGLNGGVS QVANEMNHLPAHSQSLQ RLFTEDQDVDEGLVYDTVF KHFKRHKLEISNAIKKTFPFL EGLRDRELITNK | A*02:03,A*24:10,A*33:0 3,C*03:02 |
| SEQ ID NO: 1931 | ENSG00000185686.13 | MERRRLWGSIQSRYISMS VWTSPRRLVELAGQSLLKD EALAIAALELLPRELFPPLF MAAFDGRHSQTLKAMVQ AWPFTCLPLGVLMKGQHL HLETFKAVLDGLDVLLAQE VRPRRWKLQVLDLRKNSH QDFWTVWSGNRASLYSFP EPEAAQPMTKKRKVDGLS TEAEQPFIPVEVLVDLFLKE GACDELFSYLIEKVKRKKNV LRLCCKKLKIFAMPMQDIK MILKMVQLDSIEDLEVTCT WKLPTLAKFSPYLGQMINL RRLLLSHIHASSYISPEKEEQ YIAQFTSQFLSLQCLQALYV DSLFFLRGRLDQLLRHVMN PLETLSITNCRLSEGDVMHL SQSPSVSQLSVLSLSGVML TDVSPEPLQALLERASATL QDLVFDECGITDDQLLALL PSLSHCSQLTTLSFYGNSISI SALQSLLQHLIGLSNLTHVL YPVPLESYEDIHGTLHLERL AYLHARLRELLCELGRPSM VWLSANPCPHCGDRTFYD PEPILCPCFMPN | A*02:03,A*11:01,A*11:0 2,A*24:10,A*33:03,B*15: 01,B*39:01,B*40:01,B*5 8:01,C*03:02,C*03:04,C* 14:02 |
| SEQ ID NO: 1932 | ENSG00000185989.9 | MAVEDEGLRVFQSVKIKIG EAKNLPSYPGPSKMRDCYC TVNLDQEEVFRTKIVEKSLC PFYGEDFYCEIPRSFRHLSF YIFDRDVFRRDSIIGKVAIQ KEDLQKYHNRDTWFQLQH VDADSEVQGKVHLELRLSE VITDTGVVCHKLATRIVEC QGLPIVNGQCDPYATVTLA GPFRSEAKKTKVKRKTNNP QFDEVFYFEVTRPCSYSKKS HFDFEEEDVDKLEIRVDLW NASNLKFGDEFLGELRIPLK VLRQSSSYEAWYFLQPRD NGSKSLKPDDLGSLRLNVV YTEDHVFSSDYYSPLRDLLL KSADVEPVSASAAHILGEV CREKQEAAVPLVRLFLHYG RVVPFISAIASAEVKRTQDP NTIFRGNSLASKCIDETMKL AGMHYLHVTLKPAIEEICQ SHKPCEIDPVKLKDGENLE NNMENLRQYVDRVFHAIT ESGVSCPTVMCDIFFSLREA AAKRFQDDPDVRYTAVSSF IFLRFFAPAILSPNLFQLTPH HTDPQTSRTLTLISKTVQTL GSLSKSKSASFKESYMATFY EFFNEQKYADAVKNFLDLIS SSGRRDPKSVEQPIVLKEG | A*02:03,A*11:01,A*11:0 2,A*24:02,A*24:07,A*24: 10,A*33:03,B*15:01,B*1 5:27,B*39:01,B*40:01,B* 58:01,C*03:02,C*03:04,C *07:02,C*12:02,C*14:02 |
| SEQ ID NO: 1933 | ENSG00000196961.8 | MPAVSKGDGMRGLAVFIS DIRNCKSKEAEIKRINKELA NIRSKFKGDKALDGYSKKK YVCKLLFIFLLGHDIDFGHM EAVNLLSSNKYTEKQIGYLFI SVLVNSNSELIRLINNAIKN DLASRNPTFMCLALHCIAN VGSREMGEAFAADIPRILV AGDSMDSVKQSAALCLLRL YKASPDLVPMGEWTARVV HLLNDQHMGVVTAAVSLI TCLCKKNPDDFKTCVSLAV SRLSRIVSSASTDLQDYTYY FVPAPWLSVKLLRLLQCYP PPEDAAVKGRLVECLETVL NKAQEPPKSKKVQHSNAK NAILFETISLIIHYDSEPNLLV RACNQLGQFLQHRETNLR YLALESMCTLASSEFSHEAV | A*02:03,A*11:01,A*11:0 2,A*24:02,A*24:07,A*24: 10,A*33:03,A*34:01,B*1 5:01,B*15:27,B*39:01,B* 40:01,B*40:06,B*58:01,C *03:02,C*03:04,C*03:67, C*08:01,C*12:02,C*14:0 2,C*15:02 |
| | | KTHIDTVINALKTERDVSVR QRAADLLYAMCDRSNAKQ IVSEMLRYLETADYAIREEIV LKVAILAEKYAVDYSWYVD TILNLIRIAGDYVSEEVWYR VLQIVTNRDDVQGYAAKT VFEALQAPACHENMVKVG GYILGEFGNLIAGDPRSSPP VQFSLLHSKFHLCSVATRAL LLSTYIKFINLFPETKATIQG VLRAGSQLRNADVELQQR AVEYLTLSSVASTDVLATVL EEMPPFPERESSILAKLKRK KGPGAGSALDDGRRDPSS NDINGGMEPTPSTVSTPSP SADLLGLRAAPPPAAPPAS AGAGNLLVDVFDGPAAQP SLGPTPEEAFLSPGPEDIGP PIPEADELLNKFVCKNNGV LFENQLLQIGVKSEFRQNL GRMYLFYGNKTSVQFQNF SPTVVHPGDLQTQLAVQT KRVAAQVDGGAQVQQVL NIECLRDFLTPPLLSVRFRY GGAPQALTLKLPVTINKFF QPTEMAAQDFFQRWKQL SLPQQEAQKIFKANHPMD AEVTKAKLLGFGSALLDNV DPNPENFVGAGIIQTKALQ VGCLLRLEPNAQAQMYRL TLRTSKEPVSRHLCELLAQQ F | |
| SEQ ID NO: 1934 | ENSG00000197530.8 | MAGALRRGRALGSRPSGP TVSSRRSPQCPVAQEGLGA RSRPRVAPRSLARCGPSSRL MGWKPSEARGQSQSFQA SGLQPRSLKAARRATGRPD RSRAAPPNMDPDPQAGV QVGMRVVRGVDWKWGQ QDGGEGGVGTVVELGRH GSPSTPDRTVVVQWDQG TRTNYRAGYQGAHDLLLYD NAQIGVRHPNIICDCCKKH GLRGMRWKCRVCLDYDLC TQCYMHNKHELAHAFDRY ETAHSRPVTLSPRQGLPRIP LRGIFQGAKVVRGPDWE WGSQDGGEGKPGRVVDI RGWDVETGRSVASVTWA | A*02:03,A*11:01,A*11:0 2,A*24:02,A*24:07,A*24: 10,A*33:03,B*15:01,B*3 9:01,B*40:01,B*58:01,C* 03:02,C*03:04,C*07:02,C *12:02,C*14:02 |
| | | DGTTNVYRVGHKGKVDLK CVGEAAGGFYYKDHLPRLG KPAELQRRVSADSQPFQH GDKVKCLLDTDVLREMQE GHGGWNPRMAEFIGQTG TVHRITDRGDVRVQFNHE TRWTFHPGALTKHHSFWV GDVVRVIGDLDTVKRLQA GHGEWTDDMAPALGRVG KVVKVFGDGNLRVAVAGQ RWTFSPSCLVAYRPEEDAN LDVAERARENKSSLSVALD KLRAQKSDPEHPGRLVVEV ALGNAARALDLLRRRPEQV DTKNQGRTALQVAAYLGQ VELIRLLLQARAGVDLPDDE GNTALHYAALGNQPEATR VLLSAGCRADAINSTQSTA LHVAVQRGFLEVVRALCER GCDVNLPDAHSDTPLHSAI SAGTGASGIVEVLTEVPNID VTATNSQGFTLLHHASLKG HALAVRKILARARQLVDAK KEDGFTALHLAALNNHREV AQILIREGRCDVNVRNRKL QSPLHLAVQQAHVGLVPLL VDAGCSVNAEDEEGDTAL HVALQRHQLLPLVADGAG GDPGPLQLLSRLQASGLPG SAELTVGAAVACFLALEGA DVSYTNHRGRSPLDLAAEG RVLKALQGCAQRFRERQA GGGAAPGPRQTLGTPNTV TNLHVGAAPGPEAAECLV CSELALLVLFSPCQHRTVCE ECARRMKKCIRCQVVVSKK LRPDGSEVASAAPAPGPPR QLVEELQSRYRQMEERITC PICIDSHIRLVFQCGHGACA PCGSALSACPICRQPIRDRI QIFV | |
| SEQ ID NO: 1935 | ENSG00000204839.4 | MAGGVWGRSRAREAPVG ALTLTALTEGIRARQGQPQ GPPSAGPQPKSWEVKPEA EPQTQALTAPSEAEPGRGA TVPEAGSEPCSLNSALEPAP EGPHQVPQSSWEEGVLAD LALYTAACLEEAGFAGTQA TVLTLSSALEARGERLEDQV | A*02:03,A*11:01,A*11:0 2,A*24:02,A*24:07,A*24: 10,A*33:03,B*39:01,B*4 0:01,B*58:01,C*03:02,C* 03:04,C*14:02 |
| | | HALVRGLLAQVPSLAEGRP WRAALRVLSALALEHARD VVCALLPRSLPADRVAAEL WRSLSRNQRVNGQVLVQL LWALKGASGPEPQALAAT RALGEMLAVSGCVGATRG FYPHLLLALVTQLHKLARSP CSPDMPKIWVLSHRGPPH SHASCAVEALKALLTGDGG RMVVTCMEQAGGWRRLV GAHTHLEGVLLLASAMVA HADHHLRGLFADLLPRLRS ADDPQRLTAMAFFTGLLQ SRPTARLLREEVILERLLTW QGDPEPTVRWLGLLGLGH LALNRRKVRHVSTLLPALLG ALGEGDARLVGAALGALR RLLLRPRAPVRLLSAELGPR LPPLLDDTRDSIRASAVGLL GTLVRRGRGGLRLGLRGPL RKLVLQSLVPLLLRLHDPSR DAAESSEWTLARCDHAFC WGLLEELVTVAHYDSPEAL SHLCCRLVQRYPGHVPNFL SQTQGYLRSPQDPLRRAA AVLIGFLVHHASPGCVNQD LLDSLFQDLGRLQSDPKPA VAAAAHVSAQQVA | |
| SEQ ID NO: 1936 | ENSG00000205277.5 | MLVIWILTLALRLCASVTTV TPGSTVNTSIGGNTTSASTP SSSDPFTTFSDYGVSVTFIT GSTATKHFLDSSTNSGHSE ESTVSHSGPGATGTTLFPS HSATSVFVGEPKTSPITSAS METTALPGSTTTAGLSEKS TTFYSSPRSPDRTLSPARTT SSGVSEKSTTSHSRPGPTHT IAFPDSTTMPGVSQESTAS HSIPGSTDTTLSPGTTTPSSL GPESTTFHSSPGYTKTTRLP DNTTTSGLLEASTPVHSST GSPHTTLSPSSSTTHEGEPT TFQSWPSSKDTSPAPSGTT SAFVKLSTTYHSSPSSTPTT HFSASSTTLGHSEESTPVHS SPVATATTPPPARSATSGH VEESTAYHRSPGSTQTMHF PESSTTSGHSEESATFHGST THTKSSTPSTTAALAHTSYH | A*02:03,A*11:01,A*11:0 2,A*24:02,A*24:10,A*33: 03,B*15:01,B*39:01,B*4 0:01,B*55:02,B*58:01,C* 03:02,C*03:04,C*03:67,C *07:02,C*12:02,C*14:02, C*15:02 |
| | | SSLGSTETTHFRDSSTISGRS EESKASHSSPDAMATTVLP AGSTPSVLVGDSTPSPISSG SMETTALPGSTTKPGLSEKS TTFYSSPRSPDTTHLPASM TSSGVSEESTTSHSRPGSTH TTAFPGSTTMPGLSQESTA SHSSPGPTDTTLSPGSTTAS SLGPEYTTFHSRPGSTETTL LPDNTTASGLLEASMPVHS STRSPHTTLSPAGSTTRQG ESTTFHSWPSSKDTRPAPP TTTSAFVEPSTTSHGSPSSIP TTHISARSTTSGLVEESTTY HSSPGSTQTMHFPESDTTS GRGEESTTSHSSTTHTISSA PSTTSALVEEPTSYHSSPGS TATTHFPDSSTTSGRSEEST ASHSSQDATGTIVLPARSTT SVLLGESTTSPISSGSMETT ALPGSTTTPGLSERSTTFHS SPRSPATTLSPASTTSSGVS EESTTSRSRPGSTHTTAFPD STTTPGLSRHSTTSHSSPGS TDTTLLPASTTTSGPSQEST TSHSSSGSTDTALSPGSTTA LSFGQESTTFHSNPGSTHT TLFPDSTTSSGIVEASTRVH SSTGSPRTTLSPASSTSPGL QGESTAFQTHPASTHTTPS PPSTATAPVEESTTYHRSP GSTPTTHFPASSTTSGHSEK STIFHSSPDASGTTPSSAHS TTSGRGESTTSRISPGSTEIT TLPGSTTTPGLSEASTTFYSS PRSPTTTLSPASMTSLGVG EESTTSRSQPGSTHSTVSPA STTTPGLSEESTTVYSSSRG STETTVFPHSTTTSVHGEEP TTFHSRPASTHTTLFTEDST TSGLTEESTAFPGSPASTQT GLPATLTTADLGEESTTFPS SSGSTGTKLSPARSTTSGLV GESTPSRLSPSSTETTTLPGS PTTPSLSEKSTTFYTSPRSPD ATLSPATTTSSGVSEESSTS HSQPGSTHTTAFPDSTTTS DLSQEPTTSHSSQGSTEATL SPGSTTASSLGQQSTTFHSS | |
| | | PGDTETTLLPDDTITSGLVE ASTPTHSSTGSLHTTLTPAS STSAGLQEESTTFQSWPSS SDTTPSPPGTTAAPVEVST TYHSRPSSTPTTHFSASSTT LGRSEESTTVHSSPGATGT ALFPTRSATSVLVGEPTTSP ISSGSTETTALPGSTTTAGLS EKSTTFYSSPRSPDTTLSPAS TTSSGVSEESTTSHSRPGST HTTAFPGSTTMPGVSQEST ASHSSPGSTDTTLSPGSTTA SSLGPESTTFHSSPGSTETT LLPDNTTASGLLEASTPVHS STGSPHTTLSPAGSTTRQG ESTTFQSWPSSKDTMPAP PTTTSAFVELSTTSHGSPSS TPTTHFSASSTTLGRSEEST TVHSSPVATATTPSPARSTT SGLVEESTAYHSSPGSTQT MHFPESSTASGRSEESRTS HSSTTHTISSPPSTTSALVEE PTSYHSSPGSTATTHFPDSS TTSGRSEESTASHSSQDAT GTIVLPARSTTSVLLGESTTS PISSGSMETTALPGSTTTPG LSEKSTTFHSSPRSPATTLSP ASTTSSGVSEESTTSHSRPG STHTTAFPDSTTTPGLSRHS TTSHSSPGSTDTTLLPASTT TSGPSQESTTSHSSPGSTDT ALSPGSTTALSFGQESTTFH SSPGSTHTTLFPDSTTSSGI VEASTRVHSSTGSPRTTLSP ASSTSPGLQGESTAFQTHP ASTHTTPSPPSTATAPVEES TTYHRSPGSTPTTHFPASST TSGHSEKSTIFHSSPDASGT TPSSAHSTTSGRGESTTSRI SPGSTEITTLPGSTTTPGLSE ASTTFYSSPRSPTTTLSPAS MTSLGVGEESTTSRSQPGS THSTVSPASTTTPGLSEEST TVYSSSPGSTETTVFPRTPT TSVRGEEPTTFHSRPASTH TTLFTEDSTTSGLTEESTAFP GSPASTQTGLPATLTTADL GEESTTFPSSSGSTGTTLSP ARSTTSGLVGESTPSRLSPS | |
| | | STETTTLPGSPTTPSLSEKST TFYTSPRSPDATLSPATTTS SGVSEESSTSHSQPGSTHT TAFPDSTTTPGLSRHSTTSH SSPGSTDTTLLPASTTTSGP SQESTTSHSSPGSTDTALSP GSTTALSFGQESTTFHSSPG STHTTLFPDSTTSSGIVEAST RVHSSTGSPRTTLSPASSTS PGLQGESTTFQTHPASTHT TPSPPSTATAPVEESTTYHR SPGSTPTTHFPASSTTSGHS EKSTIFHSSPDASGTTPSSA HSTTSGRGESTTSRISPGST EITTLPGSTTTPGLSEASTTF YSSPRSPTTTLSPASMTSLG VGEESTTSRSQPGSTHSTV SPASTTTPGLSEESTTVYSSS PGSTETTVFPRSTTTSVRGE EPTTFHSRPASTHTTLFTED STTSGLTEESTAFPGSPAST QTGLPATLTTADLGEESTTF PSSSGSTGTTLSPARSTTSG LVGESTPSRLSPSSTETTTLP GSPTTPSLSEKSTTFYTSPRS PDATLSPATTTSSGVSEESS TSHSQPGSTHTTAFPDSTT TSGLSQEPTASHSSQGSTE ATLSPGSTTASSLGQQSTTF HSSPGDTETTLLPDDTITSG LVEASTPTHSSTGSLHTTLT PASSTSAGLQEESTTFQSW PSSSDTTPSPPGTTAAPVE VSTTYHSRPSSTPTTHFSAS STTLGRSEESTTVHSSPGAT GTALFPTRSATSVLVGEPTT SPISSGSTETTALPGSTTTA GLSEKSTTFYSSPRSPDTTLS PASTTSSGVSEESTTSHSRP GSTHTTAFPGSTTMPGVS QESTASHSSPGSTDTTLSP GSTTASSLGPESTTFHSGPG STETTLLPDNTTASGLLEAS TPVHSSTGSPHTTLSPAGST TRQGESTTFQSWPNSKDT TPAPPTTTSAFVELSTTSHG SPSSTPTTHFSASSTTLGRS EESTTVHSSPVATATTPSPA RSTTSGLVEESTTYHSSPGS | |
| | | TQTMHFPESDTTSGRGEES TTSHSSTTHTISSAPSTTSAL VEEPTSYHSSPGSTATTHFP DSSTTSGRSEESTASHSSQ DATGTIVLPARSTTSVLLGE STTSPISSGSMETTALPGST TTPGLSEKSTTFHSSPRSPA TTLSPASTTSSGVSEESTTS HSRPGSTHTTAFPDSTTTP GLSRHSTTSHSSPGSTDTTL LPASTTTSGSSQESTTSHSS SGSTDTALSPGSTTALSFG QESTTFHSSPGSTHTTLFPD STTSSGIVEASTRVHSSTGS PRTTLSPASSTSPGLQGEST AFQTHPASTHTTPSPPSTA TAPVEESTTYHRSPGSTPTT HFPASSTTSGHSEKSTIFHS SPDASGTTPSSAHSTTSGR GESTTSRISPGSTEITTLPGS TTTPGLSEASTTFYSSPRSP TTTLSPASMTSLGVGEESTT SRSQPGSTHSTVSPASTTTP GLSEESTTVYSSSPGSTETT VFPRSTTTSVRREEPTTFHS RPASTHTTLFTEDSTTSGLT EESTAFPGSPASTQTGLPA TLTTADLGEESTTFPSSSGS TGTKLSPARSTTSGLVGEST PSRLSPSSTETTTLPGSPTTP SLSEKSTTFYTSPRSPDATLS PATTTSSGVSEESSTSHSQP GSTHTTAFPDSTTTSGLSQ EPTTSHSSQGSTEATLSPGS TTASSLGQQSTTFHSSPGD TETTLLPDDTITSGLVEASTP THSSTGSLHTTLTPASSTST GLQEESTTFQSWPSSSDTT PSPPSTTAVPVEVSTTYHSR PSSTPTTHFSASSTTLGRSE ESTTVHSSPGATGTALFPTR SATSVLVGEPTTSPISSGSTE TTALPGSTTTAGLSEKSTTF YSSPRSPDTTLSPASTTSSG VSEESTTSHSRPGSMHTTA FPSSTTMPGVSQESTASHS SPGSTDTTLSPGSTTASSLG PESTTFHSSPGSTETTLLPD NTTASGLLEASTPVHSSTGS | |
| | | PHTTLSPAGSTTRQGESTT FQSWPNSKDTTPAPPTTTS AFVELSTTSHGSPSSTPTTH FSASSTTLGRSEESTTVHSS PVATATTPSPARSTTSGLVE ESTTYHSSPGSTQTMHFPE SNTTSGRGEESTTSHSSTTH TISSAPSTTSALVEEPTSYHS SPGSTATTHFPDSSTTSGRS EESTASHSSQDATGTIVLPA RSTTSVLLGESTTSPISSGS METTALPGSTTTPGLSEKST TFHSSPSSTPTTHFSASSTTL GRSEESTTVHSSPVATATTP SPARSTTSGLVEESTAYHSS PGSTQTMHFPESSTASGRS EESRTSHSSTTHTISSPPSTT SALVEEPTSYHSSPGSIATT HFPESSTTSGRSEESTASHS SPDTNGITPLPAHFTTSGRI AESTTFYISPGSMETTLAST ATTPGLSAKSTILYSSSRSPD QTLSPASMTSSSISGEPTSL YSQAESTHTTAFPASTTTSG LSQESTTFHSKPGSTETTLS PGSITTSSFAQEFTTPHSQP GSALSTVSPASTTVPGLSEE STTFYSSPGSTETTAFSHSN TMSIHSQQSTPFPDSPGFT HTVLPATLTTTDIGQESTAF HSSSDATGTTPLPARSTAS DLVGEPTTFYISPSPTYTTLF PASSSTSGLTEESTTFHTSPS FTSTIVSTESLETLAPGLCQE GQIWNGKQCVCPQGYVG YQCLSPLESFPVETPEKLNA TLGMTVKVTYRNFTEKMN DASSQEYQNFSTLFKNRM DVVLKGDNLPQYRGVNIR RLLNGSIVVKNDVILEADYT LEYEELFENLAEIVKAKIMN ETRTTLLDPDSCRKAILCYSE EDTFVDSSVTPGFDFQEQC TQKAAEGYTQFYYVDVLD GKLACVNKCTKGTKSQMN CNLGTCQLQRSGPRCLCPN TNTHWYWGETCEFNIAKS LVYGIVGAVMAVLLLALIILI ILFSLSQRKRHREQYDVPQ | |
| | | EWRKEGTPGIFQKTAIWE DQNLRESRFGLENAYNNF RPTLETVDSGTELHIQRPE MVASTV | |
| SEQ ID NO: 1937 | ENSG00000205744.5 | MESRAEGGSPAVFDWFFE AACPASLQEDPPILRQFPP DFRDQEAMQMVPKFCFP FDVEREPPSPAVQHFTFAL TDLAGNRRFGFCRLRAGT QSCLCILSHLPWFEVFYKLL NTVGDLLAQDQVTEAEELL QNLFQQSLSGPQASVGLEL GSGVTVSSGQGIPPPTRGN SKPLSCFVAPDSGRLPSIPE NRNLTELVVAVTDENIVGL FAALLAERRVLLTASKLSTLT SCVHASCALLYPMRWEHV LIPTLPPHLLDYCCAPMPYL IGVHASLAERVREKALEDV VVLNVDANTLETTFNDVQ ALPPDVVSLLRLRLRKVALA PGEGVSRLFLKAQALLFGG YRDALVCSPGQPVTFSEEV FLAQKPGAPLQAFHRRAV HLQLFKQFIEARLEKLNKGE GFSDQFEQEITGCGASSGA LRSYQLWADNLKKGGGAL LHSVKAKTQPAVKNMYRS AKSGLKGVQSLLMYKDGD SVLQRGGSLRAPALPSRSD RLQQRLPITQHFGKNRPLR PSRRRQLEEGTSEPPGAGT PPLSPEDEGCPWAEEALDS SFLGSGEELDLLSEILDSLSM GAKSAGSLRPSQSLDCCHR GDLDSCFSLPNIPRWQPD DKKLPEPEPQPLSLPSLQN ASSLDATSSSKDSRSQLIPS ESDQEVTSPSQSSTASADP SIWGDPKPSPLTEPLILHLT PSHKAAEDSTAQENPTPW LSTAPTEPSPPESPQILAPTK PNFDIAWTSQPLDPSSDPS SLEDPRARPPKALLAERAHL QPREEPGALNSPATPTSNC QKSQPSSRPRVADLKKCFE G | A*02:03,A*11:01,A*11:0 2,A*24:10,A*33:03,B*15: 01,B*39:01,B*40:01,B*5 5:02,B*58:01,C*03:02,C* 03:04,C*14:02 |
| SEQ ID NO: 1938 | ENSG00000213420.3 | MSALRPLLLLLLPLCPGPGP GPGSEAKVTRSCAETRQVL | A*02:03,A*11:01,A*11:0 2,A*24:02,A*24:10,A*33:03,B*15:01,B*15:27,B*3 8:02,B*39:01,B*40:01,B* 58:01,C*03:02,C*03:04,C *12:02,C*14:02,C*15:02 |
| | | GARGYSLNLIPPALISGEHL RVCPQEYTCCSSETEQRLIR ETEATFRGLVEDSGSFLVHT LAARHRKFDEFFLEMLSVA QHSLTQLFSHSYGRLYAQH ALIFNGLFSRLRDFYGESGE GLDDTLADFWAQLLERVF PLLHPQYSFPPDYLLCLSRL ASSTDGSLQPFGDSPRRLR LQITRTLVAARAFVQGLET GRNVVSEALKVPVSEGCSQ ALMRLIGCPLCRGVPSLMP CQGFCLNVVRGCLSSRGLE PDWGNYLDGLLILADKLQ GPFSFELTAESIGVKISEGL MYLQENSAKVSAQVFQEC GPPDPVPARNRRAPPPRE EAGRLWSMVTEEERPTTA AGTNLHRLVWELRERLAR MRGFWARLSLTVCGDSR MAADASLEAAPCWTGAG RGRYLPPVVGGSPAEQVN NPELKVDASGPDVPTRRRR LQLRAATARMKTAALGHD LDGQDADEDASGSGGGQ QYADDWMAGAVAPPARP PRPPYPPRRDGSGGKGGG GSARYNQGRSRSGGASIGF HTQTILILSLSALALLGPR | |
| SEQ ID NO: 1939 | ENSG00000225485.3 | MNGVAFCLVGIPPRPEPRP PQLPLGPRDGCSPRRPFP WQGPRTLLLYKSPQDGFG FTLRHFIVYPPESAVHCSLK EEENGGRGGGPSPRYRLEP MDTIFVKNVKEDGPAHRA GLRTGDRLVKVNGESVIGK TYSQVIALIQNSDDTLELSI MPKDEDILQLAYSQDAYLK GNEPYSGEARSIPEPPPICY PRKTYAPPARASTRATMVP EPTSALPSDPRSPAAWSDP GLRVPPAARAHLDNSSLG MSQPRPSPGAFPHLSSEPR TPRAFPEPGSRVPPSRLEC QQALSHWLSNQVPRRAG ERRCPAMAPRARSASQDR LEEVAAPRPWPCSTSQDAL SQLGQEGWHRARSDDYLS RATRSAEALGPGALVSPRF | A*02:03,A*11:01,A*11:0 2,A*24:02,A*24:07,A*24: 10,B*15:01,B*39:01,B*4 0:01,B*55:02,B*58:01,C* 03:02,C*03:04,C*03:67,C *12:02,C*14:02,C*15:02 |
| | | ERCGWASQRSSARTPACP TRDLPGPQAPPPSGLQGL DDLGYIGYRSYSPSFQRRT GLLHALSFRDSPFGGLPTF NLAQSPASFPPEASEPPRV VRPEPSTRALEPPAEDRGD EVVLRQKPPTGRKVQLTPA RQMNLGFGDESPEPEASG RGERLGRKVAPLATTEDSL ASIPFIDEPTSPSIDLQAKHV PASAVVSSAMNSAPVLGT SPSSPTFTFTLGRHYSQDCS SIKAGRRSSYLLAITTERSKS CDDGLNTFRDEGRVLRRLP NRIPSLRMLRSFFTDGSLDS WGTSEDADAPSKRHSTSD LSDATFSDIRREGWLYYKQI LTKKGKKAGSGLRQWKRV YAALRARSLSLSKERREPGP AAAGAAAAGAGEDEAAPV CIG | |
| SEQ ID NO: 1940 | ENSG00000243449.2 | MFRAALEDSVEKKSSLKET ETTSKGTSKYDRERETEMK TVMGMKMHFWVRTPAS GRGRGGSDHARSRAAPLP LLA | A*02:03,A*24:10,A*33:0 3,B*27:04,B*38:02,B*39: 01,B*40:01,C*01:02,C*0 3:02,C*03:04,C*03:67,C* 04:01,C*07:02,C*14:02,C*15:02 |
| SEQ ID NO: 1941 | ENSG00000261787.1 | MDRGRPAGSPLSASAEPA PLAAAIRDSRPGRTGPGPA GPGGGSRSGSGRPAAANA ARERSRVQTLRHAFLELQR TLPSVPPDTKLSKLDVLLLA TTYIAHLTRSLQDDAEPA DAGLGALRGDGYLHPVKK WPMRSRLYIGATGQFLKH SVSGEKTNHDNTPTDSQP | A*02:03,A*24:02,A*24:1 0,A*33:03,B*40:01,C*03: 02,C*03:04,C*12:02,C*14:02 |

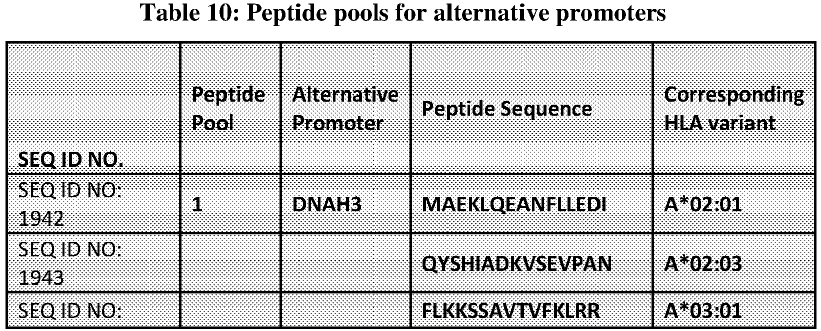

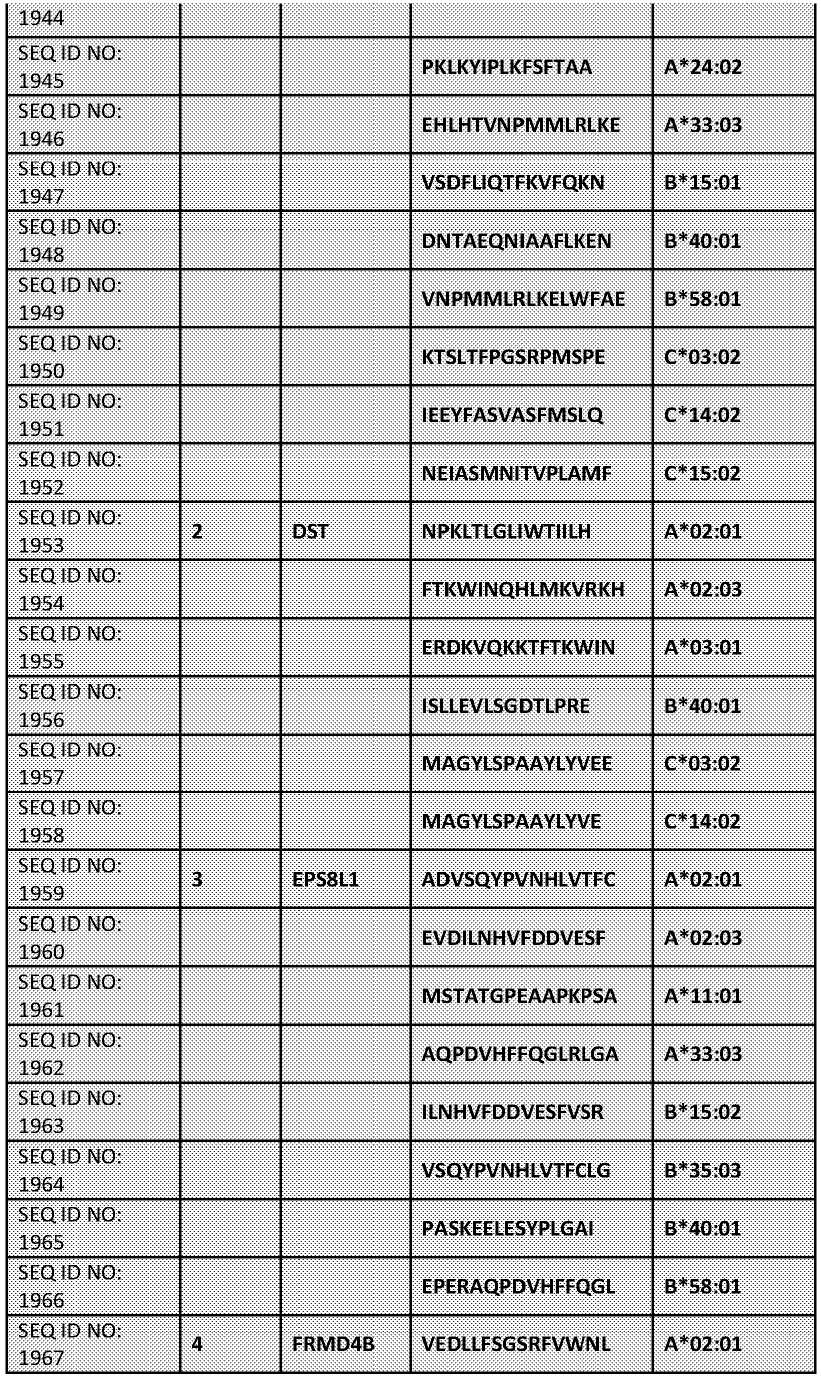

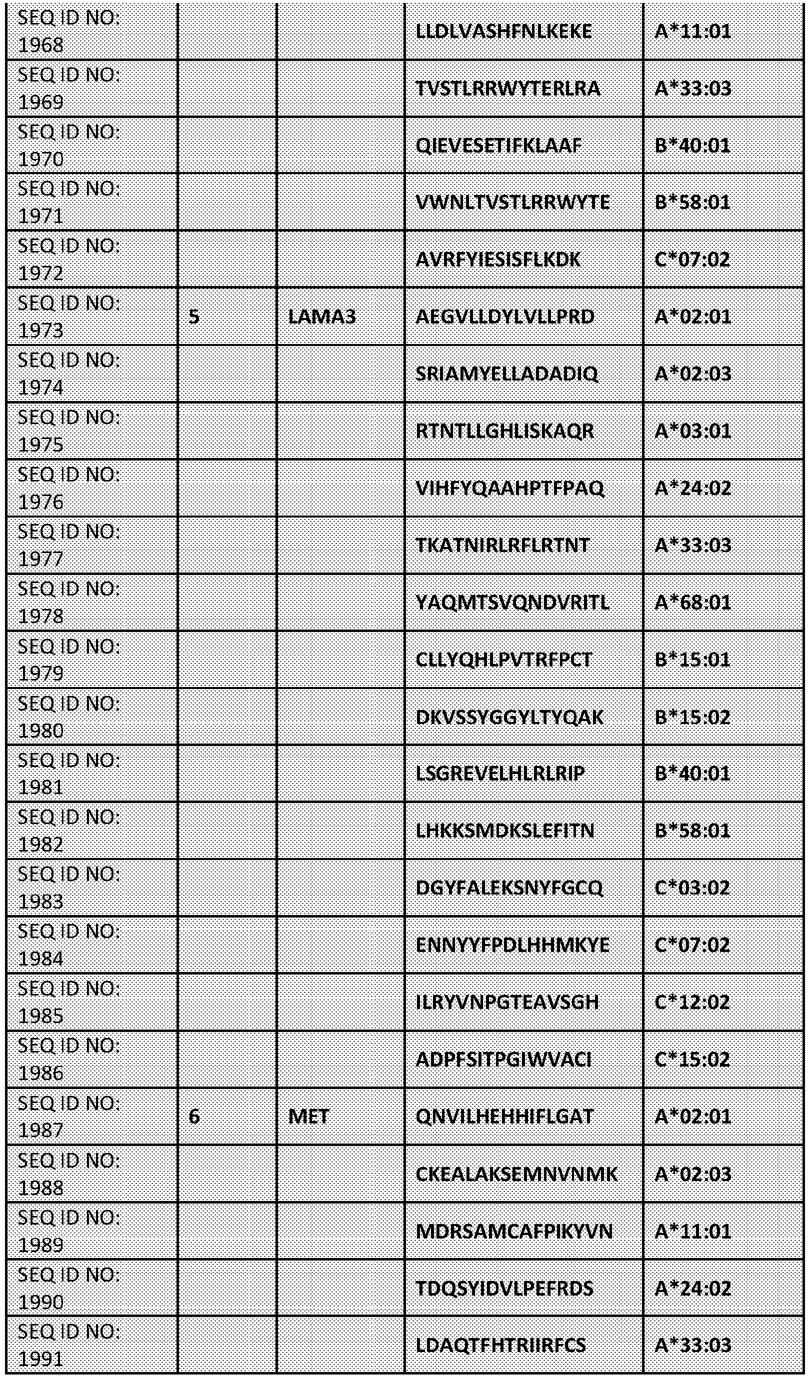

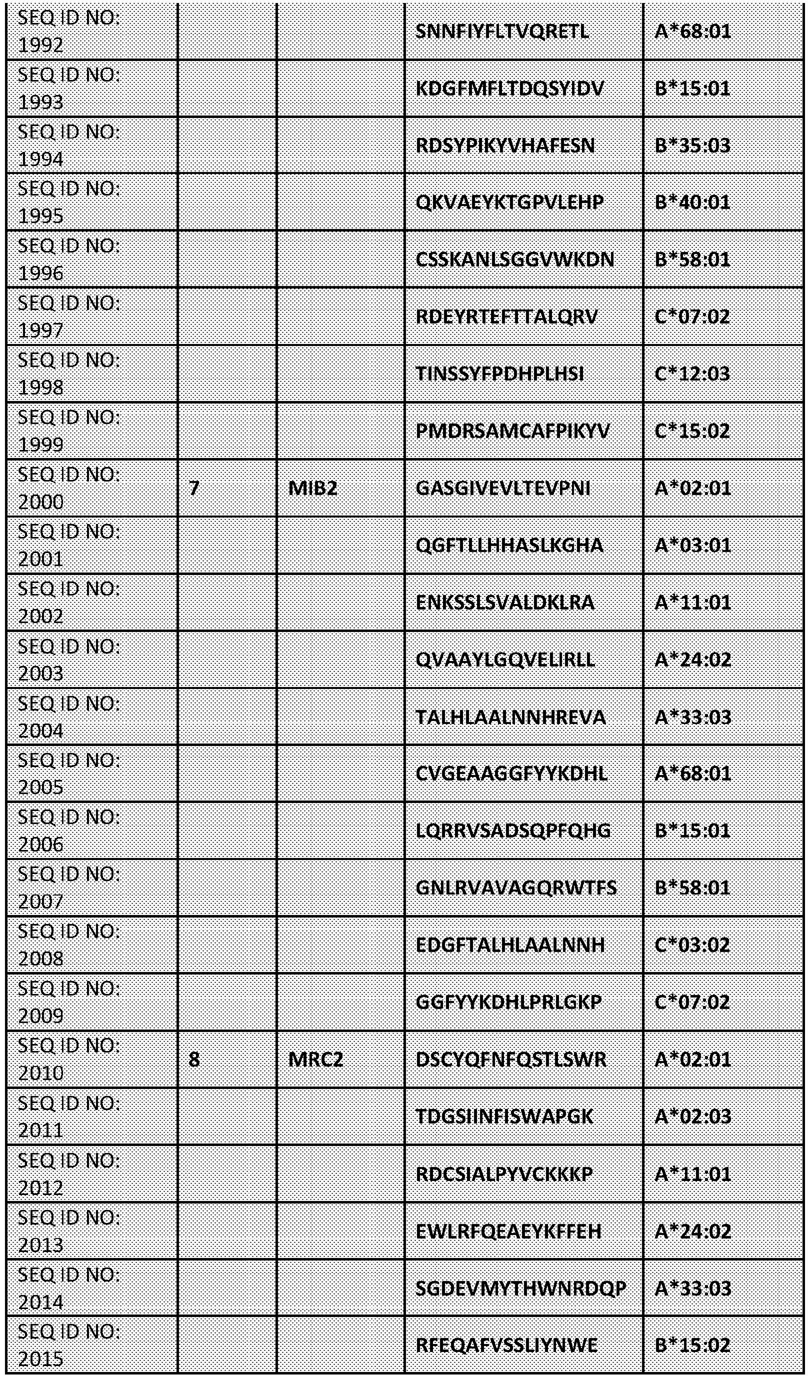

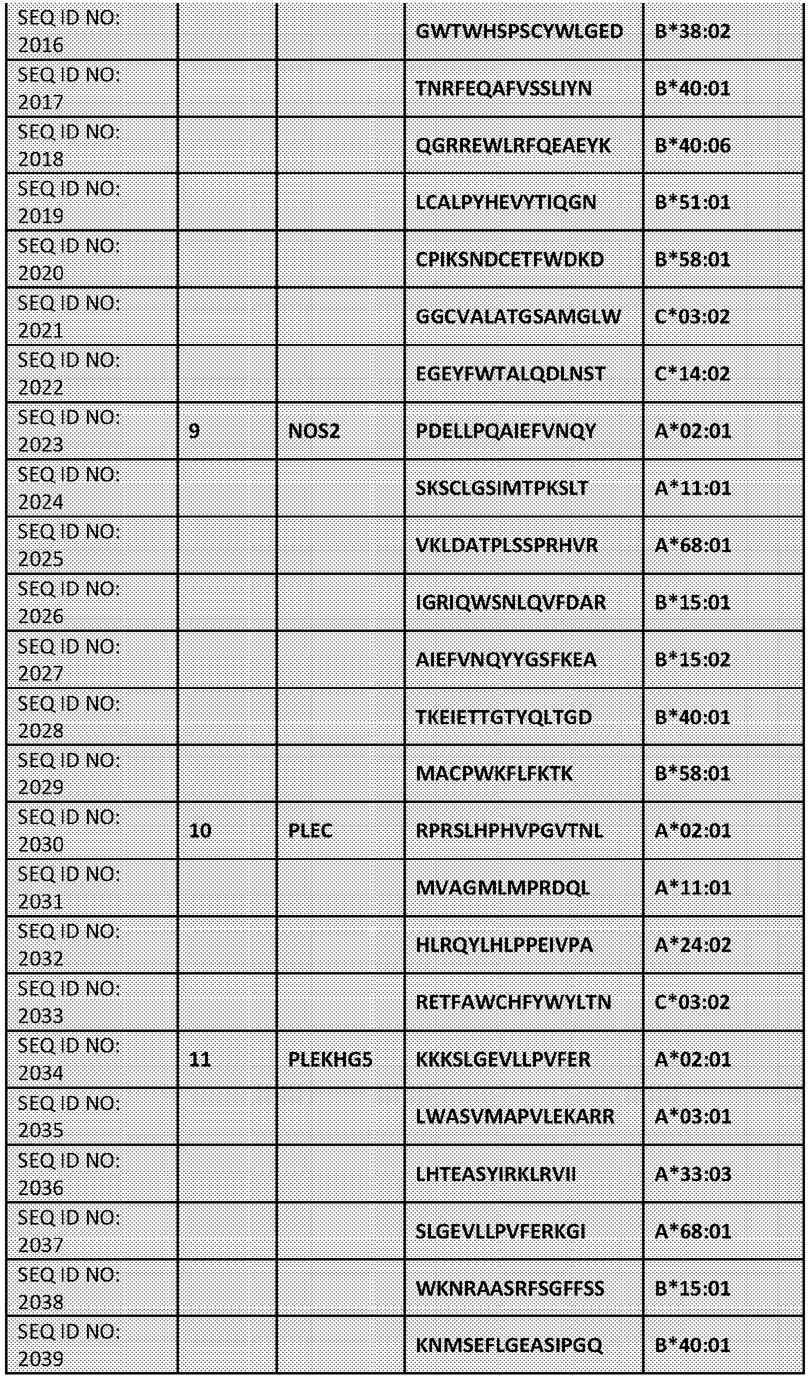

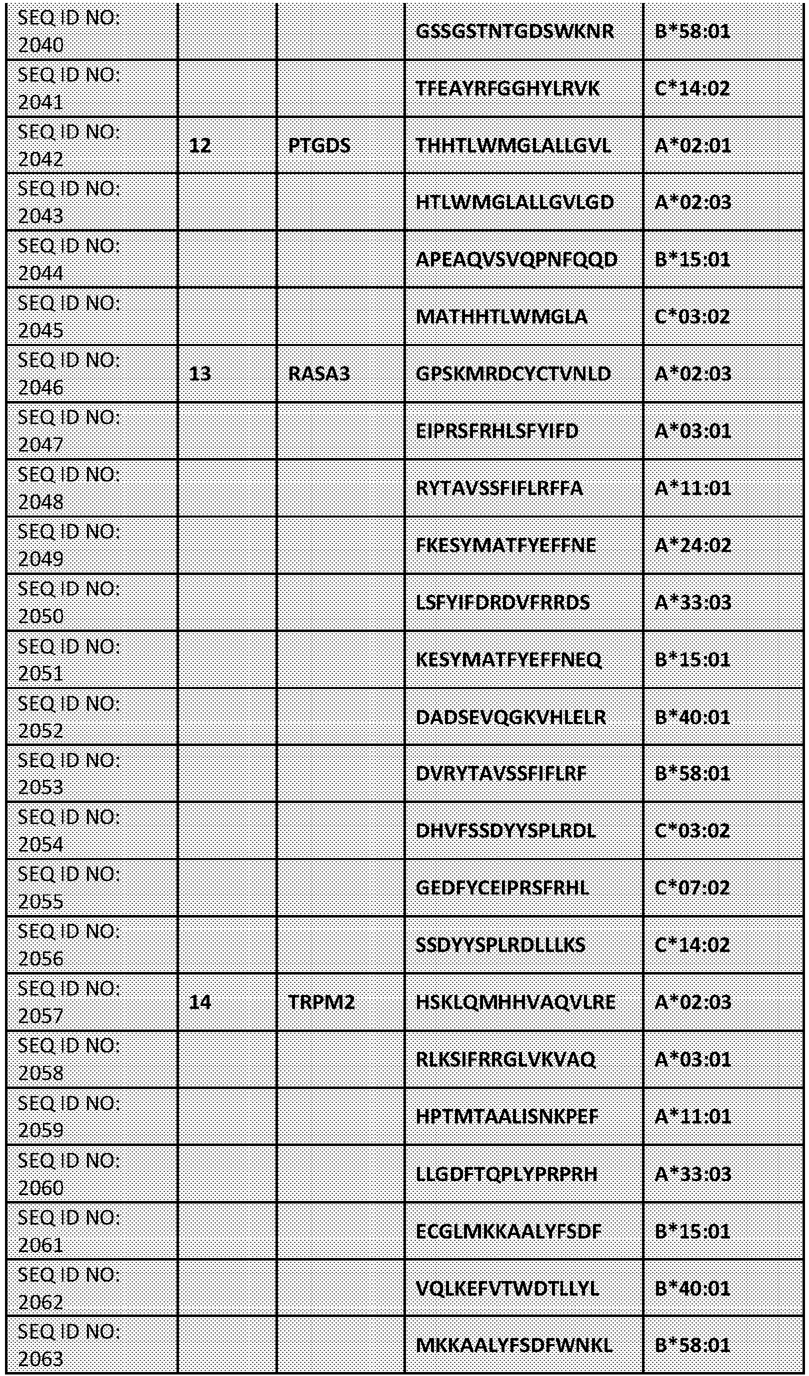

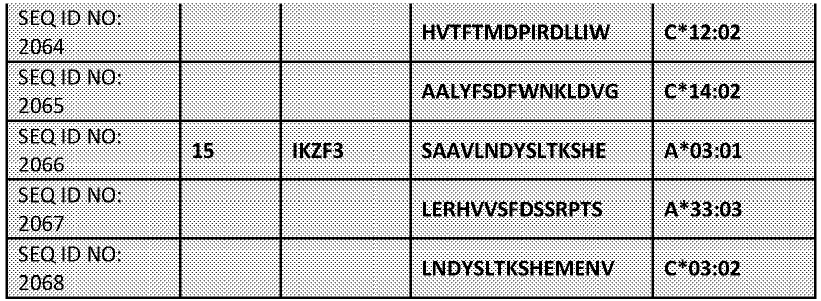

To explore if somatic promoters might contribute to reducing tumor antigen burden and immunoreactivity *in vivo,* we proceeded to examine correlations between promoter alterations and intra-tumor T-cell activity in various primary GC cohorts. First, to detect promoter alterations in a cohort of 95 GC-normal pairs (SG cohort), we generated a customized Nanostring panel targeting the top 95 recurrent GC somatic promoters, measuring transcripts associated with either the canonical promoter or the alternative promoter. There was a significant correlation between the Nanostring data and RNA-seq (Figure 16, r=0.65, P<0.001), with -35% of transcripts driven by alternate promoters upregulated in more than half of the GCs (Fig. 4D). Second, to examine markers of T-cell activity in these same GC samples, we analyzed previously published microarray data to measure *CD8A* (a measure of CD8+ tumor infiltrating lymphocytes), and granzyme A *(GZMA)* and perforin (*PRF1*)*,* which are both T-cell effectors and validated markers of T-cell cytolytic activity. We confirmed that these three genes *(CD8A, GZMA,* and *PRF1*) were not themselves associated with somatic promoters. Comparing the top and bottom quartiles, GCs with high somatic promoter usage exhibited significantly lower *GZMA* and *P7PF1* levels (P<0.001 and P=0.01, Wilcoxon Test) indicating lower T-cell cytolytic activity (Fig. 4E, top left), and also a trend towards lower *CD8A* levels (P=0.14, Wilcoxon one sided test). Using two different algorithms (ASCAT and ESTIMATE), we further confirmed that the decreased *GZMA* and *PRF1* levels are independent of tumor purity differences between GCs (Figure 16). Similar results were obtained upon splitting the GC samples based on median promoter usage score (*GZMA,* P<0.001 and *PRF1,* P=0.03). Patients with GCs exhibiting high somatic promoter usage (top 25%) also showed poor survival compared to patients with GCs with low somatic promoter usage (bottom 25%) (Fig. 4e top right, HR 2.55, P=0.02). Again, dividing patients by their median somatic promoter usage score also showed similar survival differences (Figure 11, HR=1.81, P=0.04).

To validate these findings, we then analyzed two other prominent GC cohorts - one from TCGA, and another from the Asian Cancer Research Group (ACRG). In the TCGA cohort, availability of RNA-seq data allowed us to infer somatic promoter usage directly from next-generation sequencing (NGS) data (Fig. 2c). Similar to the Singapore cohort, TCGA GCs with high somatic promoter usage (top 25%) exhibited decreased *CD8A* (P=0.002, Wilcoxon one sided test), *GZMA* (P= 0.001, Wilcoxon one sided test) and *PRF1* levels (P= 0.005, Wilcoxon one sided test, Fig. 4e bottom left) compared to GCs with low somatic promoter usage (bottom 25%) in a manner independent of tumor purity (Figure 16). Notably, as previous studies have suggested that somatic mutation burden may also correlate with intra-tumor T-cell cytolytic response, we further repeated the analysis after adjusting for the total number of missense mutations in each sample using a regression based approach. Even after correcting for somatic mutation burden, we still observed decreased *CD8A* (P=0.02, Wilcoxon one sided test), *GZMA* (P=0.01, Wilcoxon one sided test) and *PRF1* expression (P=0.03, Wilcoxon one sided test) in samples with high somatic promoter usage (top 25% against bottom 25%) (Figure 11).

We leveraged a third independent cohort of GC samples from ACRG. Using NanoString to target 89 canonical and alternative promoters along with various immune markers, we profiled 264 primary GC samples from the ACRG cohort. 40% of alternative promoter transcripts showed tumor specific expression in more than half of the samples (Figure 11). Once again, samples with high somatic promoter usage (top 25%) showed significantly lower expression of T-cell cytolytic activity markers including *CD8A* (P=0.035, Wilcoxon one sided test), *CD4A* (P=0.005, Wilcoxon one sided test), *GZMA* (P=0.001, Wilcoxon one sided test) and *PRF1* (P=0.025, Wilcoxon one sided test) (Fig. 4e, bottom right) (Figure 16). Similar results were obtained upon splitting the GC samples based on median promoter usage score (Table 11) Also, after adjusting for mutational burden (for cases where information is available), samples with high somatic promoter usage still showed decreased *CD8A* (P=0.167, Wilcoxon one sided test), *GZMA* (P=0.009, Wilcoxon one sided test), and *PRF1* (P=0.03, Wilcoxon one sided test) expression (Figure 11). Taken collectively, these results, observed across multiple GC cohorts and assessed using diverse technologies (microarray, RNA-seq, Nanostring) all support a significant association between somatic promoter usage and reduced tumor immunity levels. Importantly, the decreased levels of T-cell cytolytic activity associated with somatic promoter usage are likely independent of tumor purity and mutational load.

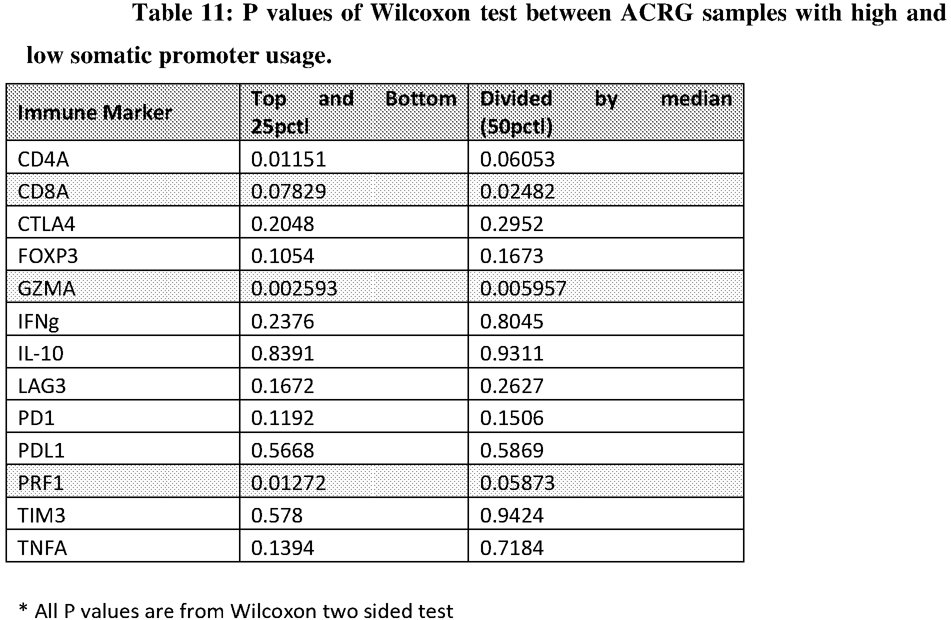

### Somatic Promoter Associated Peptides are Immunogenic in vitro

To functionally test the ability of N-terminal peptides depleted in GC to elicit immune responses, we conducted *in-vitro* assays using the high-throughput EPIMAX **(*****EPItope*** MAXimum) platform, which allows multi-epitope testing for both T cell proliferation and cytokine production. First, we identified N terminal peptides predicted to exhibit high HLA-binding affinities across a pool of healthy PBMC (peripheral blood mononuclear cell) donors. Second, selecting 15 alternative promoter-associated peptides for testing, we generated peptide pools for each peptide (Tables 9 and 10, Methods), which were then used to stimulate PBMCs from 9 healthy donors. T cell proliferation and cytokine production levels were measured and benchmarked against control peptides (Table 12). Across all 135 exposures (15 peptides across 9 donors), we observed strong cytokine responses for 79 peptide pools (58%; FC≥2 relative to Actin peptides) (Fig. 4g) inducing complex Th1, Th2 and Thl7 polarizations in a donor dependent fashion (Figure 17).

**Table 12: Cytokine Responses of N terminal Peptides**

| **Sample** | **Treatment** | **Analyte concentration (pg/ml)** | | | | | | | | | | | | | **Total analytes (pg /ml)** | **Fold change of total cytokine response (normalized against Actin control)** |
|---|---|---|---|---|---|---|---|---|---|---|---|---|---|---|---|---|
| | | **GM-CSF** | **IFNg** | **IL-2** | **IL-3** | **IL-4** | **IL-7** | **IL-9** | **IL-10** | **IL-13** | **IL-15** | **IL-17A** | **sCD40L** | **TNFa** | | |
| Donor 1 | DNAH3 | 99.39 | 228.45 | 89 | 6.35 | 2.12 | 0.085 | 7.32 | 24.91 | 228.24 | 0. 925 | 1.88 | 4.47 | 264.89 | 958.03 | 2.89 |
| Donor 1 | DST | 114.18 | 149.87 | 58.02 | 11.41 | 0.03 | 0.085 | 14.11 | 57.29 | 311.22 | 0. 925 | 1.58 | 8.97 | 251.98 | 979.67 | 2.96 |
| Donor 1 | EPS8L1 | 153.07 | 351.34 | 100.97 | 11.8 | 0.03 | 0.085 | 28.88 | 33.71 | 431.94 | 0. 925 | 0.02 | 6.17 | 434.22 | 1553.16 | 4.69 |
| Donor 1 | FRMD4B | 55.53 | 121.17 | 76.42 | 10.54 | 0.03 | 1.43 | 16.77 | 36.13 | 198.37 | 0. 925 | 0.93 | 3.76 | 186.12 | 708.13 | 2.14 |
| Donor 1 | LAMA3 | 67.29 | 152.66 | 99.6 | 4.83 | 1.72 | 0.085 | 9.11 | 25.85 | 264.85 | 0. 925 | 0.02 | 2.8 | 506.25 | 1135.99 | 3.43 |
| Donor 1 | MET | 54.4 | 93.08 | 96.36 | 6.27 | 0.03 | 0.085 | 5.52 | 25.85 | 179.02 | 0. 925 | 0.02 | 3.76 | 606.67 | 1071.99 | 3.23 |
| Donor 1 | MIB2 | 97.14 | 201.48 | 94.37 | 5.92 | 0.03 | 0.085 | 18.62 | 27 | 381.6 | 0. 925 | 0.67 | 1.81 | 684.34 | 1513.99 | 4.57 |
| Donor 1 | MRC2 | 52.57 | 63.61 | 53.15 | 5.58 | 0.03 | 0.085 | 3.32 | 37.5 | 184.11 | 0. 925 | 0.76 | 1.81 | 290.69 | 694.14 | 2.09 |
| Donor 1 | NOS2 | 31.72 | 130.64 | 26.25 | 3.51 | 0.03 | 0.085 | 5.04 | 28.47 | 133.76 | 0. 925 | 0.02 | 1.62 | 154.92 | 516.99 | 1.56 |
| Donor 1 | PLEC | 107.71 | 393.6 | 96.29 | 14.5 | 10.68 | 0.085 | 27.93 | 59.1 | 413.41 | 0. 925 | 0.02 | 7.78 | 337.55 | 1469.58 | 4.43 |
| Donor 1 | PLEKHG5 | 74.89 | 128.23 | 96.23 | 9.37 | 3.33 | 0.085 | 9.16 | 40.97 | 207.45 | 0. 925 | 4.22 | 3.64 | 236.32 | 814.82 | 2.46 |
| Donor 1 | PTGDS | 29.12 | 223.36 | 63.06 | 2.73 | 0.03 | 0.085 | 10.02 | 48.05 | 254.29 | 0. 925 | 0.02 | 0.01 | 395.74 | 1027.44 | 3.10 |
| Donor 1 | RASA3 | 33.95 | 50.06 | 58.28 | 3.84 | 0.03 | 0.085 | 8.6 | 39.39 | 196.78 | 0. 925 | 0.02 | 0.01 | 157.88 | 549.85 | 1.66 |
| Donor 1 | TRPM2 | 121.32 | 323.62 | 90.23 | 6.24 | 2.53 | 0.085 | 18.26 | 51.65 | 368.92 | 0. 925 | 0.02 | 7.61 | 428.91 | 1420.32 | 4.29 |
| Donor 1 | IKZF3 | 9.53 | 59.94 | 23.36 | 0.94 | 0.03 | 0.085 | 1.22 | 42.98 | 76.06 | 0. 925 | 0.02 | 0.01 | 48.83 | 263.93 | 0.80 |
| Donor 1 | Actin | 19.75 | 147.18 | 34.21 | 1.46 | 0.03 | 0.085 | 1.22 | 10.1 | 14.2 | 0. 925 | 0.02 | 0.78 | 101.44 | 331.40 | 1.00 |
| Donor 2 | DNAH3 | 279.27 | 1324.9 | 24 | 0.5 | 0.03 | 0.085 | 1.22 | 18.44 | 156.05 | 0.925 | 2.26 | 4.59 | 130.71 | 1942.98 | 28.04 |
| Donor 2 | DST | 773.57 | 6732.16 | 46.6 | 2 | 0.03 | 0.085 | 1.22 | 23.76 | 370.78 | 0.925 | 2.56 | 3.88 | 257.33 | 8214.90 | 118.57 |
| Donor 2 | EPS8L1 | 427.99 | 1030.19 | 85.97 | 3.33 | 4.33 | 0.085 | 18.4 | 21.15 | 386.22 | 0.925 | 0.76 | 4.3 | 167.42 | 2151.07 | 31.05 |
| Donor 2 | FRMD4B | 390.31 | 1070.19 | 94.99 | 3.93 | 10.28 | 1.27 | 1.22 | 19.9 | 415.04 | 0.925 | 0.02 | 5.24 | 159.4 | 2172.72 | 31.36 |
| Donor 2 | LAMA3 | 358.14 | 643.22 | 67.18 | 2.34 | 0.03 | 0.085 | 1.22 | 11.66 | 362.67 | 0.925 | 0.02 | 0.17 | 109.58 | 1557.24 | 22.48 |
| Donor 2 | MET | 302.2 | 256.37 | 64.56 | 1.53 | 0.91 | 0.085 | 1.22 | 14.16 | 312.32 | 0.925 | 2.39 | 4.24 | 84.79 | 1045.70 | 15.09 |
| Donor 2 | MIB2 | 173.84 | 141.37 | 17.97 | 0.73 | 0.03 | 0.085 | 1.22 | 13.23 | 153.31 | 0.925 | 0.02 | 0.65 | 61.99 | 565.37 | 8.16 |
| Donor 2 | MRC2 | 1401.1 | 5545.58 | 205.47 | 5.98 | 6.32 | 0.085 | 13.83 | 14.06 | 889.87 | 0.925 | 6.68 | 4.59 | 531.62 | 8626.11 | 124.50 |
| Donor 2 | NOS2 | 342.89 | 462.07 | 83.01 | 2.88 | 10.88 | 2.29 | 15.36 | 21.57 | 288.7 | 0.925 | 5.91 | 3.82 | 89.68 | 1329.99 | 19.20 |
| Donor 2 | PLEC | 280.02 | 357.65 | 74.41 | 2.44 | 0.03 | 0.085 | 19.79 | 24.07 | 343.1 | 0.925 | 5.46 | 2.49 | 83.91 | 1194.38 | 17.24 |
| Donor 2 | PLEKHG5 | 236.12 | 757.03 | 103.14 | 2.69 | 4.13 | 0.085 | 1.22 | 24.39 | 155.22 | 0.925 | 1.54 | 6.63 | 89.39 | 1382.51 | 19.95 |
| Donor 2 | PTGDS | 142.7 | 621.5 | 33.17 | 1.39 | 0.03 | 0.17 | 1.22 | 13.75 | 63.73 | 0.925 | 2.39 | 4.83 | 57.06 | 942.87 | 13.61 |
| Donor 2 | RASA3 | 630.2 | 2755.29 | 67.63 | 0.98 | 4.53 | 0.085 | 15.24 | 36.44 | 363.46 | 0.925 | 0.02 | 3.28 | 281.27 | 4159.35 | 60.03 |
| Donor 2 | TRPM2 | 495.45 | 1211.48 | 60.61 | 2.96 | 0.03 | 0.085 | 2.44 | 5.29 | 542.44 | 0.925 | 0.02 | 3.28 | 143.48 | 2468.49 | 35.63 |
| Donor 2 | IKZF3 | 427.38 | 1705.57 | 71.33 | 1.36 | 0.03 | 0.085 | 21.04 | 43.4 | 419.93 | 0.925 | 0.02 | 4.77 | 116.74 | 2812.58 | 40.59 |
| Donor 2 | Actin | 15.58 | 7.71 | 11.28 | 0.76 | 0.03 | 1.73 | 1.22 | 5.29 | 13.75 | 0.925 | 0.02 | 1.81 | 9.18 | 69.29 | 1.00 |
| Donor 3 | DNAH3 | 42.21 | 664.34 | 19.01 | 0.005 | 0.03 | 0.085 | 1.22 | 5.08 | 15.32 | 0.925 | 0.02 | 0.01 | 29.25 | 777.51 | 4.56 |
| Donor 3 | DST | 100.36 | 273.74 | 14.76 | 0.005 | 0.03 | 0.085 | 1.22 | 27 | 58.89 | 0.925 | 7.41 | 1.17 | 63.68 | 549.28 | 3.22 |
| Donor 3 | EPS8L1 | 208.07 | 530.49 | 41.94 | 1.07 | 3.73 | 0.085 | 1.22 | 13.12 | 107.94 | 0.925 | 0.85 | 0.01 | 50.21 | 959.66 | 5.63 |
| Donor 3 | FRMD4B | 143.55 | 211.78 | 47.51 | 0.73 | 0.03 | 0.085 | 1.22 | 17.71 | 91.8 | 0.925 | 0.02 | 1.11 | 53.79 | 570.26 | 3.35 |
| Donor 3 | LAMA3 | 100.19 | 509.46 | 23.21 | 1.08 | 0.03 | 0.085 | 1.22 | 36.97 | 34.67 | 0.925 | 1.19 | 0.01 | 50.95 | 759.99 | 4.46 |
| Donor 3 | MET | 143.98 | 322.33 | 34.04 | 1.99 | 0.03 | 0.085 | 1.22 | 12.39 | 29.84 | 0.925 | 2.64 | 0.01 | 54.62 | 604.10 | 3.55 |
| Donor 3 | MIB2 | 113.31 | 127.71 | 16.28 | 0.05 | 0.03 | 0.085 | 1.22 | 9.27 | 39.67 | 0.925 | 0.02 | 0.01 | 39.41 | 347.9 9 | 2.04 |
| Donor 3 | MRC2 | 150.52 | 323.25 | 48.19 | 0.96 | 0.03 | 0.085 | 1.22 | 11.66 | 54.63 | 0.925 | 0.58 | 0.09 | 74.36 | 666.50 | 3.91 |
| Donor 3 | NOS2 | 186.72 | 328.5 | 75.34 | 4.54 | 0.03 | 0.085 | 1.22 | 18.02 | 95.19 | 0.925 | 1.96 | 2.06 | 69.18 | 783.77 | 4.60 |
| Donor 3 | PLEC | 132.57 | 235.34 | 52.69 | 0.76 | 0.03 | 0.085 | 1.22 | 27.21 | 69.82 | 0.925 | 2.93 | 1.05 | 43.28 | 567.91 | 3.33 |
| Donor 3 | PLEKHG5 | 275.71 | 343.92 | 56.78 | 0.69 | 0.03 | 0.085 | 1.22 | 14.06 | 132.99 | 0.925 | 0.49 | 0.01 | 118.75 | 945.66 | 5.55 |
| Donor 3 | PTGDS | 185.73 | 186.82 | 57.3 | 0.005 | 0.28 | 0.085 | 1.22 | 18.44 | 127.35 | 0.925 | 0.02 | 0.01 | 90.73 | 668.92 | 3.93 |
| Donor 3 | RASA3 | 133.59 | 93.84 | 40.44 | 0.01 | 0.06 | 0.085 | 1.22 | 9.68 | 73.67 | 0.925 | 2.3 | 1.49 | 53.69 | 411.00 | 2.41 |
| Donor 3 | TRPM2 | 176.42 | 154.05 | 46.74 | 1.05 | 0.03 | 1.43 | 1.22 | 10.93 | 133.4 | 0.925 | 0.02 | 0.01 | 72 | 598.23 | 3.51 |
| Donor 3 | IKZF3 | 32.69 | 169.24 | 18.82 | 0.005 | 0.03 | 0.085 | 1.22 | 10.52 | 16.55 | 0.925 | 0.02 | 0.01 | 21.41 | 271.53 | 1.59 |
| Donor 3 | Actin | 56.66 | 60.86 | 13.4 | 0.56 | 4.53 | 0.085 | 1.22 | 2.56 | 5.96 | 0.925 | 2.89 | 0.01 | 20.69 | 170.35 | 1.00 |
| Donor 4 | DNAH3 | 0.66 | 0.005 | 2.21 | 0.005 | 0.03 | 0.085 | 1.22 | 0.41 | 0.58 | 0.925 | 0.02 | 0.01 | 2.38 | 8.54 | 1.24 |
| Donor 4 | DST | 1.83 | 1.05 | 1.06 | 0.005 | 0.03 | 0.085 | 1.22 | 3.61 | 2.32 | 0.925 | 0.02 | 0.01 | 19.23 | 31.40 | 4.55 |
| Donor 4 | EPS8L1 | 0.66 | 1.35 | 0.98 | 0.005 | 0.03 | 2.01 | 1.22 | 4.24 | 1.95 | 0.925 | 0.02 | 0.01 | 1.86 | 15.26 | 2.21 |
| Donor 4 | FRMD4B | 0.66 | 0.005 | 2.01 | 0.07 | 0.03 | 0.085 | 1.22 | 2.02 | 1.19 | 0.925 | 0.02 | 0.01 | 0.6 | 8.85 | 1.28 |
| Donor 4 | LAMA3 | 0.66 | 2.26 | 1.99 | 0.005 | 0.03 | 0.085 | 1.22 | 0.09 | 1.25 | 0.925 | 0.02 | 0.01 | 2.34 | 10.89 | 1.58 |
| Donor 4 | MET | 0.66 | 0.3 | 1.19 | 0.005 | 0.03 | 0.085 | 1.22 | 4.77 | 2.69 | 0.925 | 0.13 | 0.01 | 1.61 | 13.63 | 1.98 |
| Donor 4 | MIB2 | 0.66 | 0.005 | 1.6 | 0.005 | 0.03 | 0.085 | 1.22 | 6.55 | 0.03 | 0.925 | 0.02 | 0.01 | 2.12 | 13.26 | 1.92 |
| Donor 4 | MRC2 | 0.66 | 1.05 | 0.98 | 0.005 | 0.03 | 0.085 | 1.22 | 4.77 | 0.3 | 0.925 | 0.02 | 0.01 | 2.08 | 12.14 | 1.76 |
| Donor 4 | NOS2 | 0.66 | 2.49 | 1.02 | 0.005 | 0.03 | 0.085 | 1.22 | 6.55 | 2.14 | 0.925 | 0.02 | 0.01 | 1.47 | 16.63 | 2.41 |
| Donor 4 | PLEC | 1.42 | 0.005 | 1.66 | 0.005 | 0.03 | 0.085 | 1.22 | 5.29 | 0.79 | 0.925 | 0.31 | 0.02 | 16.87 | 28.63 | 4.15 |
| Donor 4 | PLEKHG5 | 0.66 | 0.005 | 1.15 | 0.005 | 0.03 | 0.085 | 1.22 | 3.19 | 1.19 | 0.925 | 0.02 | 0.01 | 0.8 | 9.29 | 1.35 |
| Donor 4 | PTGDS | 0.66 | 3.65 | 2.26 | 0.005 | 0.03 | 0.085 | 1.22 | 3.19 | 2.08 | 0.925 | 0.02 | 0.01 | 10.06 | 24.20 | 3.51 |
| Donor 4 | RASA3 | 0.66 | 0.01 | 2.55 | 0.005 | 0.03 | 0.085 | 1.22 | 3.3 | 1.44 | 0.925 | 0.02 | 0.01 | 1.81 | 12.07 | 1.75 |
| Donor 4 | TRPM2 | 0.66 | 1.35 | 1.32 | 0.005 | 0.03 | 0.085 | 1.22 | 4.98 | 1.05 | 0.925 | 0.02 | 0.01 | 1.7 | 13.36 | 1.94 |
| Donor 4 | IKZF3 | 0.66 | 0.9 | 1.21 | 0.005 | 0.03 | 0.085 | 1.22 | 2.56 | 3.12 | 0.925 | 0.02 | 0.01 | 3.25 | 14.00 | 2.03 |
| Donor 4 | Actin | 0.66 | 0.01 | 1.27 | 0.005 | 0.03 | 0.085 | 1.22 | 0.18 | 0.99 | 0.925 | 0.02 | 0.01 | 1.49 | 6.90 | 1.00 |
| Donor 5 | DNAH3 | 0.66 | 0.005 | 1.66 | 0.84 | 0.03 | 0.085 | 1.22 | 2.87 | 1.05 | 0.925 | 0.27 | 0.01 | 2.82 | 12.45 | 0.78 |
| Donor 5 | DST | 0.66 | 0.6 | 0.79 | 0.005 | 0.03 | 0.085 | 1.22 | 3.61 | 3.18 | 0.925 | 0.02 | 0.01 | 2.06 | 13.20 | 0.82 |
| Donor 5 | EPS8L1 | 0.66 | 0.16 | 1.93 | 0.005 | 0.03 | 1.43 | 1.22 | 3.4 | 1.19 | 0.925 | 0.58 | 0.01 | 3.54 | 15.08 | 0.94 |
| Donor 5 | FRMD4B | 0.66 | 2.03 | 1.71 | 0.005 | 0.03 | 0.085 | 1.22 | 0.09 | 0.3 | 0.925 | 0.02 | 0.01 | 1.86 | 8.95 | 0.56 |
| Donor 5 | LAMA3 | 0.66 | 0.01 | 1.93 | 0.005 | 0.03 | 2.29 | 1.22 | 0.41 | 0.3 | 0.925 | 0.22 | 0.01 | 1.86 | 9.87 | 0.62 |
| Donor 5 | MET | 0.66 | 0.005 | 1.69 | 0.005 | 0.03 | 0.085 | 1.22 | 0.09 | 1.44 | 0.925 | 0.02 | 0.01 | 2.54 | 8.72 | 0.54 |
| Donor 5 | MIB2 | 0.66 | 0.005 | 2.44 | 0.005 | 0.03 | 0.95 | 1.22 | 1.71 | 0.06 | 0.925 | 0.02 | 0.01 | 2.71 | 10.75 | 0.67 |
| Donor 5 | MRC2 | 0.66 | 0.005 | 3.06 | 0.005 | 0.03 | 0.085 | 1.22 | 0.09 | 0.92 | 0.925 | 0.02 | 0.01 | 1.38 | 8.41 | 0.52 |
| Donor 5 | NOS2 | 0.66 | 1.2 | 1.9 | 0.005 | 0.03 | 0.085 | 1.22 | 0.09 | 1.89 | 0.925 | 1.11 | 0.01 | 3.63 | 12.76 | 0.80 |
| Donor 5 | PLEC | 0.66 | 0.01 | 1.56 | 0.005 | 0.03 | 0.085 | 1.22 | 1.28 | 0.03 | 0.925 | 0.85 | 0.01 | 2.06 | 8.73 | 0.54 |
| Donor 5 | PLEKHG5 | 0.66 | 0.005 | 1.77 | 0.54 | 0.49 | 0.085 | 1.22 | 0.09 | 1.19 | 0.925 | 0.93 | 0.01 | 3.21 | 11.13 | 0.69 |
| Donor 5 | PTGDS | 0.66 | 0.005 | 0.48 | 0.005 | 0.03 | 0.085 | 1.22 | 2.66 | 2.57 | 0.925 | 1.71 | 0.01 | 2.08 | 12.44 | 0.78 |
| Donor 5 | RASA3 | 0.66 | 0.3 | 2.21 | 0.005 | 0.03 | 0.085 | 1.22 | 1.49 | 1.44 | 0.925 | 0.02 | 0.01 | 1.9 | 10.30 | 0.64 |
| Donor 5 | TRPM2 | 0.66 | 0.005 | 1.1 | 0.005 | 0.03 | 0.085 | 1.22 | 0.09 | 0.03 | 0.925 | 0.02 | 0.01 | 0.92 | 5.10 | 0.32 |
| Donor 5 | IKZF3 | 0.66 | 4.81 | 2.52 | 0.005 | 0.03 | 2.94 | 1.22 | 4.66 | 0.03 | 0.925 | 0.02 | 0.01 | 1.52 | 19.35 | 1.21 |
| Donor 5 | Actin | 0.66 | 1.65 | 1.4 | 0.005 | 0.03 | 0.085 | 1.22 | 5.5 | 1.44 | 0.925 | 0.02 | 0.01 | 3.08 | 16.03 | 1.00 |
| Donor 6 | DNAH3 | 59.45 | 150.57 | 19.71 | 0.58 | 0.91 | 1.73 | 1.22 | 26.38 | 150.33 | 0.925 | 28.58 | 5.59 | 367.48 | 813.46 | 3.66 |
| Donor 6 | DST | 44.3 | 186.38 | 22.05 | 1.56 | 0.03 | 0.085 | 28.27 | 21.57 | 149.86 | 0.925 | 6.68 | 4.12 | 170.36 | 636.19 | 2.86 |
| Donor 6 | EPS8L1 | 47.7 | 132.54 | 24.08 | 2.42 | 0.03 | 0.085 | 1.22 | 23.24 | 53.62 | 0.925 | 10.24 | 4.59 | 322.88 | 623.57 | 2.81 |
| Donor 6 | FRMD4B | 12.51 | 94.1 | 18.98 | 0.5 | 4.13 | 0.78 | 1.22 | 27 | 33.89 | 0.925 | 0.8 | 0.24 | 24.26 | 219.34 | 0.99 |
| Donor 6 | LAMA3 | 47.4 | 31 | 11.77 | 0.54 | 0.03 | 0.085 | 1.22 | 15 | 48.92 | 0.925 | 8.14 | 0.01 | 254.81 | 419.85 | 1.89 |
| Donor 6 | MET | 36.59 | 255.47 | 19.03 | 1.92 | 0.03 | 0.4 | 1.22 | 59.85 | 64.07 | 0.925 | 3.14 | 4.24 | 56.57 | 503.46 | 2.27 |
| Donor 6 | MIB2 | 28.73 | 46.26 | 15.32 | 1.69 | 7.7 | 0.085 | 1.22 | 16.35 | 44.57 | 0.925 | 1.58 | 0.58 | 202.54 | 367.55 | 1.65 |
| Donor 6 | MRC2 | 30.56 | 173.28 | 11.42 | 0.3 | 0.03 | 0.085 | 1.22 | 15.31 | 25.45 | 0.925 | 13.84 | 2.86 | 70.54 | 345.82 | 1.56 |
| Donor 6 | NOS2 | 70.25 | 513.42 | 21.89 | 2.25 | 0.03 | 1.11 | 1.22 | 72.8 | 117.93 | 1.85 | 2.77 | 2.06 | 197.11 | 1004.69 | 4.52 |
| Donor 6 | PLEC | 52.82 | 69.38 | 21.92 | 1.42 | 0.03 | 0.085 | 1.22 | 20.11 | 58.11 | 0.925 | 16.23 | 2.43 | 262.58 | 507.26 | 2.28 |
| Donor 6 | PLEKHG5 | 23.2 | 140.24 | 15.8 | 0.19 | 0.03 | 0.085 | 1.22 | 20.73 | 55.53 | 0.925 | 1.96 | 0.17 | 136.4 | 396.48 | 1.78 |
| Donor 6 | PTG DS | 44.5 | 194.94 | 14.38 | 1.12 | 0.03 | 0.085 | 1.22 | 30.35 | 54.69 | 0.925 | 6.64 | 2.43 | 125.84 | 477.15 | 2.15 |
| Donor 6 | RASA3 | 67.6 | 91.21 | 19.34 | 1.53 | 0.03 | 0.085 | 7.62 | 43.82 | 212.13 | 0.925 | 14.56 | 2.18 | 273.27 | 734.30 | 3.31 |
| Donor 6 | TRPM2 | 24.72 | 145.01 | 12.57 | 0.005 | 0.03 | 0.085 | 1.22 | 22.4 | 16.66 | 0.925 | 1.5 | 3.28 | 67.52 | 295.93 | 1.33 |
| Donor 6 | IKZF3 | 63.92 | 108.75 | 23.63 | 1.97 | 0.03 | 0.085 | 5.1 | 46.57 | 131.23 | 0.925 | 22.4 | 2.86 | 116.65 | 524.12 | 2.36 |
| Donor 6 | Actin | 18.81 | 135.48 | 11.03 | 0.5 | 0.03 | 0.085 | 1.22 | 4.66 | 8.77 | 0.925 | 2.22 | 0.01 | 38.39 | 222.13 | 1.00 |
| Donor 7 | DNAH3 | 25.1 | 28.72 | 2.1 | 0.005 | 0.03 | 0.085 | 1.22 | 7.49 | 2.45 | 0.925 | 0.02 | 0.09 | 48.76 | 117.00 | 1.64 |
| Donor 7 | DST | 20.84 | 93.16 | 3.11 | 0.005 | 0.03 | 0.085 | 1.22 | 10.1 | 4.73 | 0.925 | 1.02 | 0.01 | 80.77 | 216.01 | 3.03 |
| Donor 7 | EPS8L1 | 1.32 | 0.9 | 2.84 | 0.005 | 0.03 | 0.085 | 1.22 | 3.4 | 0.03 | 0.925 | 0.63 | 0.01 | 7.74 | 19.14 | 0.27 |
| Donor 7 | FRMD4B | 12.7 | 21.99 | 3.25 | 0.005 | 0.03 | 0.085 | 1.22 | 2.66 | 1.7 | 0.925 | 0.02 | 0.01 | 27.73 | 72.33 | 1.01 |
| Donor 7 | LAMA3 | 2.88 | 3.49 | 3.13 | 0.005 | 0.03 | 0.085 | 1.22 | 1.06 | 2.32 | 0.925 | 0.02 | 0.38 | 7.3 | 22.85 | 0.32 |
| Donor 7 | MET | 0.66 | 1.05 | 1.82 | 0.005 | 0.03 | 0.085 | 1.22 | 3.09 | 0.22 | 0.925 | 0.02 | 0.01 | 8.53 | 17.67 | 0.25 |
| Donor 7 | MIB2 | 44.9 | 19.98 | 7.32 | 0.005 | 0.03 | 0.085 | 1.22 | 0.63 | 8.89 | 0.925 | 0.02 | 0.01 | 30.68 | 114.70 | 1.61 |
| Donor 7 | MRC2 | 4.99 | 6.61 | 2.17 | 0.005 | 0.03 | 0.085 | 1.22 | 0.09 | 2.2 | 0.925 | 0.02 | 0.01 | 15.08 | 33.44 | 0.47 |
| Donor 7 | NOS2 | 64.4 | 61.11 | 9.55 | 0.38 | 0.03 | 2.29 | 1.22 | 3.93 | 10.2 | 0.925 | 0.18 | 0.01 | 29.13 | 183.36 | 2.57 |
| Donor 7 | PLEC | 68.55 | 449.86 | 8.19 | 0.005 | 0.03 | 0.085 | 1.22 | 6.34 | 13.64 | 0.925 | 0.02 | 1.43 | 36.75 | 587.05 | 8.23 |
| Donor 7 | PLEKHG5 | 39.34 | 37.86 | 7.75 | 0.005 | 0.03 | 0.085 | 1.22 | 7.6 | 5.31 | 0.925 | 0.02 | 2.92 | 55.5 | 158.57 | 2.22 |
| Donor 7 | PTGDS | 32.88 | 24.01 | 4.51 | 0.005 | 2.73 | 0.085 | 1.22 | 7.6 | 3.9 | 0.925 | 0.02 | 0.01 | 45.13 | 123.03 | 1.73 |
| Donor 7 | RASA3 | 42.8 | 44.03 | 7.54 | 0.005 | 0.03 | 0.085 | 1.22 | 7.8 | 14.2 | 0.925 | 0.02 | 0.31 | 36.75 | 155.72 | 2.18 |
| Donor 7 | TRPM2 | 29.69 | 140.85 | 2.97 | 0.005 | 0.03 | 0.085 | 1.22 | 25.75 | 3.72 | 0.925 | 0.02 | 0.01 | 124.46 | 329.74 | 4.62 |
| Donor 7 | IKZF3 | 43.4 | 29.69 | 8.26 | 0.005 | 0.03 | 0.085 | 1.22 | 5.71 | 6.88 | 0.925 | 0.02 | 0.45 | 37.8 | 134.48 | 1.89 |
| Donor 7 | Actin | 3.31 | 6.53 | 0.77 | 0.01 | 0.03 | 2.29 | 1.22 | 7.7 | 0.14 | 0.925 | 0.02 | 0.01 | 48.35 | 71.31 | 1.00 |
| Donor 8 | DNAH3 | 110.13 | 191.67 | 72.91 | 1.32 | 0.03 | 4.85 | 3.47 | 9.27 | 105.51 | 0.925 | 0.4 | 0.78 | 121.93 | 623.20 | 47.79 |
| Donor 8 | DST | 58.57 | 75.26 | 15.34 | 0.38 | 0.49 | 0.085 | 1.22 | 12.81 | 45.35 | 0.925 | 0.02 | 2.43 | 79.79 | 292.67 | 22.44 |
| Donor 8 | EPS8L1 | 88.89 | 63.7 | 41.38 | 1.19 | 0.03 | 0.085 | 6.26 | 10.1 | 121.32 | 0.925 | 0.02 | 4.24 | 92.38 | 430.52 | 33.02 |
| Donor 8 | FRMD4B | 29.4 | 65.37 | 9.26 | 0.42 | 0.03 | 0.085 | 6.48 | 8.43 | 53.96 | 0.925 | 0.22 | 1.68 | 53.45 | 229.71 | 17.62 |
| Donor 8 | LAMA3 | 197.84 | 534.58 | 80.04 | 6.66 | 5.92 | 0.085 | 11.96 | 16.25 | 222.4 | 0.925 | 0.49 | 0.01 | 173.02 | 1250.18 | 95.87 |
| Donor 8 | MET | 166.16 | 260.07 | 34.37 | 1.29 | 0.03 | 0.95 | 6.15 | 19.79 | 180.96 | 0.925 | 3.81 | 0.01 | 150.63 | 825.15 | 63.28 |
| Donor 8 | MIB2 | 55.58 | 97.75 | 8.09 | 3.34 | 0.03 | 0.4 | 10.38 | 14.37 | 48.48 | 0.925 | 4.22 | 0.01 | 70.89 | 314.47 | 24.12 |
| Donor 8 | MRC2 | 18.72 | 20.86 | 7.27 | 0.005 | 0.03 | 0.085 | 1.22 | 5.92 | 27.67 | 0.925 | 0.02 | 0.01 | 27.96 | 110.70 | 8.49 |
| Donor 8 | NOS2 | 79.04 | 62.03 | 23.6 | 1.36 | 0.03 | 0.085 | 8.21 | 11.98 | 120.62 | 0.925 | 1.28 | 0.01 | 53.5 | 362.67 | 27.81 |
| Donor 8 | PLEC | 190.8 | 360.99 | 57.12 | 8.89 | 0.03 | 0.085 | 33.62 | 22.19 | 218.93 | 0.925 | 0.67 | 0.58 | 135.11 | 1029.94 | 78.98 |
| Donor 8 | PLEKHG5 | 30.37 | 80.65 | 6.89 | 0.005 | 0.03 | 0.085 | 1.22 | 12.39 | 12.62 | 0.925 | 0.08 | 0.01 | 34.21 | 179.49 | 13.76 |
| Donor 8 | PTGDS | 17.08 | 7.78 | 5.28 | 0.005 | 1.92 | 0.085 | 1.22 | 13.44 | 25.12 | 0.925 | 0.67 | 2.31 | 25.09 | 100.93 | 7.74 |
| Donor 8 | RASA3 | 125.64 | 123.92 | 31.79 | 2.26 | 0.03 | 0.085 | 51.42 | 14.69 | 295.64 | 0.925 | 3.02 | 1.3 | 122.48 | 773.20 | 59.29 |
| Donor 8 | TRPM2 | 24.34 | 6.76 | 9.28 | 0.54 | 0.03 | 0.085 | 1.22 | 10.62 | 36.72 | 0.925 | 0.76 | 0.38 | 38.24 | 129.90 | 9.96 |
| Donor 8 | IKZF3 | 91.55 | 147.61 | 33.66 | 1.15 | 0.03 | 0.085 | 3.39 | 9.16 | 104.46 | 0.925 | 1.02 | 2.8 | 80.67 | 476.51 | 36.54 |
| Donor 8 | Actin | 0.66 | 1.12 | 1.9 | 0.22 | 0.03 | 0.085 | 1.22 | 3.61 | 0.03 | 0.925 | 0.02 | 0.58 | 2.64 | 13.04 | 1.00 |
| Donor 9 | DNAH3 | 18.58 | 8.02 | 1.45 | 0.005 | 0.91 | 0.085 | 1.22 | 12.71 | 4.02 | 0.925 | 0.18 | 0.78 | 106.41 | 155.30 | 2.24 |
| Donor 9 | DST | 18.02 | 15.32 | 3.89 | 0.17 | 0.03 | 0.085 | 1.22 | 8.22 | 1.19 | 0.925 | 0.02 | 0.01 | 64.97 | 114.07 | 1.64 |
| Donor 9 | EPS8L1 | 0.66 | 3.49 | 16.23 | 0.005 | 0.03 | 0.085 | 1.22 | 2.77 | 3.18 | 0.925 | 0.58 | 0.01 | 7.16 | 36.35 | 0.52 |
| Donor 9 | FRMD4B | 5.93 | 3.18 | 2.93 | 0.005 | 0.03 | 0.085 | 1.22 | 0.09 | 0.92 | 0.925 | 0.04 | 0.01 | 12.73 | 28.10 | 0.40 |
| Donor 9 | LAMA3 | 0.66 | 4.03 | 2.75 | 0.005 | 0.03 | 2.01 | 1.22 | 1.28 | 1.51 | 0.925 | 0.02 | 0.01 | 6.68 | 21.13 | 0.30 |
| Donor 9 | MET | 2.43 | 0.005 | 2.88 | 0.005 | 0.03 | 0.085 | 1.22 | 4.66 | 0.92 | 0.925 | 0.02 | 0.01 | 15.76 | 28.95 | 0.42 |
| Donor 9 | MIB2 | 13.91 | 10.55 | 5.42 | 0.005 | 0.03 | 0.085 | 1.22 | 6.55 | 4.25 | 0.925 | 0.02 | 0.01 | 63.45 | 106.43 | 1.53 |
| Donor 9 | MRC2 | 0.66 | 15.32 | 5.84 | 0.005 | 0.03 | 0.085 | 1.22 | 9.06 | 3.42 | 0.925 | 0.02 | 0.01 | 11.63 | 48.23 | 0.69 |
| Donor 9 | NOS2 | 27.96 | 18.69 | 4.86 | 0.005 | 0.03 | 0.085 | 1.22 | 22.19 | 2.01 | 0.925 | 1.19 | 0.01 | 220.43 | 299.61 | 4.32 |
| Donor 9 | PLEC | 3.36 | 4.73 | 2.7 | 0.005 | 0.03 | 2.01 | 1.22 | 1.92 | 0.65 | 0.925 | 0.02 | 0.01 | 15.95 | 33.53 | 0.48 |
| Donor 9 | PLEKHG5 | 1.42 | 1.35 | 2.97 | 0.56 | 4.13 | 0.085 | 1.22 | 4.03 | 0.51 | 0.925 | 0.22 | 0.01 | 8.07 | 25.50 | 0.37 |
| Donor 9 | PTGDS | 9.72 | 1.5 | 2.15 | 0.005 | 0.03 | 0.085 | 1.22 | 5.71 | 1.95 | 0.925 | 0.02 | 0.01 | 47.71 | 71.04 | 1.02 |
| Donor 9 | RASA3 | 2.48 | 6.14 | 2.12 | 0.005 | 0.03 | 0.085 | 1.22 | 4.03 | 0.03 | 0.925 | 1.19 | 0.01 | 14.78 | 33.05 | 0.48 |
| Donor 9 | TRPM2 | 5.56 | 0.9 | 4.77 | 0.38 | 0.03 | 0.085 | 1.22 | 4.03 | 1.32 | 0.925 | 0.02 | 0.01 | 10.04 | 29.29 | 0.42 |
| Donor 9 | IKZF3 | 9.67 | 0.005 | 6.18 | 0.005 | 0.03 | 1.43 | 1.22 | 5.08 | 1.32 | 0.925 | 0.08 | 0.01 | 31.98 | 57.94 | 0.83 |
| Donor 9 | Actin | 0.66 | 3.49 | 0.77 | 0.36 | 0.03 | 2.01 | 1.22 | 2.13 | 1.05 | 0.925 | 0.58 | 0.01 | 56.18 | 69.42 | 1.00 |

To test the immunogenic capacity of specific N-terminal peptides in a more cellular setting, we then assessed responses of T cells previously primed to recognize either altered or wild-type peptides, when co-cultured with HLA-matched isogenic GC cells expressing either altered or wild-type peptides respectively (Figure 12). By MHC-I affinity screening, a VMCDIFFSL nonamer in the WT *RASA3* N-terminus was predicted to exhibit high MHC-I affinity binding for both the HLA-A02:01 (IC50=6.93nm) and HLA-A02:06 (IC50=9.74nm) alleles. Using HLA-A*02:06 T cells that are cross-reactive to HLA-A*02:01-positive AGS cells, we tested release of interferon gamma (IFNγ) from primed T cells after exposure to AGS lysates expressing either RASA3 CanT or SomT isoforms. ELISA assays demonstrated that T cells primed to recognize *RASA3* CanT released significantly more IFNγ when co-cultured with *RASA3* CanT- expressing AGS cells than when co-cultured with *RASA3* SomT-expressing AGS cells. In contrast, T-cells primed with *RASA3* SomT did not exhibit appreciable IFNγ release when co-cultured with *RASA3* SomT expressing AGS cells, indicating that *RASA3* SomT is less immunogenic (Figure 12). Taken collectively, these *in vitro* results demonstrate that peptides predicted to be depleted in GCs through somatic promoter alterations can produce immunogenic responses, with the magnitude of immune responses depending on both peptide sequence and host immune background.

### Somatic Promoters are Associated with EZH2 Occupancy

To identify potential oncogenic mechanisms driving somatic promoter alterations, we intersected the genomic locations of the somatic promoters with transcription factor binding sites (TFBS) of 237 transcription factors from 83 different tissues. Regions exhibiting somatic promoters were significantly enriched in regions associated with EZH2 (P<0.01) and SUZ12 (P<0.01) binding (Fig. 6a, Table 13), confirming earlier findings on a smaller cohort. Both EZH2 and SUZ12 are components of the PRC2 epigenetic regulator complex, which is upregulated in many cancer types including GC. To validate these findings, we then performed EZH2 Chip-sequencing on HFE-145 normal gastric epithelial cells (Methods and Materials). Concordant with the previous findings, we observed significant enrichment of EZH2 binding sites at somatic promoters compared to all promoters (Enrichment score 27 vs. 13 for all promoters, P<0.01), and this EZH2 enrichment remained significant when the gained somatic (Enrichment Score 28, P<0.01) and lost somatic promoters (Enrichment Score 24, P<0.01) were analyzed separately (Figure 18).

**Table 13: Somatic Promoters Overlapping EZH2/SUZ12 Binding Sites**

| **Loci** | **Annotation Status** | **Associated Gene** |
|---|---|---|
| chrX:136647100-136648150 | Known | ZIC3 |
| chr13:100634350-100638150 | Known | ZIC2 |
| chr13:100630200-100634000 | Known | ZIC2 |
| chr20:50719850-50723350 | Known | ZFP64 |
| chr18:45660800-45664950 | Known | ZBTB7C |
| chr1:185226150-185227950 | Known | Y_RNA |
| chr3:13920600-13921250 | Known | WNT7A |
| chr2:71126100-71129800 | Known | VAX2 |
| chr5:6448050-6451150 | Known | UBE2QL1 |
| chr8:72986650-72987850 | Known | TRPA1 |
| chr22:17082250-17084550 | Known | TPTEP1 |
| chr19:55657350-55658650 | Known | TNNT1 |
| chr19:55666950-55668450 | Known | TNNI3 |
| chr22:42320400-42323750 | Known | TNFRSF13C |
| chr8:119962100-119965650 | Known | TNFRSF11B |
| chr21:42873650-42881750 | Known | TMPRSS2 |
| chr20:1164650-1168700 | Known | TMEM74B |
| chr17:53797250-53803100 | Known | TMEM100 |
| chr11:119291200-119294700 | Known | THY1 |
| chr20:55203450-55206500 | Known | TFAP2C |
| chr6:10409250-10419650 | Known | TFAP2A;TFAP2A-AS1 |
| chr6:85471550-85475350 | Known | TBX18 |
| chr20:46411750-46414250 | Known | SULF2 |
| chr8:70403800-70408450 | Known | SULF1 |
| chr5:172753250-172757450 | Known | STC2 |
| chr14:38675750-38681750 | Known | SSTR1 |
| chr7:20824950-20827850 | Known | SP8 |
| chr13:95362100-95368650 | Known | SOX21;SOX21-AS1 |
| chr3:181428150-181434750 | Known | SOX2 |
| chr8:101660950-101662650 | Known | SNX31 |
| chr20:10197250-10201300 | Known | SNAP25;SNAP25-AS1 |
| chr20:48598400-48604100 | Known | SNAI1 |
| chr14:70346050-70347700 | Known | SMOC1 |
| chr12:85303950-85307700 | Known | SLC6A15 |
| chr19:17981100-17986400 | Known | SLC5A5 |
| chr2:228580350-228583450 | Known | SLC19A3 |
| chr3:121656650-121658300 | Known | SLC15A2 |
| chr6:100910100-100913300 | Known | SIM1 |
| chr21:44842150-44848700 | Known | SIK1 |
| chr7:37953600-37956950 | Known | SFRP4 |
| chr4:154708850-154714150 | Known | SFRP2 |
| chr16:23193600-23197800 | Known | SCNN1G |
| chr16:23312800-23315350 | Known | SCNN1B |
| chr2:200326950-200329550 | Known | SATB2 |
| chr20:50415800-50419950 | Known | SALL4 |
| chr20:981750-984100 | Known | RSPO4 |
| chr1:148247000-148248800 | Known | RP11-89F3.2 |
| chr12:54472600-54477950 | Known | RP11-834C11.6;RP11-834C11.7 |
| chr5:72746300-72748200 | Known | RP11-79P5.7 |
| chr1:61103800-61106600 | Known | RP11-776H12.1 |
| chr11:134335600-134339750 | Known | RP11-627G23.1 |
| chr11:69830350-69834850 | Known | RP11-626H12.1 |
| chr16:89987550-89991500 | Known | RP11-566K11.4;TUBB3 |
| chr16:86319900-86321550 | Known | RP11-514D23.1 |
| chr3:50191700-50195800 | Known | RP11-493K19.3;SEMA3F |
| chr3:132756350-132758550 | Known | RP11-469L4.1;TMEM108 |
| chr6:26613750-26615600 | Known | RP11-457M11.6 |
| chr3:87841650-87842700 | Known | RP11-451B8.1 |
| chr1:113391350-113395900 | Known | RP11-426L16.8;RP3-522D1.1 |
| chr12:85711250-85713200 | Known | RP11-408B11.2 |
| chr6:106807450-106809950 | Known | RP11-404H14.1 |
| chr1:149230550-149232000 | Known | RP11-403I13.5 |
| chr1:222138950-222144050 | Known | RP11-400N13.2 |
| chr3:178577000-178578500 | Known | RP11-385J1.2 |
| chr17:46721450-46725800 | Known | RP11-357H14.17 |
| chr5:522450-524750 | Known | RP11-310P5.2;SLC9A3 |
| chr15:80542500-80545200 | Known | RP11-2E17.1 |
| chr5:74343750-74351250 | Known | RP11-229C3.2 |
| chr5:63460450-63463050 | Known | RNF180 |
| chr1:228742450-228743450 | Known | RNA5SP19 |
| chr1:228781900-228785450 | Known | RNA5S17;RNA5SP18 |
| chr21:38379100-38379750 | Known | RIPPLY3 |
| chr21:43180350-43189850 | Known | RIPK4 |
| chr8:104510350-104514700 | Known | RIMS2;RP11-1C8.4 |
| chr10:62758000-62762450 | Known | RHOBTB1 |
| chr15:90039550-90040150 | Known | RHCG |
| chr2:86564650-86566000 | Known | REEP1 |
| chr4:82964050-82966400 | Known | RASGEF1B;RP11-689K5.3 |
| chr3:75707050-75708850 | Known | RARRES2P1 |
| chr8:85093500-85097700 | Known | RALYL |
| chr8:128805200-128810000 | Known | PVT1 |
| chr1:29562850-29565950 | Known | PTPRU |
| chr7:158378250-158380350 | Known | PTPRN2 |
| chr1:170630400-170636550 | Known | PRRX1;RP1-79C4.4 |
| chr6:150463250-150464400 | Known | PPP1R14C |
| chr12:133264050-133266950 | Known | POLE;PXMP2;RP13-672B3.2 |
| chr5:74990850-74992350 | Known | POC5 |
| chr20:56280450-56287350 | Known | PMEPA1 |
| chr16:57315850-57319550 | Known | PLLP |
| chr1:6544500-6545600 | Known | PLEKHG5 |
| chr14:69950300-69951550 | Known | PLEKHD1 |
| chr1:201251800-201254650 | Known | PKP1 |
| chr2:42275400-42282950 | Known | PKDCC |
| chr12:130823500-130825600 | Known | PIWIL1 |
| chr4:111557000-111559350 | Known | PITX2 |
| chr7:32107350-32111900 | Known | PDE1C |
| chr1:55504650-55507550 | Known | PCSK9 |
| chr15:102029650-102031300 | Known | PCSK6 |
| chr3:142606500-142609050 | Known | PCOLCE2 |
| chr14:37129750-37133800 | Known | PAX9 |
| chr1:17443850-17446850 | Known | PADI2 |
| chr8:99951150-99961750 | Known | OSR2;RP11-44N12.5;STK3 |
| chr1:161991300-161994850 | Known | OLFML2B |
| chr7:8473050-8474100 | Known | NXPH1 |
| chr9:87282200-87286150 | Known | NTRK2 |
| chr19:15309800-15311950 | Known | NOTCH3 |
| chr4:56500900-56504300 | Known | NMU |
| chr1:183385400-183388500 | Known | NMNAT2 |
| chr8:41502400-41510150 | Known | NKX6-3 |
| chr10:134596450-134599400 | Known | NKX6-2;RP11-288G11.3 |
| chr4:85417400-85421400 | Known | NKX6-1 |
| chr2:233791350-233792700 | Known | NGEF |
| chrX:107016000-107021000 | Known | NCBP2L;TSC22D3 |
| chr11:1150000-1157350 | Known | MUC5AC |
| chr7:100607850-100613600 | Known | MUC12;MUC3A;RP11-395B7.2 |
| chr16:56699800-56705700 | Known | MT1G;MT1H |
| chr12:132313150-132317650 | Known | MMP17 |
| chr7:73036850-73039200 | Known | MLXIPL |
| chr19:54482850-54485950 | Known | MIR935 |
| chr9:21554500-21561150 | Known | MIR31HG |
| chr17:46800050-46802400 | Known | MIR3185;PRAC1;PRAC2 |
| chr1:1562700-1565700 | Known | MIB2 |
| chr1:205537050-205540700 | Known | MFSD4 |
| chr13:31480150-31483050 | Known | MEDAG |
| chr2:132152200-132153000 | Known | MED15P3 |
| chr3:150959500-150960300 | Known | MED12L |
| chr2:149894250-149897500 | Known | LYPD6B |
| chr11:1889150-1894600 | Known | LSP1 |
| chr1:156896950-156898350 | Known | LRRC71 |
| chr11:61275250-61276400 | Known | LRRC10B;MIR4488 |
| chr9:103789900-103792650 | Known | LPPR1 |
| chr16:1013250-1015550 | Known | LMF1 |
| chr1:2980250-2991900 | Known | LINC00982;PRDM16 |
| chr3:75719150-75723200 | Known | LINC00960 |
| chr20:21085550-21087550 | Known | LINC00237 |
| chr19:55127750-55130550 | Known | LILRB1 |
| chr7:103968400-103969950 | Known | LHFPL3 |
| chr1:202182400-202184350 | Known | LGR6 |
| chr1:202161700-202163400 | Known | LGR6 |
| chr1:65991250-65992850 | Known | LEPR |
| chr1:205424550-205426850 | Known | LEMD1;RP11-576D8.4 |
| chr20:9494050-9498000 | Known | LAMP5;RP5-1119D9.4 |
| chr6:129203450-129207800 | Known | LAMA2 |
| chr19:51485750-51487700 | Known | KLK7 |
| chr3:126073900-126077300 | Known | KLF15 |
| chr1:245315950-245321950 | Known | KIF26B |
| chr1:180880350-180883200 | Known | KIAA1614 |
| chr15:81070500-81075050 | Known | KIAA1199 |
| chr20:43728950-43730250 | Known | KCNS1 |
| chr14:88788450-88791000 | Known | KCNK10 |
| chr7:119911950-119914550 | Known | KCND2 |
| chr1:111210100-111218300 | Known | KCNA3 |
| chr16:31366400-31369100 | Known | ITGAX |
| chr20:13200350-13202100 | Known | ISM1 |
| chr16:54316250-54322800 | Known | IRX3 |
| chr5:2748900-2751450 | Known | IRX2 |
| chr17:38016450-38022250 | Known | IKZF3 |
| chr22:23229500-23237350 | Known | IGLC1;IGLJ1;IGLL5 |
| chr19:46579500-46581300 | Known | IGFL4 |
| chr7:45927300-45929150 | Known | IGFBP1 |
| chr7:23506000-23515500 | Known | IGF2BP3 |
| chr6:87646350-87648250 | Known | HTR1E |
| chr5:175084150-175086850 | Known | HRH2 |
| chr3:11195250-11198600 | Known | HRH1 |
| chr4:175439400-175445700 | Known | HPGD |
| chr12:54386800-54395700 | Known | HOXC6;HOXC9;HOXC-AS1;HOXC-AS2 |
| chr12:54421700-54423400 | Known | HOXC6 |
| chr12:54410150-54413050 | Known | HOXC4;HOXC6;RP11-834C11.14 |
| chr12:54446200-54449350 | Known | HOXC4 |
| chr12:54331500-54334550 | Known | HOXC13;HOXC-AS5 |
| chr12:54375250-54381900 | Known | HOXC10; HOXC-AS3; RP11-834C11.12 |
| chr17:46701450-46705000 | Known | HOXB9 |
| chr17:46804450-46808100 | Known | HOXB13 |
| chr7:27159450-27164850 | Known | HOXA3;HOXA-AS2 |
| chr7:27208400-27220700 | Known | HOXA10;HOXA9;HOXA-AS4;MIR196B;RP1-170019.20 |
| chr7:27221300-27251300 | Known | HOTTIP;HOXA11;HOXA11-AS;HOXA13;RP1-170019.14 |
| chr12:54365950-54373250 | Known | HOTAIR;HOXC11 |
| chr1:6478800-6480950 | Known | HES2 |
| chr11:2016000-2021350 | Known | H19 |
| chr11:45942850-45946400 | Known | GYLTL1B |
| chr9:140056700-140058300 | Known | GRIN1 |
| chr15:72488700-72491050 | Known | GRAMD2 |
| chr17:72425800-72433550 | Known | GPRC5C |
| chr5:89854500-89855350 | Known | GPR98 |
| chrX:133117900-133120700 | Known | GPC3 |
| chr19:2700850-2702900 | Known | GNG7 |
| chr7:99526050-99527900 | Known | GJC3;RP4-604G5.1 |
| chr8:75230900-75235150 | Known | GDAP1;JPH1 |
| chr7:74379400-74380400 | Known | GATSL1 |
| chr20:61046800-61052500 | Known | GATA5;RP13-379024.3 |
| chr8:11533800-11540650 | Known | GATA4 |
| chr8:11557150-11568950 | Known | GATA4 |
| chr11:11640700-11644650 | Known | GALNT18 |
| chr12:130645350-130646800 | Known | FZD10;FZD10-AS1 |
| chr6:96460900-96466650 | Known | FUT9 |
| chr13:39259850-39263000 | Known | FREM2 |
| chr16:86600550-86601800 | Known | FOXC2;RP11-463O9.5 |
| chr6:1608550-1611700 | Known | FOXC1 |
| chr14:38051900-38070050 | Known | FOXA1;TTC6 |
| chr17:39965500-39970950 | Known | FKBP10;LEPREL4 |
| chr9:133813800-133816150 | Known | FIBCD1 |
| chr11:69630950-69635350 | Known | FGF3 |
| chr3:13973700-13975200 | Known | FGD5P1 |
| chr10:95325600-95329150 | Known | FFAR4 |
| chr7:121942750-121947900 | Known | FEZF1;FEZF1-AS1 |
| chr16:86529000-86534050 | Known | FENDRR |
| chr21:42687850-42691150 | Known | FAM3B |
| chr17:66593700-66598900 | Known | FAM20A |
| chr1:179711850-179712600 | Known | FAM163A |
| chr8:53476650-53479500 | Known | FAM150A |
| chr4:187025100-187028650 | Known | FAM149A |
| chr12:124778800-124786100 | Known | FAM101A |
| chr7:27281600-27284150 | Known | EVX1;EVX1-AS |
| chrX:103498450-103500200 | Known | ESX1 |
| chr1:216892850-216898200 | Known | ESRRG |
| chr19:55590850-55593800 | Known | EPS8L1 |
| chr8:144950100-144953650 | Known | EPPK1 |
| chr17:48608600-48615100 | Known | EPN3 |
| chr1:23037600-23041300 | Known | EPHB2 |
| chr9:112080500-112082950 | Known | EPB41L4B |
| chr7:155250600-155253200 | Known | EN2 |
| chr19:14885900-14888350 | Known | EMR2 |
| chr22:37821950-37823900 | Known | ELFN2;RP1-63G5.5 |
| chr19:1286150-1288700 | Known | EFNA2;MUM1 |
| chr20:57874800-57877300 | Known | EDN3 |
| chr15:45399500-45410700 | Known | DUOX2;DUOXA2 |
| chr16:30021900-30023950 | Known | DOC2A |
| chr7:96633500-96636700 | Known | DLX6;DLX6-AS1;DLX6-AS2 |
| chr7:96652750-96654900 | Known | DLX5 |
| chr19:6474700-6477300 | Known | DENND1C |
| chr10:94831200-94834300 | Known | CYP26A1 |
| chr4:48987500-48989500 | Known | CWH43 |
| chr8:104382100-104385900 | Known | CTHRC1 |
| chr5:174177950-174179050 | Known | CTD-2532K18.1;MIR4634 |
| chr14:19924450-19925600 | Known | CTD-2314B22.3 |
| chr14:19640850-19641750 | Known | CTD-2314B22.1 |
| chr15:97838750-97841300 | Known | CTD-2147F2.1 |
| chr5:134912900-134915350 | Known | CTC-321K16.1;CXCL14 |
| chr5:134371700-134375750 | Known | CTC-276P9.1 |
| chr16:21288600-21290700 | Known | CRYM |
| chr2:102002650-102005250 | Known | CREG2 |
| chr15:78632500-78634200 | Known | CRABP1 |
| chr3:9745600-9747050 | Known | CPNE9 |
| chr16:89640950-89643950 | Known | CPNE7 |
| chr3:99355450-99359900 | Known | COL8A1 |
| chr6:33160200-33161450 | Known | COL11A2 |
| chr6:35754500-35755750 | Known | CLPSL1 |
| chr21:36041150-36045150 | Known | CLIC6 |
| chr17:7161850-7167950 | Known | CLDN7;RP1-4G17.5 |
| chr7:73181100-73185850 | Known | CLDN3 |
| chr3:190034900-190041800 | Known | CLDN1;CLDN16 |
| chr7:29184550-29187650 | Known | CHN2;CPVL |
| chr2:27340450-27342750 | Known | CGREF1 |
| chr13:28538700-28543950 | Known | CDX2 |
| chr5:149545100-149550500 | Known | CDX1 |
| chr16:68677900-68681200 | Known | CDH3;RP11-615I2.2 |
| chr16:68770300-68774200 | Known | CDH1 |
| chr11:6279800-6283200 | Known | CCKBR |
| chr18:57363700-57365350 | Known | CCBE1;RP11-2N1.2 |
| chr8:76189900-76191050 | Known | CASC9 |
| chr6:17392850-17396100 | Known | CAP2 |
| chr1:20808950-20814450 | Known | CAMK2N1 |
| chr7:44265350-44266400 | Known | CAMK2B |
| chr8:86350000-86351450 | Known | CA3 |
| chr5:2751850-2754050 | Known | C5orf38;IRX2 |
| chr3:138664900-138667100 | Known | C3orf72; FOXL2 |
| chr17:77019250-77024000 | Known | C1QTNF1;C1QTNF1-AS1 |
| chr1:223565950-223567600 | Known | C1orf65 |
| chr1:190440800-190450200 | Known | BRINP3;RP11-161I10.1;RP11-547I7.2 |
| chr2:198650550-198651850 | Known | BOLL |
| chr15:83952250-83953300 | Known | BNC1 |
| chr4:42152300-42155900 | Known | BEND4 |
| chr17:47209750-47211400 | Known | B4GALNT2 |
| chr11:134279600-134282050 | Known | B3GAT1 |
| chr4:94748600-94754050 | Known | ATOH1 |
| chr9:120175650-120177900 | Known | ASTN2 |
| chr9:133319400-133324650 | Known | ASS1 |
| chr11:2285750-2292550 | Known | ASCL2 |
| chr16:329250-332250 | Known | ARHGDIG |
| chr8:145908800-145912600 | Known | ARHGAP39 |
| chr4:86395150-86399900 | Known | ARHGAP24 |
| chr18:24443050-24445900 | Known | AQP4;AQP4-AS1 |
| chr11:71318250-71320050 | Known | AP000867.1 |
| chr5:79864800-79866650 | Known | ANKRD34B |
| chr2:133014850-133015750 | Known | ANKRD30BL;MIR663B |
| chr12:85672750-85675650 | Known | ALX1 |
| chr6:168195400-168198750 | Known | AL009178.1;C6orf123 |
| chr10:4867450-4870200 | Known | AKR1E2 |
| chr16:3232300-3234150 | Known | AJ003147.8 |
| chr8:11203650-11206800 | Known | AF131216.5;TDH |
| chr17:15847250-15850800 | Known | ADORA2B |
| chr7:5601050-5603800 | Known | ACTB |
| chr7:100490350-100495550 | Known | ACHE |
| chr3:18734950-18736300 | Known | AC144521.1 |
| chr2:131593950-131595800 | Known | AC133785.1;ARHGEF4 |
| chr4:44447900-44452050 | Known | AC131951.1;KCTD8 |
| chr17:7982650-7984350 | Known | AC129492.6;ALOX12B |
| chr5:1003400-1005850 | Known | AC116351.2;RP11-43F13.4 |
| chr2:100721300-100722600 | Known | AC092667.2;AFF3 |
| chr2:286750-288600 | Known | AC079779.4;FAM150B |
| chr2:132121200-132122150 | Known | AC073869.1 |
| chr2:233282700-233286450 | Known | AC068134.5;AC068134.6 |
| chr16:31495650-31500700 | Known | AC026471.6;SLC5A2 |
| chr12:54348250-54351050 | Known | AC012531.23;HOXC12 |
| chr2:118561200-118562150 | Known | AC009312.1 |
| chr16:51182700-51185700 | Known | AC009166.5;SALL1 |
| chr2:171671550-171676200 | Known | AC007405.8;GAD1 |
| chr2:66801200-66811950 | Known | AC007392.3 |
| chr2:71113350-71116800 | Known | AC007040.5 |
| chr7:15720950-15728900 | Known | AC005550.4;MEOX2 |
| chr6:1611750-1616000 | Unknown | - |
| chr15:96958950-96961350 | Unknown | - |
| chr2:66652100-66655200 | Unknown | - |
| chr2:8833050-8834200 | Unknown | - |
| chr9:17905350-17908250 | Unknown | - |
| chr5:2746900-2748550 | Unknown | - |
| chr7:45001800-45003250 | Unknown | - |
| chr12:52257150-52258000 | Unknown | - |
| chr2:218874000-218875450 | Unknown | - |
| chr19:30214300-30216100 | Unknown | - |
| chr8:140717350-140719650 | Unknown | - |
| chr7:27264550-27266100 | Unknown | - |
| chr19:48900250-48904400 | Unknown | - |
| chr16:51186150-51187850 | Unknown | - |
| chr9:132458700-132461300 | Unknown | - |
| chr11:44337850-44339250 | Unknown | - |
| chr17:46694850-46697150 | Unknown | - |
| chr10:124898400-124900700 | Unknown | - |
| chr6:10382900-10384750 | Unknown | - |
| chr8:144489000-144490750 | Unknown | - |
| chr20:49837550-49839250 | Unknown | - |
| chr3:193921100-193922050 | Unknown | - |
| chr13:100619800-100623100 | Unknown | - |
| chr1:165320950-165322700 | Unknown | - |
| chr1:180203650-180205650 | Unknown | - |
| chr1:23543800-23544900 | Unknown | - |
| chr8:144842350-144844000 | Unknown | - |
| chr5:174162150-174163450 | Unknown | - |
| chr1:184632450-184634700 | Unknown | - |
| chr13:21295150-21296450 | Unknown | - |
| chr1:156893100-156894550 | Unknown | - |
| chr20:46434400-46435400 | Unknown | - |
| chr11:33398050-33400750 | Unknown | - |
| chr6:134216650-134218050 | Unknown | - |
| chr2:45176050-45177700 | Unknown | - |
| chr13:36044350-36045800 | Unknown | - |
| chr2:45227500-45229600 | Unknown | - |
| chr10:43427950-43429950 | Unknown | - |
| chr1:152079200-152081300 | Unknown | - |
| chr7:54731350-54733200 | Unknown | - |
| chr20:4201500-4202700 | Unknown | - |
| chr8:145555300-145556800 | Unknown | - |
| chr7:64733800-64735500 | Unknown | - |
| chrX:119124000-119127100 | Unknown | - |
| chr3:14642850-14644150 | Unknown | - |
| chr10:102488400-102492200 | Unknown | - |
| chr5:42999400-43001150 | Unknown | - |
| chr21:38063750-38066650 | Unknown | - |
| chr2:131010400-131011600 | Unknown | - |
| chr19:30018700-30020150 | Unknown | - |
| chr5:72731550-72734700 | Unknown | - |
| chr8:102092150-102094400 | Unknown | - |
| chr4:4867350-4869600 | Unknown | - |
| chr4:4854350-4855850 | Unknown | - |
| chr7:156735150-156736500 | Unknown | - |
| chr1:161442450-161443650 | Unknown | - |
| chr12:54356450-54358100 | Unknown | - |
| chr1:48174300-48176650 | Unknown | - |
| chr7:25900700-25903050 | Unknown | - |
| chr10:102830000-102833650 | Unknown | - |
| chr6:137310350-137312150 | Unknown | - |
| chr1:152081400-152084100 | Unknown | - |
| chr7:27274550-27276500 | Unknown | - |
| chr12:113904650-113906650 | Unknown | - |
| chr1:17024500-17028900 | Unknown | - |
| chr5:72528750-72529950 | Unknown | - |
| chr9:99481850-99483650 | Unknown | - |
| chr1:46954600-46956800 | Unknown | - |
| chr17:26119900-26121850 | Unknown | - |
| chr1:2253650-2254650 | Unknown | - |
| chr7:73060250-73063150 | Unknown | - |
| chr19:1754200-1758750 | Unknown | - |
| chr9:29211200-29215700 | Unknown | - |
| chr7:31375200-31377000 | Unknown | - |
| chr1:165344500-165346650 | Unknown | - |
| chr10:57389650-57391700 | Unknown | - |
| chr1:163441550-163443100 | Unknown | - |
| chr1:200842700-200844850 | Unknown | - |
| chr20:44639000-44640950 | Unknown | - |
| chr2:176952400-176953750 | Unknown | - |
| chr20:6031700-6033850 | Unknown | - |
| chr5:2738550-2740800 | Unknown | - |
| chr3:74662150-74664400 | Unknown | - |
| chr10:134600350-134602350 | Unknown | - |
| chr1:152084900-152085650 | Unknown | - |
| chr8:52520450-52521550 | Unknown | - |
| chr1:121279850-121280850 | Unknown | - |
| chr13:37729350-37731000 | Unknown | - |
| chr7:8390700-8392150 | Unknown | - |
| chr12:32818500-32820350 | Unknown | - |
| chr16:15350450-15351950 | Unknown | - |
| chr2:58342200-58346950 | Unknown | - |
| chr3:112383300-112384750 | Unknown | - |
| chr19:1682300-1683350 | Unknown | - |
| chr4:27077050-27078000 | Unknown | - |
| chr8:23507850-23509050 | Unknown | - |
| chr4:10782250-10783600 | Unknown | - |
| chr17:12927950-12928650 | Unknown | - |
| chr2:11989300-11990550 | Unknown | - |
| chr7:23074700-23076100 | Unknown | - |
| chr22:28479200-28480250 | Unknown | - |
| chr9:36763800-36766950 | Unknown | - |
| chr6:28757250-28758600 | Unknown | - |
| chr1:50032150-50033200 | Unknown | - |
| chr6:4334150-4335300 | Unknown | - |
| chr1:195732150-195733300 | Unknown | - |
| chr6:170483200-170484200 | Unknown | - |
| chr12:38447100-38448600 | Unknown | - |
| chr7:86667750-86669950 | Unknown | - |
| chr16:9683650-9684650 | Unknown | - |
| chr1:171342100-171343300 | Unknown | - |
| chr20:47203350-47204450 | Unknown | - |
| chr20:62030950-62034000 | Unknown | - |
| chr1:168323150-168325650 | Unknown | - |
| chr6:10133900-10134950 | Unknown | - |
| chr4:71924850-71926200 | Unknown | - |
| chrX:130711450-130713600 | Unknown | - |
| chr12:38549550-38551600 | Unknown | - |
| chr2:131094200-131095000 | Unknown | - |
| chr1:183626800-183628050 | Unknown | - |
| chr6:28918100-28918850 | Unknown | - |
| chr2:198504700-198507250 | Unknown | - |
| chr11:71350450-71351500 | Unknown | - |
| chr20:47001000-47003900 | Unknown | - |
| chr21:10600500-10603150 | Unknown | - |
| chr3:34131250-34132150 | Unknown | - |
| chr5:7170200-7171750 | Unknown | - |
| chr17:50486700-50487400 | Unknown | - |
| chr2:122809550-122810150 | Unknown | - |
| chr8:57178000-57179050 | Unknown | - |
| chr4:142803450-142805000 | Unknown | - |
| chr10:118367950-118370350 | Unknown | - |
| chrX:115004100-115005700 | Unknown | - |
| chr3:53961050-53963000 | Unknown | - |
| chr6:28920750-28922800 | Unknown | - |
| chr17:11769750-11770850 | Unknown | - |
| chr6:1594950-1595600 | Unknown | - |
| chr15:79783300-79784500 | Unknown | - |
| chr7:83684250-83685650 | Unknown | - |
| chr18:2246500-2247900 | Unknown | - |
| chr10:36147250-36148500 | Unknown | - |
| chr7:91023500-91025650 | Unknown | - |
| chr2:79337900-79339650 | Unknown | - |
| chrX:115002950-115003900 | Unknown | - |
| chr1:34557900-34558600 | Unknown | - |
| chr19:523250-524300 | Unknown | - |
| chr13:91315500-91317200 | Unknown | - |
| chr6:26330700-26333000 | Unknown | - |
| chr9:115565950-115567400 | Unknown | - |
| chr14:42380150-42381450 | Unknown | - |
| chr7:76356350-76358750 | Unknown | - |
| chr13:108578200-108579350 | Unknown | - |
| chr8:90569800-90570900 | Unknown | - |
| chr3:185842600-185844550 | Unknown | - |
| chr1:207903150-207904800 | Unknown | - |
| chr2:14988000-14988950 | Unknown | - |
| chr12:47819700-47821500 | Unknown | - |
| chr1:83728350-83730000 | Unknown | - |
| chr11:105384700-105387850 | Unknown | - |
| chr3:88557900-88558600 | Unknown | - |
| chr6:142290050-142291600 | Unknown | - |
| chr3:83265600-83268250 | Unknown | - |

To experimentally test if inhibiting EZH2/PRC2 activity might modulate somatic promoter usage in GC, we treated IM95 GC cells with GSK126, a highly selective small-molecule inhibitor of EZH2 methyltransferase activity. This line was selected as it has previously shown to be sensitive to EZH2 depletion (Figure 14). RNA-seq analysis of GSK126-treated IM95 cells at two treatment time points (Day 6 and 9) confirmed that genes upregulated upon EZH2 inhibition are enriched in previously identified PRC2 target gene sets (Figure 18). GSK126 treatment caused deregulation of 2134 promoters in total. Of 1959 promoters exhibiting somatic alterations in primary GCs (Fig. 1D), GSK126 treatment caused deregulation of 251 somatic promoters in IM95 cells (12.8%). This proportion was significantly greater than the proportion of unaltered promoters exhibiting deregulation after GSK126 challenge (8.8%, OR 1.46 P<0.001, Fisher Test, Fig. 5B), suggesting heightened sensitivity of somatic promoters to EZH2 inhibition. The proportion of somatic promoters deregulated after EZH2 inhibition was also greater than the total proportion of genes (as defined by Gencode) regulated by GSK126 (1.5%, OR 9.21, P<0.001, Fig. 5B). Of those promoters exhibiting both GSK126 deregulation and also mapping to somatic promoters lost in primary GC, 89.6% were reactivated following GSK126 administration (78/87, FC>=2, qval <0.1, Methods and Materials), consistent with EZH2 functioning to repress these promoters. For example, Fig. 5C and 5D highlights two lost somatic promoters (*SLC9A9* and *PSCA*), exhibiting expression gain after GSK126 treatment (Fig. 5). These results thus suggest a general role for EZH2 in regulating epigenomic promoter alterations in GC.

### Somatic Promoters Reveal Novel Cancer-associated Transcripts

Finally, when analyzing the altered somatic promoters with respect to both proximity to known genes, we found that somatic promoters could be classified into *annotated* and *unannotated* categories. Annotated promoters were defined as promoters mapping close (<500bp) to a known Gencode transcription start site (TSS), while unannotated promoters refer to those mapping to genomic regions devoid of known Gencode TSSs. The majority of promoters present in non-malignant tissues, and also promoters unchanged between tumors and normal tissues, mapped closely to previously annotated TSSs (72%-92%). In contrast, only 41% of promoters mapped to annotated promoter locations, while the remaining 59% mapped to "unannotated" locations, distant from Gencode TSSs and in many cases 2-10 kb away (Fig. 6a).

To test the functional relevance of these unannotated promoters, we used GenoCanyon, a nucleotide level quantification of genomic functional potential that integrates multiple levels of conservation and epigenomic information. We observed that 81% of the unannotated promoter regions exhibited a maximum genome wide functional score of greater than 0.9 (range 0-1), indicating high functional potential. To ascertain tissue type specificities, we then applied tissue specific annotations using GenoSkyline, an extension of the GenoCanyon framework integrating Roadmap Epigenomics data We observed that GI tissues had the 3^{rd} highest median score after ESC and fetal tissues, consistent with our tumors being gastric in lineage and also de-differentiated (Fig.5b). In a separate analysis, recent studies have also suggested that endogenous repeat elements in the human genome may contribute significantly to regulatory element variation, and hypomethylation of repeat elements can induce cancer-associated transcription. We found that unannotated promoters, were also significantly enriched for the repeat elements ERV1 (P<0.0001 Unannotated vs. All) and L1 (P<0.0001 Unannotated vs. All, Figure 13).

Compared to annotated promoters, unannotated promoters exhibited weaker H3K27ac signals suggesting that the former might have lower activity and decreased gene expression levels (Figure 13). Supporting this, somatic promoters, even those supported by CAGE tags (indicating true promoters), exhibited significantly lower RNA-seq expression levels compared CAGE tag supported all promoters (Fig. 5c). We thus hypothesized that unannotated promoters might be associated with low transcript levels, thereby rendering them more challenging to detect by conventional depth transcriptome sequencing given the very wide dynamic range of cellular transcriptomes (10-10,000 transcripts per cell for different genes) (Fig. 5d). To test this possibility, we employed both down-sampling and up-sampling analysis. Not surprisingly, decreasing levels of RNA-seq depth caused a concomitant decrease in detected somatic promoter transcripts. For example, downsampling to ∼40M reads caused ∼250 transcripts (FPKM>0, Fig. 5e) to be rendered undetectable at somatic promoters. More convincingly, in the reciprocal experiment, we experimentally generated deep RNA-seq data for matched 5 GC/normal pairs (average read depth 140M compared to standard 100M), and confirmed the additional detection of 435 new somatic promoter-associated transcripts (FPKM>0) (Fig. 5e). We estimate that usage of deep RNA-sequencing data allowed us to discover additional transcripts for 22% of the unannotated promoters, not previously detectible at regular depth RNA-seq (Fig. 5f). These results demonstrate that despite being associated with *bona-fide* cancer associated transcripts, many somatic promoters defined by epigenomic profiling may have been missed by conventional-depth RNA-seq.

### Discussion

Identifying somatically-altered *cis*-regulatory elements, and understanding how these elements direct cancer-associated gene expression represents a critical scientific goal. Here, we defined close to 2000 promoters exhibiting altered activity in GC, indicating that somatic promoters in GC are pervasive. Promoters are canonically defined as proximal *cis-*regulatory elements that recruit general transcription factors to initiate transcription. However, selection and activation of TSSs by RNA polymerase at core promoters is dependent on multiple factors. Core promoters are differentially distributed between genes of different functions, and chromatin distributions and epigenetic landscapes of core promoter regions can also differ in a tissue specific manner. Presence of multiple transcription initiation sites within the same gene can generate distinct transcript isoforms with different 5'UTRs that can act as switches to regulate gene expression, and usage of alternative 5'UTRs can also impact both translation and protein stability of cancer associated genes such as *BRCA1, TGF-β* and *ERG* Such findings demonstrate that specific promoter element activity is complex and cell context dependent, with impact on downstream transcriptional, translational, and functional processes.

A significant proportion (∼18%) of somatic promoters corresponded to alternative promoters. In cancer, alternative promoter utilization is of major relevance, as increasing numbers of genes (e.g. *LEF1*, *TP53*, *TGFB3*) are now being shown to exhibit distinct alternative-promoter associated isoforms that differentially affect malignant growth. In the current study, we identified alternative promoters in genes both known and novel to GC biology with significant clinical and translational implications. For example, we discovered an alternative promoter at the *EpCAM* gene locus specifically activated in gastric tumors. In GC, *EpCAM* encodes a transmembrane glycoprotein which has been proposed as a marker for circulating tumor cells and *EpCAM* expression levels have been correlated with GC patient prognosis. However, little is known about the specific cellular mechanisms driving high *EpCAM* expression in GC. Our finding that *EpCAM* is regulated in GC not through its canonical promoter, but instead through a cancer-specific alternative promoter may lend credence to recent reports suggesting that in addition to acting as an experimentally convenient surface marker, *EpCAM* may actually play a more direct pro-oncogenic role in stimulating cellular proliferation.

Another novel example of an alternative promoter-associated gene, identified for the first time in our study, was *RASA3.* While a functional role for *RASA3* in cancer remains to definitely established, studies from other biological fields have shown that *RASA3* can inhibit *RAP1*, which in turn has been implicated in invasion and metastasis in various cancers. *RASA3* depletion can enhance signaling by integrins and mitogen-activated protein kinases, and the possibility that *RASA3* can act as tumor suppressor has also been recently suggested through independent cross-species cancer studies. A plausible role for *RASA3* as a potential tumor suppressor is consistent with our own results where expression of wild-type *RASA3* potently inhibited cell migration and invasion in GC cell lines, while N-terminal variant *RASA3* enhanced migration and invasion in normal gastric epithelial cells. A third example of an alternative-promoter driven genes was *MET,* which has been extensively investigated as a target for cancer therapy. While we and others have previously reported expression of an N-terminal truncated MET variant in cancer, functional implications of this truncated MET variant have remained unclear. In the present study, experimental assessment of MET wild-type and variant signaling revealed that truncated MET variants may have different downstream signaling effects compared to full-length MET isoforms. Under the experimental conditions used, we observed significant differences in phosphorylation patterns of ERK, STAT3 and GAB1, in a manner consistent with MET-Var being more pro-oncogenic compared to MET-Var, as both ERK, STAT3, and GAB1 have been shown to facilitate MET-induced signaling. The MET signaling pathway is known to be particularly complex with multiple feedback loops, and understanding how expression of the N terminal short *MET* isoform might modulate downstream survival signaling will be an important subject of future research, particularly in light of recent clinical trials targeting *MET* in lung cancer using antibodies which have been unsuccessful.

Our study also revealed an unexpected relationship between somatic promoters and tumor immunity. Specifically, we discovered that alternative promoter isoforms overexpressed in GC were significantly depleted of N-terminal peptides predicted to be potentially immunogenic, based on computational predictions of high-affinity MHC Class I binding and other immunological assays. We believe that finding is relevant to cancer immunity, as it builds on previous findings from the literature establishing the existence of self-reactive T-cells, the potential immunogenicity of overexpressed tumor antigens, and the process of tumor immunoediting. First, while the majority of self-reactive T-cells are clonally deleted during early development, numerous groups have also demonstrated the frequent persistence of self-reactive T cells in the periphery. For example, analysis of transgenic mice has shown that 25-40% of autoreactive T cells are likely to escape clonal deletion even in the presence of the deleting ligand, and in humans, Yu et al has demonstrated that clonal deletion prunes the T-cell repertoire but does not fully eliminate self-reactive T-cell clones. Importantly, while such self-reactive T-cells are typically low-avidity and are not capable of recognizing self-antigens under normal physiological conditions, they still retain the ability to become activated and to produce effector and memory cells under conditions of appropriate stimulation, such as infection and the mounting of anti-tumor responses.

Second, in cancer, several studies have shown that self-reactive T-cells can exhibit immunologic activity towards overexpressed tumor antigens, even if these antigens are also expressed at lower levels in normal tissues. One well-known example is the melanocyte differentiation antigen Melan-A/MART-1, which is expressed by both normal melanocytes and overexpressed in malignant melanoma cells. T-cell recognition of Melan-A/MART-1 has been detected in 50% of melanoma patients, and even healthy individuals have been shown to exhibit a disproportionately high frequency of Melan-A/MART-1-specific T cells in the peripheral blood. Besides Melan-A/MART-1, other examples of tumor associated self-antigens inducing immunological recognition in both healthy individuals and cancer patients include tyrosinase-related proteins (TRP-1 and TRP-2) and glycoprotein (gp)100 in melanoma, and P1A in mastocytoma cells. Such examples clearly demonstrate that in certain cases, normally expressed proteins can still become immunogenic when overexpressed in cancer. Third, tumor immunoediting - the acquired capacity of developing tumors to escape immune control, is a recognized hallmark of cancer. Tumor immune escape can occur via different mechanisms, such as through upregulation of immune checkpoint inhibitors (eg PD-L1), and altered transcription of antigen presenting genes or tumor-specific antigens. For example, decreased expression of melanoma antigens (eg gp100, MART-1, and P1A) has been associated with melanoma progression to later disease stages. Besides overt downregulation of the entire gene, it is thus highly plausible that transcriptional changes affecting splice forms and promoter variants may also contribute to tumor immunoediting. For example, very recent work in B-cell acute lymphoblastic leukemia (B-ALL) has described the production of N-terminally truncated CD19 transcript variants in response to CD19 CART (chimeric antigen receptor-armed T cells) therapy, clearly showing that promoter transcript variants can indeed arise as a consequence of immunologic pressure. Taken collectively, we believe that these previously established findings all point to a plausible role for alternative promoters in reducing the immunogenic potential of tumors. In this regard, our observation that regions exhibiting somatic promoter alterations showed a significant overlap with binding targets of the Polycomb repressive complex 2 (*PRC2*) epigenetic regulator complex, and are particularly sensitive to EZH2 inhibition, suggests that pharmacologic approaches for reawakening somatic promoter-associated epitopes might represent an attractive strategy for increasing anti-tumor T-cell immunoreactivity and anti-tumor activity.

In conclusion, our study indicates an important role for somatic somatic promoters in GC. We also note that a significant portion (52%) of the somatic promoters localized to unannotated TSSs, consistent with recent studies indicating the existence of hundreds of transcript loci remaining to be annotated. Interestingly, a large portion of the human transcriptome has been shown to originate from repetitive elements that can exhibit promoter activity and/or express noncoding RNAs. Unannotated promoters activated in our GC study were found to be enriched in ERV-1 and L1 repeat elements which have been shown to be associated with stage specific transcription in early human embryonic cells, suggesting a yet unknown functional role for these promoters. Analysis of these unannotated promoters is likely to provide fertile ground for new and hitherto unanticipated insights into mechanisms of GC development and progression.

## Claims

1. A method for determining the presence or absence of at least one promoter in a cancerous biological sample relative to a non-cancerous biological sample, comprising:
contacting the cancerous biological sample with at least one antibody specific for histone modification H3K4me3 and at least one antibody specific for histone modification H3K4me1;
isolating nucleic acid from the cancerous biological sample having a signal ratio of H3K4me3 relative to H3K4mel greater than 1, wherein the isolated nucleic acid comprises at least one region specific to said histone modifications;
detecting a signal intensity of H3K4me3 in the isolated nucleic acid; and
determining the presence or absence of at least one promoter in the cancerous biological sample based on the change in the signal intensity of H3K4me3 relative to the signal intensity of H3K4me3 in a non-cancerous biological sample.

2. The method of claim 1, wherein the cancerous and non-cancerous biological sample comprises a single cell, multiple cells, fragments of cells, body fluid or tissue; optionally wherein the cancerous and non-cancerous biological sample is obtained from the same subject; optionally wherein the cancerous and non-cancerous biological sample are each obtained from different subjects; optionally wherein the contacting step comprises the immunoprecipitation of chromatin with the antibodies specific for the histone modifications.

3. The method of claim 1 or 2, further comprising mapping at least one promoter from the cancerous biological sample against at least one reference nucleic acid sequence to identify a gene transcript associated with the at least one promoter; optionally wherein the at least one reference nucleic acid sequence comprises a nucleic acid sequence derived from:
i) an annotated genome sequence;
ii) a *de novo* transcriptome assembly; and/or
iii) a non-cancerous nucleic acid sequence library or database.

4. The method of claim 1, wherein the change of signal intensity of H3K4me3 is greater than a 1.5 fold increase or decrease relative to the signal intensity of H3K4me3 in the non-cancerous biological sample; optionally wherein a change of signal intensity of H3K4me3 greater than a 1.5 fold increase relative to the signal intensity of H3K4me3 in a non-cancerous biological sample, correlates to the presence of at least one cancer-associated promoter in the cancerous biological sample.

5. The method of claim 4, wherein the activity of the at least one cancer-associated promoter correlates with an increase of SUZ12 or EZH2 binding sites relative to the total promoter population; optionally wherein the increase of SUZ12 or EZH2 binding sites correlates with an upregulation of activity of the at least one cancer-associated promoter; optionally wherein the increase of SUZ12 or EZH2 binding sites correlates with a downregulation of activity of the at least one cancer-associated promoter.

6. The method of claim 5, wherein the gene transcript start site is associated with one or more of a cell-type specification gene, a cell adhesion gene, a cell mediated immunity gene, a gastric cancer-associated or deregulated gene, a PRC2 target gene or a transcription factor; optionally wherein the gene transcript start site is associated with an oncogene; optionally wherein the gene transcript start site is associated with a gene selected from the group consisting of *MYC*, *MET*, *CEACAM6*, *CLDN7*, *CLDN3*, *HOTAIR*, *PVT1*, *HNF4α*, *RASA3*, *GRIN2D*, *EpCAM* and a combination thereof.

7. The method of any of claims 1-6, wherein the cancer is gastric cancer or colon cancer.

8. The method of any of claims 1-6, wherein the at least one promoter is an alternative promoter that is associated with a canonical promoter, wherein the canonical promoter is present in both the cancerous biological sample and the non-cancerous biological sample, and wherein the alternative promoter is only present in the cancerous biological sample, or wherein the alternative promoter is only absent in the cancerous biological sample.

9. The method of any of claims 1-5, wherein the at least one promoter is an unannotated promoter that is positioned more than 500bp away from a gene transcript start site.

10. The method of claim 8, further comprising:
measuring the expression level of the at least one alternative promoter in the cancerous biological sample and non-cancerous biological sample, wherein the measuring comprises digital profiling of reporter probes; and
determining the differential expression level of the at least one alternative promoter relative to the non-cancerous biological sample, based on the digital profiling of the reporter probes, to validate the presence or absence of at least one alternative promoter in the cancerous biological sample relative to a non-cancerous biological sample; optionally wherein said step of measuring is conducted using a NanoString™ platform.

11. A method for determining the prognosis of cancer in a subject, comprising:
contacting a cancerous biological sample obtained from the subject with at least one antibody specific for histone modification H3K4me3 and at least one antibody specific for histone modification H3K4me1;
isolating nucleic acid from the cancerous biological sample having a signal ratio of H3K4me3 relative to H3K4mel greater than 1, wherein the isolated nucleic acid comprises at least one region specific to said histone modifications;
detecting a signal intensity of H3K4me3 in the isolated nucleic acid; and
determining the presence or absence of at least one cancer-associated promoter in the cancerous biological sample based on the change in the signal intensity of H3K4me3 relative to the signal intensity of H3K4me3 in a reference nucleic acid sequence, wherein the presence or absence of the at least one cancer-associated promoter in the cancerous biological sample is indicative of the prognosis of the cancer in the subject.

12. The method of claim 11, wherein the at least one cancer-associated promoter is an alternative promoter that is associated with a canonical promoter, wherein the canonical promoter is present in both the cancerous biological sample and the reference nucleic acid sequence, and wherein the alternative promoter is only present in the cancerous biological sample or wherein the alternative promoter is only absent in the cancerous biological sample; optionally wherein the presence or absence of the at least one alternative promoter in the cancerous sample is indicative of a poor prognosis of cancer survival in the subject.

13. The method of claim 12, further comprising:
measuring the expression level of the at least one alternative promoter in the cancerous biological sample and the reference nucleic acid sequence, wherein the measuring comprises digital profiling of reporter probes; and
determining the differential expression level of the at least one alternative promoter relative to the non-cancerous biological sample, based on the digital profiling of the reporter probes, to validate the presence or absence of at least one alternative promoter in the cancerous biological sample relative to the reference nucleic acid sequence.

14. The method of claim 13, wherein said step of measuring is conducted using a NanoString™ platform.

15. A method for determining the presence or absence of at least one cancer-associated promoter in a cancerous biological sample relative to a non-cancerous biological sample, comprising:
contacting the cancerous biological sample with at least one antibody specific for histone modification H3K4me3 and at least one antibody specific for histone modification H3K4me1;
isolating nucleic acid from the cancerous biological sample having a signal ratio of H3K4me3 relative to H3K4mel greater than 1, wherein the isolated nucleic acid comprises at least one region specific to said histone modifications;
detecting a signal intensity of H3K4me3 in the isolated nucleic acid at a read depth of 20M; and
determining the presence or absence of at least one cancer-associated promoter in the cancerous biological sample based on the change in the signal intensity of H3K4me3 relative to the signal intensity of H3K4me3 in a non-cancerous biological sample.

## Patentansprüche

1. Verfahren zum Bestimmen der Anwesenheit oder Abwesenheit von mindestens einem Promotor in einer krebsartigen biologischen Probe relativ zu einer nicht krebsartigen biologischen Probe, das Folgendes umfasst:
Berühren der krebsartigen biologischen Probe mit mindestens einem Antikörper, der für Histonmodifikation H3K4me3 spezifisch ist, und mindestens einem Antikörper, der für Histonmodifikation H3K4me1 spezifisch ist;
Isolieren von Nukleinsäure aus der krebsartigen biologischen Probe, die ein Signalverhältnis von H3K4me3 relativ zu H3K4me1 von mehr als 1 aufweist, wobei die isolierte Nukleinsäure mindestens einen Bereich umfasst, der für die Histonmodifikationen spezifisch ist;
Detektieren einer Signalintensität von H3K4me3 in der isolierten Nukleinsäure; und
Bestimmen der Anwesenheit oder Abwesenheit von mindestens einem Promotor in der krebsartigen biologischen Probe auf Grundlage der Änderung in der Signalintensität von H3K4me3 relativ zu der Signalintensität von H3K4me3 in einer nicht krebsartigen Probe.

2. Verfahren nach Anspruch 1, wobei die krebsartige und die nicht krebsartige biologische Probe eine einzelne Zelle, mehrere Zellen, Fragmente von Zellen, Körperflüssigkeit oder -gewebe umfassen; wahlweise wobei die krebsartige und die nicht krebsartige biologische Probe von demselben Subjekt erlangt sind; wahlweise wobei die krebsartige und die nicht krebsartige biologische Probe jeweils von unterschiedlichen Subjekten erlangt sind; wahlweise wobei der Berührungsschritt die Immunpräzipitation von Chromatin mit den Antikörpern umfasst, die für die Histonmodifikation spezifisch sind.

3. Verfahren nach Anspruch 1 oder 2, das ferner Zuordnen von mindestens einem Promotor aus der krebsartigen biologischen Probe gegen mindestens eine Referenznukleinsäuresequenz umfasst, um ein Gen-Transkript zu identifizieren, das mit dem mindestens einen Promotor zusammenhängt; wahlweise wobei die mindestens eine Referenznukleinsäuresequenz eine Nukleinsäuresequenz umfasst, die von Folgenden abgeleitet ist:
i) einer annotierten Genomsequenz;
ii) einer *De*-*Novo*-Transkriptom-Assemblierung; und/oder
iii) einer Bibliothek oder Datenbank von nicht krebsartigen Nukleinsäuresequenzen.

4. Verfahren nach Anspruch 1, wobei die Änderung der Signalintensität von H3K4me3 größer als eine 1,5 malige Erhöhung oder Verringerung relativ zu der Signalintensität von H3K4me3 in der nicht krebsartigen biologischen Probe ist; wahlweise, wobei eine Änderung der Signalintensität von H3K4me3 von mehr als eine 1,5 malige Erhöhung relativ zu der Signalintensität von H3K4me3 in einer nicht krebsartigen biologischen Probe mit der Anwesenheit von mindestens einem mit Krebs zusammenhängenden Promotor in der krebsartigen biologischen Probe korreliert.

5. Verfahren nach Anspruch 4, wobei die Aktivität des mindestens einen mit Krebs zusammenhängenden Promotors mit einer Erhöhung von SUZ12- oder EZH2-Bindungsstellen relativ zu der gesamten Promotorpopulation korreliert; wahlweise wobei die Erhöhung von SUZ12- oder EZH2-Bindestellen mit einer Hochregulierung von Aktivität des mindestens einen mit Krebs zusammenhängenden Promotors korreliert; wahlweise wobei die Erhöhung von SUZ12- oder EZH2-Bindestellen mit einer Herabregulierung von Aktivität des mindestens einen mit Krebs zusammenhängenden Promotors korreliert.

6. Verfahren nach Anspruch 5, wobei die Gen-Transkript-Startstelle mit einem oder mehreren eines Zelltypspezifizierungsgens, eines Zelladhäsionsgens, eines Gens von zellvermittelter Immunität, eines mit Magenkrebs zusammenhängenden oder deregulierten Gens, eines PRC2-Zielgens oder eines Transkriptionsfaktors zusammenhängt; wahlweise, wobei die Gen-Transkript-Startstelle mit einem Onkogen zusammenhängt; wahlweise wobei die Gen-Transkript-Startstelle mit einem Gen zusammenhängt, das ausgewählt ist aus der Gruppe bestehend aus *MYC*, *MET*, *CEACAM6*, *CLDN7*, *CLDN3*, *HOTAIR*, *PVT1*, *HNF4α*, *RASA3*, *GRIN2D*, *EpCAM* und einer Kombination davon.

7. Verfahren nach einem der Ansprüche 1-6, wobei der Krebs Magenkrebs oder Darmkrebs ist.

8. Verfahren nach einem der Ansprüche 1-6, wobei der mindestens eine Promotor ein alternativer Promotor ist, der mit einem kanonischen Promotor zusammenhängt, wobei der kanonische Promotor in sowohl der krebsartigen biologischen Probe als auch der nicht krebsartigen biologischen Probe anwesend ist und wobei der alternative Promotor nur in der krebsartigen biologischen Probe anwesend ist oder wobei der alternative Promotor nur in der krebsartigen biologischen Probe abwesend ist.

9. Verfahren nach einem der Ansprüche 1-5, wobei der mindestens eine Promotor ein nicht annotierter Promotor ist, der mehr als 500 bp von der Gen-Transkript-Startstelle entfernt positioniert ist.

10. Verfahren nach Anspruch 8, das ferner Folgendes umfasst:
Messen des Expressionsniveau des mindestens einen alternativen Promotors in der krebsartigen biologischen Probe und der nicht krebsartigen biologischen Probe, wobei das Messen ein digitales Profiling von Reportersonden umfasst; und
Bestimmen des differentiellen Expressionsniveaus des mindestens einen alternativen Promotors relativ zu der nicht krebsartigen biologischen Probe, auf Grundlage des digitalen Profilings der Reportersonden, um die Anwesenheit oder Abwesenheit von mindestens einem alternativen Promotor in der krebsartigen biologischen Probe relativ zu einer nicht krebsartigen biologischen Probe zu validieren; wahlweise, wobei der Schritt des Messens unter Verwendung einer NanoString™-Plattform durchgeführt wird.

11. Verfahren zum Bestimmen der Prognose von Krebs in einem Subjekt, das Folgendes umfasst:
Berühren einer krebsartigen biologischen Probe, die von dem Subjekt erlangt wurde, mit mindestens einem Antikörper, der für die Histonmodifikation H3K4me3 spezifisch ist, und mindestens einem Antikörper, der für die Histonmodifikation H3K4me1 spezifisch ist;
Isolieren von Nukleinsäure von der krebsartigen biologischen Probe, die ein Signalverhältnis von H3K4me3 relativ zu H3K4me1 von mehr als 1 aufweist, wobei die isolierte Nukleinsäure mindestens einen Bereich umfasst, der für die Histonmodifikationen spezifisch ist;
Detektieren einer Signalintensität von H3K4me3 in der isolierten Nukleinsäure; und
Bestimmen der Anwesenheit oder Abwesenheit von mindestens einem mit Krebs zusammenhängenden Promotor in der krebsartigen biologischen Probe auf Grundlage der Änderung in der Signalintensität von H3K4me3 relativ zu der Signalintensität von H3K4me3 in einer Referenznukleinsäuresequenz, wobei die Anwesenheit oder Abwesenheit des mindestens einen mit Krebs zusammenhängenden Promotors in der krebsartigen biologischen Probe die Prognose des Krebs in dem Subjekt angibt.

12. Verfahren nach Anspruch 11, wobei der mindestens eine mit Krebs zusammenhängende Promotor ein alternativer Promotor ist, der mit einem kanonischen Promotor zusammenhängt, wobei der kanonische Promotor in sowohl der krebsartigen biologischen Probe als auch der Referenznukleinsäuresequenz anwesend ist und wobei der alternative Promotor nur in der krebsartigen biologischen Probe anwesend ist oder wobei der alternative Promotor nur in der krebsartigen biologischen Probe abwesend ist; wahlweise, wobei die Anwesenheit oder Abwesenheit des mindestens einen alternativen Promotors in der krebsartigen Probe eine schlechte Prognose von Krebsüberleben in dem Subjekt angibt.

13. Verfahren nach Anspruch 12, das ferner Folgendes umfasst:
Messen des Expressionsniveaus des mindestens einen alternativen Promotors in der krebsartigen biologischen Probe und der Referenznukleinsäuresequenz, wobei das Messen ein digitales Profiling von Reportersonden umfasst; und
Bestimmen des differentiellen Expressionsniveaus des mindestens einen alternativen Promotors relativ zu der nicht krebsartigen biologischen Probe, auf Grundlage des digitalen Profilings der Reportersonden, um die Anwesenheit oder Abwesenheit von mindestens einem alternativen Promotor in der krebsartigen biologischen Probe relativ zu der Referenznukleinsäure zu validieren.

14. Verfahren nach Anspruch 13, wobei der Schritt des Messens unter Verwendung einer NanoString™-Plattform durchgeführt wird.

15. Verfahren zum Bestimmen der Anwesenheit oder Abwesenheit von mindestens einem mit Krebs zusammenhängenden Promotor in einer krebsartigen biologischen Probe relativ zu einer nicht krebsartigen biologischen Probe, die Folgendes umfasst:
Berühren der krebsartigen biologischen Probe mit mindestens einem Antikörper, der für die Histonmodifikation H3K4me3 spezifisch ist, und mindestens einem Antikörper, der für die Histonmodifikation H3K4mel spezifisch ist;
Isolieren der Nukleinsäure von der krebsartigen biologischen Probe, die ein Signalverhältnis von H3K4me3 relativ zu H3K4mel von mehr als 1 aufweist, wobei die isolierte Nukleinsäure mindestens einen Bereich umfasst, der für die Histonmodifikationen spezifisch ist;
Detektieren einer Signalintensität von H3K4me3 in der isolierten Nukleinsäure mit einer Messtiefe von 20M; und
Bestimmen der Anwesenheit oder Abwesenheit von mindestens einem mit Krebs zusammenhängenden Promotor in der krebsartigen biologischen Probe auf Grundlage der Änderung in der Signalintensität von H3K4me3 relativ zu der Signalintensität von H3K4me3 in einer nicht krebsartigen biologischen Probe.

## Revendications

1. Procédé pour déterminer la présence ou l'absence d'au moins un promoteur dans un échantillon biologique cancéreux par rapport à un échantillon biologique non cancéreux, comprenant :
la mise en contact de l'échantillon biologique cancéreux avec au moins un anticorps spécifique pour la modification des histones H3K4me3 et au moins un anticorps spécifique pour la modification des histones H3K4mel ;
l'isolation de l'acide nucléique de l'échantillon biologique cancéreux ayant un rapport de signal de H3K4me3 par rapport à H3K4mel supérieur à 1, dans lequel l'acide nucléique isolé comprend au moins une région spécifique auxdites modifications des histones ;
la détection d'une intensité de signal de H3K4me3 dans l'acide nucléique isolé ; et
la détermination de la présence ou de l'absence d'au moins un promoteur dans l'échantillon biologique cancéreux sur la base du changement de l'intensité du signal de H3K4me3 par rapport à l'intensité du signal de H3K4me3 dans un échantillon biologique non cancéreux.

2. Procédé selon la revendication 1, dans lequel l'échantillon biologique cancéreux et non cancéreux comprend une seule cellule, plusieurs cellules, des fragments de cellules, un fluide ou un tissu corporel ; éventuellement dans lequel l'échantillon biologique cancéreux et non cancéreux est obtenu à partir du même sujet ; éventuellement dans lequel l'échantillon biologique cancéreux et non cancéreux est chacun obtenu à partir de sujets différents ; éventuellement dans lequel l'étape de mise en contact comprend l'immunoprécipitation de la chromatine avec les anticorps spécifiques pour les modifications des histones.

3. Procédé selon la revendication 1 ou 2, comprenant en outre le mappage d'au moins un promoteur de l'échantillon biologique cancéreux par rapport à au moins une séquence d'acide nucléique de référence pour identifier une transcription génétique associée à l'au moins un promoteur ; éventuellement dans lequel l'au moins une séquence d'acide nucléique de référence comprend une séquence d'acide nucléique dérivée :
i) d'une séquence génomique annotée ;
ii) d'un assemblage de transcriptome *de novo* ; et/ou
iii) d'une banque ou d'une base de données de séquences d'acides nucléiques non cancéreuses.

4. Procédé selon la revendication 1, dans lequel le changement d'intensité de signal de H3K4me3 est supérieur à une augmentation ou une diminution de 1,5 fois par rapport à l'intensité de signal de H3K4me3 dans l'échantillon biologique non cancéreux ; éventuellement dans lequel un changement d'intensité de signal de H3K4me3, supérieur à une augmentation de 1,5 fois par rapport à l'intensité de signal de H3K4me3 dans un échantillon biologique non cancéreux, est corrélé à la présence d'au moins un promoteur associé au cancer dans l'échantillon biologique cancéreux.

5. Procédé selon la revendication 4, dans lequel l'activité de l'au moins un promoteur associé au cancer est corrélé à une augmentation des sites de liaison SUZ12 ou EZH2 par rapport à la population totale de promoteurs ; éventuellement dans lequel l'augmentation des sites de liaison SUZ12 ou EZH2 est corrélée à une régulation positive de l'activité de l'au moins un promoteur associé au cancer ; éventuellement dans lequel l'augmentation des sites de liaison SUZ12 ou EZH2 est corrélée à une régulation négative de l'activité de l'au moins un promoteur associé au cancer.

6. Procédé selon la revendication 5, dans lequel le site d'initiation de transcription génétique est associé à un ou plusieurs parmi un gène de spécification de type cellulaire, un gène d'adhésion cellulaire, un gène d'immunité à médiation cellulaire, un gène associé au cancer gastrique ou dérégulé, un gène cible PRC2 ou un facteur de transcription ; éventuellement dans lequel le site d'initiation de transcription génétique est associé à un oncogène ; éventuellement dans lequel le site d'initiation de transcription génétique est associé à un gène choisi dans le groupe constitué de *MYC*, *MET*, *CEACAM6*, *CLDN7*, *CLDN3*, *HOTAIR*, *PVT1*, *HNF4α*, *RASA3*, *GRIN2D*, *EpCAM* et une combinaison de ceux-ci.

7. Procédé selon l'une quelconque des revendications 1 à 6, dans lequel le cancer est un cancer gastrique ou un cancer du côlon.

8. Procédé selon l'une quelconque des revendications 1 à 6, dans lequel l'au moins un promoteur est un promoteur alternatif qui est associé à un promoteur canonique, dans lequel le promoteur canonique est présent à la fois dans l'échantillon biologique cancéreux et dans l'échantillon biologique non cancéreux, et dans lequel le promoteur alternatif n'est présent que dans l'échantillon biologique cancéreux, ou dans lequel le promoteur alternatif est uniquement absent dans l'échantillon biologique cancéreux.

9. Procédé selon l'une quelconque des revendications 1 à 5, dans lequel l'au moins un promoteur est un promoteur non annoté qui est positionné à plus de 500 pb d'un site d'initiation de transcription génétique.

10. Procédé selon la revendication 8, comprenant en outre :
la mesure du niveau d'expression de l'au moins un promoteur alternatif dans l'échantillon biologique cancéreux et l'échantillon biologique non cancéreux, dans lequel la mesure comprend le profilage numérique de sondes rapporteurs ; et
la détermination du niveau d'expression différentiel de l'au moins un promoteur alternatif par rapport à l'échantillon biologique non cancéreux, sur la base du profilage numérique des sondes rapporteurs, pour valider la présence ou l'absence d'au moins un promoteur alternatif dans l'échantillon biologique cancéreux par rapport à un échantillon biologique non cancéreux ; éventuellement dans lequel ladite étape de mesure est effectuée à l'aide d'une plateforme NanoString™.

11. Procédé pour déterminer le pronostic du cancer chez un sujet, comprenant :
la mise en contact d'un échantillon biologique cancéreux obtenu du sujet avec au moins un anticorps spécifique pour la modification des histones H3K4me3 et au moins un anticorps spécifique pour la modification des histones H3K4me1 ;
l'isolation de l'acide nucléique de l'échantillon biologique cancéreux ayant un rapport de signal de H3K4me3 par rapport à H3K4me1 supérieur à 1, dans lequel l'acide nucléique isolé comprend au moins une région spécifique auxdites modifications des histones ;
la détection d'une intensité de signal de H3K4me3 dans l'acide nucléique isolé ; et
la détermination de la présence ou de l'absence d'au moins un promoteur associé au cancer dans l'échantillon biologique cancéreux sur la base du changement de l'intensité du signal de H3K4me3 par rapport à l'intensité de signal de H3K4me3 dans une séquence d'acide nucléique de référence, dans lequel la présence ou l'absence de l'au moins un promoteur associé au cancer dans l'échantillon biologique cancéreux est indicatif du pronostic du cancer chez le sujet.

12. Procédé selon la revendication 11, dans lequel l'au moins un promoteur associé au cancer est un promoteur alternatif qui est associé à un promoteur canonique, dans lequel le promoteur canonique est présent à la fois dans l'échantillon biologique cancéreux et dans la séquence d'acide nucléique de référence, et dans lequel le promoteur alternatif n'est présent que dans l'échantillon biologique cancéreux ou dans lequel le promoteur alternatif est uniquement absent dans l'échantillon biologique cancéreux ; éventuellement dans lequel la présence ou l'absence de l'au moins un promoteur alternatif dans l'échantillon cancéreux est indicative d'un mauvais pronostic de survie au cancer chez le sujet.

13. Procédé selon la revendication 12, comprenant en outre :
la mesure du niveau d'expression de l'au moins un promoteur alternatif dans l'échantillon biologique cancéreux et la séquence d'acide nucléique de référence, dans lequel la mesure comprend le profilage numérique de sondes rapporteurs ; et
la détermination du niveau d'expression différentiel de l'au moins un promoteur alternatif par rapport à l'échantillon biologique non cancéreux, sur la base du profilage numérique des sondes rapporteurs, pour valider la présence ou l'absence d'au moins un promoteur alternatif dans l'échantillon biologique cancéreux par rapport à la séquence d'acide nucléique de référence.

14. Procédé selon la revendication 13, dans lequel ladite étape de mesure est effectuée à l'aide d'une plateforme NanoString™.

15. Procédé pour déterminer la présence ou l'absence d'au moins un promoteur associé au cancer dans un échantillon biologique cancéreux par rapport à un échantillon biologique non cancéreux, comprenant :
la mise en contact de l'échantillon biologique cancéreux avec au moins un anticorps spécifique pour la modification des histones H3K4me3 et au moins un anticorps spécifique pour la modification des histones H3K4me1 ;
l'isolation de l'acide nucléique de l'échantillon biologique cancéreux ayant un rapport de signal de H3K4me3 par rapport à H3K4me1 supérieur à 1, dans lequel l'acide nucléique isolé comprend au moins une région spécifique auxdites modifications des histones ;
la détection d'une intensité de signal de H3K4me3 dans l'acide nucléique isolé à une profondeur de lecture de 20 M ; et
la détermination de la présence ou de l'absence d'au moins un promoteur associé au cancer dans l'échantillon biologique cancéreux sur la base du changement de l'intensité de signal de H3K4me3 par rapport à l'intensité de signal de H3K4me3 dans un échantillon biologique non cancéreux.
